# EUROPEAN PATENT SPECIFICATION

(11) **EP 3 934 594 B1**
(45) Date of publication and mention of the grant of the patent: **28.05.2025**
(21) Application number: 20766528.2
(22) Date of filing: 06.02.2020
(51) Int. Cl.: A61N 5/10, A61F 7/00, A61K 9/51, A61K 31/16, A61N 5/06, A61K 41/00, C09K 11/02, A23L 3/50, A23L 3/26, A23L 5/30, A23L 2/50, C09K 11/77, H01L 31/055, C09J 11/04, C09J 11/06, H01L 23/00, C09J 11/02, H01L 31/054, C08K 3/00, C09J 5/00, C09J 9/00

(54) **ENERGY AUGMENTATION STRUCTURES FOR USE IN NON-INVASIVE IN-SITU PHOTOBIOMODULATION**
ENERGIEERHÖHUNGSSTRUKTUREN ZUR VERWENDUNG IN NICHT-INVASIVER IN-SITU-PHOTOBIOMODULATION
STRUCTURES D'AUGMENTATION D'ÉNERGIE, DESTINÉES À ÊTRE UTILISÉES DANS UNE PHOTOBIOMODULATION IN SITU NON INVASIVE

(30) Priority: 04.03.2019 US 201962813390 P; 31.05.2019 US 201962855508 P; 09.09.2019 US 201962897677 P; 11.12.2019 US 201962946648 P; 31.12.2019 US 201962955533 P
(43) Date of publication of application: 12.01.2022
(73) Proprietor: Immunolight, LLC., Detroit, MI 48226 (US)
(72) Inventor: BOURKE, Jr., Frederic A., Detroit, Michigan 48226 (US); WALDER, Harold, Detroit, Michigan 48226 (US); FATHI, Zakaryae, Detroit, Michigan 48226 (US); BEYER, Wayne F., Detroit, Michigan 48226 (US); RUDDER, Ronald A., Bristow, Virginia 20136 (US); SIMMONS, Joseph H., Detroit, Michigan 48226 (US)
(74) Representative: Tostmann, Holger Carl
(86) International application number: PCT/US2020/016999
(87) International publication number: WO 2020/180451

(56) References cited:
- WO-A1-2015/164485
- US-A1- 2015 251 016
- US-A1- 2017 121 472
- US-A1- 2018 185 518
- US-A1- 2018 311 355
- US-B1- 7 245 196

## Description

### BACKGROUND OF THE INVENTION

### Field of the Invention:

The disclosure relates to methods, systems, and devices for energy augmentation, with and without an energy modulation agent/energy conversion agent present, and uses particularly for generating or enhancing photon or electron emission and/or for enhancing light or photon collection, and their use in non-invasive systems and methods for performing in-situ (or *in-vivo*) photobiomodulation. Document WO 2015/164485 discloses a kit for modifying a target structure which mediates or is associated with a biological activity according to the preamble of claim 1.

### Discussion of the Background:

Presently, light (i.e., electromagnetic radiation from the radio frequency through the visible to the X-ray wavelength range) is used in a number of industrial, communication, electronic, and pharmaceutical processes. Light in the infrared and visible range is typically generated from an electrical energy source which for example either heats a material to extremely high temperatures where black body emission occurs (as in an incandescent lamp). Light in the visible and ultraviolet range is typically generated by heating a gas to an electrical discharge where transitions from one electronic state of the gas atom or molecule occur with the emission of light. There are also semiconductor based light sources (as in light emitting diodes and semiconducting lasers) where electrons/holes in a material recombine to produce light emission.

Visible light is defined as the electromagnetic radiation with wavelengths between 380 nm and 750 nm. In general, electromagnetic radiation including light is generated by the acceleration and deceleration or changes in movement (vibration) of electrically charged particles, such as parts of molecules (or adjacent atoms) with high thermal energy, or electrons in atoms (or molecules).

For reference purposes, infra-red (IR) radiation just beyond the red end of the visible region; and, ultra-violet (UV) radiation has a shorter wavelength than violet light. The UV portion of the spectrum is divided into three regions: UVA (315 - 400 nm), UVB (280 - 315 nm) and UVC (100 - 280 nm).

Industrial lamps used in lighting applications cover the visible range of wavelengths for proper white perception. Thermal sources like heated filaments can be made of different type conductors, including W-filaments, halogen-protected W-filaments, and electrically induced high temperature plasmas (arc lamps).

The power (energy emitted per second) of a radiant source is frequently expressed in watts (W), but light can also be expressed in lumens (lm) to account for the varying sensitivity of the eye to different wavelengths of light. The derived relevant units are the radiance (luminance) of a source in W/m² (lm/m²) in a certain direction per steradian (unit of solid angle) and the irradiance (illuminance) of a surface in W/m² (lm/m² or lux).

With the development of ultraviolet sources, ultraviolet radiation is being increasingly utilized for industrial, chemical, and pharmaceutical purposes. For example, UV light is known to sterilize media and is known to drive a number of photo-activated chemical processes such as the cross-linking of polymers in adhesives or coatings. Typically, ultraviolet sources use gas discharge lamps to generate emitted light in the ultraviolet range. The emitted light is then optically filtered to remove many of not all of the non-ultraviolet frequencies. Ultraviolet light can also be produced in semiconductor phosphors from the excitation of these phosphors from high energy sources such as, for example, X-ray irradiation.

With the development of infrared radiation sources, infrared radiation is being increasingly utilized for communications and signaling purposes. Typically, infrared sources use broad spectrum light sources referred to as glowbars to generate a broad spectrum of light centered in the infrared range or use lasers to emit very specific infrared wavelengths. For the broad band sources, the emitted light is optically filtered to remove many, if not all, of the non-infrared frequencies.

It is generally desirable to have devices, materials, and capabilities to convert light from one frequency range to another. Down conversion has been one way to convert higher energy light to lower energy, as used in the phosphors noted above. Up conversion has also been shown where lower energy light is converted to higher energy light. Typically, this process is a multi-photon absorption process where two or more photons are used to promote an excited electronic state in a host medium which in turn radiates at a wavelength of light that has a higher energy than the energy of the incident light which promoted the multi-photon absorption process. Both down conversion and up conversion have been studied and documented in the past.

Indeed, workers have studied the phenomenon of photoluminescence and fluorescence, which is the ability of certain solids to emit light when driven or charged by an external energy source. Many well-known phosphors and fluorescors are triggered by high-energy electrons or photons and emit photons of lower energy. It has been recognized that certain infrared phosphors can convert infrared light to light in the visible range (violet through red).

The properties of light such as its radiance is particularly important in reading or display applications where the human eye has to perceive and discern temporary images or permanent images (as for example shown by road and highway signs) formed with visible light. Televisions, computer monitors, displays, and signs use a cathode ray technology (CRT) technology where high energy electrons impinge on phosphors that emit visible light. Televisions, computer monitors, displays, and signs more recently have used liquid crystal display or plasma display technology to generate visible images discernable to the human eye.

In these and other reading or display applications, attempts have been made to develop displays with relatively high contrast images while minimizing the amount of broadband light emitted or reflected from a display, which may detract from the contrast of the image displayed.

In general, the up conversion and the down conversion discussed above have been used in a number of fields to in effect convert an incident wavelength of light to a different wavelength. In one example, high energy photons such as X-rays are converted by absorption in phosphors of the x-ray energy, and luminescence from the phosphors in the ultraviolet, visible, and/or near infrared spectrum has been used for driving photoactive reactions. In other examples, infrared or near infrared light has been up converted by absorption in phosphors of the infrared or near infrared light, and luminescence from the phosphors in the visible and/or ultraviolet spectrum. In other examples, light within the visible region can be down converted or up converted (depending on the phosphors chosen) to a different band within the visible wavelengths. This shifting (energy conversion) can be for color enhancement and can be used in solar cells to convert one part of the solar spectrum to another part more favorable for a photovoltaic device to generate power.

In many of these prior applications, metallic structures have been placed on the phosphors or in a vicinity of the phosphors to generate a plasmonics effect which essentially is an amplification of the local field very nearby the outside of the metallic structures. Plasmonic effects can enhance coupling of incident light into the phosphors and/or enhance the reactivity of the converted light tons nearby receptor. While the plasmons in the metal can propagate along the metal, the plasmons decay evanescently in the z direction normal to the metal/dielectric interface with 1/e decay length of the order of half the wavelength (~200 nm for wavelengths in the visible range).

In some prior applications, photonic band gap structures have been used In a photonics band gap structure, the materials thereof consist or photonic crystals (PhCs) are materials with a periodic dielectric profile, which can prevent light of certain frequencies or wavelengths from propagating in one, two or any number of directions within the materials. In this way, light not suitable or detrimental to a process can be rejected while light more suitable for a process can be confined within the photonic band gap structure or better confined within the photovoltaic converter.

In the field of solar cells, the addition of plasmonics, photonics band gap, and up and down conversion is known in the literature. Additionally, antireflection coatings and concentrators are well known in the literature.

The problem with the plasmonics effect is that, as noted above, the plasmons and the electric field enhancement decays rapidly with distance away from the metal structure meaning that the effect is only useful for a small volume of interaction.

The problem with antireflection coatings is that, although sun light is not scattered away as much as if there were no coatings, the light transmitted is still predominantly that of wavelengths that are not optimum for power generation.

The problem with concentrators is that, besides concentrating light which can be converted to power, a concentrator also concentrates light which does not generate power, which in general makes for waste heat.

While photonic band gap structures can serve to reflect or confine light, they have no effective way to gain power from the discarded light.

### SUMMARY OF THE INVENTION

The invention is defined in the following claims. Other embodiments, examples, substances, kits and, in particular, therapeutic methods are not a part of the invention.

### BRIEF DESCRIPTION OF THE FIGURES

A more complete appreciation of the invention and many of the attendant advantages thereof will be readily obtained as the same becomes better understood by reference to the following detailed description when considered in connection with the accompanying drawings, wherein:
Figure 1 is a schematic depicting an energy augmentator system with optional inclusion of an energy converter;
Figure 2 is a schematic depicting a folded resonator as an illustrative energy augmentation structure of the invention;
Figure 3 is a diagram depicting the basic concepts underlying one of the energy augmentation structures of this invention;
Figure 4 is a schematic depicting a staggered antenna configuration as an illustrative energy augmentation structure of the invention;
Figure 5 is a schematic depicting the effect of electrode spacing in the folded resonator of the invention;
Figure 6 is a diagram showing a pattern of ¾ 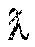 folded resonators distributed in space;
Figures 7A-7C are diagrams showing a pattern of ¾ 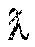 folded resonators distributed in a plane or otherwise along a surface of an object;
Figure 8 is a diagram showing a pattern of ¾ 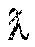 folded resonators distributed in a plane or otherwise along a surface of an object and having a different orientation than in Figure 7A;
Figure 9 is a diagram showing a pattern of ¾ 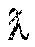 folded resonators having a light or photon or electron emitting material deposited in the region of between the opposing electrodes;
Figure 10 is a diagram showing a pattern of ¾ 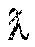 folded resonators having a patterned deposit of a light or photon or electron emitting material in the region of between the opposing electrodes;
Figure 11 is a diagram showing a pattern of ¾ 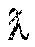 folded resonators having a patterned deposit of a light or photon or electron emitting material in the region of between the opposing electrodes;
Figure 12 is a diagram showing a pattern of ¾ 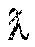 folded resonators having for the metal traces a fractal pattern for the electrical path that loops around to connect the opposing electrodes;
Figure 13 is a diagram showing a fractal antenna segment where the straight-line sides of the metal pads have locally intensified electric field;
Figure 14 is a diagram showing a repeated pattern of the fractal antenna segment of Figure 13
Figure 15 is a diagram showing a pattern of bowtie fractal antenna segments;
Figure 16 is a diagram showing a paired three-dimensional fractal structure with an intensified electric field in between;
Figure 17 is a diagram showing a pattern of the paired three-dimensional fractal structures;
Figure 18 is a diagram showing a ¾ wavelength resonator with the distal ends of the resonator antenna protruding outwardly while maintaining parallelism;
Figure 19 is a diagram showing a packing configuration for three different ¾ wavelength resonators, that are maintained in plane with no overlapping distal ends;
Figure 20 is a diagram showing another packing configuration for three different ¾ wavelength resonators, that are maintained in plane with overlapping distal ends;
Figure 21 is a diagram showing yet another packing configuration for the ¾ wavelength resonators, with an off (or out of) plane axial symmetry;
Figure 22 is a diagram showing a multi-level packing configuration in parallel planes for the folded ¾ wavelength resonator shown;
Figure 23 is a diagram showing a multi-level packing configuration in parallel planes with distal ends protruding out for the ¾ wavelength resonator in Figure 22;
Figure 24A is a diagram showing a different in-plane packing configuration;
Figure 24B is a diagram showing another different in-plane packing configuration;
Figure 25A is a schematic illustrating a distributed point light collector/transmitter of the invention;
Figure 25B is a schematic of a cross section of the collector/transmitter of Figure 25A.
Figure 26 is a schematic illustrating various converter structures of the invention;
Figure 27 is a schematic illustration of plasmon resonance as a function of shell thickness;
Figure 28A is a schematic illustrating other various converter structures of the invention;
Figure 28B is a further schematic illustrating other various converter structures of the invention;
Figures 28C is a schematic illustrating various plasmonics-active converter structures of the invention;
Figure 28D is a schematic illustration of photo-active molecules linked to plasmonics-active upconverter structures of the invention;
Figure 29 is a diagram showing a mechanoluminescent emitter
Figure 30 is a diagram showing a composite piezoelectric/electroluminescent emitter
Figure 31 is a diagram showing a distribution of the composite emitters of Figure 30 across a surface for light emission;
Figure 32 is a diagram showing a distribution of the composite emitters of Figure 30 within a target region for light emission;
Figure 33 is a graphical representation of DNA gel products in particular a-aggregated ds genomic DNA and ds genomic DNA;
Figure 34 is a graphical representation of DNA gel products in particular b-DNA fragments and ssDNA;
Figure 35 is a graphical representation of DNA gel products in particular ssDNA and crosslinked DNA;
Figure 36 is a schematic depicting the signals associated with crosslinked DNA and the signal associated with single strand DNA (represented by the smear pattern);
Figure 37 is a table depicting experimental conditions for the effect of temperature and distance from the X-Ray source;
Figure 38 is a schematic depicting gel electrophoresis results post a DNA crosslinking attempt using various experimental conditions having the total delivered energy as a variable;
Figure 39 is another table depicting experimental conditions for testing the effect of total delivered energy (some conditions had constant power and some conditions had constant flux)
Figure 40 is another table depicting the luminosity results from the ds DNA and the ss DNA (with the higher the number the higher brightness);
Figure 41 is a depiction of the sum of all the brightness results from two minute and six minute X-Ray irradiation treatments;
Figure 42 is another table depicting the total energy delivered per experimental condition;
Figure 43 is a schematic depicting gel electrophoresis results post a DNA crosslinking attempt using varying phosphor concentrations at kVp values at or below 80 kVp, with all conditions yielding a ds-DNA signal;
Figure 44 is another table depicting experimental conditions for testing the effect of phosphor concentration variation;
Figure 45 is a schematic depicting gel electrophoresis results post a DNA crosslinking attempt using kVp levels of 80 kVp for S1, 40 kVp for S2, 20 kVp for S3, and 10 kVp for S4.
Figure 46 is a schematic illustration of how photo-catalytic light works cooperatively with non-ionizing radiation to potentiate the activation of bio-therapeutics;
Figure 47A is a schematic of a test set up devised to channel an external radiation source into the x-ray radiation system;
Figure 47B is a schematic of a weakly coupled fiber bundle for combining different wavelengths of ionizing and non-ionizing radiation;
Figure 47C is a schematic of the combination of X-Ray and a fiber optic for simultaneous use of X-Ray energy with external light sources having potentiating effects;
Figure 48 is a schematic of the combination of X-Ray and a microwave guide allowing the simultaneous use of X-Ray energy and microwave energy to interact with a target or reactive site;
Figure 49 is a schematic of x-ray spectra for various kVp;
Figure 50 is a schematic of the absorption of psoralen measured in different solvents and over a broad range extending from the UVB, the UVA, and part of the visible;
Figure 51 is another table depicting the color spectrum;
Figure 52 is another table depicting the properties of various x-ray phosphors;
Figure 53 is a schematic of the spectral emission of YTaO₄ (reported to have a peak emission at 337 nm under X-Ray excitation) showing emission at 327 nm;
Figure 54 is a schematic of the spectral emission of LaF₃:Ce (reported to have a peak emission at 337 nm under X-Ray excitation) showing emission at 300 nm;
Figure 55 is a schematic of the spectral emission of LaOBr:Tm₃⁺ coated with silica suitable for a phosphor chemistry capable of emission in the UVB, UVA and the visible light regions;
Figure 56 is a schematic of the spectral output of a visible CaWO₄ phosphor under X-Ray excitation from different energy level and different flux x-rays;
Figure 57 is a schematic of the spectral output of a visible Y₂SiO₅:Ce phosphor under X-Ray excitation from different energy level and different flux x-rays;
Figure 58 is a schematic of the spectral output of a visible phosphor (BASF commercial phosphor XYMARA MARKER BLUE LF2A) under X-Ray excitation from different energy level and different flux x-rays;
Figure 59 is a schematic of the spectral output of an 'Y₂O₂S:Tm phosphor capable of emission in the UVA and in the visible light regions;
Figure 60 is a schematic of the spectral output of a BaSO4:Eu phosphor capable of emission in the UVA and in the visible light regions;
Figure 61 is a schematic of the spectral output of an YTaO₄ phosphor capable of emission in the UVA and in the visible light regions;
Figure 62 is a schematic of the spectral output of an YTaO₄ phosphor chemistry capable of emission in the UVA and CaWO₄ capable of emitting in the UVA and in the visible;
Figure 63 is another table depicting properties of various phosphors for mixing;
Figure 64 is a schematic depicting a typical spectrum from a commercial UV light source which has been used to activate psoralens;
Figure 65 is a schematic of the emission spectra under X-Ray excitation for a powder mixture of CaWO and YTaO₄;
Figure 66 is a schematic of the emission spectra under X-Ray excitation for the combination of CaWO₄ and YTaO₄ mixture;
Figure 67 is a schematic of the emission spectra under X-Ray for various materials including. Y₂O₃, CaWO₄, YaTO₄, YaTO₄:Nb, BaSO₄:Eu, La₂O₂S:Tb, BaSi₂O₅:Pb for various voltages between the filament and the target;
Figure 68 is a schematic of emission spectra under X-ray excitation for scintillators;
Figure 69 is a schematic of emission spectra of lutetium oxyorthosilicate LSO under different excitation sources;
Figure 70 is a schematic depiction of a dilution process for cell assay analysis;
Figure 71 is another table depicting properties of various phosphors and the names of various phosphors;
Figure 72 is a schematic of the results from a clonogenic assay for an YTaO₄:Nb phosphor with and without a silica coating;
Figure 73 is a schematic of the results from a clonogenic assay for a BaSO₄:Eu phosphor with and without a silica coating;
Figure 74 is a schematic of the results from a clonogenic assay for a BaSi₂O₃:Pb phosphor with and without a silica coating;
Figure 75 is a schematic showing the effect of X-ray from a voltage of 160 kVp and 1 mg/ml concentration of the YTaO₄ phosphor showing a XRT and Phosphor effect, and further cell kill when adding trimethyl psoralen (TMP);
Figure 76 is a schematic of the results from a clonogenic assay for a YTaO₄ phosphor with and without a silica coating for three different concentrations added to a B16 mouse melanoma cells with TMP;
Figure 77 is a schematic of the results from a clonogenic assay for a YTaO₄ phosphor (uncoated) at 0.75 mg/ml +/- 2 gray XRT at 160 kVp or 320 kVp;
Figure 78 is a schematic of the results from a clonogenic assay for an YTaO₄:Nb phosphor (uncoated) at 0.75 mg/ml , +/- 2 gray XRT at 160 kVp and 320 kVp;
Figure 79 is a schematic of the results from a clonogenic assay for a LaOBr:Tm phosphor (coated with SiO₂);
Figure 80 is a schematic of the results from a clonogenic assay for a LaOBr:Tm phosphor (coated with SiO₂) with Phosphor-Alone Toxicity using at 0.75mg/ml and phosphor plus TMP at 80 kVp XRT for 1 or 4 minutes total;
Figure 81 is a schematic of the results from a clonogenic assay for a LaOBr:Tm phosphor (coated with SiO₂) with Phosphor-Alone Toxicity using at 0.75mg/ml and phosphor plus TMP at 40 kVp XRT for 1 or 4 minutes total;
Figure 82 is a schematic of a cell kill assay performed with a CaWO₄ phosphor combined with the Y₂O₃ particles;
Figure 83 is a schematic of the results from a clonogenic assay for B16 mouse melanoma cells treated with a CaWO₄ phosphor;
Figure 84 is a schematic of the results from a clonogenic assay for B16 mouse melanoma cells treated with a CaWO₄ phosphor by varying the X-ray voltage;
Figure 85 is a schematic of a container including a solution containing nano-particles;
Figure 86 is a schematic of a solution containing nano particles applied to a quartz wafer through the process of spin coating;
Figure 87 is a schematic of a wafer dried to produce a thin layer of nanoparticles dispersed across the surface of the wafer;
Figure 88 is a schematic of a wafer a taken to a physical vapor deposition system
Figure 89 is a schematic of a wafer placed onto a stage and inserted into physical vapor deposition system for applying a coating on a top half of the nano particles;
Figure 90 is a schematic of a cross section of the quartz wafer coated with nanoparticles;
Figure 91 is a schematic of the half coated phosphor particles placed back in solution inside a container that has a biased stage;
Figure 92 is a schematic of the half coated phosphor particles placed back in solution inside a container that has a RF biased stage;
Figure 93 is a schematic of the half coated phosphor particles placed back in solution inside a container that has a RF biased micro-electrode structure;
Figure 94 is a schematic of the half coated phosphor particles disposed around a metallic nano rod and heated to sufficient temperatures to alloy the metallic coating with the metallic nano rod;
Figure 95 is a schematic of mass transport being used to form a neck between particles;
Figure 96 is a schematic showing alignment of a magnetic particle under a magnetic field and followed by joining the phosphor and the magnetic particles with a lateral field configuration;
Figure 97 is a schematic showing the joining of a magnetic particle and phosphor through a necking process;
Figure 98 is a schematic showing the joining of a magnetic particle and phosphor through an adhesion process by surface modification of at least one of the particles;
Figure 99 is a schematic showing a lipid envelope around the adhered phosphor and nano magnetic particle;
Figure 100 is a schematic showing the alignment of a magnetic particle under a magnetic field and followed by joining the phosphor and the magnetic particles (orthogonal field configuration);
Figure 101 is a schematic showing that, after joining the particles in an orthogonal field configuration, the particles would have a tendency to self-assemble in a recto-linear fashion; and
Figure 102 is a schematic showing that, after joining the particles in a lateral field configuration.

### DETAILED DESCRIPTION OF THE INVENTION

Reference will now be made in detail to a number of embodiments of the invention, examples of which are illustrated in the accompanying drawings, in which like reference characters refer to corresponding elements.

As noted above, energy converters such up conversion materials and down conversion materials have been used in a number of fields in effect to convert an incident wavelength of light to a different wavelength. Metallic structures have been placed on the phosphors or in a vicinity of the phosphors to generate a plasmonics effect which essentially is an amplification of the local field very nearby the outside of the metallic structures. In some applications, photonic band gap structures have been used in solar cell applications to prevent light of certain frequencies or wavelengths from propagating in one, two or any number of directions

within the materials. Additionally, antireflection coatings and concentrators are well known in the literature.

The present inventors recognized that the shortcomings of these structures could be addressed by use of the energy augmentation structures described herein used in conjunction with energy converters.

### A. ENERGY AUGMENTATION STRUCTURES

In the present invention, the term "energy augmentation" means effecting some change in one or more wavelengths of electromagnetic energy in at least one property, including, but not limited to, intensity, power, associated electrical field, associated magnetic field, wave amplitude, photonic flux, magnetic flux, phase, coherence, propagation direction, etc. The structure performing the energy augmentation can be termed an "energy augmentation structure" or an "energy augmentator". These terms are used interchangeably herein. Preferably the energy augmentation structure is a non-plasmonic structure (a structure that does not exhibit plasmonic properties).

The energy augmentator are antennas, such as fractal antennas.

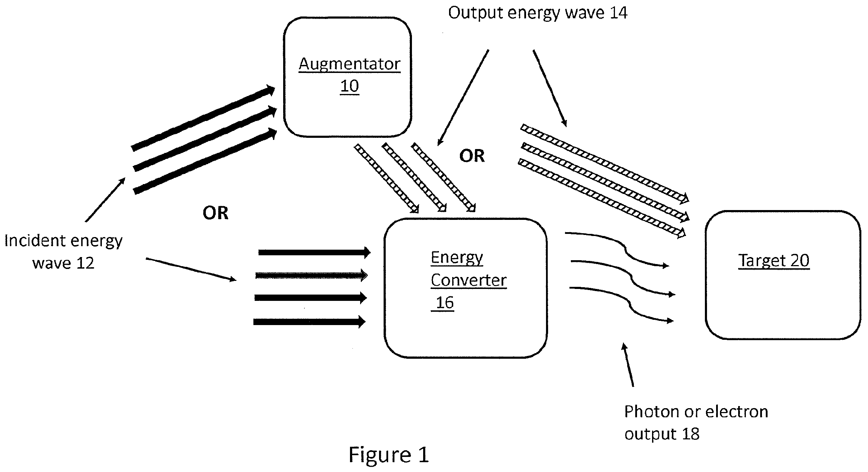

In one embodiment, as shown schematically in Figure 1, an energy augmentator 10 is provided that is capable of receiving or capturing one or more wavelengths of electromagnetic energy representing an incident energy wave 12. Having received or captured the incident energy wave 12, the energy augmentator 10 is capable of augmenting the one or more wavelengths of received or captured energy wave flux 12 in at least one property. As shown in Figure 1, in one embodiment, energy augmentator 10 then outputs an energy wave 14 with the at least one property augmented, with the augmented energy wave 14 incident on target 20. Details of the augmentation are described below.

In another embodiment, the output (augmented) energy wave 14 (i.e., one or more output wavelengths of electromagnetic energy) can be incident on an energy converter 16 (such as the up conversion materials and down conversion materials noted above). The energy converter 16 can output photons or electrons 18 which can be directed to target 20. In these embodiments, target 20 may receive the photons or electrons 18 or the output augmented energy wave 14 simultaneously or separately.

In one embodiment, the energy augmentator 10 may be one or more of an electromagnetic resonator structure, a folded resonator structure, and a fractal structure having a region of an intensified electromagnetic field within those structures.

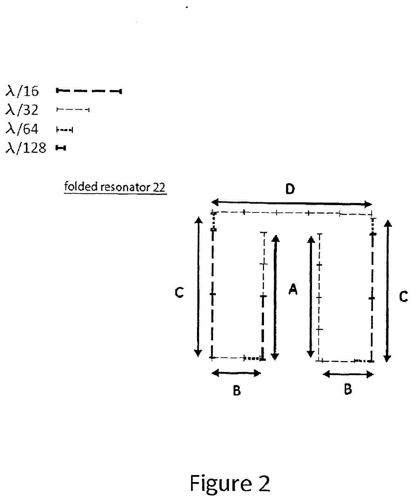

Figure 2 below is a diagram depicting a folded resonator structure 22 of this invention.

The resonator in one embodiment of the present invention is a 3/4λ metal structure bent, as shown in Figure 2 having a "folded" structure making for opposing electrodes between which an intense electric field is developed. Exemplary characteristics of the "folded structure" antenna are listed in the following table:

**Table 1**

| Wavelength (nm) | | | | | | | | | | | |
|---|---|---|---|---|---|---|---|---|---|---|---|
| Antenna Side | 1400 | 1300 | 1200 | 1100 | 1000 | 900 | 800 | 700 | 600 | 500 | 400 |
| A | 175.0 | 162.5 | 150.0 | 137.5 | 125.0 | 112.5 | 100.0 | 87.5 | 75.0 | 62.5 | 50.0 |
| B | 65.6 | 60.9 | 56.3 | 52.6 | 46.9 | 42.2 | 37.5 | 32.8 | 28.1 | 23.4 | 18.8 |
| C | 196.9 | 182.8 | 168.8 | 154.7 | 140.6 | 126.6 | 112.5 | 98.4 | 84.4 | 78.3 | 56.3 |
| D | 218.8 | 203.1 | 187.5 | 171.9 | 156.3 | 140.6 | 125.0 | 109.4 | 33.8 | 78.2 | 62.5 |
| Total | 2093.8 | 1015.5 | 937.5 | 859.4 | 781.3 | 703.1 | 625.0 | 546.9 | 468.8 | 390.6 | 312.5 |
| 3/4 lambda | 1050 | 975 | 900 | 825 | 750 | 675 | 600 | 525 | 450 | 375 | 300 |

The calculations of a theoretical 3/4 λ and the slightly oversized antenna to account for all the bending corners involved in making the antenna would result in this structure having a size between the theoretical 0.75*λ and the upper oversized limit 0.78*λ.

While the resonators shown in most of the drawings could be characterized as having a rectangular-shape loop connecting the opposing antenna sections or electrodes together, the invention is not so limited. Other "loop" shapes could be used, so long as the opposing electrodes are parallel and coplanar with one another, with the loop forming an electrical path having a length of ½ λ, with the opposing electrodes having a length of 1/8 λ each, thereby making the ¾ λ resonator.

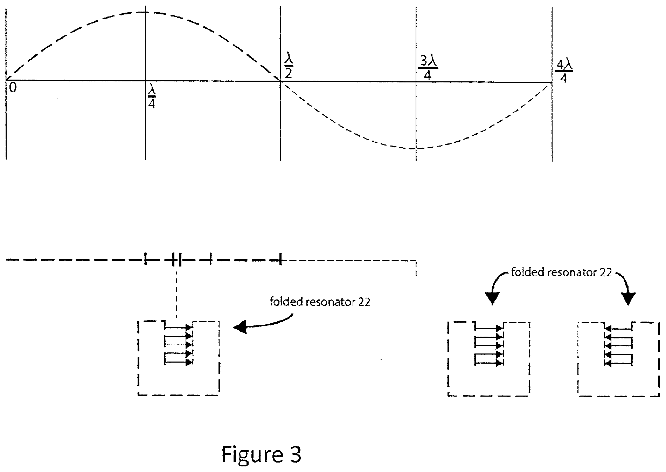

Figure 3 is a diagram depicting the basic concepts underlying one of the energy augmentation structures of this invention. In the depiction in Figure 3 is a sinusoidal wave representing for example an instantaneous waveform of a light wave (an incident energy flux 12). The depiction shows the length of ¾ of the wavelength 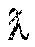 , and how in one embodiment a ¾ 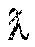 resonator is constructed with the open ends of the resonator "folded" together to form in this embodiment a ¾ 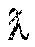 folded resonator 22. As shown in Figure 3, the folded ends form a region of an intensified, amplified electric field denoted by the horizontally directed arrows between the opposing open ends. When light nominally of a wavelength 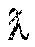 (or harmonics thereof 2 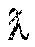 , 3 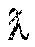 , 4 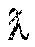 , etc.) is incident on the folded antenna structure, a fraction-a of the light will be coupled into this structure establishing the amplified electric field. Since the light from sun comes continuously and at different rotational polarizations, subsequent light waves will continue to "pump" the electric fields in the resonant structure until some "loss" mechanism caps the strength of the electric fields. For resonators made of low loss materials, high Q-factors are obtained which, in this case, could mean that the electric field strength between the opposing electrodes may be for example 100 to 1000 times the peak amplitude of the electric field vector of the incident waveform.

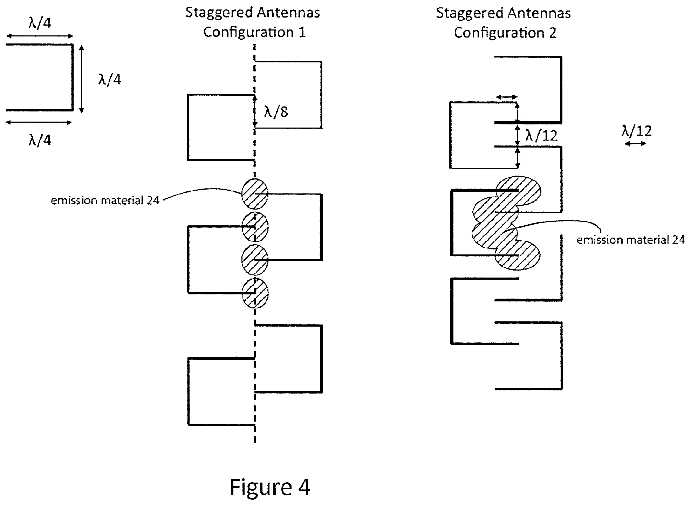

In another embodiment, a resonating antenna could have the configuration below shown in Figure 4. Here, the ¾ 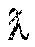 structures oppose and are interdigitated together without a "folded" structure. In the depiction in Figure 4, the horizontal stubs are ¾ 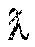 long, the vertical extending connectors are ¼ long, and the vertical spacing between the horizontal stubs and the extend of interdigitation varies as shown between configuration 1 and configuration 2. In one embodiment of the invention, an energy converter, a light or electron emitting material, or a color emitting or color converter material (i.e., emissive material 24) is placed inside or around the region of an intensified electric field, as shown in Figure 4.

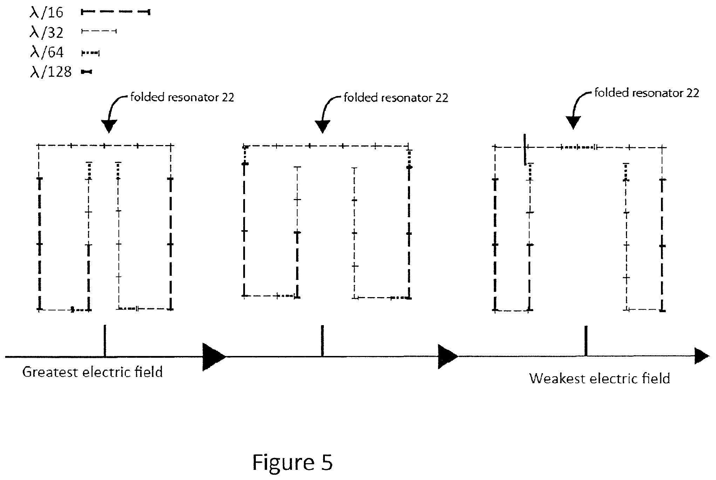

Figure 5 shows that different ¾ 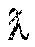 folded resonators can be made having different distances between the opposing electrodes and thus different electric field strengths. In this way, the folded resonators of the invention can be adjusted such that the strength of the electric field between the opposing electrodes does not exceed the dielectric strength of any material in between. Exceeding the dielectric strength of any material in between could result in destruction of that material as intense current (e.g., a micro-arc) would flow during any time that the dielectric strength was exceeded, thus breaking the material down. As shown, here the opposing sides need not have an exact length of 1/8 λ.

In one embodiment of the invention, an energy converter, a light or electron emitting material, or a color emitting or color converter material (i.e., an emissive material) 24 is placed inside or around the regions of intensified electric field near/between the opposing electrodes. In one embodiment of the invention, the color emitting or color converter material may itself be absorbing a color light such as for example blue light and emitting lower energy, down-shifted red light. In this case, a red phosphor could be the color emitting or color converter material.

While the ¾ 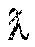 folded resonator in one embodiment could be designed to resonate at blue light ( 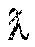 = 420 to 440 nm), the resonator is preferably designed to resonate from light at a different frequency than the blue light that is being absorbed by the red phosphor. In one embodiment, for color enhancement for objects under solar light, the ¾ 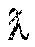 folded resonator could be designed to be driven by infrared light from the solar spectrum (e.g. 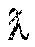 = 700 to 1000 nm) to generate the intensified electric field, and the red phosphor disposed in the region of intensified electric field would have a brighter red emission than if the intensified electric field were not present.

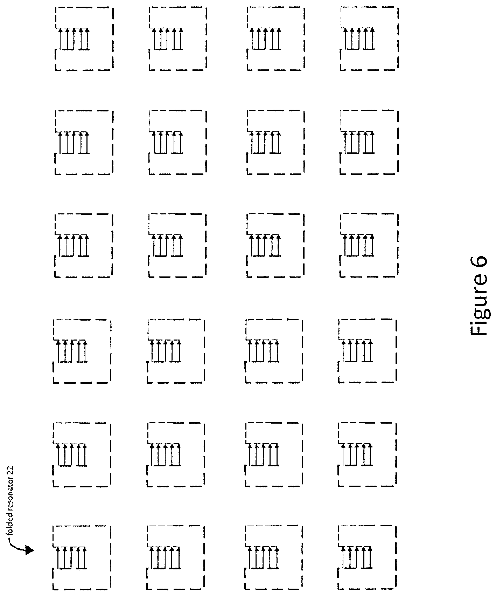

Figure 6 is diagram showing a pattern of ¾ 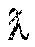 folded resonators 22 distributed in space. As to be discussed in more detail later, there are numerous ways to distribute the ¾ 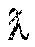 folded resonators. The present invention is not limited to the regular, uniformly spaced and sized resonators shown in Figure 6. There is no requirement that the distribution be regular, uniformly spaced, uniformly sized, or uniformly oriented. Differently sized, spaced, and oriented resonators may provide better utilization of the full spectrum of the sun or any other light source incident on the object.

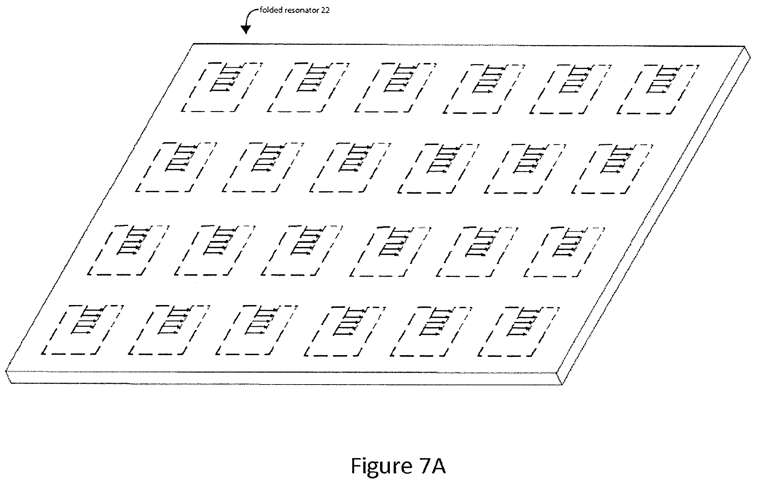

Figure 7A is a diagram showing a pattern of ¾ 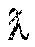 folded resonators 22 distributed in a plane or otherwise along a surface of an object. In one embodiment, this pattern could be formed by lithographic or stamping processes onto a planar surface such as a glass plate or onto a curved sheet type product. In one embodiment, the glass plate could itself be a phosphorescent plate or could have sections of different phosphorescent material deposited in a pattern that would align/match the respective positions of the opposing electrodes on each resonator. In one embodiment, the sheet product could be a laminate type of product applied to for example a nominally white object. Upon solar irradiation, the infrared part of the solar spectrum (normally only heating the surface) would generate the intensified electric field regions. In those regions, down converting phosphors converting deep blue and ultraviolet light to visible light would convert the deep blue and ultraviolet light of the solar spectrum to visible light, and the intensified electric field would enhance greater visible light emission.

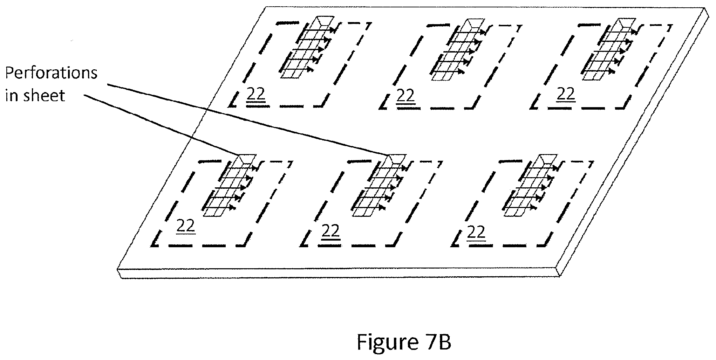

In one embodiment, the energy augmentators could be disposed on a perforated sheet, as shown in Figure 7B. The perforations in one embodiment are in the regions of intensified electric field such that phosphors or other energy converting materials or devices could be disposed in the perforations.

In one embodiment (for color enhancement), the sheet product could be a laminate type of product applied to for example a nominally green object. Upon solar irradiation, the infrared part of the solar spectrum (normally only heating the surface) would generate the intensified electric field regions. In those regions, down converting phosphors converting blue, deep blue and ultraviolet light to green light would convert the blue, deep blue, and ultraviolet light of the solar spectrum to green light and the intensified electric field would enhance greater green light emission.

In one embodiment, the sheet product could be a laminate type of product applied to for example a nominally red object. Upon solar irradiation, the infrared part of the solar spectrum (normally only heating the surface) would generate the intensified electric field regions. In those regions, down converting phosphors converting green, blue, deep blue and ultraviolet light to red light would convert the green, blue, deep blue and ultraviolet light of the solar spectrum to red light and the intensified electric field would enhance greater red light emission.

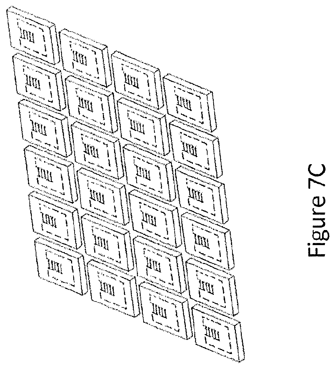

In one embodiment, the energy augmentators could be disposed on a sheet and then separated into distinct pieces, as shown in Figure 7C, which could be readily added and mixed into a medium to be processed.

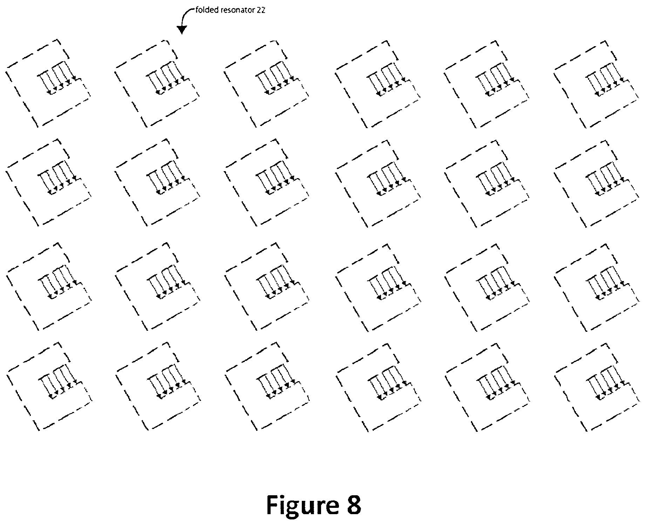

Figure 8 is a diagram showing a pattern of ¾ 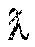 folded resonators 22 distributed in a plane or otherwise along a surface of an object and having a different orientation than in Figure 7. By having different orientations, the rotating polarized sun light waves which may at one instance not have an electric field alignment conducive to driving the ¾ 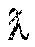 folded resonators, would have their electric field alignment conducive to driving resonators of a different orientation and therefore better aligned. Accordingly, if the sheet type products were used, layers of differently oriented ¾ 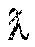 folded resonators could be stacked together.

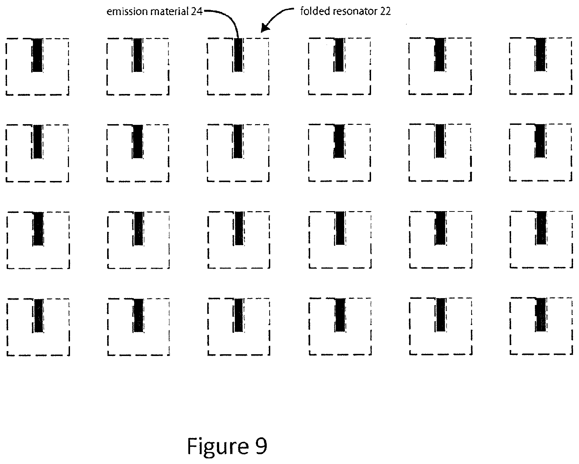

Figure 9 is a diagram showing a pattern of ¾ 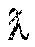 folded resonators 22 having an energy converter, a light or electron emitting material, or a color emitting or color converter material (i.e., emissive material 24) deposited in the region of between the opposing electrodes. Here, while shown in a plan view, the color converting or enhancing material deposited in the region of between the opposing electrodes may be deposited such that the color converting or enhancing material has an upper surface raised above the metal traces of the ¾ 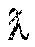 folded resonators. In this embodiment, the raised sections would intercept fringing fields of the intensified electric field between the opposing electrodes.

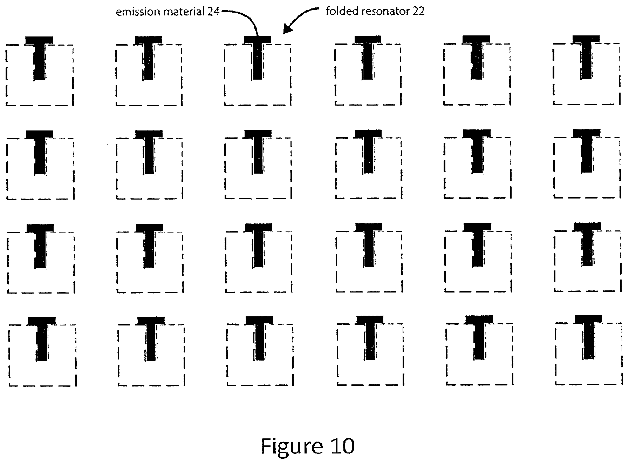

Figure 10 is a diagram showing a pattern of ¾ 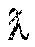 folded resonators 22 having an energy converter, a light or electron emitting material, or a color emitting or color converter material (i.e., emissive material 24) deposited in the region of between the opposing electrodes. Here, as before, the color converting or enhancing material deposited in the region of between the opposing electrodes may be deposited such that the color converting or enhancing material has an upper surface raised above the metal traces of the ¾ 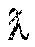 folded resonators. In this embodiment, the raised sections would intercept fringing fields of the intensified electric field between the opposing electrodes. In this embodiment, the raised sections would extend around the corners where geometrically the corners would further intensify the electric field.

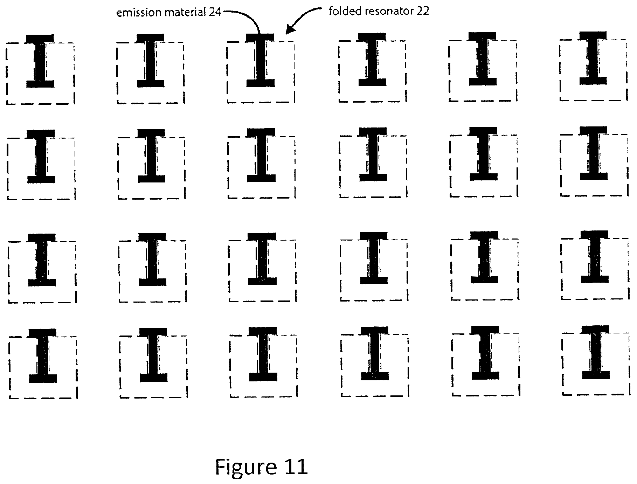

Figure 11 is a diagram showing a pattern of ¾ 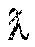 folded resonators 22 having an energy converter, a light or electron emitting material, or a color emitting or color converter material (i.e., emissive material 24) deposited in the region of between the opposing electrodes. Here, as before, the color converting or enhancing material deposited in the region of between the opposing electrodes may be deposited such that the color converting or enhancing material has an upper surface raised above the metal traces of the ¾ 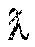 folded resonators. In this embodiment, the raised sections would intercept fringing fields of the intensified electric field between the opposing electrodes. In this embodiment, the raised sections would extend around the corners where geometrically the corners would further intensify the electric field and would extend around the ends of the opposing electrodes.

In these embodiments shown in Figures 9, 10, and 11, the energy converters, or light or electron emitting materials, or color emitting or color converter materials (i.e., emissive materials 24) are disposed in a vicinity of one or more energy augmentation structures (i.e., the ¾ 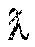 folded resonators). As such, the energy augmentation structures preferably are in a region of intensified electric field. The intensified electric field may represent a region of intensified energy especially if there is electrical current flow conductively coupling the energy converter to the one energy augmentation structures. In later embodiments, conductively coupling the energy converter to the one energy augmentation structures has advantages. Accordingly, the energy converters or color converting or enhancing materials disposed in a vicinity of one or more energy augmentation structures may have a physical conductive connection between the energy converter and the at least one energy augmentation structure. Alternatively, the coupling may be more that of radiatively or capacitively coupling the electric fields from the resonant structure into energy converters or color converting or enhancing materials disposed inside the energy augmentation structure, outside the energy augmentation structure, in a layer with the energy augmentation structure, or in a layer above or below the energy augmentation structure.

As used herein, in a vicinity of refers to the disposition of one thing inside the structure of another thing, outside and nearby or adjacent the structure of the other thing, and can include the disposition of one thing above or below the other thing in any three dimensional direction. Accordingly, in one embodiment of the present invention, the color converting or enhancing materials are disposed in a vicinity of the energy augmentation structures

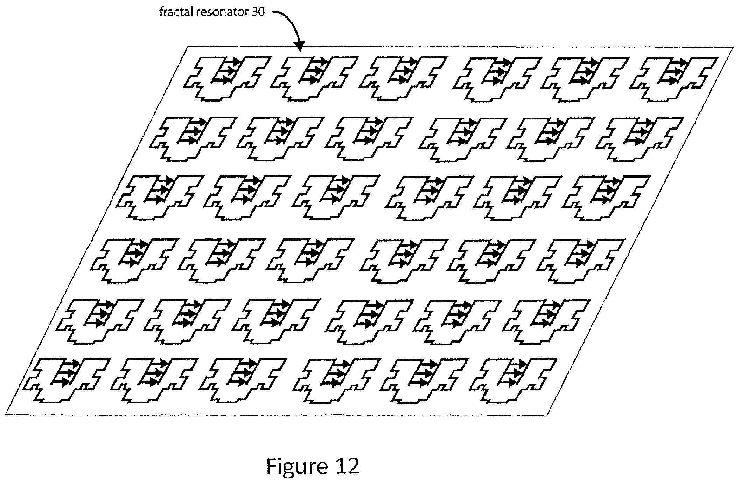

Figure 12 is a diagram showing a pattern of ¾ 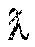 folded resonators 30 having for its metal traces a fractal pattern for the electrical path that loops around to connect the opposing electrodes. A fractal pattern for the electrical path with this pattern means that the metal trace can support various wavelengths resonating with the ¾ 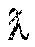 characteristics because of the multiplicity of possible loop paths available because the widths of each segment of the conductive path vary in width permitting electrical paths of different physical lengths to exist around the loop.

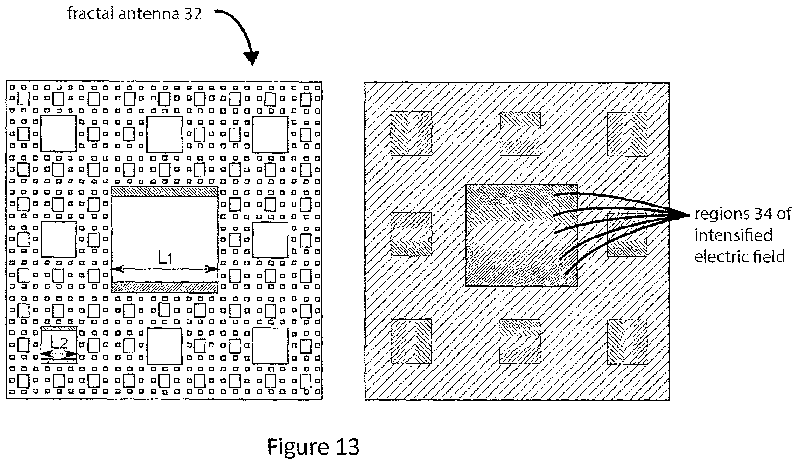

Figure 13 is a diagram showing another fractal antenna segment 32 where the straight-line sides of the metal pads have regions 24 of locally intensified electric field. Here, in one embodiment, the fractal antenna segment is designed for resonance in the infrared range, with the intensifies electric field regions 34 (for example as shown toward the straight-line sides of the metal pads being the place where blue phosphors and red phosphors (or other emissive materials 24) would be deposited such that their emission, would be enhanced the intensified electric fields in those regions 34.

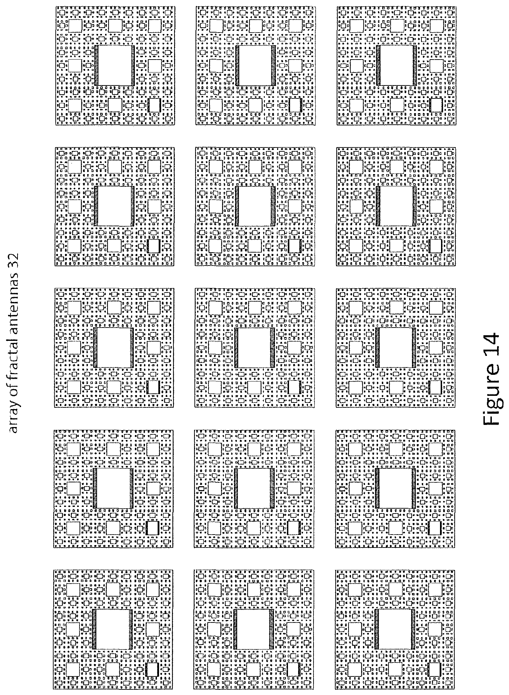

Figure 14 is a diagram showing a repeated pattern (array) of the fractal antenna segments 32 of Figure 13.

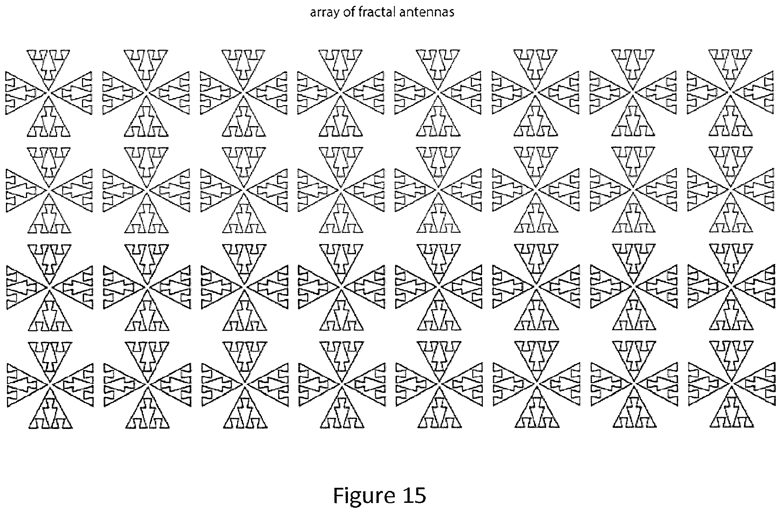

Figure 15 is a diagram showing a pattern (array) of bowtie fractal antenna segments, providing an alternative embodiment to the fractal antenna segments of Figure 14.

In one embodiment of the invention, the resonant structures can comprise three-dimensional fractal patterns. Known in the art is the fabrication of three-dimensional fractal structures by nanoscale anisotropic etching of silicon such as described in Nanoscale etching of 3d fractal structures with many applications including 3d fractal antennas and structures for filters, by Brian Wang, June 22, 2013, in the Journal of Micromechanics and Microengineering, (available at wvw.nextbigfuture.com/2013/06/nanoscale-etching-of-3d-fractal.html). In one embodiment of the invention, metal is deposited over a silicon three-dimensional fractal structure to form a multi-dimensional light collector.

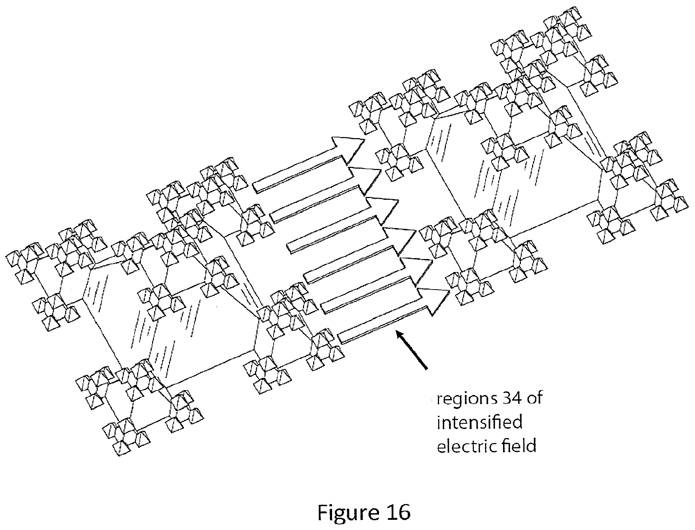

Figure 16 is a diagram showing a paired three-dimensional fractal structure with regions 34 of an intensified electric field in between the pairs. The paired three-dimensional fractal structure is a color enhancement structure according to one embodiment of the invention. In one embodiment of the invention, these pyramidal type structures would be metallized with opposing faces metalized, a first loop conductor formed around the other sides of the first pyramid, then connecting across a region between the pair, and then a second loop formed around the sides of the second pyramid to the metallized opposing face of the second pyramid, to mimic (as seem from above) the ¾ 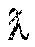 folded resonators shown in Figure 3.

In one embodiment, converter (emissive) materials 24 would be disposed nearby different sections of the pyramidal type structures and preferably between the opposing faces of the pair where the intensified electric field (depicted by the arrows) exists. With the three-dimensional aspect of this invention, red, yellow, green, and blue converters (or other designated emitters) could be disposed at different levels within this region of intensified electric field.

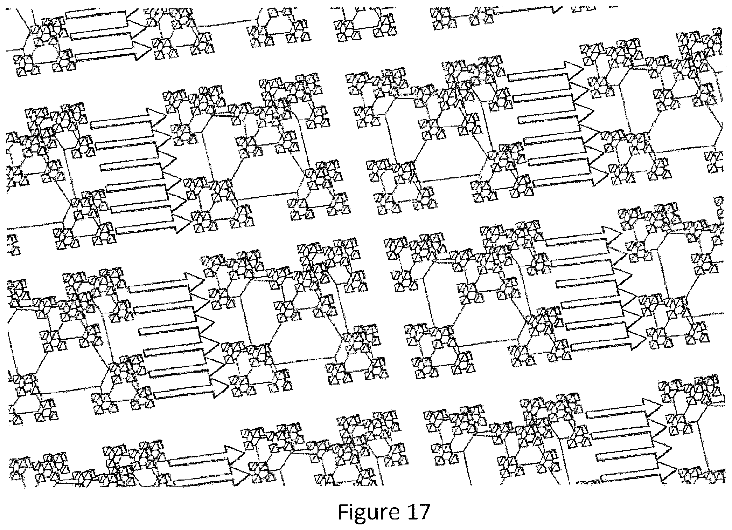

Figure 17 is a diagram showing a pattern (array) of the paired three-dimensional fractal structures of Figure 16.

In these embodiments shown in Figures 12 to 17, the energy converters, or light or electron emitting materials, or color emitting or color converter materials (i.e., emissive materials 24) are disposed in a vicinity of one or more energy augmentation structures (i.e., the ¾ 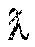 folded resonators). As such, the energy augmentation structures preferably are in a region of intensified electric field. The intensified electric field may represent a region of intensified energy especially if there is electrical current flow conductively coupling the energy converter to the one energy augmentation structures. In later embodiments, conductively coupling the energy converter to the one energy augmentation structures has advantages. Accordingly, the energy converters, or light or electron emitting materials, or color emitting or color converter materials disposed in a vicinity of one or more energy augmentation structures may have a physical conductive connection between the energy converter and the at least one energy augmentation structure. Alternatively, the coupling may be more that of radiatively coupling the electric fields from the resonant structure into energy converters or color converting or enhancing materials disposed inside the energy augmentation structure, outside the energy augmentation structure, in a layer with the energy augmentation structure, or in a layer above or below the energy augmentation structure.

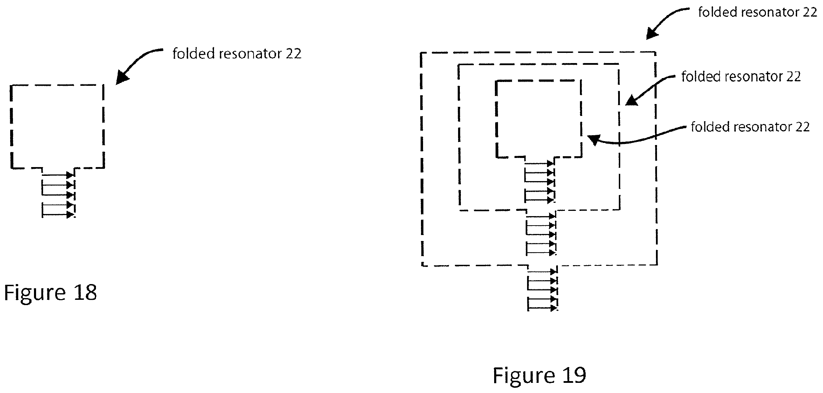

The energy augmentation structures are not limited to those shown above. Other variants are possible. Moreover, in one embodiment of the invention, the ¾ 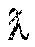 folded resonators need not to have the "folded sections" which fold inwards as shown in Figure 3. Instead, as shown in Figure 18, the ¾ 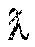 resonators of the invention can have folded sections which fold outward with the regions of intensified electric field being outside of the "loop" of the resonator. The distal ends of the antenna protrude outwardly while maintaining parallelism. Specifically, Figure 8 is a schematic of a ¾ 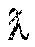 external-electrode folded resonator 22. This external, opposed electrode pair design follows the general apportioning, scaling aspects, converter material placement, etc., shown in Figures 5 through 11 but with the internal folded sections being replaced by the external-electrode pair.

In one embodiment of the invention, the ¾ 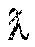 external-electrode folded resonator 22 provides the capability to be packed in a concentric-type arrangement with progressively increasing or decreasing size resonators. These resonators are maintained in plane with no overlapping distal ends. Figure 19 is a schematic of a plurality of concentric-type ¾ 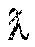 external-electrode folded resonators 22. Since each of the ¾ 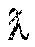 external-electrode folded resonators 22 has a different electrical length, the plurality of concentric-type ¾ 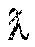 external-electrode resonators will be "tuned" to the different wavelengths associated with the respective electrical lengths. Three different frequencies are therefore focused between the distal ends of the antennas.

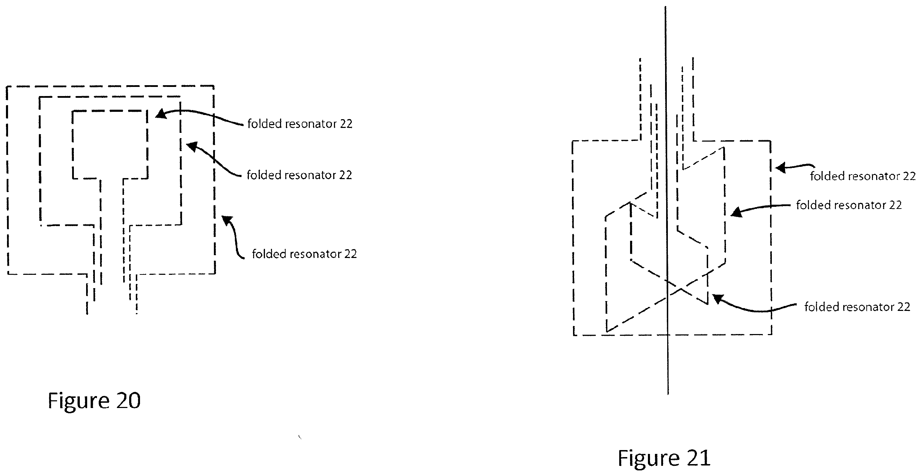

In another embodiment, Figure 20 is a schematic of a plurality of concentric-type ¾ 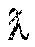 external-electrode folded resonators 22 with overlapping electrodes. In one embodiment, the overlapping provides a more concentrated/enhanced field region than in the non-overlapping arrangement of Figure 19.

The present invention is not limited to planar concentric type packing arrangements as shown in Figures 19 or 20. The three different ¾ wavelength resonators in Figure 20 are maintained in plane with overlapping distal ends. These antennas are inductively coupled. In one embodiment, the present invention utilizes an off plane configuration with axial symmetry where the antennas are in an axially rotated, multiple frequency, interleaved ¾ wave resonator structure. Figure 21 is a schematic of an axially rotated, multiple frequency, interleaved ¾ wave resonators 22 showing (in this example) three differently sized resonators for multiple frequency resonance disposed about/along a common axis but axially rotated. In one embodiment, in this configuration, the resultant electric field is concentrated without one electrode section perturbing the electric fields from another.

In a further embodiment, there is provided an energy collector comprising at least one energy augmentation structure; and at least one energy converter capable of receiving an applied electromagnetic energy, converting the applied electromagnetic energy and emitting therefrom an emitted electromagnetic energy shifted in wavelength or energy from the applied electromagnetic energy and the energy converter being disposed in a vicinity of the at least one energy augmentation structure such that the emitted electromagnetic energy is emitted with at least one augmented property compared to if the energy converter were remote from the at least one energy augmentation structure.

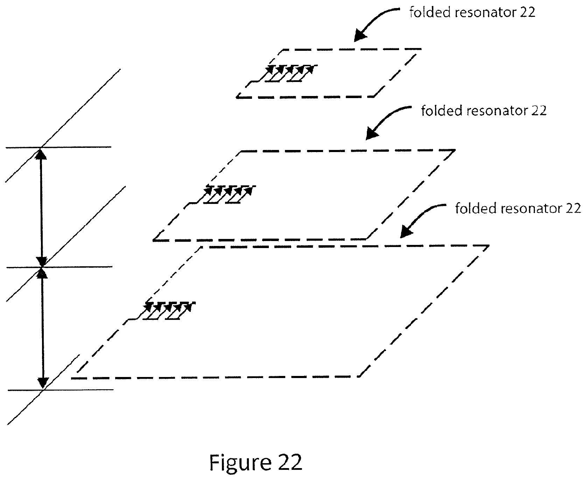

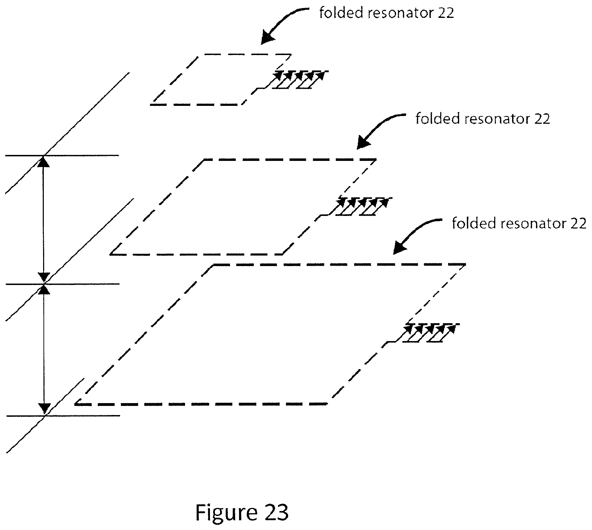

In one embodiment, the present invention can use different levels for disposing ¾ 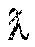 resonators thereon regardless of the resonators being ¾ 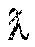 internally-folded resonators or ¾ 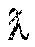 external-electrode resonators. This packing is shown in Figures 22 and 23 for configuration in parallel planes with distal ends folded in or protruding out respectively.

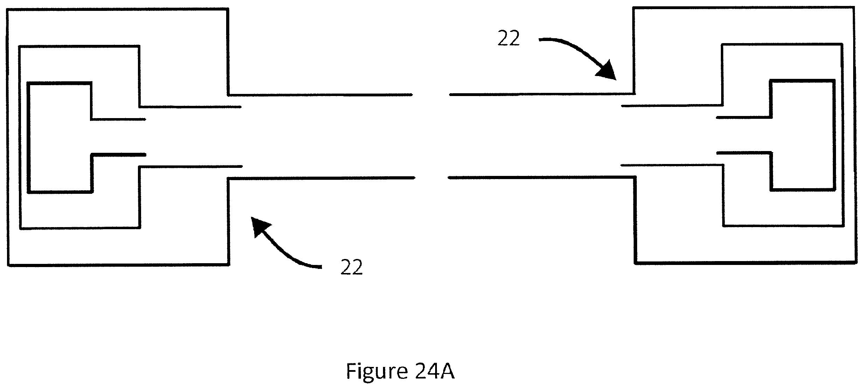

In the embodiment of the invention depicted in Fig. 20 having a plurality of concentric-type ¾ 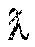 external-electrode resonators 22, the antennas are inductively decoupled. This configuration allows the electric field to be focused from three different frequencies in a longer path. This configuration can be used to create a mirror image configuration to extend the length of focused electric field as is illustrated in Fig. 24A.

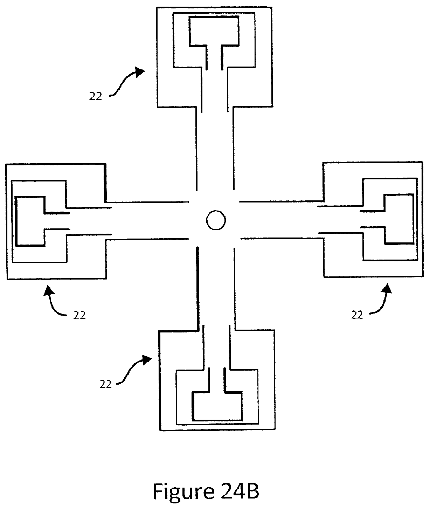

The resonator configuration in this case is mirror imaged with another set of antennas (folded resonators 22) to create a longer path (doubled) of focused electric field. Furthermore, the resonator antenna configuration can be placed in more creative ways to enhance the electric field focusing around a target as is illustrated in the Figure 24B.

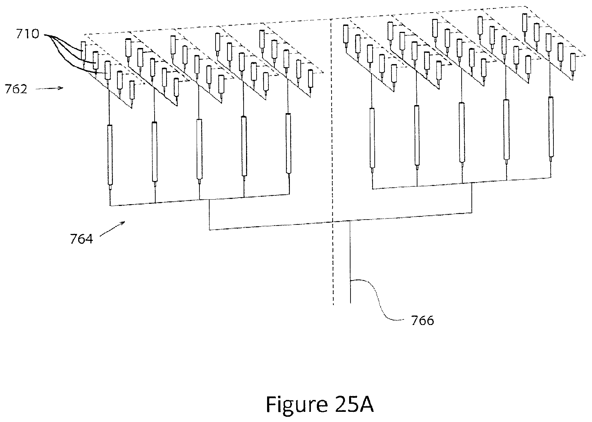

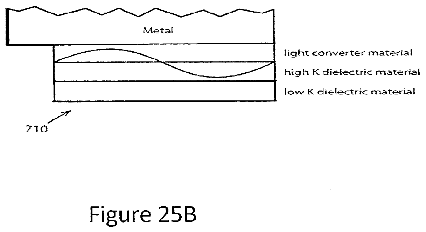

The configuration in Figure 25 allows the surrounding of a target within the plane of the resonator structure/antenna for the purpose of heating and focusing energy around the target. This prevents heat dissipation in silicon where the thermal conductivity is high. The silicon substrate in such an instance can be single crystalline, polycrystalline or amorphous.

In one embodiment of the present invention, an "energy augmentation structure" represents a structure whereby a spatial region of the energy collector contains a converter material (or other light or electron emitting material) exposed to energy which stimulates emission of light at a different energy (wavelength) from that to which it is exposed while being in a spatial area/volume (e.g., between or around or in a vicinity of the folded structures or the external-electrode pairs) where there is an artificially induced higher electrical field and/or a higher energy density. These artificial regions can be produced for example by use of structures including, but not limited to, multiple level collection optics, resonators, fractal antennas, and electrical grid (or electrode) patterns.

By having the light or electron emitting materials disposed in a vicinity of the energy augmentation structures of this invention, regardless of the whether the energy augmentation structure is in a region of intensified electric field or otherwise outside the region of intensified electric field, the energy augmentation structures of the invention are able to produce light which can be used for a variety of applications, in particular for photostimulation of biological, chemical, and physical reactions such as for example photoactivation of photoreactive drugs, photoactivation of photosensitive materials such as adhesives or lithographic photoresists, or for direct interaction with biological and chemical agents in the environment of the augmentation structures, as in sterilization.

In one embodiment, the light or electron emitting materials noted above are disposed with an energy augmentation structure comprising one or more of an electromagnetic resonator structure, a folded resonator structure, and a fractal resonating structure, any of which having a region of an intensified electromagnetic field within the resonating structures.

In one embodiment, the energy converter or light or electron emitting materials noted above includes one or more luminescing materials. As described herein, there are uses of the energy augmentation structure and/or energy collector embodiments which enhance bioluminescence, chemo-luminescence, photoluminescence, fluorescence, mechano-luminescence, and/or electron emission.

In one embodiment, the energy converter or light emitting materials noted above includes for the one or more luminescent materials phosphorescent materials, fluorescent materials, electroluminescent materials, chemo-luminescent materials, bioluminescent materials, and mechano-luminescent materials used in conjunction with or not in conjunction with the energy augmentation structure noted above. When used in conjunction with the energy augmentation structure noted above, the emitted electromagnetic energy from the luminescent material is emitted with at least one augmented property compared to if the energy converter (e.g., the luminescent material) were remote from the at least one energy augmentation structure.

In one embodiment, the bioluminescent materials are UV-emitting bioluminescent materials such as catalyzed luciferase and luminescent proteins.

In one embodiment, the energy converter or light emitting materials noted above includes for the one or more luminescing materials phosphorescent materials, fluorescent materials, electroluminescent materials, chemo-luminescent materials, bioluminescent materials, and mechano-luminescent materials used in conjunction with or not in conjunction with the energy augmentation structure noted above and which emit one of ultra-violet, visible, near infrared, and infrared light. In this embodiment, UV-emitting electroluminescent materials or mechano-luminescent devices and materials can be used. In this embodiment, UV-emitting bioluminescent materials can be used.

In some embodiments, metallic patterns form a folded resonator having opposing electrodes with electric fields directed in between, and a converter is positioned between the opposing electrodes or within fringing electric field of the opposing electrodes or otherwise in a vicinity of the opposing electrodes. In one example, the folded resonator is a ¾ 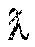 folded resonator. In one example, metallic patterns comprise at least one of Au, Ag, Cu, Al, or transparent metal oxides. In another example, the metallic patterns can be formed with refractory metals such for example Ti, W, and Mo.

In some embodiments, the metallic patterns referenced above comprise an external external-electrode pair structure having opposing electrodes with electric fields directed in between, and a converter is positioned between the opposing electrodes or within fringing electric field of the opposing electrodes or otherwise in a vicinity of the opposing electrodes. In one example, the resonator is a ¾ 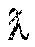 external-electrode pair resonator. In one example, metallic patterns comprise at least one of Au, Ag, Cu, Al, or transparent metal oxides. In another example, the metallic patterns can be formed with refractory metals such for example Ti, W, and Mo.

In some embodiments, plural resonators and plural converters are disposed at multiple positions throughout a light collector. In one example, the plural converters are positioned to convert light being internally scattered within the light collector.

In some embodiments of the energy augmentation structures, a first level of metallic patterns (or a second level of metallic patterns) comprises a metal core cladded with a high-K dielectric and a subsequent cladding of a low-K dielectric. In some embodiments of the energy augmentation structures, the first level of metallic patterns noted above (or the second level of metallic patterns) comprises a radial pattern of conductors. In some embodiments of the energy augmentation structures, the first level of metallic patterns noted above (or the second level of metallic patterns) comprises a fractal pattern. In one example, the fractal pattern is embedded within a dielectric material.

In some embodiments of the energy augmentation structures, the first level of metallic patterns noted above (or the second level of metallic patterns) comprises a three-dimensional fractal structure.

In some embodiments of the energy augmentation structures, there is provided a panel with the first level of metallic patterns and the second level of metallic patterns and optionally multiple converters formed therein or thereon. In some embodiments of the augmentation structures, there is provided a sheet with the first level of metallic patterns and the second level of metallic patterns and optionally multiple converters formed therein or thereon.

In some embodiments of the energy augmentation structures, the first level of metallic patterns noted above (or the second level of metallic patterns) is of different sizes and/or orientations to each other of the first level of metallic patterns or with respect to the second level of metallic patterns.

In another embodiment, the energy augmentator can collect or distribute light.

Figure 25A is a schematic illustrating a distributed point light collector/transmitter of the invention showing a distribution of branches that can either collect light from distributed points 710 or conversely can distribute light from a central source 766 to the distributed points 710. The section of the collector/transmitter is shown in Figure 25B showing a core metal, an optional light converter material, a high K dielectric, and a low K dielectric. In this arrangement, as shown, light is confined and not loss to scatter out of the collector/transmitter, except at the ends.

### B. ENERGY CONVERTERS

In various embodiments of the invention, energy converters are used with the energy augmentators described above. In some embodiments, the converters are for up conversion of light e.g., from the IR regime into visible electromagnetic radiation and for down conversion of light e.g., from the UV range into visible electromagnetic radiation. The invention in various embodiments up converts energy, preferably light in the visible spectrum. The invention encompasses a variety of applications where the up and down conversion materials with or without the energy augmentators are included to enhance electromagnetic energy emission, preferably light or photon emission. An energy augmentator may be separate from or connected to the energy converter. In certain embodiments, the energy converter can have the energy augmentator formed on its surface through chemical vapor deposition ("CVD") or physical vapor deposition ("PVD") processes or other nanoscale "printing" methods. Such embodiments may be particularly useful in methods for treating human or animal patients, in which having such energy augmentators "imprinted" on a surface of the energy converter can guarantee proximity between the energy augmentator and the energy converter to maximize the interaction with the energy being applied. Alternatively, the energy augmentator can be formed on a surface of an inert non-energy converting particle, formed, for example, from silica or formed from a non-energy converting particle coated with an biologically and/or chemically inert coating (such as, for example, diamond, diamond-like carbon, or similar inert materials). Such an energy augmentator can then be co-administered with the energy converter to the human or animal patient.

Suitable energy modulation agents or energy converters (the two terms are used interchangeably herein) of the invention include, but are not limited to, a biocompatible fluorescing metal nanoparticle, fluorescing dye molecule, gold nanoparticle, a water soluble quantum dot encapsulated by polyamidoamine dendrimers, a luciferase (bioluminescence), a biocompatible phosphorescent molecule, a combined electromagnetic energy harvester molecule, and a lanthanide chelate capable of intense luminescence.

Alternatively, the energy modulation agent or energy converter can emit energy in a form suitable for absorption at a target site or receptor. For example, the initiation energy source may be acoustic energy and one energy converter may be capable of receiving acoustic energy and emitting photonic energy (e.g. sonoluminescent molecules) to be received by another energy converter that is capable of receiving photonic energy. Other examples include energy converters that receive energy at x-ray wavelength and emit energy at UV wavelength, preferably at UV-A wavelength. A plurality of such energy converters may be used to form a cascade to transfer energy from initiation energy source via a series of energy converters.

Resonance Energy Transfer (RET) is an energy transfer mechanism between two molecules having overlapping emission and absorption bands. Electromagnetic emitters are capable of converting an arriving wavelength to a longer wavelength. For example, UV-B energy absorbed by a first molecule may be transferred by a dipole-dipole interaction to a UV-A-emitting molecule in close proximity to the UV-B-absorbing molecule. Alternatively, a material absorbing a shorter wavelength may be chosen to provide RET to a non-emitting molecule that has an overlapping absorption band with the transferring molecule's emission band. Alternatively, phosphorescence, chemiluminescence, or bioluminescence may be used to transfer energy to a target site or a receptor such as a photoactivatable agent.

In a further embodiment, a biocompatible emitting source, such as a fluorescing metal nanoparticle or fluorescing dye molecule, is selected as an energy converter that emits in the UV-A band. In another embodiment, an energy converter comprising a UV-A emitting source can be a gold nanoparticle comprising for example a cluster of 5 gold atoms.

In another embodiment, an energy converter comprising a UV- or light-emitting luciferase is selected as the emitting source. A luciferase may be combined with ATP or another molecule, which may then be oxygenated with additional molecules to stimulate light emission at a desired wavelength. Alternatively, a phosphorescent emitting source may be used as the energy converter. One advantage of a phosphorescent emitting source is that the phosphorescent emitting molecules or other source may be electroactivated or photoactivated prior to insertion into the tumor either by systemic administration or direct insertion into the region of the tumor. Phosphorescent materials may have longer relaxation times than fluorescent materials, because relaxation of a triplet state is subject to forbidden energy state transitions, storing the energy in the excited triplet state with only a limited number of quantum mechanical energy transfer processes available for returning to the lower energy state. Energy emission is delayed or prolonged from a fraction of a second to several hours. Otherwise, the energy emitted during phosphorescent relaxation is not otherwise different than fluorescence, and the range of wavelengths may be selected by choosing a particular phosphor.

In one embodiment, the energy converters of the invention can include persistent after-glow phosphor materials emitting light in the visible to near ultraviolet and ultraviolet range. In one embodiment, Eu-doped strontium aluminate is used as an energy converter in which deep UV light or x-ray or electron beans "charge" the photoluminescence such that these phosphors can be charged outside for example a patient and then injected into target or diseased site where UV photons would be emitted. In another embodiment, gadolinium strontium magnesium aluminate is used as an energy converter in which deep UV light or x-ray or electron beans "charge" the photoluminescence such that these phosphors can be charged outside for example a patient and then injected into target or diseased site where UV photons would be emitted. U.S. Pat. Appl. Publ. No. 20070221883 describes specifically gadolinium-activated strontium magnesium aluminate having an excitation maximum at about 172 nm, and which emits in a narrow-band UV emission at about 310 nm. The '883 publication also describes other useful energy converters for this invention, making note of emission spectra between 300 nm and 320 nm for a Sr(Al,Mg)₁₂O₁₉:Gd phosphor and two 312 nm line emitting phosphors, YMgB₅O₁₀:Gd, Ce and YMgB₅O₁₀:Gd, Ce, Pr. WO2016200349 describes long lasting yellowish-green emitting phosphorescent pigments in the strontium aluminate (SrAl2O4) system, which could serve as energy converters in the present invention. WO 2016200348 describes long lasting bluish-green emitting phosphorescent pigments in the strontium aluminate (Sr4Al14O25) system, which could serve as energy converters in the present invention. Xiong et al in "Recent advances in ultraviolet persistent phosphors," Optical Materials X 2 (2019) describes a number of ultraviolet persistent phosphors that could as energy converters in the present invention. The table below provides a listing of such persistent phosphors:

| | | |
|---|---|---|
| SrO:Pb²⁺ | 390 | > 1 h |
| CaAl₂O₄:Ce³⁺Tb³⁺ | 400 | > 10 h |
| CaAl₂O₄:Ce³⁺ Tb³⁺ | 413 | > 10 h |
| Sr₂Al₂SiO₂:Ce³⁺ | 400 | several minutes |
| SrZrO₃ | 395 | < 1000 s |
| BaZsO₃:Mg²⁺ | 400 | > 2400 s |
| SrZrO₃:Pr³⁺ | 356 | |
| CdSiO₃:B1³⁺ | 360 | |
| CdSiO₃:Bi³⁺ Dy³⁺ | 360 | |
| CdSiO₃:Bi³⁺ Gd³⁺ | 344 | > 6 h |
| Sr₂MgGe₂O₇:Pb²⁺ | 370 | > 12 h |
| NaLuGeO₄:Bi³⁺ Eu³⁺ | 400 | > 63 h |
| CaZnGe₂O₆: Bi³⁺ | 300-700 | > 12 h |
| Cs₂NaYF₆:Pr³⁺ | 250 | > 2 h |

In one embodiment, the phosphor described by Xiong et al as CaAl₂O₄:Ce³⁺having an emission peak of 400 nm and a persistent time of more than 10 h could be used, where it would be charged by x-ray irradiation outside a patient and then injected at a diseased site to provide internally generated UV light.

In one embodiment, the persistent phosphors noted could be activated *ex vivo* and introduced along with psoralen (or other photoactivatable drug) into the patient by exchange of a bodily fluid or for example by supplying the persistent phosphors and the photoactivatable drug into a patient's blood stream.

In one embodiment, the persistent phosphors noted could be activated *in vivo* by injection of the phosphors into a diseased site and then exposure to x-rays.

In another embodiment, a combined electromagnetic energy harvester molecule is designed, such as the combined light harvester disclosed in J. Am. Chem. Soc. 2005, 127, 9760-9768. By combining a group of fluorescent molecules in a molecular structure, a resonance energy transfer cascade may be used to harvest a wide band of electromagnetic radiation resulting in emission of a narrow band of fluorescent energy. In another embodiment, a Stokes shift of an emitting source or a series of emitting sources arranged in a cascade is selected to convert a shorter wavelength energy, such as X-rays, to a longer wavelength fluorescence emission such an optical or UV-A.

In one embodiment, a lanthanide chelate capable of intense luminescence is used as an energy converter. In another embodiment, a biocompatible, endogenous fluorophore emitter is selected as an energy converter.

In one embodiment, the energy converters of the invention can include visible and UV-light emitting bioluminescent materials. In one embodiment, bioluminescent materials such as coelenterate-type luciferin analogues could be used including amide monoanion known to emit at 480 nm and oxyluciferin known to emit at 395 nm.

Among various materials, luminescent nanoparticles have attracted increasing technological and industrial interest. In the context of the invention, nanoparticle refers to a particle having a size less than one micron. While the description of the invention describes specific examples using nanoparticles, the invention in many embodiments is not limited to particles having a size less than one micron. However, in many of the embodiments, the size range of less than one micron, and especially less than 100 nm produces properties of special interest such as for example emission lifetime luminescence quenching, luminescent quantum efficiency, and concentration quenching and such as for example diffusion, penetration, and dispersion into mediums where larger size particles would not migrate.

This invention in various embodiments can use a wide variety of down conversion materials (or mixtures of down conversion materials) with or without the energy augmentators to enhance light or photon emission. These down conversion materials can include quantum dots, semiconductor materials, alloys of semiconductor materials, scintillation and phosphor materials, materials that exhibit X-ray excited luminescence (XEOL), organic solids, metal complexes, inorganic solids, crystals, rare earth materials (lanthanides), polymers, scintillators, phosphor materials, etc., and materials that exhibit excitonic properties. Accordingly, the down conversion materials to enhance light or photon emission can convert energy from one of ultraviolet light, x-rays, and high energy particles to visible light. The down conversion materials to enhance light or photon emission can convert energy from higher energy visible light to lower energy visible light.

In one embodiment of the invention, a quantum dot mixture with or without the energy augmentators can be used for the multiple nanoparticles. Quantum dots are in general nanometer size particles whose energy states in the material of the quantum dot are dependent on the size of the quantum dot. For example, quantum dots are known to be semiconductors whose conducting characteristics are closely related to the size and shape of the individual crystal. Generally, the smaller the size of the crystal, the larger the band gap, the greater the difference in energy between the highest valence band and the lowest conduction band becomes. Therefore, more energy is needed to excite the dot, and concurrently, more energy is released when the crystal returns to its resting state. In fluorescent dye applications, this equates to higher frequencies of light emitted after excitation of the dot as the crystal size grows smaller, resulting in a color shift from red to blue in the light emitted. Quantum dots represent one way to down convert ultraviolet light of the spectrum to a targeted wavelength or energy emission. Quantum dots represent one way to down convert blue light of the spectrum to a targeted wavelength or energy emission.

As described in U.S. Pat. No. 6,744,960 different size quantum dots produce different color emissions. In that work and applicable to this invention, quantum dots can comprise various materials including semiconductors such as zinc selenide (ZnSe), cadmium selenide (CdSe), cadmium sulfide (CdS), indium arsenide (InAs), and indium phosphide (InP). Another material that may suitably be employed is titanium dioxide (TiO₂). The size of the particle, i.e., the quantum dot 18, may range from about 2 to 10 nm. Since the size of these particles is so small, quantum physics governs many of the electrical and optical properties of the quantum dot. One such result of the application of quantum mechanics to the quantum dot 18 is that quantum dots absorb a broad spectrum of optical wavelengths and re-emit radiation having a wavelength that is longer than the wavelength of the absorbed light. The wavelength of the emitted light is governed by the size of the quantum dot. For example, CdSe quantum dots 5.0 nm in diameter emit radiation having a narrow spectral distribution centered about 625 nm while quantum dots 18 including CdSe 2.2 nm in size emit light having a center wavelength of about 500 nm. Semiconductor quantum dots comprising CdSe, InP, and InAs, can emit radiation having center wavelengths in the range between 400 nm to about 1.5 µm. Titanium dioxide TiO₂also emits in this range. The linewidth of the emission, i.e., full-width half-maximum (FWHM), for these semiconductor materials may range from about 20 to 30 nm. To produce this narrowband emission, quantum dots simply need to absorb light having wavelengths shorter than the wavelength of the light emitted by the dots. For example, for 5.0 nm diameter CdSe quantum dots light having wavelengths shorter than about 625 nm is absorbed to produce emission at about 625 nm while for 2.2 nm quantum dots comprising CdSe light having wavelengths smaller than about 500 nm is absorbed and re-emitted at about 500 nm. In practice, however, the excitation or pump radiation is at least about 50 nanometers shorter than the emitted radiation.

Specifically, in one embodiment of the invention, a quantum dot mixture (QDM) coating can be deposited using CVD and or sol-gel techniques using standard precipitation techniques. The QDM coating can be made of a silicate structure that does not diminish UV output. Within the silicate family, silica (SiO₂) is suitable since it maximizes UV transmission through the coating. The coating can further include a second layer of a biocompatible glass. Such bio-compatible glass and glass ceramic compositions can contain calcium, a lanthanide or yttrium, silicon, phosphorus and oxygen. Other biocompatible materials and techniques are described in the following patents : U.S. Pat. Nos. 5,034,353; 4,786,617; 3,981,736; 3,922,155; 4-,120,730; and U.S. Pat. Appl. Nos. 2008/0057096; 2006/0275368; and 2010/0023101.

Further, the down conversion materials for the invention described here can be coated with insulator materials such as for example silica which will reduce the likelihood of any chemical interaction between the luminescent particles and the medium the particles are included therein. These and the other conversion materials described here can be used with or without energy augmentators. For biocompatible applications of inorganic nanoparticles, one of the major limiting factors is their toxicity. Generally speaking, all semiconductor nanoparticles are more or less toxic. For biocompatible applications, nanoparticles with toxicity as low as possible are desirable or else the nanoparticles have to remain separated from the medium. Pure TiO₂, ZnO, and Fe₂O₃ are biocompatible. CdTe and CdSe are toxic, while ZnS, CaS, BaS, SrS and Y₂O₃ are less toxic. In addition, the toxicity of nanoparticles can result from their inorganic stabilizers, such as TGA, or from dopants such as Eu ²⁺, Cr ³⁺ or Nd ³⁺. Other suitable down conversion materials which would seem the most biocompatible are zinc sulfide, ZnS:Mn²⁺, ferric oxide, titanium oxide, zinc oxide, zinc oxide containing small amounts of Al₂O₃, and AgI nanoclusters encapsulated in zeolite. For non-medical applications, where toxicity may not be as critical a concern, the following materials (as well as those listed elsewhere) are considered suitable: lanthanum and gadolinium oxyhalides activated with thulium; Er³⁺ doped BaTiO₃ nanoparticles, Yb³⁺ doped CsMnCl₃ and RbMnCl₃, BaFBr:Eu²⁺ nanoparticles, Cesium Iodine, Bismuth Germanate, Cadmium Tungstate, and CsBr doped with divalent Eu.

In various embodiments of the invention, the following luminescent polymers with or without energy augmentators are also suitable as conversion materials: poly(phenylene ethynylene), poly(phenylene vinylene), poly(p-phenylene), poly(thiophene), poly(pyridyl vinylene), poly(pyrrole), poly(acetylene), poly(vinyl carbazole), poly(fluorenes), and the like, as well as copolymers and/or derivatives thereof.

In various embodiments of the invention, the following materials with or without energy augmentators can be used similar to that detailed in U.S. Pat. No. 7,090,355, For down-conversion, the following materials can be used. Inorganic or ceramic phosphors or nano-particles, including but not limited to metal oxides, metal halides, metal chalcogenides (e.g. metal sulfides), or their hybrids, such as metal oxo-halides, metal oxo-chalcogenides. Laser dyes and small organic molecules, and fluorescent organic polymers. Semiconductor nano-particles, such as II-VI or III-V compound semiconductors, e.g. fluorescent quantum dots. Organometallic molecules including at least a metal center such as rare earth elements (e.g. Eu, Tb, Ce, Er, Tm, Pr, Ho) and transitional metal elements such as Cr, Mn, Zn, Ir, Ru, V, and main group elements such as B, Al, Ga, etc. The metal elements are chemically bonded to organic groups to prevent the quenching of the fluorescence from the hosts or solvents. Phosphors can be used including the Garnet series of phosphors: (Y ₘ A ₁₋ₘ) ₃ (Al ₙ B ₁₋ₙ ) ₅ O ₁₂, doped with Ce; where 0 ≦m, n≦1, where A includes other rare earth elements, B includes B, Ga. In addition, phosphors containing metal silicates, metal borates, metal phosphates, and metal aluminates hosts can be used. In addition, nano-particulates phosphors containing common rare earth elements (e.g. Eu, Tb, Ce, Dy, Er, Pr, and Tm) and transitional or main group elements (e.g. Mn, Cr, Ti, Ag, Cu, Zn, Bi, Pb, Sn, and Tl) as the fluorescent activators, can be used. Materials such as Ca, Zn, Cd in tungstates, metal vanadates, ZnO, etc. can be used.

The commercial laser dye materials obtained from several laser dye vendors, including Lambda Physik, and Exciton, etc. can also be used with or without energy augmentators. A partial list of the preferred laser dye classes includes: Pyrromethene, Coumarin, Rhodamine, Fluorescein, other aromatic hydrocarbons and their derivatives, etc.. In addition, there are many polymers containing unsaturated carbon-carbon bonds, which also serve as fluorescent materials and find many optical and fluorescent applications. For example, MEH-PPV, PPV, etc. have been used in opto-electronic devices, such as polymer light emitting diodes (PLED). Such fluorescent polymers can be used directly as the fluorescent layer of the transparent 2-D display screen with and without energy augmentators.

As noted above, semiconductor nanoparticles (e.g., quantum dots) can be used with or without energy augmentators. The terms "semiconductor nanoparticles," in the art refers to an inorganic crystallite between 1 nm and 1000 nm in diameter, preferably between 2 nm to 50 nm. A semiconductor nano-particle is capable of emitting electromagnetic radiation upon excitation (i.e., the semiconductor nano-particle is luminescent). The nanoparticle can be either a homogeneous nano-crystal, or comprises of multiple shells. For example, the nanoparticle can include a "core" of one or more first semiconductor materials, and may be surrounded by a "shell" of a second semiconductor material. The core and/or the shell can be a semiconductor material including, but not limited to, those of the group II-VI (ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, and an alloy or a mixture thereof.

Fluorescent organometallic molecules containing rare earth or transitional element cations can be used for down conversion materials with or without energy augmentators. Such molecules include a metal center of rare earth elements including Eu, Tb, Er, Tm, Ce protected with organic chelating groups. The metal center may also include transitional elements such as Zn, Mn, Cr, Ir, etc. and main group elements such as B, Al, Ga. Such organometallic molecules can readily dissolve in liquid or transparent solid host media. Some examples of such fluorescent organometallic molecules include: 1. 'I'ris(dibenzoylmethane)mono(phenanthroline)europium(III), 2. Tris(8-hydroxyquinoline)erbium; 3. Tris(1-phenyl-3-methyl-4-(2,2-dimethylpropan-1-oyl)pyrazolin -5-one)terbium(III); 4. Bis(2-methyl-8-hydroxyquinolato)zinc; 5. Diphenylborane-8-hydroxyquinolate.

Specific examples of down-conversion materials for red emission include those discussed above and europium complexes such as those described in JP Laid-open Patent Publication (Kokai) No. 2003-26969, constructed such that β-diketone ligand is coordinated to europium forming an europium complex capable of emitting red fluorescence. Other specific examples of the rare earth element complexes include complexes include lanthanum (Ln), europium (Eu), terbium (Tb), and gadolinium (Gd) and combinations thereof. An europium (Eu) complex is capable of emitting red fluorescence when irradiated with ultraviolet rays having a wavelength ranging from 365 nm to 410 nm. Terbium (Tb) is capable of emitting green fluorescence when irradiated with ultraviolet rays having a wavelength of 365 nm.

In other down-conversion embodiments, the down conversion materials which emit red light may include europium, light emitting particles which emit green light may include Terbium, and light emitting particles which emit blue or yellow light may include cerium (and/or thulium). In up-conversion embodiments, up conversion materials which emit red light may include praseodymium, light emitting particles which emit green light may include erbium, and light emitting particles which emit blue light may include thulium. In embodiments, the conversion materials can be light emitting particles made of fluorescent molecules that emit different colors (e.g. red, green, and blue), or different wavelengths or energies of light. In embodiments, the conversion materials can be light emitting particles made of pure organic or organo-metallic dyes with or without energy augmentators.

In addition to the combinations of rare earth complexes, such as a combination of a europium complex and a terbium complex, it is also possible employ a combination of a europium complex and a green-emitting fluorescent substance which is not a complex, or a combination of a terbium complex and a red-emitting fluorescent substance which is not a complex.

Other down converter materials (which can be used with or without energy augmentators) include for example ZnS, PbS, SbS₃, MoS₂, PbTe, PbSe, BeO, MgO. Li₂CO₃, Ca(OH)₂, MoO₃, SiO₂, Al₂O₃, TeO₂, SnO₂, KBr, KCl, and NaCl. These materials can include dopants to tailor the emission properties, as noted above. Examples of doped (or alloyed) glass systems suitable for the include Y₂O₃:Gd, Y₂O₃:Dy, Y₂O₃:Tb, Y₂O₃:Ho, Y₂O₃:Er, Y₂O₃:Tm, Gd₂O₃:Eu, Y₂O₂S:Pr, Y₂O₂S:Sm, Y₂O₂S:Eu, Y₂O₂S:Tb, Y₂O₂S:Ho, Y₂O₂S:Er, Y₂O₂S:Dy, Y₂O₂S:Tm, ZnS:Ag:Cl (blue), ZnS:Cu:Al (green), Y₂O₂S:Eu (red), Y₂O₃:Eu (red), YVO₄:Eu (red), and Zn₂SiO₄:Mn (green).

With regard more specifically to down converter materials suitable for the invention, U.S. Pat. No. 4,705,952 describes an infrared-triggered phosphor that stores energy in the form of visible light of a first wavelength and released energy in the form of visible light of a second wavelength when triggered by infrared light. The phosphors in U.S. Pat. No. 4,705,952 were compositions of alkaline earth metal sulfides, rare earth dopants, and fusible salts. The phosphors in U.S. Pat. No. 4,705,952 were more specifically phosphors made from strontium sulfide, barium sulfide and mixtures thereof; including a dopant from the rare earth series and europium oxide, and mixtures thereof; and including a fusible salt of fluorides, chlorides, bromides, and iodides of lithium, sodium, potassium, cesium, magnesium, calcium, strontium, and barium, and mixtures thereof. The materials described in U.S. Pat. No. 4,705,952 are useful in various embodiments of the invention with or without energy augmentators. In one example, the infrared-triggered phosphors would be used in conjunction with the folded resonators, and the receipt of a microwave or IR signal would locally heat and trigger emission. (This application would be particularly well suited for color enhancement and/or security applications.)

In other embodiments of the invention, the down converter materials (or mixtures of down converters materials (which can be used with or without energy augmentators) can include Y₂O₃: Li. Sun et al "Luminescent properties of Li+ doped nanosized Y2O3:Eu," Solid State Comm. 119 (2001) 393-396 describe such materials. Hou et al "Luminescent properties nano-sized Y2O3:Eu fabricated by co-precipitation method," Journal of Alloys and Compounds, vol. 494, issue 1-2, 2 April 2010, pages 382-385 describe that nano-sized yttria (Y₂O₃) powders have been successfully synthesized by a co-precipitation method. The powders were well crystallized, and the grains were almost spherical with good dispersibility. The quenching concentration of Eu³⁺ ions is 9 mol% which is much higher than micro-scaled powders. The incorporation of Li+ ions greatly improved the luminescence intensity. The highest emission intensity was observed with 4 mol% Li+ doped Y₂O₃:Eu powder ((Y_{0.87}Eu_{0.09}Li_{0.04})₂O₃) and the fluorescence intensity was increased by as much as 79%. Yi et al "Improved cathodoluminescent characteristics of Y2O3:Eu3+ thin films by Li-doping," Appl. Phys. A 87, 667-671 (2007) describe cathodoluminescent spectra for both Y₂O₃:Eu³⁺ and Li-doped Y₂O₃:Eu³⁺ films and methods for making these materials.

Specific downconverting materials may also include at least one or more of Y₂O₃, Y₂O₃:Gd, Y₂O₂S, NaYF₄, NaYbF₄, YAG, YAP, Nd₂O₃, LaF₃, LaCl₃, La₂O₃, TiO₂, LuPO₄, YVO₄, YbF₃, YF₃, Na-doped YbF₃, ZnS, ZnSe, MgS, CaS, Zn₂SiO₄:Mn, LaOBr:Tm and alkali lead silicate including compositions of SiO₂, B₂O₃, Na₂O, K₂O, PbO, MgO, or Ag, and combinations or alloys or layers thereof. Furthermore, the down-converting materials can be sulfur containing phosphors, which can help for example in the rubber vulcanization or other photoactivated processes. An example of such a sulfur containing phosphor is: (Sr,Ca)Ga₂S₄. Other examples wherein said phosphor particles comprise a thiogallate host material selected from the group consisting of SrGa₂S₄, CaGa₂S₄ BaGa₂S₄, MgGa₂S₄ and solid solutions thereof. The particle size of such phosphor can be controlled from 25 nm to 300 microns in size as described in US6153123A. The downconverting materials can include a dopant including at least one of Er, Eu, Yb, Tm, Nd, Mn, Sb, Tb, Ce, Y, U, Pr, La, Gd and other rare-earth species or a combination thereof. The dopant can be included at a concentration of 0.01%-50% by mol concentration. At times it is preferable to have a combination of dopants rather than one dopant such is the case for a Mn and Sb in silicate matrices.

The invention in other embodiments can use a wide variety of up conversion materials (or mixtures of up converters) with or without the energy augmentators to enhance a particular wavelength or energy of light emitted from a material or surface. These up conversion materials can include similar materials as discussed above with regard to down conversion but typically included doped or impurity states in a host crystal that provide a mechanism for up conversion pumping. Accordingly, the up conversion materials to enhance wavelength or energy emission can convert energy from one of near infrared, infrared, and microwave irradiation. The upconversion materials to enhance color emission can convert energy from lower energy visible light to higher energy visible light.

In one example, a nanoparticle of a lanthanide doped oxide can be excited with near infrared light such as laser light at 980 nm and 808 nm to produce visible light in different parts of the red, green, blue spectrum (different wavelengths or energies) depending on the dopant trivalent rare earth ion(s) chosen, their concentration, and the host lattice.

The lanthanide doped oxides suitable for this invention differ from more traditional multi-photon up conversion processes where the absorption of, for example, two photons is needed in a simultaneous event to promote an electron from a valence state directly into an upper level conduction band state where relaxation across the band gap of the material produces fluorescence. Here, the co-doping produces states in the band gap of the NaYF₄ such that the Yb³⁺ ion has an energy state at ²F_{5/2} pumpable by a single photon event and from which other single photon absorption events can populate even higher states. Once in this exited state, transitions to higher energy radiative states are possible, from which light emission will be at a higher energy than that of the incident light pumping the ²F_{5/2} energy state. In other words, the energy state at ²F_{3/2} of the Yb³⁺ ion is the state that absorbs 980 nm light permitting a population build up serving as the basis for the transitions to the higher energy states such as the ⁴F_{7/2} energy state. Here, transitions from the ⁴F_{7/2} energy state produce visible emissions.

U.S. Pat. No. 7,008,559 describes the upconversion performance of ZnS where excitation at 767 nm produces emission in the visible range. The materials described in U.S. Pat. No. 7,008,559 (including the ZnS as well as Er³⁺ doped BaTiO₃ nanoparticles and Yb³⁺ doped CsMnCl₃) are suitable in various embodiments of the invention with or without the energy augmentators.

Further, materials specified for up conversion materials in the invention (with or without energy augmentation) include CdTe, CdSe, ZnO, CdS, Y₂O₃, MgS, CaS, SrS and BaS. Such up conversion materials may be any semiconductor and more specifically, but not by way of limitation, sulfide, telluride, selenide, and oxide semiconductors and their nanoparticles, such as Zn₁₋ₓMnₓS_{y}, Zn₁₋ₓMnₓSe_{y}, Zn₁₋ₓMnₓTe_{y}, Cd₁₋ₓMnS_{y}. Cd₁₋ₓMnₓSe_{y}, Cd₁₋ₓMnₓTe_{y}, Pb₁₋ₓMnₓS_{y}, Pb₁₋ₓMnₓSe_{y}, Pb₁₋ₓMnₓTe_{y}, Mg₁₋ₓMnS_{y}, Ca₁₋ₓMnₓS_{y}, Ba₁₋ₓMnₓS_{y} and Sr₁₋ₓ, etc. (wherein, 0<x≤1, and 0<y≤1). Complex compounds of the above-described semiconductors are also contemplated for use in the invention--e.g. (M_{1-z}N_{z})₁₋ₓMnₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, Se, Te, O; 0<x≦ 1, 0<y≦ 1, 0<z≦ 1). Two examples of such complex compounds are Zn_{0.4}Cd_{0.4}Mn_{0.2}S and Zn_{0.9}Mn_{0.1}S_{0.8}Se_{0.2}. Additional conversion materials include insulating and nonconducting materials such as BaF₂, BaFBr, and BaTiO₃, to name but a few exemplary compounds. Transition and rare earth ion co-doped semiconductors suitable for the invention include sulfide, telluride, selenide and oxide semiconductors and their nanoparticles, such as ZnS; Mn; Er; ZnSe; Mn, Er; MgS; Mn, Er; CaS; Mn, Er; ZnS; Mn, Yb; ZnSe; Mn, Yb; MgS; Mn, Yb; CaS; Mn,Yb etc., and their complex compounds: (M_{1-z}N_{z})₁₋ₓ(Mn_{q}R_{1-q})ₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, ....0<z≦1, o≦q51).

Some nanoparticles such as ZnS:Tb³⁺, Er³⁺; ZnS:Tb³⁺; Y₂O₃:Tb3⁺; Y₂O₃:Tb³⁺, Er3⁺; ZnS:Mn²⁺; ZnS:Mn,Er³⁺ are known in the art to function for both down-conversion luminescence and upconversion luminescence and would be suitable for the invention with or without energy augmentators. In up-conversion embodiments, light emitting particles which emit red light may include praseodymium, light emitting particles which emit green light may include erbium, and light emitting particles which emit blue light may include thulium.

In general, the upconversion process generally requires one of more rare-earth dopants, such as Er, Eu, Yb, Tm, Nd, Tb, Ce, Y, U, Pr, La, Gd and other rare-earth species or a combination thereof, doped into a dielectric crystal (of any size >0.1nm), including at least one of Y₂O₃, Y₂O₂S, NaYF₄, NaYbF₄, YAG, YAP, Nd₂O₃, LaF₃, LaCl₃, La₂O₃, TiO₂, LuPO₄, YVO₄, YbF₃, YF₃, Na-doped YbF₃, or SiO₂, where incident radiation is at longer wavelength than emissive radiation from the crystal. The wavelength emitted in based entirely on the dopant ion(s) chosen and their associated and relative concentration in the host crystal. For the example of upconversion in a Y₂O₃ host crystal, to achieve a blue emission (~450 - 480 nm) one could synthesize [Y₂O₃; Yb (3%), Tm (0.2%)], where the Yb and Tm are the percentages doped in the crystal relative to the Y atoms being 100%. Likewise, typical green upconversion materials are [Y₂O₃; Yb (5%), Ho (1%)] and [Y₂O₃; Yb (2%), Er (1%)], and typical red upconversion materials are [Y₂O₃; Yb (10%), Er (1%)] and [Y₂O₃; Yb (5%), Eu (1%)]. The concentrations of dopants relative to each other and the crystal matrix must be tuned for every combination, and there are multiple ways to achieve multiple wavelength or energy emissions from even the same dopants.

Up-conversion of red light with a wavelength of about 650 nm in Tm³⁺ doped flourozirconate glasses can be used in the invention to produce blue light. In this system, the blue light consists of two emission bands; one at 450 nm which is ascribed to the 1D2→3H4 transition, the others at 475 nm is ascribed to the 1G4→3H6 transition. The emission intensities of both bands have been observed by others to vary quadratically with the excitation power. For glasses with a Tm³⁺ concentration of 0.2 mol% and greater, cross-relaxation processes occur which decrease the up-conversion efficiency.

The emission of visible light upon excitation in the near-infrared (NIR) has been observed in optically clear colloidal solutions of LuPO₄:Yb³⁺, Tm³⁺, and YbPO₄:Er³⁺ nanocrystals in chloroform. Excitation at 975 nm has been shown by others to produce visible emission in the blue, green, or red spectral regions.

Tellurium and germanium oxides (tellurites and germanates) are also suitable upconverters. These glasses can be doped with Tm, Yb, Ho, Er, Pr, for example.

Yb³⁺ doped BaZrO₃ is also suitable for upconversion. Er³⁺ and/or Tm³⁺ doping are also suitable for tailoring the emission wavelengths.

In another embodiment, Nd³⁺:Cs₂NaGdCl₆ and Nd³⁺, Yb³⁺:Cs₂NaCdCl₆ polycrystalline powder samples prepared by Morss method have been reported to be up converters and are suitable for the present invention. These materials, under 785 nm irradiation, have shown upconversion emissions near 538 nm (Green), 603 nm (Orange), and 675 nm (Red) were observed and assigned to 4G7/2→419/2, (4G7/2→4I11/2; 4G5/2→4I9/2), and (4G7/2→4I13/2; 4G5/2→4I11/2), respectively.

In another embodiment, Nd³⁺ and Ho³⁺ co-doped -based ZrF₄ fluoride glasses under 800 nm excitation have been reported to be up converters and are suitable for the present invention. Among the up-conversion luminescences for the ZrF₄ fluoride glasses, the green emission was seen to be extremely strong and the blue and red emission intensities were very weak.

In another embodiment, Tm³⁺/Yb³⁺ -codoped TeO₂-Ga₂O₃-R₂O (R=Li, Na, K) glasses have been reported to be up converters and are suitable for the present invention. These materials, under excitation at 977 nm, showed intense blue upconversion emission centered at 476 nm along with a weak red emission at 650 nm.

In another embodiment, metal-to-ligand charge transfer (MLCT) transition in [Ru(dmb)₃]²⁺ (dmb = 4,4'-dimethyl-2,2'-bipyridine) in the presence of anthracene or 9,10-diphenylanthracene have been reported to be up converters and are suitable for the present invention. Upconverted singlet fluorescence resulting from triplet-triplet annihilation at low excitation power has been reported. In particular 9,10-diphenylanthracene (DPA) (substituted for anthracene) showed higher efficiencies for upconversion. In these experiments, workers with this material system assumed that DPA's increased singlet fluorescence quantum yield ( = 0.95) relative to anthracene ( = 0.27)7. This work lead to an approximate 24.4 ± 6.1 enhancement of green-to-blue light upconversion permitting direct visualization of the process at low excitation power, for example by a commercial green laser pointer (*λ*ₑₓ= 532 nm, <5 mW peak power).

In certain embodiments, further energy converters include, but are not limited to, (not ranked by order of preference or utility):
CaF₂, ZnF₂, KMgF₃, ZnGa₂O₄, ZnAl₂O₄, Zn₂SiO₄, Zn₂GeO₄, Ca₃(PO₄)₃F, Sr₃(PO₄)₃F, CaSiO₃, MgSiO₃, ZnS, MgGa₂O₄, LaAl₁₁O₁₈, Zn₂SiO₄, Ca₅(PO₄)₃F, Mg₄Ta₂O₉, CaF₂, LiAl₅O₈, LiAlO₂, CaPO₃, AlF₃, and LuPO₄:Pr³⁺ Examples further include the alkali earth chalcogenide phosphors which are in turn exemplified by the following non-inclusive list: MgS:Eu³⁺, CaS:Mn²⁺, CaS:Cu, CaS:Sb, CaS:Ce³⁺, CaS:Eu²⁺, CaS:Eu²⁺Ce³⁺, CaS:Sm³⁺, CaS:Pb²⁺, CaO:Mn²⁺, CaO:Pb²⁺

Further examples include the ZnS type phosphors that encompass various derivatives: ZnS:Cu,Al(Cl), ZnS:Cl(Al), ZnS:Cu, I(Cl), ZnS:Cu, ZnS:Cu,In.

Also included are the compound IIIb-Vb phosphors which include the group IIIb and Vb elements of the periodic table. These semiconductors include BN, BP, BSb, AlN, AlP, AlAs, AlSb, GaN, GaP, GaAs, GaSb, InN, InP, InAs, InSb and these materials may include donors and acceptors that work together to induce light emission diodes. These donors include, but are not limited to, Li, Sn, Si, Li, Te, Se, S, O and acceptors include, but are not limited to, C, Be, Mg, Zn, Cd, Si, Ge. Further included are the major GaP light emitting diodes which include, but are not limited to, GaP:Zn,O, GaP:NN, Gap:N and GaP, which emit colors Red, Yellow, Green and Pure Green respectively.

The materials can further include such materials as GaAs with compositional variation of the following sort: In_{1-y}(Ga₁₋ₓAlₓ)_{y}P.

Also included is silicon carbide SiC, which has commercial relevancy as a luminescent platform in blue light emitting diodes. These include the polytypes 3C-SiC, 6H-SiC, 4H-SiC with donors such as N and Al and acceptors such as Ga and B.

Further examples include multiband luminescent materials include, but not limited to, the following compositions (Sr, Ca, Ba)₅(PO₄)₃Cl:Eu²⁺, BaMg₂Al]₆O₂₇:Eu²⁺, CeMgAl₁₁O₁₉:Ce^{3÷}:Tb³⁺, LaPO₄:Ce³⁺:Tb³⁺, GdMgB₅O₁₀:Ce₃:Tb³⁺, Y₂O₃:Eu³⁺, (Ba,Ca,Mg)₅(PO₄)₃Cl:Eu²⁺, 2SrO_{0.34}P2O50.16B2O3:Eu²⁺, Sr₄Al₁₄o₂₅:Eu²⁺.

Materials typically used for fluorescent high pressure mercury discharge lamps are also included. These can be excited with X-Ray and are exemplified by way of family designation as follows: Phosphates (Sr, M)(PO₄)₂:Sn²⁺, Mg or Zn activator, Germanate 4MgO.GeO₂:Mₙ⁴⁺, 4(MgO, MgF₂)GeO₂:Mn⁴⁺, Yttrate Y₂O₃:Eu³⁺, Vanadate YVO₄:Eu³⁺, Y(P,V)O₄:Eu³⁺, Y(P,V)O₄:In⁺, Halo-Silicate Sr₂Si₃O₈₂SrCl₂:Eu²⁺, Aluminate (Ba,Mg)₂Al₁₆O₂₄:Eu²⁺, (Ba, Mg)₂Al₁₆O₂₄:Eu²⁺,Mn²⁺, Y₂O₃Al₂O₃:Tb³⁺.

Another grouping by host compound includes chemical compositions in the halophosphates phosphors, phosphate phosphors, silicate phosphors, aluminate phosphors, borate phosphors, tungstate phosphors, and other phosphors. The halophosphates include, but are not limited to: 3Ca₃(PO₄)₂.Ca(F,Cl)₂:Sb³⁺, 3Ca₃(PO₄)₂.Ca(F,Cl)₂: Sb³⁺/Mn²⁺ Sr₁₀(PO₄)₆Cl₂:Eu²⁺, (Sr,Ca)₁₀(PO₄)ₛCl₂:Eu²⁺, (Sr,Ca)₁₀(PO₄)₆.nB₂O₃:Eu³⁺, (Sr, Ca,Mg)₁₀(PO₄)₆Cl₂:Eu²⁺. The phosphate phosphors include, but are not limited to: Sr₂P₂O₇:Sn²⁺, (Sr,Mg)₃(PO₄)₂:Sn²⁺, Ca₃(PO₄)₂.Sn²⁺, Ca₃(PO₄)₂:Tl⁺, (Ca,Zn)₃(PO₄)₂:Tl⁺, Sr₂P₂O₇:Eu²⁺, SrMgP₂O₇:Eu²⁺, Sr₃(PO₄)₂:Eu²⁺, LaPO₄:Ce³⁺, Tb³⁺, La₂O₃.0.2SiO₂.0.9P₂O₅:Ce³⁺.Tb³⁺, BaO.TiO₂.P₂O₅. The silicate phosphors Zn₂SiO₄:Mn²⁺ CaSiO₃:Pb²⁺/Mn²⁺, (Ba, Sr, Mg) 3Si₂O₇:Pb²⁺, BaSi₂O₅:Pb²⁺, Sr₂Si₃O_{8.}2SrCl₂:Eu²⁺, Ba₃MgSi₂O₈:Eu²⁺, (Sr,Ba)Al₂Si₂O₈:Eu²⁺.

The aluminate phosphors include, but are not limited to: LiAlO₂:Fe³⁺, BaAl₈O₁₃:Eu²⁺, BaMg₂Al₁₆O₂₇:Eu²⁺, BaMg₂Al₁₆O₂₇:Eu²⁺/Mn²⁺, Sr₄Al₁₄O₂₅:Eu²⁺, CeMgAl₁₁O₁₉:Ce³⁺/Tb³⁺.

The borate phosphors include: Cd₂B₂O₅:Mn²⁺, SrB₄O₇F:Eu²⁺, GdMgB₅O₁₀:Ce³⁺/Tb³⁺, GdMgB₅O₁₀:Ce³⁺/Mn³⁺, GdMgB₅O₁₀:Ce³⁺/Tb³⁺/Mn²⁺.

The tungstate phosphors include, but are not limited to: CaWO₄, (Ca,Pb)WO₄, MgWO₄. Other phosphors Y₂O₃:Eu³⁺, Y(V,P)O₄:Eu²⁺, YVO₄:Dy³⁺, MgGa₂O₄:Mn²⁺, 61MgO.As₂O₅:Mn²⁺, 3.5MgO.0.5MgF₂.GeO₂:Mn⁴⁺.
The activators to the various doped phosphors include, but are not limited to: Tl⁺, Pb²⁺, Ce³⁺, Eu²⁺, WO₄²-, Sn²⁺, Sb³⁺, Mn²⁺, Tb³⁺, Eu³⁺, Mn⁴⁺, Fe³⁺ The luminescence center Tl⁺ is used with a chemical composition such as: (Ca,Zn)₃(PO₄)₂:Tl⁺, Ca₃(PO₄)₂:Tl⁺. The luminescence center Mn²⁺ is used with chemical compositions such as MgGa₂O₄:Mn²⁺ BaMg₂Al₁₆O₂₇:Eu²⁺/Mn²⁺, Zn₂SiO₄:Mn²⁺, 3Ca₃(PO₄)₂.Ca(F,Cl)₂:Sb²⁺/Mn²⁺, CaSiO₃:Pb²⁺/Mn²⁺, Cd₂B₂O₅:Mn²⁺, CdB₂O₃:Mn²⁺, GdMgB₅O_{10L}:Ce³⁺/Mn²⁺, GdMgB₃O₁₀:Ce³⁺/Tb³⁺/Mn²⁺. The luminescence center Sn2+ is used with chemical compositions such as: Sr₂P₂O₇:Sn²⁺, (Sr,Mg)₃(PO₄)2:Sn²⁺. The luminescence center Eu²⁺ is used with chemical compositions such as: SrB₄O₇F:Eu²⁺, (Sr,Ba)Al₂Si₂O₈:Eu²⁺ Sr₃(PO₄)₂:Eu²⁺, Sr₂P₂O₇:Eu²⁺, Ba₃MgSi₂O₈:Eu²⁺, Sr₁₀(PO₄)₆Cl₂:Eu²⁺, BaMg₂Al₁₆O₂₇:Eu²⁺/Mn²⁺, (Sr,Ca)₁₀(PO₄)₆Cl₂:Eu²⁺. The luminescence center Pb²⁺ is used with chemical compositions such as: (Ba,Mg,Zn)₃Si₂O₇:Pb²⁺, BaSi₂O₅:Pb²⁺, (Ba,Sr)₃Si₂O₇:Pb²⁺.

The luminescence center Sb²⁺ is used with chemical compositions such as: 3Ca₃(PO₄)₂.Ca(F,Cl)₂:Sb³⁺, 3Ca₃(PO₄)₂.Ca(F,Cl)₂:Sb³⁺/Mn²⁺

The luminescence center Tb³⁺ is used with chemical compositions such as: CeMgAl₁₁O₁₉:Ce³⁺/Tb³⁺, LaPO₄:Ce³⁺/Tb³⁺, Y₂SiO₅:Ce³⁺/Tb³⁺, GdMgB₅O₁₀:Ce³⁺/Tb³⁺. The luminescence center Eu³⁺ is used with chemical compositions such as: Y₂O₃:Eu³⁺, Y(V,P)O₄:Eu³⁺. The luminescence center Dy³⁺ is used with chemical compositions such as: YVO₄:Dy³⁺ The luminescence center Fe³⁺ is used with chemical compositions such as: LiAlO₂:Fe³⁺. The luminescence center Mn⁴⁺ is used with chemical compositions such as: 6MgO.As₂O₅:Mn⁴⁺, 3.5MgO0.5MgF₂.GeO₂:Mn⁴⁺ The luminescence center Ce³⁺ is used with chemical compositions such as: Ca₂MgSi₂O₇:Ce³⁺ and Y₂SiO₃:Ce³⁺ The luminescence center WO₄²⁻ is used with chemical compositions such as: CaWO₄, (Ca,Pb)WO₄, MgWO₄. The luminescence center TiO₄⁴⁻ is used with chemical compositions such as: BaO.TiO_{2.}P₂O₅.

Additional phosphor chemistries of interest using X-Ray excitations include, but are not limited to, the k-edge of these phosphors. Low energy excitation can lead to intense luminescence in materials with low k-edge. Some of these chemistries and the corresponding k-edge are listed below:

| | |
|---|---|
| BaFCl:Eu²⁺ | 37.38 keV |
| BaSO₄:Eu²⁺ | 37.38 keV |
| CaWO₄ | 69.48 keV |
| Gd₂O₂S:Tb³⁺ | 50.22 keV |
| LaOBr:Tb³⁺ | 38.92 keV |
| LaOBr:Tm³⁺ | 38.92 keV |
| La₂O₂S:Tb³⁺ | 38.92 keV |
| Y₂O₂S:Tb³⁺ | 17.04 keV |
| YTaO₄ | 67.42 keV |
| YTaO₄:Nb | 67.42 keV |
| ZnS:Ag | 9.66 keV |
| (Zn,Cd)S:Ag | 9.66/26.7 keV |

These materials can be used alone or in combinations of two or more. A variety of compositions can be prepared to obtain the desired output wavelength or spectrum of wavelengths.

In the present invention, the phosphor selection could be chosen such that under x-ray or other high energy source irradiation, the light emitted from the phosphors could, for example, have exemplary characteristics including:
Emissions in 190 -250 nm wavelength range;
Emissions in the 330-340 nm wavelength range.

### Mechanoluminescent materials (organic and inorganic):

Mechano-luminescent materials can be used as energy converters

Mechano-luminescent energy converters are not a part of the invention and are disclosed for illustrative purposes only.

Mechano-luminescent materials convert ultrasonic or mechanical energy (such as vibrations naturally existing on an article such as motor or vibrations from driven by transducers) into visible light. Here, for example, the mechano-luminescent materials would be placed in a vicinity (e.g., between or around or inside) the folded structures or the external-electrode pairs.

In one embodiment, an electromagnetic wave energy augmentator captures one or more wavelengths of electromagnetic energy, and augments the one or more wavelengths of electromagnetic energy in at least one property (such as electric field intensity in a vicinity of the mechano-luminescent materials), while at the same time the mechano-luminescent materials can be considered an energy converter converting the ultrasonic or mechanical energy into electromagnetic radiation (i.e., emitted light).

In one embodiment the increased electric field in the folded structure or the external electrode pair increases the luminescence of the mechano-luminescent materials. The energy used to build the electric field in the folded structure or the external electrode pair being provided separately from the mechanical energy driving the mechano-luminescence.

Various mechano-luminescent materials suitable for the present invention with or without energy augmentators include ZnS:Mn²⁺, SrAl₂O₄:Eu²⁺, ZnS:Cu, SrAMgSi₂O₇:Eu²⁺ (A = Ca, Sr, Ba), KCl, KI, KBr, NaF, NaCl, LiF, RbCl, RbBr, RbI, MgO, SrAl₂O₄, CaAl₂O₄, Sr₁₋ₓBaₓAl₂O₄ (x = 0, 0.1, 0.2, 0.4), Sr_{0.9}Ca_{0.1}Al₂O₄, Zn₂Ge_{0.9}Si_{0.1}O₄, MgGa₂O₄, ZnGa₂O₄, ZnAl₂O₄, ZnS, ZnTe, (ZnS)₁₋ₓ(MnTe)ₓ(x < ¼), CaZnOS, BaZnOS, Ca₂MgSi₂O₇, Sr₂MgSi₂O₇, Ba₂MgSi₂O₇, SrCaMgSi₂O₇, SrBaMgSi₂O₇, SrₙMgSi₂O₅₊ₙ(1 ≤ n ≤ 2) , Ca₂Al₂SiO₇, Sr₂Al₂SiO₇, CaYAl₃O₇, CaAl₂Si₂O₈, Ca₁₋ₓSrₓAl₂Si₂O₈(x < 0.8) , SrMg₂(PO₄)₂, Ba₁₋ₓCaₓTiO₃ (0.25 < x < 0.8) , Ba₁₋ₓCaₓTiO₃, LiNbO₃, Sr₂SnO₄, (Ca, Sr, Ba)₂SnO₄, Sr₃Sn₂O₇, Sr₃(Sn, Si)₂O₇, Sr₃(Sn, Ge)₂O₇, Ca₃Ti₂O₇, CaNb₂O₆, Ca₂Nb₂O₇, Ca₃Nb₂O₈, BaSi₂O₂N₂, SrSi₂O2N₂, CaZr(PO₄)₂, ZrO₂.

In one embodiment, a europium-holmium co-doped strontium aluminate can be used as a mechano-luminescent material (i.e., an energy converter) alone or in conjunction with the energy augmentators. The europium-holmium co-doped strontium aluminate and the other mechano-luminescent materials convert sonic or acoustic energy into photon emissions which may or may not be placed in a vicinity of the energy augmentators.

Yanim Jia, in "Novel Mechano-Luminescent Sensors Based on Piezoelectric/Electroluminescent Composites," Sensors (Basel). 2011; 11(4): 3962-396, describes a mechanoluminescent composite made of a piezoelectric material and an electroluminescent material. In this composite device, when a stress is applied to the piezoelectric layer, electrical charges will be induced at both the top and bottom faces of piezoelectric layer due to the piezoelectric effect. These induced electrical charges will result in a light output from the electroluminescent layer due to the electroluminescent effect.

Here, in one embodiment with or without energy augmentators, such composites made of a piezoelectric material and an electroluminescent material, hereinafter "composite mechano-luminescent emitters," provides a structure that, upon stimulation with mechanical or vibrational energy such as from an acoustic or ultrasonic transducer, emit light.

Electroluminescent and phosphorescent materials (organic and inorganic): The present disclosure in various embodiments can utilize organic fluorescent molecules or inorganic particles capable or fluorescence and phosphorescence having crystalline, polycrystalline or amorphous micro-structures for the converters (optionally including the energy augmentation structures described above). Such molecules or particles are not a part of the invention.

The list of inorganic molecules that can be used with or without energy augmentators for the electroluminescence and phosphorescent materials described below include but is not limited to the following inorganic electroluminescent phosphor materials:

SrS:Ce ³⁺

CaGa₂S₄:Ce³⁺

SrS:Cu⁺

CaS:Pb²⁺

BaAl₂S₄:EU²⁺

ZnS:Tb³⁺

ZnMgS:Mn²⁺

SrGa₂S₄:Eu²⁺

CaAl₂S₄:Eu²⁺

BaAl₂S₄:Eu²⁺

ZnS:Mn²⁺

MgGa₂O₄:Eu³⁺

(Ca, Sr)Y₂S₄:Eu²⁺

BaAl₂S₄:Eu²⁺

Organic molecules that can phosphoresce under the influence of an electric field are also of interest in the present application. The organic fluorescent compounds with high quantum yield include by way of illustration:
Naphthalene,
Pyrene,
Perylene,
Anthracene,
Phenanthrene,
p-Terphenyl,
p-Quartphenyl,
Trans-stilbene,
Tetraphenylbutadiene,
Distyrylbenzene,
2,5-Diphenyloxazole,
4-Methyl-7-diethylaminocoumarin,
2-Phenyl-5-(4-biphenyl)-1,3,4-oxadiazole,
3-Phenylcarbostyryl,
1,3,5-Triphenyl-2-pyrazoline,
1,8-Naphthoylene -1', 2'-bezimidazole,
4-Amino-N-phenyl-naphthalimide.

The inorganic fluorescent and phosphorescent materials detailed here are numerous, and various examples are given by way of illustration rather than limitation and can be used with or without energy augmentators. Furthermore, these materials can be doped with specific ions (activators or a combination of activators) that occupy a site in the lattice structure in the case of crystalline or polycrystalline materials and could occupy a network forming site or a bridging and/or non-bridging site in amorphous materials. These compounds could include (not ranked by order of preference or utility) the following material examples:
CaF₂, ZnF₂, KMgF₃, ZnGa₂O₄, ZnAl₂O₄, Zn₂SiO₄, Zn₂GeO₄, Ca₅(PO₄)₃F, Sr₅(PO₄)₃F, CaSiO₃, MgSiO₃, ZnS, MgGa₂O₄, LaAl₁₁O₁₈, Zn₂SiO₄, Ca₅(PO₄)₃F, Mg₄Ta₂O₉, CaF₂, LiAl₅O₈, LiAlO₂, CaPO₃, AlF₃.

Further included are alkali earth chalcogenide phosphors which are in turn exemplified by the following non-inclusive list:
MgS:Eu³⁺, CaS:Mn²⁺, CaS:Cu, CaS:Sb, CaS:Ce³⁺, CaS:Eu²⁺, CaS: Eu²⁺ Ce³⁺, CaS: Sm³⁺, CaS:Pb²⁺, CaO:Mn²⁺, CaO:Pb²⁺.

The examples include the ZnS type phosphors that encompass various derivatives:
ZnS:Cu,Al(Cl), ZnS:Cl(Al), ZnS:Cu,I(Cl), ZnS:Cu, ZnS:Cu,In.

Compound IIIb-Vb phosphors which include the group IIIb and Vb elements of the periodic table are suitable for converter materials. These semiconductors include BN, BP, BSb, AlN, AlP, AlAs, AlSb, GaN, GaP, GaAs, GaSb, InN, InP, InAs, InSb and these materials have donors and acceptors that work in together to induce light emission diodes. The donors include Li, Sn, Si, Li, Te, Se, S, O, and acceptors include C, Be, Mg, Zn, Cd, Si, Ge. As an example, GaP light emitting diodes include GaP:Zn, O, GaP:NN, Gap:N and GaP which emit colors Red, Yellow, Green and Pure Green respectively.

The compounded materials further include such materials as GaAs with compositional variation of the following sort: In1-y(Ga1-xAlx)yP (provides a simple example).

Silicon Carbide SiC as a luminescent platform has commercial relevancy if the blue light emitting diodes. These include the polytypes 3C-SiC, 6H-SiC, 4H-SiC with donors such as N and Al and acceptors such as Ga and B.

Multiband luminescent materials suitable for converter materials include for example the following compositions:
(Sr, Ca, Ba)₅(PO₄)₃Cl:Eu²⁺, BaMg₂Al₁₆O₂₇:Eu²⁺, CeMgAl₁₁O₁₉:Ce³⁺:Tb³⁺, LaPO₄:Ce³⁺:³⁺, GdMgB₅O₁₀:Ce³⁺:Tb³⁺, Y₂O₃:Eu³⁺, (Ba,Ca,Mg)₅(PO₄)₃Cl:Eu²⁺, 2SrO_{0.84}P₂O₅-0.16B₂O₃:Eu²⁺, Sr₄Al₁₄O₂₅:Eu²⁺.

Other materials suitable for converter materials include those materials used for fluorescent high pressure mercury discharge lamps can be excited with X-Ray and are exemplified by way of family designation as follows:
Phosphates (Sr, M)(PO₄)₂:Sn²⁺, Mg or Zn activator, Germanate 4MgO.GeO₂:Mn⁴⁺, 4(MgO, MgF₂)GeO₂:Mn⁴⁺, Yttrate Y₂O₃:Eu³⁺, Vanadate YVO₄:Eu³⁺, Y(P,V)O₄:Eu³⁺, Y(P,V)O₄:In⁺, Halo-Silicate Sr2Si3O₈·2SrCl₂:Eu²⁺, Aluminate (Ba,Mg)₂Al₁₆O₂₄:Eu²⁺, (Ba, Mg)₂Al₁₆O₂₄:Eu²⁺,Mn²⁺, Y₂O₃Al₂O₃:Tb³⁺.

Another grouping of materials suitable for converter materials by host compound include chemical compositions in the Halophosphates phosphors, Phosphate phosphors, Silicate phosphors, Aluminate phosphors, Borate phosphors, Tungstate phosphors, and other phosphors.

The halophosphates include by way of illustration:
3Ca₃(PO₄)₂.Ca(F,Cl)₂:Sb³⁺, 3Ca₃(PO₄)₂.Ca(F,Cl)₂:Sb³⁺/Mn²⁺, Sr₁₀(PO₄)₆Cl₂:Eu²⁺, (Sr,Ca)₁₀(PO₄)₆Cl₂:Eu²⁺, (Sr,Ca)₁₀(PO₄)₆.nB₂O₃:Eu³⁺, (Sr, Ca,Mg)₁₀(PO₄)₆Cl₂:Eu²⁺. The phosphate phosphors include by way of illustration Sr₂P₂O₇:Sn²⁺, (Sr,Mg)₃(PO₄)₂:Sn²⁺, Ca₃(PO₄)₂.Sn²⁺, Ca₃(PO₄)₂:Tl⁺, (Ca,Zn)₃(PO₄)₂:Tl⁺, Sr₂P₂O₇:Eu²⁺, SrMgP₂O₇:Eu²⁺, Sr₃(PO₄)₂:Eu²⁺, LaPO₄:Ce³⁺, Tb³⁺, La₂O₃·0.2SiO₂·0.9P₂O₅:Ce³⁺.Tb³⁺, BaO·TiO₂·P₂O₅. The silicate phosphors Zn₂SiO₄:Mn²⁺, CaSiO₃:Pb²⁺/Mn²⁺, (Ba, Sr, Mg)·3Si₂O₇:Pb²⁺, BaS₂O₅:Pb²⁺, Sr₂Si₃O₈·2SrCl₂:Eu²⁺, Ba₃MgSi₂O₈:Eu²⁺, (Sr,Ba)Al₂Si₂O₈:Eu²⁺.

The aluminate phosphors include:
LiAlO₂:Fe³⁺, BaAl₈O₁₃:Eu²⁺, BaMg₂Al₁₆O₂₇:Eu²⁺, BaMg₂Al_{1c}O₂₇:Eu²⁺/Mn²⁺, Sr₄Al₁₄O₂₅:Eu²⁺, CeMgAl₁₁O₁₉:Ce³⁺/Tb³+.

The borate phosphors include:
Cd₂B₂O₅:Mn²⁺, SrB₄O₇F:Eu²⁺, GdMgB₅O₁₀:Ce³⁺/Tb³⁺, GdMgB₅O₁₀:Ce³⁺/Mn³⁺, GdMgB₅O₁₀:Ce³⁺/Tb³⁺/Mn²⁺.

The tungstate phosphors include:
CaWO₄, (Ca,Pb)WO₄, MgWO₄. Other phosphors Y₂O₃:Eu³⁺, Y(V,P)O₄:Eu²⁺ YVO₄:Dy³⁺, MgGa₂O₄:Mn²⁺, 6MgO·As₂O₅:Mn²⁺ 3.5MgO·0.5MgF₂·GeO₂:Mn⁴⁺.

Activators of relevance to the various doped phosphors include the following list:
Tl⁺, Pb²⁺, Ce³⁺, Eu²⁺, WO₄²⁻, Sn²⁺, Sb³⁺, Mn²⁺, Tb³⁺, Eu³⁺, Mn⁴⁺, Fe³⁺.

In various embodiments, the luminescence center Tl+ can be used with a chemical composition such as:
(Ca,Zn)₃(PO₄)₂:Tl⁺, Ca₃(PO₄)₂:Tl⁺.

Similarly, the luminescence center Mn2+ can be used with chemical compositions such as
MgGa₂O₄:Mn²⁺, BaMg₂Al₁₆O₂₇:Eu²⁺/Mn²⁺, Zn₂SiO₄:Mn²⁺, 3Ca₃(PO₄)₂·Ca(F,Cl)₂:Sb²⁺/Mn²⁺, CaSiO₃:Pb²⁺/Mn²⁺, Cd₂B₂O₅:Mn²⁺, CdB₂O₅:Mn²⁺, GdMgB₅O₁₀:Ce³⁺/Mn²⁺, GdMgB₃O₁₀:Ce³⁺/Tb³⁺/Mn²⁺.

Further, the luminescence center Sn²⁺ can be used with chemical compositions such as:
Sr₂P₂O₇:Sn²⁺, (Sr,Mg)₃(PO₄)₂:Sn²⁺.

The luminescence center Eu²⁺ can also be used with chemical compositions such as:
SrB₄O₇F:Eu²⁺, (Sr,Ba)Al₂Si₂O₈:Eu²⁺, Sr₃(PO₄)₂:Eu²⁺, Sr₂P₂O₇:Eu²⁺, Ba₃MgSi₂O₈:Eu²⁺, Sr₁₀(PO₄)₆Cl₂:Eu²⁺, BaMg₂Al₁₆O₂₇:Eu²⁺/Mn²⁺, (Sr,Ca)₁₀(PO₄)₆Cl₂:Eu²⁺.

The luminescence center Pb²⁺ can be used with chemical compositions such as:
(Ba,Mg,Zn)₃Si₂O₇:Pb²⁺, BaSi₂O₅:Pb²⁺, (Ba,Sr)₃Si₂O₇:Pb²⁺.

The luminescence center Sb²⁺ can be used with chemical compositions such as:
3Ca₃(PO₄)₂·Ca(F,Cl)₂:Sb³⁺, 3Ca₃(PO₄)₂·Ca(F,Cl)₂:Sb³⁺/Mn²⁺.

The luminescence center Tb3+ can be used with chemical compositions such as:
CeMgAl₁₁O₁₉:Ce³⁺/Tb³⁺, LaPO₄:Ce³⁺/Tb³⁺, Y₂SiO₅:Ce³⁺/Tb³⁺, GdMgB₅O₁₀:Ce³⁺/Tb³⁺.

The luminescence center Eu³⁺ can be used with chemical compositions such as:

Y₂O₃:Eu³⁺, Y(V,P)O₄:Eu³⁺.

The luminescence center Dy³⁺ can be used with chemical compositions such as:

YVO₄:Dy³⁺.

The luminescence center Fe³⁺ can be used with chemical compositions such as:

LiAlO₂:Fe³⁺.

The luminescence center Mn⁴⁺ can be used with chemical compositions such as:

6MgO·As₂O₅:Mn⁴⁺, 3.5MgO·0.5MgF₂·GeO₂:Mn⁴⁺.

The luminescence center Ce³⁺ can be used with chemical compositions such as:

Ca₂MgSi₂O₇:Ce³⁺ and Y₂SiO₅:Ce³⁺.

The luminescence center WO₄²⁻ can be used with chemical compositions such as:

CaWO₄, (Ca,Pb)WO₄, MgWO₄.

The luminescence center TiO₄⁴⁻ can be used with chemical compositions such as:

BaO·TiO₂·P₂O₅.

In various embodiments the phosphor chemistry utilized in x-ray excitations can be used with or without energy augmentators. Of particular interest is the k-edge of these phosphors. Low energy excitation can lead to intense luminescence in materials

with low k-edge. Some of these chemistries and the corresponding k-edge are included as follows:

| | |
|---|---|
| BaFCl:Eu²⁺ | 37.38 keV |
| BaSO₄:Eu²⁺ | 37.38 keV |
| CaWO₄ | 69.48 keV |
| Gd₂O₂S:Tb³⁺ | 50.22 keV |
| LaOBr:Tb³⁺ | 38.92 keV |
| LaOBr:Tm³⁺ | 38.92 keV |
| La₂O₂S:Tb³⁺ | 38.92 keV |
| Y₂O₂S:Tb³⁺ | 17.04 keV |
| YTaO₄ | 67.42 keV |
| YTaO₄:Nb | 67.42 keV |
| ZnS:Ag | 9.66 keV |
| (Zn,Cd)S:Ag | 9.66/26.7 keV |

In one embodiment light from these materials (excited for example by high energy particles including x-rays, gamma rays, protons, and electrons) can have their emissions modulated by having those materials included in a vicinity of (including inside) the color enhancing structures described herein. For example, in medical treatments where x-ray excites phosphorescence to photostimulate reactions in a patient, simultaneous with irradiation by the high energy particles, there could be applied infrared irradiation to drive resonance in the energy augmentation structures described herein, where the x-ray phosphors would have enhanced light emissions when in the presence of the intensified electric fields. In another example, in medical or scientific instruments, for simultaneous with irradiation by the high energy particles, there could be applied electric fields to enhance emissions from these x-ray phosphors.

**Electro Luminescent Materials:** Various materials used for the electroluminescence with or without energy augmentators can include but are not limited to:
4,4',4"-Tris[phenyl(m-tolyl)amino]triphenylamine (m-MTDATA) N,N'-Bis(3-methylphenyl)-N,N'-diphenylbenzidine (TPD)
4,4',4"-Tris[phenyl(m-tolyl)amino]triphenylamine (m-MTDATA) N,N'-Bis(3-methylphenyl)-N,N'-diphenylbenzidine (TPD)
Tris-(8-hydroxyquinoline)aluminum
2,4,6-Tris(2-pyridyl)-s-triazine (TPT) 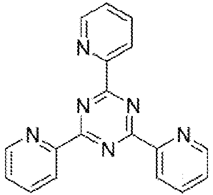
2,2',2"-(1,3,5-Benzinetriyl)-tris(1-phenyl-1-H-benzimidazole) Alq 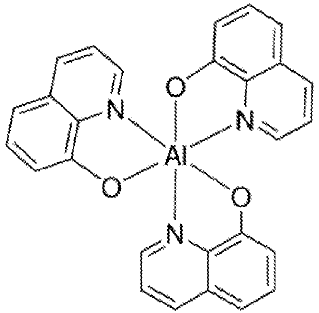
2,2',2"-(1,3,5-Benzinetriyl)-tris(1-phenyl-1-H-benzimidazole) TPBI 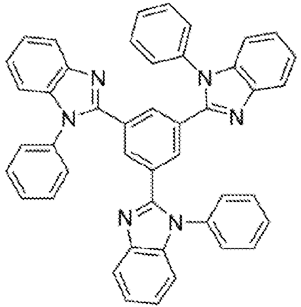
2,9-Dimethyl-4,7-diphenyl-1,10-phenanthroline, BCP2,9-Dimethyl-4,7-diphenyl-1,10-phenanthroline, BCP

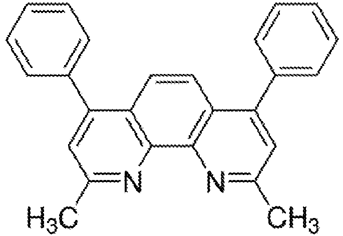

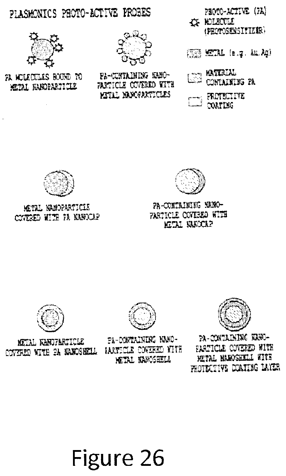

**Plasmonic enhancement structures:** Figure 26 is a schematic of a depiction of an upconverter or a down converter material (i.e., a photoactive material) according to one embodiment of the invention to be utilized in the color enhancement/augmentation structures noted herein with or without energy augmentators. Figure 26 shows a number of structural configurations for placement of a dielectric core upconverter or a down converter material (which is of a nanometer sized scale) in proximity to a metal shell. Incident light at a wavelength λ₁ interacts with the upconverting dielectric core. The interaction of light λ₁ with the dielectric core produces a secondary emission at a frequency λ₂ which has a shorter wavelength than λ₁ and accordingly has a higher energy than λ₁. While the exact physical mechanisms for the upconversion may depend on the particular upconversion material and process being used in a particular application, for the purposes for discussion and illustration, the following explanation is offered.

In the context of Figure 26, when a wavelength λ₁ interacts with a dielectric material core, three separate processes are well understood for the upconversion process involving trivalent rare earth ions. These three processes are:
1) excited state absorption whereby two photons are absorbed sequentially by the same ion to excite and populate one or more states;
2) energy transfer upconversion which is a transfer of excitation from one ion to another already in an excited state; and
3) a cooperative process of multiphotons where two nearby ions in excited states are emitting collectively from a virtual state.

Regardless of which one of these processes is occurring between the chosen ion(s) and the host lattice, the end result is a photon of energy greater than the excitation energy being emitted from the host lattice for the upconversion or down conversion process.

Therefore, the particular ion being activated (whether it be a dopant ion or a host ion of a lattice such as in the neodymium oxide) will be chosen based on the host material being processed, in order that the dopant ion or the host ion in the dielectric core provide ion states which are pumpable by a NIR source to generate the resultant emission λ₂.

Hence, the invention in one embodiment provides an upconversion or a down conversion material configured, upon exposure to a first wavelength λ₁ of radiation, to generate a second wavelength λ₂ of radiation having an energy higher or lower than the first wavelength λ₁. The system can include a metallic structure disposed in relation to the nanoparticle (e.g. a metallic shell covering a fraction of the nanoparticle). The system may include a receptor disposed in the medium in proximity to the nanoparticle. The receptor upon activation by the second wavelength λ₂ may itself fluoresce producing visible light. In one embodiment of the invention, a physical characteristic of metallic structure (such as those described above and below in the drawings) is set to a value where a surface plasmon resonance in the metallic structure resonates at a frequency which provides spectral overlap with either the first wavelength λ₁ or the second wavelength λ₂. This system with a metallic structure disposed in relation to an up-conversion or a down-conversion nanoparticle becomes the converter utilized in the color enhancement/augmentation structures noted herein.

Within the context of the invention, the term "physical characteristic" of the metallic shell or core can relate to any characteristic of the metal itself or the shell or core dimensions or shape which affects the surface plasmon resonance frequency. Such physical characteristics can include, but are not limited to, a conductivity, a radial dimension, a chemical composition or a crystalline state of the metal shell or core.

In various embodiments, the metallic structures can be a metallic shell encapsulating at least a fraction of the nanoparticle in the metallic shell wherein a conductivity, a radial dimension, or a crystalline state of the metallic shell sets the surface plasmon resonance in the metallic structure to resonate at a frequency which provides spectral overlap with either the first wavelength λ₁ or the second wavelength λ₂. In various embodiments, the metallic structures can be a multi-layer metallic shell encapsulating at least a fraction of the nanoparticle in the metallic shell wherein a conductivity, a radial dimension, or a crystalline state of the metallic shell sets the surface plasmon resonance in the metallic structure to resonate at the first wavelength λ₁ and the second wavelength λ₂. This capability permits radiation at λ₁ and λ₂ to be amplified.

In various embodiments, the metallic structures can be a metallic particle existing in one or more multiple structures. These multiple structures can have a variety of shapes including for example sphere, spheroid, rod, cube, triangle, pyramid, pillar, crescent, tetrahedral shape, star or combination thereof disposed adjacent the nanoparticle wherein a conductivity, a dimension (e.g. a lateral dimension or a thickness), or a crystalline state of the metallic structure sets the surface plasmon resonance in the metallic particle or rod to resonate at a frequency which provides spectral overlap with either the first wavelength λ₁ or the second wavelength λ₂. Such shapes are described in the present figures and in the figures in U.S. Serial No. 12/401,478. The shape choice can affect the frequency of the surface plasmon resonance. It is known that the plasmon band is changed by the shape of nanoparticles (e.g., prolate and obloid spheroids). The paper "Spectral bounds on plasmon resonances for Ag and Au prolate and oblate nanospheroids," in the Journal of Nanophotonics, Vol. 2, 029501 (26 September 2008), shows plasmon resonance shifts for shaping of Ag and plasmon resonance shifts for shaping of Au of prolate and obloid spheroids. In one embodiment of the invention, with an increasing aspect ratio for a metallic structure of the invention, the prolate spheroid resonance is red shifted relative to a sphere with no lower limit (under the assumptions of a Drude dispersion model). On the other hand, the oblate resonances are "blue shifted" as the spheroid becomes increasingly flat, but up to a limit.

In various embodiments, the metallic structures disposed in relation to an up-conversion or a down-conversion nanoparticle can be a metallic structure disposed interior to the nanoparticle wherein a conductivity or a dimension (e.g. a lateral dimension or a thickness) of the metallic structure sets the surface plasmon resonance in the metallic structure to resonate at a frequency which provides spectral overlap with either the first wavelength λ₁ or the second wavelength λ₂. In various embodiments, the metallic structures can be a metallic multi-layer structure disposed interior to the nanoparticle wherein a conductivity or a dimension (e.g. a lateral dimension or a thickness) of the metallic structure sets the surface plasmon resonance in the metallic structure to resonate at the first wavelength λ₁ and the second wavelength λ₂. This capability once again permits radiation at λ₁ and λ₂ to be amplified.

In another embodiment, the invention provides a nanoparticle structure including a sub 1000 nm dielectric core and a metallic structure disposed in relation to the nanoparticle. The dielectric core includes at least one of Y₂O₃, Y₂O₂S, NaYF₄, NaYbF₄, YAG, YAP, Nd₂O₃, LaF₃, LaCl₃, La₂O₃, TiO₂, LuPO₄, YVO₄, YbF₃, YF₃, Na-doped YbF₃, or SiO₂. Such nanoparticle structures can exhibit in certain embodiments surface plasmon resonance in the metallic structures to enhance upconversion of light from a first wavelength λ₁ to a second wavelength λ₂.

As described above, a shell (or other structure) is in particular designed with a layer thickness (or for example a lateral dimension) to enhance the photon upconversion process through plasmonic enhancement. The thickness of the shell (or other physical characteristic) is "tuned" in its thickness to the absorption process by having a dimension in which plasmons (i.e., electrons oscillations) in shell have a resonance in frequency which provides spectral overlap with the absorption band targeted. Thus, if the upconversion is to be stimulated by 980 nm NIR light, then the thickness of the shell is "tuned" in a thickness to where a plasmon resonance resonates at a frequency also of 980 nm (or in the neighborhood thereof as plasmon resonances are typically broad at these wavelengths).

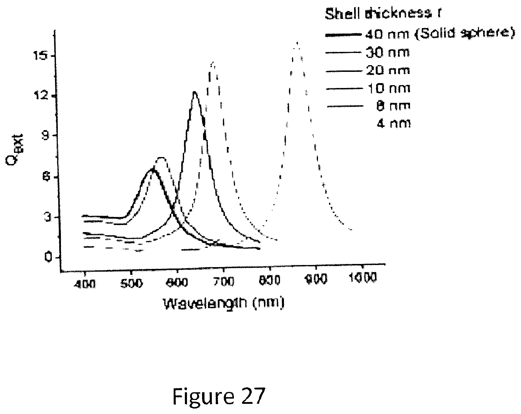

A plasmon resonating shell can be made of numerous transition metals, including though not limited to gold, silver, platinum, palladium, nickel, ruthenium, rhenium, copper, and cobalt or a combination or alloys or layers thereof. Such a plasmon resonating shell can be also made of a combination of metals and non-metals. When formed of a gold nanoshell, the recommended thickness to resonate with 980 nm light is approximately 3.5 nm surrounding an 80 nm upconverting core, as projected by extended Mie theory calculations. (See Jain et al., Nanolett. 2007, 7(9), 2854. Figure 27 is reproduced from Jain *et al* and illustrates the capability in the invention to "tune" the metal shell to have a spectral overlap with the excitation and/or emission radiation wavelengths.

In one embodiment of the invention, the metallic structures disposed in relation to an up-conversion or a down-conversion nanoparticle can be an alloy such as for example a Au:Ag alloy. The alloy content can be set to adjust the frequency of the surface plasmon resonance. In one embodiment of the invention, the metallic structures can be an alloy such as for example a Pt:Ag alloy. The alloy content can be set to adjust the frequency of the surface plasmon resonance. In one embodiment of the invention, the metallic structures can be an alloy such as for example a Pt:Au alloy. The alloy content can be set to adjust the frequency of the surface plasmon resonance.

In one embodiment of the invention, the converter nanoparticle can be an alloy of two or more materials. In this embodiment, the alloy can have a composition between the two or more materials which is set to a compositional value where excitation of the alloy at first wavelength λ₁ produces emission at the second wavelength λ₂. In one embodiment of the invention, the nanoparticle can be a zinc sulfide and zinc selenide alloy. In one embodiment of the invention, the nanoparticle can be a zinc sulfide and cadmium sulfide alloy.

In one embodiment of the invention, the zinc sulfide and zinc selenide nanoparticle alloy can have an alloy content set to provide a predetermined surface plasmon resonance. In one embodiment of the invention, the zinc sulfide and cadmium sulfide nanoparticle alloy can have an alloy content is set to provide a predetermined surface plasmon resonance.

Some techniques for producing nanoparticles and nanoparticle alloys which are suitable for the invention are described in the following documents: U.S. Pat. Nos. 7,645,318; 7,615,169; 7,468,146; 7,501,092; U.S. Pat. Appl. Publ. No. 2009/0315446; 2008/0277270; 2008/0277267; 2008/0277268; and WO 2009/133138.

In one embodiment of the invention, the thickness of the metal shell disposed in relation to an up-conversion or a down-conversion nanoparticle is set depending on the absorption frequency (or in some cases the emission frequency) of the particular dopant ions in the dielectric core to enhance the total efficiency of the emission process of the upconverted light. Accordingly, the thickness of the shell can be considered as a tool that in one instance enhances the absorption of λ₁, and in another instance can be considered as a tool that enhances the emission of λ₂, or in other situations can be considered an enhancement feature that in combination enhances the overall net process.

Additionally, plasmon-phonon coupling may be used to reduce a resonance frequency through the tuning of the bands to a degree off resonance. This may be useful in optimizing resonance energy transfer processes for the purpose of shifting the outputted color to a color desirable for a painted, colored, or displayed surface. In one example, Figure 27 shows an example of the plasmon resonance shift as a function of shell thickness.

Here, in one embodiment of the invention, the capability to produce stimulated emission at a targeted wavelength or color or energy is complemented by the ability to design nanoparticles that have designed absorption bands. Such absorption materials could for example further serve to improve the monochromaticity of light observed from a paint, ink, dye, or otherwise reflecting surface treated with the color enhancing compositions of the invention.

Details of the preparation of this nanoparticle system are included in U.S. Serial No. 12/725,108. The absorption spectrum of Y₂O₃ alone (lower trace) is fairly featureless, showing absorption due to the tri-arginine near 200 nm and a gentle slope associated with scattering and absorption by the Y₂O₃ nanoparticles extending into the visible portion of the spectrum. The gold-coated Y₂O₃ (upper trace), on the other hand, exhibit a strong absorption band at 546 nm, which is characteristic of the plasmonics resonance band due to the gold shell around the Y₂O₃ cores. The red-shifting of the plasmon absorption to 546 nm is consistent with the presence of a gold shell around a dielectric core.

In one embodiment of the invention, the converter materials for the upconverter dielectric core can include a wide variety of dielectric materials, as described above. In various embodiments of the invention, the upconverter dielectric core includes more specifically lanthanide doped oxide materials. Lanthanides include lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), and lutetium (Lu). Other suitable dielectric core materials include non-lanthanide elements such as yttrium (Y) and scandium (Sc). Hence. suitable dielectric core materials include Y₂O₃, Y₂O₂S, NaYF₄, NaYbF₄, Na-doped YbF₃, YAG, YAP, Nd₂O₃, LaF₃, LaCl₃, La₂O₃, TiO₂, LuPO₄, YVO₄, YbF₃, YF₃, or SiO₂. These dielectric cores can be doped with Er, Eu, Yb, Tm, Nd, Tb, Ce, Y, U, Pr, La, Gd and other rare-earth species or a combination thereof.

Lanthanides usually exist as trivalent cations, in which case their electronic configuration is (Xe) 4fⁿ, with n varying from 1 (Ce³⁺) to 14 (Lu³⁺). The transitions within the f-manifold are responsible for many of the photo-physical properties of the lanthanide ions, such as long-lived luminescence and sharp absorption and emission lines. The f-electrons are shielded from external perturbations by filled 5s and 5p orbitals, thus giving rise to line-like spectra. The f-f electronic transitions are LaPorte forbidden, leading to long excited state lifetimes, in the micro- to millisecond range.

Accordingly, examples of doped materials in the invention include oxides such as yttrium oxide and neodymium oxide and aluminum oxide as well as sodium yttrium fluoride and nanocrystalline perovskites and garnets such as yttrium aluminum garnet (YAG) and yttrium aluminum perovskite (YAP). Of these materials, doping is required for some, but not all of these materials, for promoting upconversion efficiencies. In various embodiments of the invention, the host nanocrystals are doped with trivalent rare earth lanthanide ions from those lanthanide series elements given above.

More specifically, in various embodiments of the invention, pairs of these dopants are introduced in order to make accessible more energy states in the host crystal. The activation and pumping of these energy states follows closely the principles discussed above. Doping concentrations in the invention can range from 0.2% to 20% roughly per ion into the host lattice or in a weight or mol% variation. The efficiency of the upconversion processes of specific bands in these materials can be modulated by the percentages doped to induce and enhance targeted emissions. Lanthanide doped upconverters while not limited to, can use the following mol percent dopant compositions: 5% Er, 10% Yb, 0.2% Tm + 3% Yb, and 1% Er + 10% Yb.

The size of the nanocrystal will also have an effect on the efficiency of the upconversion process, as a larger nanocrystal will have more sites for dopant ions to be accommodated into the host lattice, therefore enabling more emissions from the same doped host than if the nanocrystal were smaller. While the dopant percentages listed above are not rigidly fixed, these numbers provide a rudimentary teaching of the typical percentages one would use in obtaining a particular dielectric core material of the invention.

Moreover, some of these host crystals (e.g., neodymium oxide) in one embodiment of the invention may require no specific doping to facilitate upconversion, which has been seen in one instance in Nd₂O₃ with an excitation wavelength of 587 nm producing emissions at 372 nm, 402 nm, and 468 nm. See Que, W et al. Journal of Applied Physics 2001, vol 90, pg. 4865. Doping neodymium oxide with Yb³⁺, in one embodiment of the invention, would enhance upconversion through sensitizing the Nd³⁺ ions with a lower energy Yb³⁺ activator.

In one embodiment of the invention, the dielectric core is coated, such as for example with a metallic shell, to enhance electron-phonon coupling and thereby increase up conversion or down conversion efficiency, as discussed above. In another embodiment of the invention, the shell can include a SiO₂- and/or TiO₂-coating, and this coating is in one embodiment coated on doped Y₂O₃ upconverting nanoparticles to thereby, in some instances, increase the upconversion efficiency relative to an uncoated nanocrystal. In another embodiment of the invention, the shell can include a SiO₂- and/or TiO₂-coating, and this coating is in one embodiment coated on doped Y₂O₃ down converting nanoparticles to thereby, in some instances, increase the down conversion efficiency relative to an uncoated nanocrystal. Further, in one embodiment of the invention, the coating can be a polymer. In one embodiment, this coating is provided on NaYF₄:Ln/NaYF₄ dielectric core. Such coatings can increase the upconversion efficiency relative to an uncoated upconverter.

In another embodiment of the invention, phonon modes of undoped host-lattice (e.g., Y₂O₃) nanocrystals are modulated, for example, by Au, Ag, Pt, and Pd shells of varying thicknesses. In various embodiments of the invention, the upconverter dielectric core and the shell system includes as upconverting nanocrystals Y₂O₃:Ln with NaYF₄ shells, Y₂O₃:Ln with Au(Ag,Pt) shells, NaYF₄:Ln with Y₂O₃ shells, NaYF₄:Ln with Au(Ag,Pt) shells. In this system, the core diameter and shell outer/inner diameter of the metallic coatings can be set to dimensions that are expected to be tunable to a plasmon mode overlap.

In other embodiments as discussed below, the metal coating or the metallic structure disposed in relation to an up-conversion or a down-conversion nanoparticle can exist inside the dielectric and the relative position of the metal structure to the dielectric structure can enhance plasmon resonance. These structures with the metallic structure inside can be referred to as a metallic core up converter or a metallic core down converter. The metallic core technique for energy conversion is useful since it takes advantage of metal nano-particles that have improved surface morphology compared to shell coatings on core dielectrics. The metal or metallic alloy in the inner core metallic energy converter can be selected to tune its plasmonic activity. These structures with the metallic structure outside can be referred to as a core up converter or a core down converter.

In various embodiments of the invention, the upconverter or down converter dielectric core can be coated with thiol-terminated silanes to provide a coating of SiO₂ about the core of similar reactivity to Y₂O₃. In one embodiment of the invention, the above-described methodology is used to synthesize core-shell nanoparticles of Y₂O₃:Ln with NaYF₄ shells, Y₂O₃:Ln with Au(Ag,Pt) shells, NaYF₄:Ln with Y₂O₃ shells, NaYF₄:Ln with Au(Ag,Pt) shells where core and shell diameters varying from 2 to 20 nm. In these material systems, the tuned ratio of core-to-shell diameter may permit a plasmon-phonon resonance which should amplify absorption of NIR light and/or upconverted emission. In these material systems, control of the core and shell diameters is one factor determining the size dependent effect and subsequent tuning of plasmon-phonon resonance.

In one embodiment of the invention, the upconverter dielectric core can be mixed core-shell materials including for example semiconducting Y₂O₃ and NaYF₄ cores doped with various Ln series metals, which have been shown to possess large upconverting efficiencies. These doped Y₂O₃ and NaYF₄ cores will have shells of Au(Ag,Pt, Pd) or undoped Y₂O₃ and NaYF₄ matrices which have the potential to enhance or tune the phonon modes needed for energy transfer in the upconversion process. Solubility can be enhanced, for example, by addition of thiolated organics (Au shell), organic chain triethanolsilane (Y₂O₃ shell), and trioctylphosphine-oleic amine (NaYF₄ shell). All core-shell nanoparticles may further be solubilized into a colloidal suspension with the addition of triarginine peptide, polyethylene glycol, and polyethyleneimine surfactants.

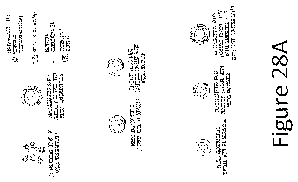

Figure 28A shows some of the various embodiments of the converter structures of the invention that can be designed: (a) a structure including upconverter (UC) molecules bound to a metal (gold) nanoparticle; (b) a structure including an UC-containing nanoparticle covered with metal nanoparticles, (c) a metal nanoparticle covered with an UC-containing nanocap; (d) an UC-containing nanoparticle covered with metal nanocap, (e) a metal nanoparticle covered with UC nanoshell, (f) an UC-containing nanoparticle covered with metal nanoshell, (g) an UC-containing nanoparticle covered with metal nanoshell with protective coating layer.

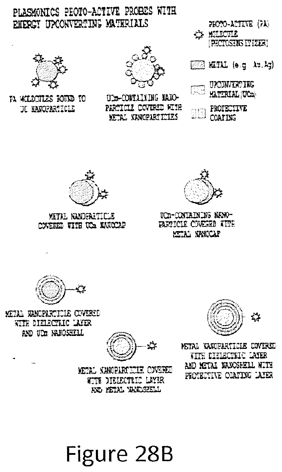

The configurations (while shown in the Figure 28A with UC-containing materials) would be applicable for enhancement for down converting materials such as the quantum dots or phosphors described herein. Moreover, in one embodiment of the invention, dielectric spacers (for examples silicates as discussed below) can be used with the structure of Figure 6A-b to space apart the particle type metallic structures. In another embodiment of the invention, dielectric spacers can be used with the structure of Figure 28A to space apart the metal layers, whether or not these layers are partial metal layers or continuous metal layers. See the schematics in Figure 28B

In various embodiments of the invention, multi-layer metallic nanoshells discussed in this application have the potential capability to enhance electromagnetically two spectral regions. Accordingly, the metallic structures of the invention can be used in the upconverting mode to enhance both the excitation at wavelength λ₁ and the emission at wavelength λ₂. This feature also can be used in the down converting to enhance primarily the emission at wavelength λ₂ and potentially the excitation at wavelength λ₁.

Such metallic structures in various embodiments of the invention include conducting materials made for example of metals, or doped glasses or doped semiconductors. These conducting materials can be in the form of pure or nearly pure elemental metals, alloys of such elemental metals, or layers of the conducting materials regardless of the constituency. The conducting materials can (as noted above) include non-metallic materials as minor components which do not at the levels of incorporation make the composite material insulating.

Similarly, in various embodiments of the invention, the up or down converting materials can include at least one of a dielectric, a glass, or a semiconductor. The up or down converting materials can include an alloy of two or more dielectric materials, an alloy of two or more glasses, or an alloy of two or more semiconductors.

Accordingly, Figure 28A represents embodiments of the invention where the dielectric core is supplemented with a shell. The shell can include a metal layer of a prescribed thickness. The metal layer can include materials such as nickel, gold, iron, silver, palladium, platinum and copper and combinations thereof. The metal layer can be also made of a combination of metals and non-metals. The shell functions as a plasmonic shell where surface plasmons can form in the metal between the dielectric core and the outer environment acting as an exterior dielectric. The shell (as shown) may not be a complete shell. Partial metallic shells or metallic shells of varying thicknesses are also acceptable in the invention.

As discussed below, the metallic shells in another embodiment of the invention serve as scattering centers for UV light where UV light which, even if absorbed in a paint or coating layer contributes at a minimum to localized heating of the paint or coating layer material, will be scattered from the paint or coated layer.

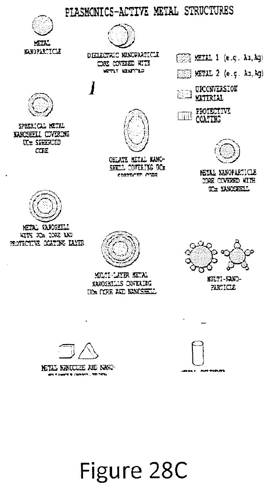

Figure 28C shows still further embodiments of plasmonics-active nanostructures having upconverting (UC) materials that can be designed: (a) a metal nanoparticle, (b) an UC nanoparticle core covered with metal nanocap, (c) a spherical metal nanoshell covering an UC spheroid core, (d) an oblate metal nanoshell covering UC spheroid core, (e) a metal nanoparticle core covered with UC nanoshell, (f) a metal nanoshell with protective coating layer, (g) multi-layer metal nanoshells covering an UC spheroid core, (h) multi-nanoparticle structures, (i) a metal nanocube and nanotriangle/nanoprism, and (j) a metal cylinder.

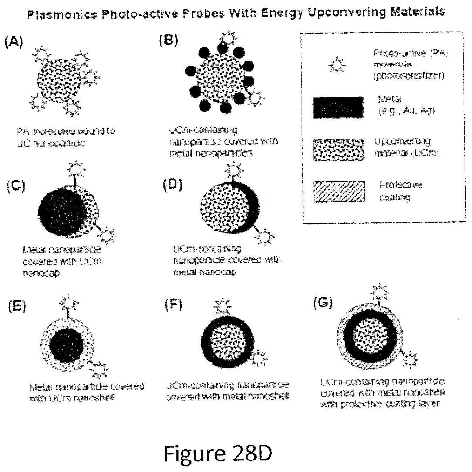

Figure 28D shows yet other embodiments of plasmonics-active nanostructures having upconverting materials with linked photo-active (PA) molecules that can be designed. For example, for the case of psoralen (as the PA molecule), the length of the linker between the PA molecule and the UC material or the metal surface is tailored such that it is sufficiently long to allow the PA molecules to be active (attach to DNA) and short enough to allow efficient excitation of light from the UC to efficiently excite the PA molecules. Figure 28D shows (a) PA molecules bound to an UC nanoparticle, (b) an UC material-containing a nanoparticle covered with metal nanoparticles, (c) a metal nanoparticle covered with UC material nanocap, (D) an UC material-containing nanoparticle covered with metal nanocap, (e) a metal nanoparticle covered with an UC material nanoshell, (f) an UC material-containing nanoparticle covered with metal nanoshell, (g) an UC material-containing nanoparticle covered with metal nanoshell with protective coating layer.

With the upconverter and down converter structures of the invention, a plasmonics effect is advantageous. A plasmonics effect can increase the local intensity of the received light or the local intensity of the emitted light from the up and/or down converter structures of the invention. A plasmonics effect can occur throughout the electromagnetic region provided the suitable nanostructures, nanoscale dimensions, metal types are used. Plasmonic effects are possible over a wide range of the electromagnetic spectrum, ranging from gamma rays and X rays throughout ultraviolet, visible, infrared, microwave and radio frequency energy. However, for practical reasons, visible and NIR light are used for metal structures such as for example silver and gold nanoparticles, since the plasmon resonances for silver and gold occur in the visible and NIR region, respectively.

In various embodiments, nanoparticles of neodymium and ytterbium doped yttrium oxide, europium and ytterbium doped yttrium oxide, and any combination of rare earth trivalent ions doped into a neodymium oxide nanocrystal can be used. The dual doped yttrium oxide of composition neodymium and ytterbium and also the dual doped europium and ytterbium are new for the yttrium oxide host lattice, although such dual doped systems have been shown to work in other host lattices such as YAG.

These dual doped lanthanide glasses have been shown to upconvert efficiently on bulk materials, and thereby can provide new upconverter structures at the nano-scale. There are advantages offered by these yttrium oxide nanostructures of the invention. The small scale synthetic methodology for creating nanoscale yttrium oxide is easier to control and produce in yttrium oxide than in YAG. The host structure of yttrium oxide scintillates by down conversion. These combinations of dopants in yttrium oxide for example can provide predetermined emission colors for the yttrium oxide nanocrystal for the color shifting of the invention.

In one embodiment of the invention, a dual dopant permits excitation of either ion in the host glass. For instance, excitation by 980 nm light excites an ytterbium ion, where through transfer of energy from one excited state of the ytterbium ion to another dopant provides a mechanism for upconversion emission of light in the visible and NIR spectral regions.

Up-conversion phosphors similar in chemical compositions to the down-conversion fluorescent materials discussed above can be used. The up-conversion phosphors can include laser dyes, e.g., the organic small molecules that can be excited by the absorption of at least two infrared photons with emission of visible light. The up-conversion phosphors can include fluorescent polymers, e.g., the class of polymers that can be excited by the absorption of at least two infrared photons with emission of visible light. The up-conversion phosphors can include inorganic or ceramic particles or nano-particles, including the conventional up-conversion phosphors (e.g. metal fluorides, metal oxides) that can be excited by the absorption of at least two infrared photons with emission of visible light. The up-conversion phosphors can include semiconductor particles, including nano-particles such as II-VI or III-V compound semiconductors, e.g. quantum dots, described in details in the "down-conversion" semiconductors above.

Fluorescent up-conversion inorganic phosphors can include but are not limited to metal oxides, metal halides, metal chalcogenides (e.g. sulfides), or their hybrids, such as metal oxo-halides, metal oxo-chalcogenides. Fluorescent up-conversion inorganic phosphors are usually doped with rare earth elements (e.g. Yb³⁺, Er³⁺, Tm³⁺). Some host examples include, but are not limited to: NaYF₄, YF₃, BaYF₅, LaF₃, La₂MoO₈, LaNbO₄, LnO₂ S; where Ln is the rare earth elements, such as Y, La, Gd).

These converters (and the other energy converters described herein which receive energy and generate light or electron emission) can optionally include any of the energy augmentation structures described above.

In various embodiments of the invention, energy converters can be used with the energy augmentators described above for color enhancement. In some embodiments, the converters are up conversion of light e.g., from the IR regime into visible electromagnetic radiation and for down conversion of light e.g., from the UV range into visible electromagnetic radiation. The invention in various embodiments up converts energy, preferably light in the visible spectrum. The invention encompasses a variety of applications where the up and down conversion materials with or without energy augmentators are included to enhance the color of the object being displayed. These application areas can include paints on signs, walls, cars, buildings, boats, airplanes. These application areas can include display monitors, computer monitors, telephone displays, watch dials, instrument dials to name but a few.

Among various materials, luminescent nanoparticles have attracted increasing technological and industrial interest. In the context of the invention, nanoparticle refers to a particle having a size less than one micron. While the description of the invention describes specific examples using nanoparticles, the invention in many embodiments is not limited to particles having a size less than one micron. However, in many of the embodiments, the size range of less than one micron, and especially less than 100 nm produces properties of special interest such as for example emission lifetime luminescence quenching, luminescent quantum efficiency, and concentration quenching and such as for example diffusion, penetration, and dispersion into mediums where larger size particles would not migrate.

This invention in various embodiments can use a wide variety of down conversion materials (or mixtures of down conversion materials) with or without the energy augmentators to enhance a particular color of light observable to an observer. These down conversion materials can include quantum dots, semiconductor materials, alloys of semiconductor materials, scintillation and phosphor materials, materials that exhibit X-ray excited luminescence (XEOL), organic solids, metal complexes, inorganic solids, crystals, rare earth materials (lanthanides), polymers, scintillators, phosphor materials, etc., and materials that exhibit excitonic properties. Accordingly, the down conversion materials to enhance color emission can convert energy from one of ultraviolet light, x-rays, and high energy particles to visible light. The down conversion materials to enhance color emission can convert energy from higher energy visible light to lower energy visible light with or without the energy augmentators.

In one embodiment of the invention, a quantum dot mixture with or without the energy augmentators can be used for color enhancement. Quantum dots are in general nanometer size particles whose energy states in the material of the quantum dot are dependent on the size of the quantum dot. For example, quantum dots are known to be semiconductors whose conducting characteristics are closely related to the size and shape of the individual crystal. Generally, the smaller the size of the crystal, the larger the band gap, the greater the difference in energy between the highest valence band and the lowest conduction band becomes. Therefore, more energy is needed to excite the dot, and concurrently, more energy is released when the crystal returns to its resting state. In fluorescent dye applications, this equates to higher frequencies of light emitted after excitation of the dot as the crystal size grows smaller, resulting in a color shift from red to blue in the light emitted. Quantum dots represent one way to down convert ultraviolet light of the spectrum to a targeted color emission, such as for example a green light emission. Quantum dots represent one way to down convert blue light of the spectrum to a targeted color emission, such as for example a green light emission.

As described in U.S. Pat. No. 6,744,960, different size quantum dots produce different color emissions. In that work and applicable to this invention, quantum dots can comprise various materials including semiconductors such as zinc selenide (ZnSe), cadmium selenide (CdSe), cadmium sulfide (CdS), indium arsenide (InAs), and indium phosphide (InP). Another material that may suitably be employed is titanium dioxide (TiO₂). The size of the particle, i.e., the quantum dot 18, may range from about 2 to 10 nm. Since the size of these particles is so small, quantum physics governs many of the electrical and optical properties of the quantum dot. One such result of the application of quantum mechanics to the quantum dot 18 is that quantum dots absorb a broad spectrum of optical wavelengths and re-emit radiation having a wavelength that is longer than the wavelength of the absorbed light. The wavelength of the emitted light is governed by the size of the quantum dot. For example, CdSe quantum dots 5.0 nm in diameter emit radiation having a narrow spectral distribution centered about 625 nm while quantum dots 18 including CdSe 2.2 nm in size emit light having a center wavelength of about 500 nm. Semiconductor quantum dots comprising CdSe, InP, and InAs, can emit radiation having center wavelengths in the range between 400 nm to about 1.5 µm. Titanium dioxide TiO₂also emits in this range. The linewidth of the emission, i.e., full-width half-maximum (FWHM), for these semiconductor materials may range from about 20 to 30 nm. To produce this narrowband emission, quantum dots simply need to absorb light having wavelengths shorter than the wavelength of the light emitted by the dots. For example, for 5.0 nm diameter CdSe quantum dots light having wavelengths shorter than about 625 nm is absorbed to produce emission at about 625 nm while for 2.2 nm quantum dots comprising CdSe light having wavelengths smaller than about 500 nm is absorbed and re-emitted at about 500 nm. In practice, however, the excitation or pump radiation is at least about 50 nanometers shorter than the emitted radiation.

Specifically, in one embodiment of the invention, a quantum dot mixture (QDM) coating can be deposited using CVD and or sol-gel techniques using standard precipitation techniques to be used with or without the energy augmentators. The QDM coating can be made of a silicate structure that does not diminish UV output. Within the silicate family, silica (SiO₂) is suitable since it maximizes UV transmission through the coating. The coating can further include a second layer of a biocompatible glass. Such bio-compatible glass and glass ceramic compositions can contain calcium, a lanthanide or yttrium, silicon, phosphorus and oxygen. Other biocompatible materials and techniques are described in the following patents U.S. Pat. Nos. 5,034,353; 4,786,617; 3,981,736; 3,922,155; 4,120,730; and U.S. Pat. Appl. Nos. 2008/0057096; 2006/0275368; and 2010/0023101.

Further, the down conversion materials for the invention described here can be coated with insulator materials such as for example silica which will reduce the likelihood of any chemical interaction between the luminescent particles and the medium the particles are included therein. These and the other conversion materials here can be used with or without the energy augmentators. For biocompatible applications of inorganic nanoparticles, one of the major limiting factors is their toxicity. Generally speaking, all semiconductor nanoparticles are more or less toxic. For biocompatible applications, nanoparticles with toxicity as low as possible are desirable or else the nanoparticles have to remain separated from the medium. Pure TiO₂, ZnO, and Fe₂O₃ are biocompatible. CdTe and CdSe are toxic, while ZnS, CaS, BaS, SrS and Y₂O₃ are less toxic. In addition, the toxicity of nanoparticles can result from their inorganic stabilizers, such as TGA, or from dopants such as Eu ²⁺, Cr ³⁺ or Nd ³⁺. Other suitable down conversion materials which would seem the most biocompatible are zinc sulfide, ZnS:Mn²⁺, ferric oxide, titanium oxide, zinc oxide, zinc oxide containing small amounts of Al₂O₃, and AgI nanoclusters encapsulated in zeolite. For non-medical applications, where toxicity may not be as critical a concern, the following materials (as well as those listed elsewhere) are considered suitable: lanthanum and gadolinium oxyhalides activated with thulium; Er³⁺ doped BaTiO₃ nanoparticles, Yb³⁺ doped CsMnCl₃ and RbMnCl₃, BaFBr:Eu²⁺ nanoparticles, Cesium Iodine, Bismuth Germanate, Cadmium Tungstate, and CsBr doped with divalent Eu.

In various embodiments of the invention, the following luminescent polymers are also suitable as conversion materials with or without the energy augmentators: poly(phenylene ethynylene), poly(phenylene vinylene), poly(p-phenylene), poly(thiophene), poly(pyridyl vinylene), poly(pyrrole), poly(acetylene), poly(vinyl carbazole), poly(fluorenes), and the like, as well as copolymers and/or derivatives thereof.

In various embodiments of the invention, the following materials can be used similar to that detailed in U.S. Pat. No. 7,090,355. For down-conversion, the following materials can be used with or without the energy augmentators. Inorganic or ceramic phosphors or nano-particles, including but not limited to metal oxides, metal halides, metal chalcogenides (e.g. metal sulfides), or their hybrids, such as metal oxo-halides, metal oxo-chalcogenides. Laser dyes and small organic molecules, and fluorescent organic polymers. Semiconductor nano-particles, such as II-VI or III-V compound semiconductors, e.g. fluorescent quantum dots. Organometallic molecules including at least a metal center such as rare earth elements (e.g. Eu, Tb, Ce, Er, Tm, Pr, Ho) and transitional metal elements such as Cr, Mn, Zn, Ir, Ru, V, and main group elements such as B, Al, Ga, etc. The metal elements are chemically bonded to organic groups to prevent the quenching of the fluorescence from the hosts or solvents. Phosphors can be used including the Garnet series of phosphors: (Y ₘ A ₁₋ₘ ) ₃(Al ₙ B ₁₋ₙ) ₅ O ₁₂, doped with Ce; where 0 ≤ m, n ≤ 1, where A includes other rare earth elements, B includes B, Ga. In addition, phosphors containing metal silicates, metal borates, metal phosphates, and metal aluminates hosts can be used. In addition, nano-particulates phosphors containing common rare earth elements (e.g. Eu, Tb, Ce, Dy, Er, Pr, Tm) and transitional or main group elements (e.g. Mn, Cr, Ti, Ag, Cu, Zn, Bi, Pb, Sn, TI) as the fluorescent activators, can be used. Materials such as Ca, Zn, Cd in tungstates, metal vanadates, ZnO, etc. can be used with or without the energy augmentators.

The commercial laser dye materials obtained from several laser dye vendors, including Lambda Physik, and Exciton, etc. can also be used with or without the energy augmentators. A partial list of the preferred laser dye classes includes: Pyrromethene, Coumarin, Rhodamine, Fluorescein, other aromatic hydrocarbons and their derivatives, etc. In addition, there are many polymers containing unsaturated carbon-carbon bonds, which also serve as fluorescent materials and find many optical and fluorescent applications For example, MEH-PPV, PPV, etc. have been used in opto-electronic devices, such as polymer light emitting diodes (PLED). Such fluorescent polymers can be used directly as the fluorescent layer of the transparent 2-D display screen.

As noted above, semiconductor nanoparticles (e.g., quantum dots) can be used with or without the energy augmentators. The terms "semiconductor nanoparticles," in the art refers to an inorganic crystallite between 1 nm and 1000 nm in diameter, preferably between 2 nm to 50 nm. A semiconductor nano-particle is capable of emitting electromagnetic radiation upon excitation (i.e., the semiconductor nano-particle is luminescent). The nanoparticle can be either a homogeneous nano-crystal, or comprises of multiple shells. For example, the nanoparticle can include a "core" of one or more first semiconductor materials, and may be surrounded by a "shell" of a second semiconductor material. The core and/or the shell can be a semiconductor material including, but not limited to, those of the group II-VI (ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, and an alloy or a mixture thereof.

Fluorescent organometallic molecules containing rare earth or transitional element cations can be used for down conversion materials with or without the energy augmentators. Such molecules include a metal center of rare earth elements including Eu, Tb, Er, Tm, Ce protected with organic chelating groups. The metal center may also include transitional elements such as Zn, Mn, Cr, Ir, etc. and main group elements such as B, Al, Ga. Such organometallic molecules can readily dissolve in liquid or transparent solid host media. Some examples of such fluorescent organometallic molecules include: 1. Tris(dibenzoylmethane)mono(phenanthroline)europium(III); 2. Tris(8-hydroxyquinoline)erbium; 3. Tris(1-phenyl-3-methyl-4-(2,2-dimethylpropan-1-oyl)pyrazolin -5-one)terbium(III); 4. Bis(2-methyl-8-hydroxyquinolato)zinc; 5. Diphenylborane-8-hydroxyquinolate.

Specific examples of down-conversion materials for red emission include those discussed above and europium complexes such as those described in JP Laid-open Patent Publication (Kokai) No. 2003-26969, constructed such that β-diketone ligand is coordinated to europium forming an europium complex capable of emitting red fluorescence. Other specific examples of the rare earth element complexes include complexes include lanthanum (Ln), europium (Eu), terbium (Tb), and gadolinium (Gd) and combinations thereof A europium (Eu) complex is capable of emitting red fluorescence when irradiated with ultraviolet rays having a wavelength ranging from 365 nm to 410 nm. Terbium (Tb) is capable of emitting green fluorescence when irradiated with ultraviolet rays having a wavelength of 365 nm.

In other down-conversion embodiments with or without the energy augmentators, the down conversion materials which emit red light may include europium, light emitting particles which emit green light may include Terbium, and light emitting particles which emit blue or yellow light may include cerium (and/or thulium). In up-conversion embodiments, up conversion materials which emit red light may include praseodymium, light emitting particles which emit green light may include erbium, and light emitting particles which emit blue light may include thulium. In embodiments, the conversion materials can be light emitting particles made of fluorescent molecules that emit different colors (e.g. red, green, and blue). In embodiments, the conversion materials can be light emitting particles made of pure organic or organo-metallic dyes with or without the energy augmentators.

In addition to the combinations of rare earth complexes, such as a combination of a europium complex and a terbium complex, it is also possible employ a combination of a europium complex and a green-emitting fluorescent substance which is not a complex, or a combination of a terbium complex and a red-emitting fluorescent substance which is not a complex.

Other down converter materials with or without the energy augmentators include for example ZnS, PbS, SbS₃, MoS₂, PbTe, PbSe, BeO, MgO. Li₂CO₃, Ca(OH), MoO₃, SiO₂, Al₂O₃, TeO₂, SnO₂, KBr, KCl, and NaCl. These materials can include dopants to tailor the emission properties, as noted above. Examples of doped (or alloyed) glass systems suitable for the include Y₂O₃:Gd, Y₂O₃:Dy, Y₂O₃:Tb, Y₂O₃:Ho, Y₂O₃:Er, Y₂O₃:Tm, Gd₂O₃:Eu, Y₂O₂S:Pr, Y₂O₂S:Sm, Y₂O₂S:Eu, Y₂O₂S:Tb, Y₂O₂S:Ho, Y₂O₂S:Er, Y₂O₂S:Dy, Y₂O₂S:Tm, ZnS:Ag:Cl (blue), ZnS:Cu:Al (green), Y₂O₂S:Eu (red), Y₂O₃:Eu (red), YVO₄:Eu (red), and Zn₂SiO₄:Mn (green).

With regard more specifically to down converter materials suitable for the invention with or without the energy augmentators, U.S. Pat. No. 4,705,952 describes an infrared-triggered phosphor that stores energy in the form of visible light of a first wavelength and released energy in the form of visible light of a second wavelength when triggered by infrared light. The phosphors in U.S. Pat. No. 4,705,952 were compositions of alkaline earth metal sulfides, rare earth dopants, and fusible salts. The phosphors in U.S. Pat. No. 4,705,952 were more specifically phosphors made from strontium sulfide, barium sulfide and mixtures thereof; including a dopant from the rare earth series and europium oxide, and mixtures thereof; and including a fusible salt of fluorides, chlorides, bromides, and iodides of lithium, sodium, potassium, cesium, magnesium, calcium, strontium, and barium, and mixtures thereof. The materials described in U.S. Pat. No. 4,705,952 are useful in various embodiments of the invention with or without the energy augmentators.

In other embodiments of the invention, the down converter materials (or mixtures of down converters materials can include Y₂O₃: Li. Sun et al "Luminescent properties of Li+ doped nanosized Y2O3:Eu," Solid State Comm. 119 (2001) 393-396 describe such materials. Hou et al "Luminescent properties nano-sized Y2O3:Eu fabricated by co-precipitation method," Journal of Alloys and Compounds, vol. 494, issue 1-2, 2 April 2010, pages 382-385 describe that nano-sized yttria (Y₂O₃) powders have been successfully synthesized by a co-precipitation method. The powders were well crystallized, and the grains were almost spherical with good dispersibility. The quenching concentration of Eu³⁺ ions is 9 mol% which is much higher than micro-scaled powders. The incorporation of Li+ ions greatly improved the luminescence intensity. The highest emission intensity was observed with 4 mol% Li+ doped Y₂O₃:Eu powder ((Y_{0.87}Eu_{0.09}Li_{0.04})₂O₃) and the fluorescence intensity was increased by as much as 79%. Yi et al "Improved cathodoluminescent characteristics of Y₂O₃:Eu³⁺ thin films by Li-doping," Appl. Phys. A 87, 667-671 (2007) describe cathodoluminescent spectra for both Y₂O₃:Eu³⁺ and Li-doped Y₂O₃:Eu³⁺ films and methods for making these materials.

The invention in other embodiments can use a wide variety of up conversion materials (or mixtures of up converters) with or without the energy augmentators to enhance a particular color of light observable from reflective material or surface. These up conversion materials can include similar materials as discussed above with regard to down conversion but typically included doped or impurity states in a host crystal that provide a mechanism for up conversion pumping. Accordingly, the up conversion materials to enhance color emission can convert energy from one of near infrared, infrared, and microwave irradiation. The upconversion materials to enhance color emission can convert energy from lower energy visible light to higher energy visible light.

Upconversion materials with or without the energy augmentators can be used in various ways to enhance visible light emission by way of conversion of infrared light from a solar spectrum (as in daylight exposure) or a black body spectrum (as in an incandescent lamp). In one example, a nanoparticle of a lanthanide doped oxide can be excited with near infrared light such as laser light at 980 nm and 808 nm to produce visible light in different parts of the red, green, blue spectrum depending on the dopant trivalent rare earth ion(s) chosen, their concentration, and the host lattice.

The lanthanide doped oxides suitable for this invention differ from more traditional multi-photon up conversion processes where the absorption of, for example, two photons is needed in a simultaneous event to promote an electron from a valence state directly into an upper level conduction band state where relaxation across the band gap of the material produces fluorescence. Here, the co-doping produces states in the band gap of the NaYF₄ such that the Yb³⁺ ion has an energy state at ²F_{5/2} pumpable by a single photon event and from which other single photon absorption events can populate even higher states. Once in this exited state, transitions to higher energy radiative states are possible, from which light emission will be at a higher energy than that of the incident light pumping the ²F_{5/2} energy state. In other words, the energy state at ²F_{5/2} of the Yb³⁺ ion is the state that absorbs 980 nm light permitting a population build up serving as the basis for the transitions to the higher energy states such as the ⁴F_{7/2} energy state. Here, transitions from the ⁴F_{7/2} energy state produce visible emissions.

U.S. Pat. No. 7,008,559 describes the upconversion performance of ZnS where excitation at 767 nm produces emission in the visible range. The materials described in U.S. Pat. No. 7,008,559 (including the ZnS as well as Er³⁺ doped BaTiO₃ nanoparticles and Yb³⁺ doped CsMnCl₃) are suitable in various embodiments of the invention with or without the energy augmentators.

Further, materials specified for up conversion materials in the invention with or without the energy augmentators include CdTe, CdSe, ZnO, CdS, Y₂O₃, MgS, CaS, SrS and BaS. Such up conversion materials may be any semiconductor and more specifically, but not by way of limitation, sulfide, telluride, selenide, and oxide semiconductors and their nanoparticles, such as Zn₁₋ₓMnₓS_{y}, Zn₁₋ₓMnₓSe_{y}, Zn₁₋ₓMnₓTe_{y}, Cd₁₋ₓMnS_{y}, Cd₁₋ₓMnₓSe_{y}, Cd₁₋ₓMnₓTe_{y}, Pb₁₋ₓMnₓS_{y}, Pb₁₋ₓMnₓSe_{y}, Pb₁₋ₓMnₓTe_{y}, Mg₁₋ₓMnS_{y}, Ca₁₋ₓMnₓS_{y}, Ba₁₋ₓMnₓS_{y} and Sr₁₋ₓ, etc. (wherein, 0<x ≦ 1, and 0<y ≦ 1). Complex compounds of the above-described semiconductors are also contemplated for use in the invention--e.g. (M_{1-z}N_{z})₁₋ₓMnₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, Se, Te, O; 0<x ≦ 1, 0<y ≦ 1, 0<z ≦ 1). Two examples of such complex compounds are Zn_{0.4}Cd_{0.4}Mn_{0.2}S and Zn_{0.9}Mn_{0..1}S_{0.8}Se_{0.2}. Additional conversion materials include insulating and nonconducting materials such as BaF₂, BaFBr, and BaTiO₃, to name but a few exemplary compounds. Transition and rare earth ion co-doped semiconductors suitable for the invention include sulfide, telluride, selenide and oxide semiconductors and their nanoparticles, such as ZnS; Mn; Er; ZnSe; Mn, Er; MgS; Mn, Er; CaS; Mn, Er; ZnS; Mn, Yb; ZnSe; Mn, Yb; MgS; Mn, Yb; CaS; Mn,Yb etc., and their complex compounds: (M_{1-z}N_{z})₁₋ₓ(Mn_{g}R_{1-q})ₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, ...0<z≤1, o<q≤1).

Some nanoparticles such as ZnS:Tb³⁺, Er³⁺; ZnS:Tb³⁺; Y₂O₃:Tb³⁺; Y₂O₃:Tb³⁺, Er3⁺; ZnS:Mn²⁺; ZnS:Mn,Er³⁺ are known in the art to function for both down-conversion luminescence and upconversion luminescence and would be suitable for the invention with or without the energy augmentators. In up-conversion embodiments, light emitting particles which emit red light may include praseodymium, light emitting particles which emit green light may include erbium, and light emitting particles which emit blue light may include thulium.

In general, the upconversion process generally requires one of more rare-earth dopants, such as Er, Eu, Yb, Tm, Nd, Tb, Ce, Y, U, Pr, La, Gd and other rare-earth species or a combination thereof, doped into a dielectric crystal (of any size >0.1nm), including at least one of Y₂O₃, Y₂O₂S, NaYF₄, NaYbF₄, YAG, YAP, Nd₂O₃, LaF₃, LaCl₃, La₂O₃, TiO₂, LuPO₄, YVO₄, YbF₃, YF₃, Na-doped YbF₃, or SiO₂, where incident radiation is at longer wavelength than emissive radiation from the crystal. The wavelength emitted in based entirely on the dopant ion(s) chosen and their associated and relative concentration in the host crystal. For the example of upconversion in a Y₂O₃ host crystal, to achieve a blue emission (~450 - 480 nm) one could synthesize [Y₂O₃; Yb (3%), Tm (0.2%)], where the Yb and Tm are the percentages doped in the crystal relative to the Y atoms being 100%. Likewise, typical green upconversion materials are [Y₂O₃; Yb (5%), Ho (1%)] and [Y₂O₃; Yb (2%), Er (1%)], and typical red upconversion materials are [Y₂O₃; Yb (10%), Er (1%)] and [Y₂O₃; Yb (5%), Eu (1%)]. The concentrations of dopants relative to each other and the crystal matrix must be tuned for every combination, and there are multiple ways to achieve multiple colors from even the same dopants with or without the energy augmentators.

Up-conversion of red light with a wavelength of about 650 nm in Tm³⁺ doped flourozirconate glasses can be used in the invention to produce blue light. In this system, the blue light consists of two emission bands; one at 450 nm which is ascribed to the 1D2→3H4 transition, the others at 475 nm is ascribed to the 1G4→3H6 transition. The emission intensities of both bands have been observed by others to vary quadratically with the excitation power. For glasses with a Tm³⁺ concentration of 0.2 mol% and greater, cross-relaxation processes occur which decrease the up-conversion efficiency.

The emission of visible light upon excitation in the near-infrared (NIR) has been observed in optically clear colloidal solutions of LuPO₄:Yb³⁺, Tm³⁺, and YbPO₄:Er³⁺ nanocrystals in chloroform. Excitation at 975 nm has been shown by others to produce visible luminescence in the blue, green, or red spectral regions.

Tellurium and germanium oxides (tellurites and germinates) are also suitable upconverters. These glasses can be doped with Tm, Yb, Ho, Er, Pr, for example.

Yb³⁺ doped BaZrO₃ is also suitable for upconversion. Er³⁺ and/or Tm³⁺ doping are also suitable for tailoring the emission wavelengths.

In another embodiment, Nd³⁺ :Cs₂NaGdCl₆ and Nd³⁺, Yb³⁺:Cs₂NaGdCl₆ polycrystalline powder samples prepared by Morss method have been reported to be up converters and are suitable for the present invention. These materials, under 785 nm irradiation, have shown upconversion emissions near 538 nm (Green), 603 nm (Orange), and 675 nm (Red) were observed and assigned to 4G7/2→4I9/2, (4G7/2→4I11/2; 4G5/2→4I9/2), and (4G7/2→4I13/2; 4G5/2→4I11/2), respectively.

In another embodiment, Nd³⁺ and Ho³⁺ co-doped -based ZrF₄ fluoride glasses under 800 nm excitation have been reported to be up converters and are suitable for the present invention. Among the up-conversion luminescences for the ZrF₄ fluoride glasses, the green emission was seen to be extremely strong and the blue and red emission intensities were very weak.

In another embodiment, Tm³+/Yb³⁺ -codoped TeO₂-Ga₁O₃-R₂O (R=Li, Na, K) glasses have been reported to be up converters and are suitable for the present invention. These materials, under excitation at 977 nm, showed intense blue upconversion emission centered at 476 nm along with a weak red emission at 650 nm.

In another embodiment, metal-to-ligand charge transfer (MLCT) transition in [Ru(dmb)₃]²⁺ (dmb = 4,4'-dimethyl-2,2'-bipyridine) in the presence of anthracene or 9,10-diphenylanthracene have been reported converters and are suitable for the present invention. Upconverted to be up converters and are suitable for the present invention. Upconverted singlet fluorescence resulting from triplet-triplet annihilation at low excitation power has been reported. In particular 9,10-diphenylanthracene (DPA) (substituted for anthracene) showed higher efficiencies for upconversion. In these experiments, workers with this material system assumed that DPA's increased singlet fluorescence quantum yield ( = 0.95) relative to anthracene ( = 0.27)7. This work lead to an approximate 24.4 ± 6.1 enhancement of green-to-blue light upconversion permitting direct visualization of the process at low excitation power, for example by a commercial green laser pointer (*λ*ₑₓ= 532 nm, <5 mW peak power).

The structures described herein for color enhancement with the energy augmentation structures are denoted as color enhancing/ energy augmentation structures or as energy enhancing/augmentation structures.

By having the energy converters or color converting or enhancing materials disposed in a vicinity of the energy augmentation structures of this invention, regardless of the whether the energy augmentation structure is in a region of intensified electric field or otherwise outside the region of intensified electric field, the color enhancing/ energy augmentation structures or the energy enhancing/augmentation structures of the invention are able to produce light which can be used for a variety of applications, in particular for photostimulation of biological, chemical, and physical reactions such as for example photoactivation of photoreactive drugs, photoactivation of photosensitive materials such as adhesives or lithographic photoresists, or for direct interaction with biological and chemical agents in the environment of the augmentation structures, as in sterilization.

Accordingly, in one embodiment of the invention, the color enhancement structures described herein can receive polychromatic light from a variety of sources such as sunlight, incandescent bulbs, fluorescent tube, and LED light sources with each having different wavelengths or wavelength bands. For these wavelength different bands, the resonators are "matched" or "tuned" to those wavelengths such that an intense electric field is established especially between the external-electrode pairs, or the folded resonator electrode pairs if used. In those regions of intense electric field can be disposed color converters (up and/or down phosphors) which can take light from one of the different wavelengths or wavelength bands, and have light of another wavelength or of different wavelength bands be emitted therefrom. In one embodiment, the intense electric field increases the intensity of the emitted light from the phosphors. Moreover, unlike the above-noted plasmonics where the electric field enhancement is restricted to regions within 100 to 200 nm of the metal, the resonators establish an increased electric field within the volume of the external-electrode pair, or the folded resonator electrode pairs if used, such that the phosphor material in a vicinity and within the external-electrode pair (or the folded resonator electrode pairs) exhibits an intensity larger than if the converter were remote from the resonator.

In view of the above, this invention is directed in general to methods and systems for color enhancement utilizing a color enhancement structure having a) an energy collector comprising at least one energy augmentation structure, and b) an energy converter capable of converting a second wavelength/quantum of electromagnetic energy into and emitting therefrom a third wavelength of light shifted in wavelength/energy from the second wavelength/quantum of electromagnetic energy. In one embodiment, the energy converter is disposed in a vicinity of the at least one energy augmentation structure such that the light shifted in wavelength is emitted with an intensity larger than if the converter were remote from the at least one energy augmentation structure. For ease of understanding, the term "wavelength" will be used to describe the electromagnetic energy entering into the energy converter, even though that electromagnetic energy may be better described in certain embodiments based upon its energy level or strength.

By having the energy converters or color converting or enhancing materials disposed in a vicinity of the energy augmentation structures of this invention, regardless of the whether the energy augmentation structure is in a region of intensified electric field or otherwise outside the region of intensified electric field, the color enhancing/augmentation structures or the energy enhancing/augmentation structures of the invention are able to enhance the conversion of one form of energy to another, as a conversion from one or more wavelengths of light to other wavelengths of light, or as a conversion from the one or more wavelengths of light to electrical energy, or as a conversion from the one or more wavelengths of light to heat.

Conversion from the one or more wavelengths of light to other wavelengths of light is useful for color shifting and color enhancement applications. Conversion from the one or more wavelengths of light to electrical energy is useful for harvesting solar energy using for example photovoltaic cells. Conversion from the one or more wavelengths of light to heat is useful also for harvesting solar energy using for example thermoelectric cells or other heat-to-electrical energy devices such as thermoelectric generators.

In some embodiments of the color enhancing/ energy augmentation structures, the color enhancing structure includes a multi-dimensional light collector comprising a first level of metallic patterns and a second level of metallic patterns offset in at least one of a lateral or axial direction from the first level of metallic patterns. At least one of the metallic patterns optionally comprises a first resonator dimensioned to be resonant with a first wavelength of light. The first resonator can be one of a folded structure or an external-electrode pair structure as noted above. The color enhancement structure has a converter capable of converting a second wavelength of light into and emitting therefrom a third wavelength of light shifted in wavelength from the second wavelength of light. The converter is disposed with the first resonator such that the light shifted in wavelength is emitted with an intensity larger than if the converter were remote from the first resonator.

In some embodiments, the energy converter being disposed in a vicinity of the at least one energy augmentation structure is conductively coupled the energy converter to the at least one energy augmentation structure.

For example, in some embodiments, the energy converter being disposed in a vicinity of the at least one energy augmentation structure comprises a physical conductive connection between the energy converter and the at least one energy augmentation structure.

In some embodiments of the color enhancing/ energy augmentation structures, the color enhancing structure, the energy converter comprises a down converter converting ultraviolet or blue light into red, yellow, or green light. In some embodiments of the color enhancing/augmentation structures, the energy converter comprises an up converter converting infrared or red light into yellow, green light, or blue light.

In some embodiments of the color enhancing/ energy augmentation structures, the metallic patterns referenced above comprises a folded resonator having opposing electrodes with electric fields directed in between, and the converter is positioned between the opposing electrodes or within fringing electric field of the opposing electrodes or otherwise in a vicinity of the opposing electrodes. In one example, the folded resonator is a ¾ 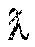 folded resonator. In one example, metallic patterns comprise at least one of Au, Ag, Cu, Al, or transparent metal oxides. In another example, the metallic patterns can be formed with refractory metals such for example Ti, W, and Mo.

In some embodiments of the color enhancing/ energy augmentation structures, the metallic patterns referenced above comprises an external external-electrode pair structure having opposing electrodes with electric fields directed in between, and the converter is positioned between the opposing electrodes or within fringing electric field of the opposing electrodes or otherwise in a vicinity of the opposing electrodes. In one example, the resonator is a ¾ 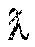 external-electrode pair resonator. In one example, metallic patterns comprise at least one of Au, Ag, Cu, Al, or transparent metal oxides. In another example, the metallic patterns can be formed with refractory metals such for example Ti, W, and Mo.

In some embodiments of the color enhancing/ energy augmentation structures, the color enhancing structure, there is an antireflection film disposed on at least one of the metallic patterns or on the converter.

In some embodiments of the color enhancing/ energy augmentation structures, the color enhancing structure, the first resonator noted above comprises plural resonators, the converter noted above comprises plural converters, and the plural converters are disposed at multiple positions throughout the light collector. In one example, the plural converters are positioned to convert light being internally scattered within the light collector.

In some embodiments of the color enhancing/ energy augmentation structures, the first level of metallic patterns noted above (or the second level of metallic patterns) comprises a metal core cladded with a high-K dielectric and a subsequent cladding of a low-K dielectric. In some embodiments of the color enhancing/ energy augmentation structures, the first level of metallic patterns noted above (or the second level of metallic patterns) comprises a radial pattern of conductors. In some embodiments of the color enhancing/ energy augmentation structures, the first level of metallic patterns noted above (or the second level of metallic patterns) comprises a fractal pattern. In one example, the fractal pattern is embedded within a dielectric material.

In some embodiments of the color enhancing/ energy augmentation structures, the first level of metallic patterns noted above (or the second level of metallic patterns) comprises a three-dimensional fractal structure.

In some embodiments of the color enhancing/ energy augmentation structures, the light collector comprises a transparent panel with the first level of metallic patterns and the second level of metallic patterns and optionally multiple converters formed therein. In some embodiments of the color enhancing/augmentation structures, the light collector comprises a transparent sheet with the first level of metallic patterns and the second level of metallic patterns and optionally multiple converters formed therein.

In some embodiments of the color enhancing/ energy augmentation structures, the first level of metallic patterns noted above (or the second level of metallic patterns) are of different sizes and/or orientations to each other of the first level of metallic patterns or with respect to the second level of metallic patterns.

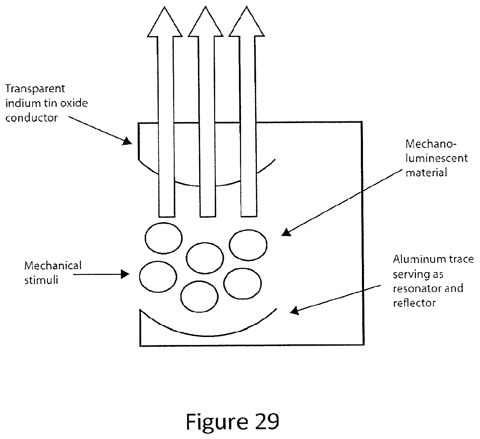

Indeed, Figure 29 is a schematic of a reflective resonator including mechano-luminescent materials, in this example the mechano-luminescent materials being placed between a folded resonator structure, although mechano-luminescent materials could be placed between an external electrode pair resonator structure. Thus, in one embodiment, an electromagnetic wave energy augmentator captures one or more wavelengths of electromagnetic energy, and augments the one or more wavelengths of electromagnetic energy in at least one property (such as electric field intensity in a vicinity of the mechano-luminescent materials), while at the same time the mechano-luminescent materials can be considered an energy converter converting the ultrasonic or mechanical energy into electromagnetic radiation (i.e., emitted light).

In one embodiment the increased electric field in the folded structure or the external electrode pair increases the luminescence of the mechano-luminescent materials. The energy used to build the electric field in the folded structure or the external electrode pair being provided separately from the mechanical energy driving the mechano-luminescence.

For example, the reflective resonator of Figure 29 could be placed adjacent an exhaust stack of an engine or other waste heat dissipating machine. In one embodiment, the reflective resonator of Figure 29 would be mounted on a stainless steel arm connected to the heat stack. The stainless steel would couple mechanical vibrations to the reflective resonator while thermally isolating the reflective resonator from the exhaust stack, thereby permitting even inorganic mechano-luminescent materials to be used.

When the engine began to show higher levels of vibration or vibrations at different frequencies, the intensity of the light emitted would change providing a visible light signal that the engine or machine was under stress from power loads or wear or mechanical failure.

In one embodiment the reflective structure shown in Figure 29 need not include the resonator and its resonating elements. In one embodiment of the invention, the reflective structure shown in Figure 29 could be placed directly on a machine operating at a relatively cold temperature around 100 °C. In this embodiment, the reflective structure need not include the resonator and its resonating elements. However, if the resonator and its resonating elements were present, a laser such as 656 nm laser could "probe" the resonator and intensify "on demand" the mechano-luminescence. In this way, early detection of developing mechanical problems could be detected.

Various mechano-luminescent materials suitable for the present invention include ZnS:Mn²⁺, SrAl₂O₄:Eu²⁺, ZnS:Cu, SrAMgSi₂O₇:Eu²⁺ (A = Ca, Sr, Ba), KCl, KI, KBr, NaF, NaCl, LiF, RbCl, RbBr, Rbl, MgO, SrAl₂O₄, CaAl₂O₄, Sr₁₋ₓBaₓAl₂O₄ (x = 0, 0.1, 0.2, 0.4), Sr_{0.9}Ca_{0.1}Al₂O₄, Zn₂Ge_{0.9}Si_{0.1}O₄, MgGa₂O₄, ZnGa₂O₄, ZnAl₂O₄, ZnS, ZnTe, (ZnS)₁₋ₓ(MnTe)ₓ (x < ¼), CaZnOS, BaZnOS, Ca₂MgSi₂O₇, Sr₂MgSi₂O₇, Ba₂MgSi₂O₇, SrCaMgSi₂O₇, SrBaMgSi₂O₇, SrₙMgSi₂O₅₊ₙ(1 ≤ n ≤ 2), Ca₂Al₂SiO₇, Sr₂Al₂SiO₇, CaYAl₃O₇, CaAl₂Si₂O₈, Ca₁₋ₓSrₓAl₂Si₂O₈ (x < 0.8) , SrMg₂(PO₄)₂, Ba₁₋ₓCaₓTiO₃ (0.25 < x < 0.8) , Ba₁₋ₓCaₓTiO₃, LiNbO₃, Sr₂SnO₄, (Ca, Sr, Ba)₂SnO₄, Sr₃Sn₂O_{y}, Sr₃(Sn, Si)₂O₇, Sr₃(Sn, Ge)₂O₇, Ca₃Ti₂O₇, CaNb₂O₆, Ca₂Nb₂O₇, Ca₃Nb₂O₈, BaSi₂O₂N₂, SrSi₂O₂N₂, CaZr(PO₄)₂, ZrO₂.

Yanim Jia, in "Novel Mechano-Luminescent Sensors Based on Piezoelectric/Electroluminescent Composites," Sensors (Basel). 2011; 11(4): 3962-396, describes a mechanoluminescent composite made of a piezoelectric material and an electroluminescent material. In this composite device, when a stress is applied to the piezoelectric layer, electrical charges will be induced at both the top and bottom faces of piezoelectric layer due to the piezoelectric effect. These induced electrical charges will result in a light output from the electroluminescent layer due to the electroluminescent effect.

Here, in one embodiment such composites made of a piezoelectric material and an electroluminescent material, hereinafter "composite mechano-luminescent emitters," provides a structure that, upon stimulation with mechanical or vibrational energy such as from an acoustic or ultrasonic transducer, emit light. Details of various electroluminescent materials that can be used for the composite mechano-luminescent emitters are provided in the next section where electroluminescent materials alone are placed in vicinity of the opposing resonator electrodes.

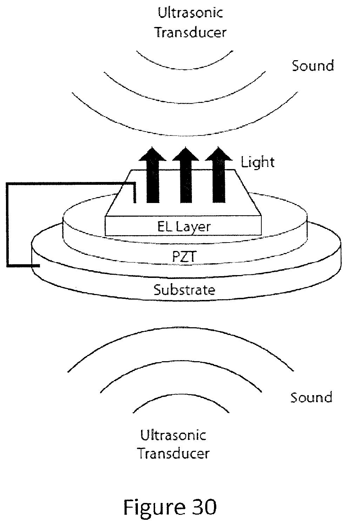

Figure 30 is a schematic of composite mechano-luminescent emitter composed of a piezoelectric material and an electroluminescent material which, in one embodiment, could be mechano-luminescent light emitters in Figure 29.

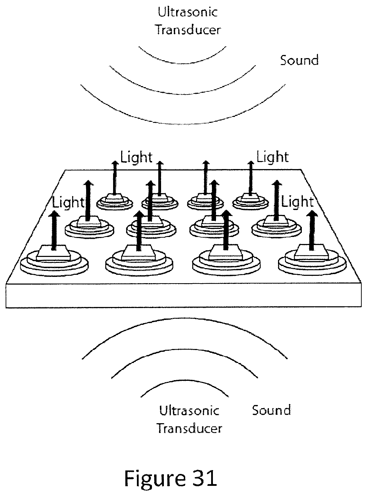

In another embodiment, the composite mechano-luminescent emitters could be used without need for any resonator structure. Figure 31 is schematic showing the composite mechano-luminescent emitters distributed across a sector of interest for generation of light therefrom. Figure 31 shows that an ultrasonic transducer can be used for stimulation/activation of these composite mechano-luminescent emitters.

In color enhancement applications, application of ultrasonic energy could change the color emission from a surface. Such applications could be for security systems where an item would contain a pattern of the composite mechano-luminescent emitters. The pattern would not be apparent until it was activated with ultrasonic or acoustic energy upon which time light of a predetermined wavelength would be emitted. The light emitted might be visible or infrared light depending on the type of detector used to detect the emitted light.

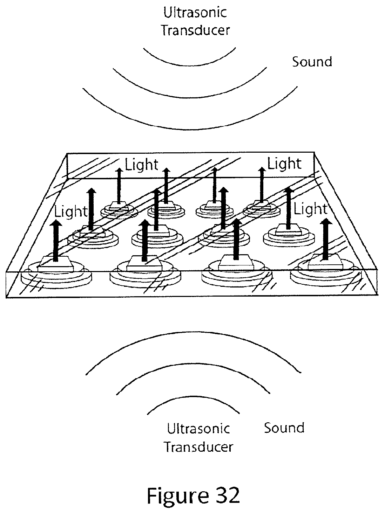

In a related application of these composite mechano-luminescent emitters, Figure 32 is schematic showing the composite mechano-luminescent emitters distributed inside a medium of interest for generation of light therein or therefrom. With the present invention, light can be turned on and off with the on/off status of an ultrasonic transducer and the intensity of the light can be varied. There are no power leads to run into the medium of interest. There is no space taken up by batteries or control elements to turn power on and off. The composite mechano-luminescent emitters can be miniaturized. The composite mechano-luminescent emitters could be agglomerated in a container. In some embodiments, the container would not be completely packed permitting the tilting of the container to relocate the composite mechano-luminescent emitters within the container.

**Electroluminescent and phosphorescent materials (organic and inorganic):** The present disclosure in various embodiments with or without energy augmentators can utilize in organic fluorescent molecules or inorganic particles capable or fluorescence and phosphorescence having crystalline, polycrystalline or amorphous micro-structures for the converters (optionally including the energy augmentation structures described above).

The list of inorganic molecules that can be used in the resonating structures to enhance the color emission include but is not limited to the following inorganic electroluminescent phosphor materials:

SrS:Ce ³⁺

CaGa₂S₄:Ce³⁺

SrS:Cu⁺

CaS:Pb²⁺

BaAl₂S₄:Eu²⁺

ZnS:Tb³⁺

ZnMgS:Mn²⁺

SrGa₂S₄:Eu²⁺

CaAl₂S₄:Eu²⁺

BaAl₂S₄:Eu²⁺

ZnS:Mn²⁺

MgGa₂O₄:Eu³⁺

(Ca, Sr)Y₂S₄:Eu²⁺

BaAl₂S₄:Eu²⁺

The organic molecules that can phosphoresce under the influence of an electric field are also of interest in the present application. The organic fluorescent compounds with high quantum yield include by way of illustration:
Naphthalene,
Pyrene,
Perylene,
Anthracene,
Phenanthrene,
p-Terphenyl,
p-Quartphenyl,
Trans-stilbene,
Tetraphenylbutadiene,
Distyrylbenzene,
2,5-Diphenyloxazole,
4-Methyl-7-diethylaminocoumarin,
2-Phenyl-5-(4-biphenyl)-1,3,4-oxadiazole,
3-Phenylcarbostyryl,
1,3,5-Triphenyl-2-pyrazoline,
1,8-Naphthoylene -1', 2'-bezimidazole,
4-Amino-N-phenyl-naphthalimide.

The inorganic fluorescent and phosphorescent materials detailed here are numerous, and various examples are given by way of illustration rather than limitation. Furthermore,, these materials can be doped with specific ions (activators or a combination of activators) that occupy a site in the lattice structure in the case of crystalline or polycrystalline materials and could occupy a network forming site or a bridging and/or non-bridging site in amorphous materials. These compounds could include (not ranked by order of preference or utility) the following material examples:

CaF₂, ZnF₂, KMgF₃, ZnGa₂O₄, ZnAl₂O₄, Zn₂SiO₄, Zn₂GeO₄, Ca₃(PO₄)₃F, Sr₃(PO₄)₃F, CaSiO₃, MgSiO₃, ZnS, MgGa₂O₄, LaAl₁₁O₁₈, Zn₂SiO₄, Ca₅(PO₄)₃F, Mg₄Ta₂O₉, CaF₂, LiAl₅O₈, LiAlO₂, CaPO₃, AlF₃.

Further included are alkali earth chalcogenide phosphors which are in turn exemplified by the following non-inclusive list:

MgS:Eu³⁺, CaS:Mn²⁺, CaS:Cu, CaS:Sb, CaS:Ce³⁺, CaS:Eu²⁺, CaS: Eu²⁺ Ce³⁺, CaS: Sm³⁺, CaS:Pb²⁺, CaO:Mn²⁺, CaO:Pb²⁺.

The examples include the ZnS type phosphors that encompass various derivatives:

ZnS:Cu,Al(Cl), ZnS:Cl(Al), ZnS:Cu,I(Cl), ZnS:Cu, ZnS:Cu,In.

Compound IIIb-Vb phosphors which include the group IIIb and Vb elements of the periodic table are suitable for converter materials.. These semiconductors include BN, BP, BSb, AlN, AlP, AlAs, AlSb, GaN, GaP, GaAs, GaSb, InN, InP, InAs, InSb and these materials have donors and acceptors that work in together to induce light emission diodes. The donors include Li, Sn, Si, Li, Te, Se, S, O, and acceptors include C, Be, Mg, Zn, Cd, Si, Ge. As an example, GaP light emitting diodes include GaP:Zn, O, GaP:NN, Gap:N and GaP which emit colors Red, Yellow, Green and Pure Green respectively.

The compounded materials further include such materials as GaAs with compositional variation of the following sort: In1-y(Ga1-xAlx)yP (provides a simple example) Silicon Carbide SiC as a luminescent platform has commercial relevancy if the blue light emitting diodes. These include the polytypes 3C-SiC, 6H-SiC, 4H-SiC with donors such as N and Al and acceptors such as Ga and B.

Multiband luminescent materials suitable for converter materials include for example the following compositions:

(Sr, Ca, Ba)₃(PO₄)₃Cl:Eu²⁺, BaMg₂Al₁₆O₂₇:Eu²⁺, CeMgAl₁₁O₁₉:Ce³⁺:Tb³⁺, LaPO₄:Ce³⁺:Tb³⁺, GdMgB₅O₁₀:Ce³⁺:Tb³⁺, Y₂O₃:Eu³⁺, (Ba,Ca,Mg)₃(PO₄)₃Cl:Eu²⁺, 2SrO_{0.84}P₂O₃·0.16B₂O₃:Eu^{2÷}, Sr₄Al₁₄O₂₅:Eu²⁺.

Other materials suitable for converter materials include those materials used for fluorescent high pressure mercury discharge lamps can be excited with X-Ray and are exemplified by way of family designation as follows:
Phosphates (Sr, M)(PO₄)₂:Sn²⁺, Mg or Zn activator, Germanate 4MgO.GeO₂:Mn⁴⁺, 4(MgO, MgF₂)GeO₂:Mn⁴⁺, Yttrate Y₂O₃:Eu³⁺, Vanadate YVO₄:Eu³⁺, Y(P,V)O₄:Eu³⁺, Y(P,V)O₄:In⁺, Halo-Silicate Sr2Si3O₈·2SrCl₂:Eu²⁺, Aluminate (Ba,Mg)₂Al₁₆O₂₄:Eu²⁺, (Ba, Mg)₂Al₁₆O₂₄:Eu²⁺,Mn²⁺, Y₂O₃Al₂O₃:Tb³⁺.

Another grouping of materials suitable for converter materials by host compound include chemical compositions in the Halophosphates phosphors, Phosphate phosphors, Silicate phosphors, Aluminate phosphors, Borate phosphors, Tungstate phosphors, and other phosphors.

The halophosphates include by way of illustration:
3Ca₃(PO₄)₂.Ca(F,Cl)₂:Sb³⁺, 3Ca₃(PO₄)₂.Ca(F,Cl)₂:Sb³⁺/Mn²⁺, Sr₁₀(PO₄)₆Cl₂:Eu²⁺, (Sr,Ca)₁₀(PO₄)₆Cl₂:Eu²⁺, (Sr,Ca)₁₀(PO₄)₆.nB₂O₃:Eu³⁺, (Sr, Ca,Mg)₁₀(PO₄)₆Cl₂:Eu²⁺. The phosphate phosphors include by way of illustration Sr₂P₂O₇:Sn²⁺, (Sr,Mg)₃(PO₄)₂:Sn²⁺, Ca₃(PO₄)₂.Sn²⁺, Ca₃(PO₄)₂:Tl⁺, (Ca,Zn)₃(PO₄)₂:Tl⁺, Sr₂P₂O₇:Eu²⁺, SrMgP₂O₇:Eu²⁺, Sr₃(PO₄)₂:Eu²⁺, LaPO₄:Ce³⁺, Tb³⁺, La₂O₃·0.2SiO₂·0.9P₂O₅:Ce³⁺.Tb³⁺, BaO·TiO₂·P₂O₅. The silicate phosphors Zn₂SiO₄:Mn²⁺, CaSiO₃:Pb²⁺/Mn²⁺, (Ba, Sr, Mg)·3Si₂O₇:Pb²⁺, BaSi₂O₅:Pb²⁺, Sr₂Si₃O₈·2SrCl₂:Eu²⁺, Ba₃MgSi₂O₈:Eu²⁺ (Sr,Ba)Al₂Si₂O₈:Eu²⁺.

The aluminate phosphors include:

LiAlO₂:Fe³⁺, BaAl₈O₁₃:Eu²⁺, BaMg₂Al₁₆O₂₇:Eu²⁺, BaMg₂Al₁₆O₂₇:Eu²⁺/Mn²⁺, Sr₄Al₄O₂₅:Eu²⁺, CeMgAl₁₁O₁₉:Ce³⁺/Tb³⁺.

The borate phosphors include:

Cd₂B₂O₅:Mn²⁺, SrB₄O₇F:Eu²⁺, GdMgB₅O₁₀:Ce³⁺/Tb³⁺, GdMgB₅O₁₀:Ce³⁺/Mn³⁺, GdMgB₅O₁₀:Ce³⁺/Tb³⁺/Mn²⁺.

The tungstate phosphors include:
CaWO₄, (Ca,Pb)WO₄, MgWO₄. Other phosphors Y₂O₃:Eu³⁺, Y(V,P)O₄:Eu²⁺, YVO₄:Dy³⁺, MgGa₂O₄:Mn²⁺, 6MgO·As₂O₅:Mn²⁺, 3.5MgO·0.5MgF₂·GeO₂:Mn⁴⁺.

Activators of relevance to the various doped phosphors include the following list:
Tl⁺, Pb²⁺, Ce³⁺, Eu²⁺, WO₄²⁻, Sn²⁺, Sb³⁺, Mn²⁺, Tb³⁺, Eu³⁺, Mn⁴⁺, Fe³⁺.

In various embodiments, the luminescence center Tl+ can be used with a chemical composition such as:

(Ca,Zn)₃(PO₄)₂:Tl⁺, Ca₃(PO₄)₂:Tl⁺.

Similarly, the luminescence center Mn2+ can be used with chemical compositions such as

MgGa₂O₄:Mn²⁺, BaMg₂Al₁₆O₂₇:Eu²⁺/Mn²⁺, Zn₂SiO₄:Mn²⁺, 3Ca₃(PO₄)₂·Ca(F,Cl)₂:Sb²⁺/Mn²⁺, CaSiO₃:Pb²⁺/Mn²⁺, Cd₂B₂O₅:Mn²⁺, CdB₂O₅:Mn²⁺, GdMgB₅O₁₀:Ce³⁺/Mn²⁺, GdMgB₅O₁₀:Ce³⁺/Tb³⁺/Mn²⁺.

Further, the luminescence center Sn²⁺ can be used with chemical compositions such as:

Sr₂P₂O₇:Sn²⁺, (Sr,Mg)₃(PO₄)₂:Sn²⁺.

The luminescence center Eu²⁺ can also be used with chemical compositions such as:

SrB₄O₇F:Eu²⁺, (Sr,Ba)Al₂Si₂O₈:Eu²⁺, Sr₃(PO₄)₂:Eu²⁺, Sr₂P₂O₇:Eu²⁺, Ba₃MgSi₂O₈:Eu²⁺, Sr₁₀(PO₄)₆Cl₂:Eu²⁺, BaMg₂Al₁₆O₂₇:Eu²⁺/Mn²⁺, (Sr,Ca)₁₀(PO₄)₆Cl₂:Eu²⁺.

The luminescence center Pb²⁺ can be used with chemical compositions such as:

(Ba,Mg,Zn)₃Si₂O₇:Pb²⁺, BaSi₂O₅:Pb²⁺, (Ba,Sr)₃Si₂O₇:Pb²⁺.

The luminescence center Sb²⁺ can be used with chemical compositions such as:

3Ca₃(PO₄)₂·Ca(F,Cl)₂:Sb³⁺, 3Ca₃(PO₄)₂·Ca(F,Cl)₂:Sb³⁺/Mn²⁺.

The luminescence center Tb3+ can be used with chemical compositions such as:

CeMgA₁₁O₁₉:Ce³⁺/Tb³⁺, LaPO₄:Ce³⁺/Tb³⁺, Y₂SiO₅:Ce³⁺/Tb³⁺, GdMgB₅O₁₀:Ce³⁺/Tb³⁺.

The luminescence center Eu³⁺ can be used with chemical compositions such as:

Y₂O₃:Eu³⁺, Y(V,P)O₄:Eu³⁺.

The luminescence center Dy³⁺ can be used with chemical compositions such as:

YVO₄:Dy³⁺.

The luminescence center Fe³⁺ can be used with chemical compositions such as:

LiAlO₂:Fe³⁺.

The luminescence center Mn⁴⁺ can be used with chemical compositions such as:

6MgO·As₂O₅:Mn⁴⁺, 3.5MgO·0.5MgF₂·GeO₂:Mn⁴⁺.

The luminescence center Ce³⁺ can be used with chemical compositions such as:

Ca₂MgSi₂O₇:Ce³⁺ and Y₂SiO₅:Ce³⁺.

The luminescence center WO₄²⁻ can be used with chemical compositions such as:

CaWO₄, (Ca,Pb)WO₄, MgWO₄.

The luminescence center TiO₄⁴⁻ can be used with chemical compositions such as:

BaO·TiO₂·P₂O₅.

In various embodiments of this invention, the phosphor chemistry utilized in X-Ray excitations can be used. Of particular interest is the k-edge of these phosphors. Low energy excitation can lead to intense luminescence in materials with low k-edge. Some of these chemistries and the corresponding k-edge are included as follows:

| | |
|---|---|
| BaFCl:Eu²⁺ | 37.38 keV |
| BaSO₄:Eu²⁺ | 37.38 keV |
| CaWO₄ | 69.48 keV |
| Gd₂O₂S:Tb³⁺ | 50.22 keV |
| LaOBr:Tb³⁺ | 38.92 keV |
| LaOBr:Tm³⁺ | 38.92 keV |
| La₂O₂S:Tb³⁺ | 38.92 keV |
| Y₂O₂S:Tb³⁺ | 17.04 keV |
| YTaO₄ | 67.42 keV |
| YTaO₄:Nb | 67.42 keV |
| ZnS:Ag | 9.66 keV |
| (Zn,Cd)S:Ag | 9.66/26.7 keV |

### PHOTOBIOMODULATION

This disclosure provides methods, systems and devices which use phosphorescing, fluorescing and scintillating materials or other of the energy conversion materials and devices described herein, with and without the energy augmentators described above for increasing the effectiveness and/or the efficiency of light emission in a subject or medium being treated by X-ray radiation or a particle beam. The invention also pertains to methods and structures for assembling nano-particles to increase their net light output under excitation. With the energy augmentators, this invention can generate local regions of intense electric fields to enhance or accelerate light, or photon, or electron emission of materials in proximity to those local regions. The methods described in this disclosure are not a part of the invention and are disclosed for illustrative purposes only.

Light modulation from a deeply penetrating radiation like X-ray to a photo-catalytic radiation like UV, opens the possibility for activating bio-therapeutic agents of various kinds within mammalian bodies. Other possibilities include the activation of photo-catalysts in mediums for cross-linking reactions in polymeric chains and polymer based adhesives. These examples are but two examples of a number of possibilities that can be more generally described as the use of a conversion material to convert an initiating radiation that is deeply penetrating to another useful radiation possessing the capability of promoting photo-based chemical reactions. The photo-chemistry is driven inside mediums of far ranging kinds including organic, inorganic or composited from organic and inorganic materials.

The photo-activation with no line of site required can be done *in-vivo* and *ex-vivo* such as those carried out in cell cultures. In turn, the photo activation of select bio-therapeutic agent, and conceivably more than one agent at a time, can lead to the onset of a desirable chemical reaction, or a cascade of reactions, that in turn lead to a beneficial therapeutic outcome. As an example, the binding of psoralen to DNA through the formation of monoadducts is well known to engender an immune response if done properly. An in-depth treatise of the subject is available in the open literature. Psoralen under the correct photo-catalytic light gains the aptitude to bind to DNA. Psoralen has been reported to react to other sites that have a suitable reactivity including and not limited to cell walls. If this reaction is of the correct kind, as is the case for psoralen-DNA monoadducts formation, the binding leads to a programmable cell death referred to as Apoptosis. Such programmable cell death, if accomplished over a sufficiently large cell population, can signal the body to mount an immune response enabling target specific cell kill throughout the body. Such immune response is of the upmost importance for various medical treatments including cancer cure.

The cascade of events described above has at its source the modulation of electromagnetic energy from the X-ray to the UV energy using phosphors in the presence of bio-therapeutic agents; these methods and the like, have been thoroughly described in various patents and patent applications such as those listed in the cross-reference section above.

In particular, in U.S. Serial No. 11/935,655, entitled "METHODS AND SYSTEMS FOR TREATING CELL PROLIFERATION DISORDERS," the use of a phosphorescent emitting source was described with the advantage of phosphorescent emitting molecules or other source may be electroactivated or photoactivated prior to insertion into the tumor either by systemic administration or direct insertion into the region of the tumor. Phosphorescent materials have longer relaxation times than fluorescent materials. Energy emission is delayed or prolonged from a fraction of a second to several hours. Otherwise, the energy emitted during phosphorescent relaxation is not otherwise different than fluorescence, and the range of wavelengths may be selected by choosing a particular phosphor.

In particular, in U.S. Serial No. 12/401,478, entitled "PLASMONIC ASSISTED SYSTEMS AND METHODS FOR INTERIOR ENERGY-ACTIVATION FROM AN EXTERIOR SOURCE," the use of phosphorescent materials as energy converters was described. The '478 application details a number of converters some having a very short energy retention time (on the order of fs-ns, e.g. fluorescent molecules) whereas others having a very long half-life (on the order of seconds to hours, e.g. luminescent inorganic molecules or phosphorescent molecules). Specific types of energy converters described in the '478 application included Y₂O₃; ZnS; ZnSe; MgS; CaS; Mn, Er ZnSe; Mn, Er MgS; Mn, Er CaS; Mn, Er ZnS; Mn,Yb ZnSe; Mn,Yb MgS; Mn, Yb CaS; Mn, Yb ZnS:Tb³⁺, Er³⁺; ZnS:Tb³⁺; Y₂O₃:Tb³⁺; Y₂O₃:Tb³⁺, Er3⁺; ZnS:Mn²⁺; ZnS:Mn,Er³⁺.

The treated condition, disorder, or disease may or may not be significantly mediated by abnormal cellular proliferation and said predetermined change does not have to substantially affect cellular proliferation with and without energy augmentators.

The target structure need not be present inside an organism, but may be one in vitro or ex vivo. The predetermined change may enhance the expression of, promote the growth of, or increase the quantity of the target structure; or the predetermined change can enhance, inhibit or stabilize the usual biological activity of the target structure compared to a similar untreated target structure. For example, the predetermined change with and without energy augmentators can alter the immunological or chemical properties of the target structure which may be a cell, cell membrane, internal cellular structure, polypeptide or non-polypeptide compound which can be modified by said predetermined change to be more or less antigenic or immunogenic. In another embodiment, modifying the target structure can be done without the need for a pharmaceutical agent.

In one embodiment, there is provided a system for light stimulation within a medium. The system has a reduced-voltage x-ray source configured to generate x-rays from a peak applied cathode voltage at or below 105 kVp, and a first plurality of energy-emitting particles in the medium which, upon radiation from the x-ray source, radiate with and without energy augmentators at a first lower energy than the x-ray source to interact with the medium or with at least one photoactivatable agent in the medium.

In one embodiment, there is provided a method for light stimulation within a medium. The method includes introducing a first plurality of energy-emitting particles into the medium, radiating the first plurality of energy-emitting particles in the medium with x-rays generated from a peak applied cathode voltage at or below 105 kVp, and emitting with and without energy augmentators a first lower energy than the x-ray source to interact with the medium or with at least one photoactivatable agent in the medium..

In one embodiment, there is provided a system for modulating biological activity within a medium with and without energy augmentators. The system includes a reduced-voltage x-ray source configured to generate x-rays from a peak applied cathode voltage at or below 105 kVp, and a plurality of energy-emitting particles in the medium which, upon radiation from the x-ray source, radiate at a lower energy than the x-ray source to alter the biological activity of the medium.

In one embodiment, there is provided a method for modulating biological activity within a medium with and without energy augmentators. The method includes introducing a plurality of energy-emitting particles into the medium, radiating the plurality of energy-emitting particles in the medium with x-rays generated from a peak applied cathode voltage at or below105 kVp, and emitting a first lower energy than the x-ray source to alter the biological activity of the medium.

In one embodiment, there is provided a system for light stimulation within a medium with and without energy augmentators. The system includes an initiation source configured to radiate an initiation energy, a first plurality of energy-emitting particles in the medium which (upon radiation from the initiation source) radiate at a first lower energy than the initiation source to interact with at least one photoactivatable agent in the medium, and a second plurality of energy-emitting particles in the medium which (upon radiation from the initiation source) radiate at a second lower energy than the initiation source to interact with at least one photoactivatable agent in the medium. A combination of emission from the first and second plurality of energy-emitting particles produces a spectrum for illumination of the at least one photoactivatable agent in the medium. The spectrum has a wavelength distribution simulating at least a part of an absorption spectrum of the at least one photoactivatable agent.

In one embodiment, there is provided a method for light stimulation within a medium v. The method includes introducing a first plurality of energy-emitting particles in with and without energy augmentators radiating the first and second plurality of energy-emitting particles in the medium with an initiation energy, emitting from the first and second plurality of energy-emitting particles a first lower energy than the initiation energy and a second lower energy than the initiation energy to interact with at least one photoactivatable agent in the medium. A combination of emission from the first and second plurality of energy-emitting particles produces a spectrum for illumination of the at least one photoactivatable agent in the medium. The spectrum has a wavelength distribution simulating at least a part of an absorption spectrum of the at least one photoactivatable agent.

In one embodiment, there is provided a system for light stimulation within a medium with and without energy augmentators. The system includes a first plurality of light-emitting particles which upon encountering an initiating excitation of light energy or particle beam energy radiate a first output energy having photocatalysis potential to activate photoactivatable agents in the medium, and a second plurality of light-emitting particles which upon encountering the initiating excitation of light energy or particle beam energy radiate a second output energy complementary to the first output. A combination of energy emission from the first and second plurality of energy emitting particles produces a combined energy capable of activating chemical agents inside the medium.
In one embodiment, there is provided a method for light stimulation within a medium with and without energy augmentators. The method includes introducing a first plurality of light-emitting particles into the medium, introducing a second plurality of light-emitting particles into the medium, exposing the first plurality of light-emitting particles to an initiating excitation of light energy or particle beam energy to produce from the first plurality of light-emitting particles a first output energy having photocatalysis potential to activate at least one photoactivatable agent in the medium, and exposing the second plurality of light-emitting particles to an initiating excitation of light energy or particle beam energy to produce from the second plurality of light-emitting particles a second output energy complementary to the first output. A combination of energy emission from the first and second plurality of energy emitting particles produces a combined energy capable of activating chemical agents inside the medium. With the energy augmentators, this invention can generate local regions of intense electric fields to enhance light emission from the first and second plurality of energy emitting particles in proximity to those local regions.

In one embodiment, there is provided a system for light stimulation within a medium with and without energy augmentators. The system has a first plurality of light-emitting particles which upon encountering an appropriate initiating excitation of light energy or particle beam energy radiate with and without energy augmentation an output energy having photocatalysis potential to activate photoactivatable agents with minimized impact on the medium. The system further has a second plurality of light-emitting particles which upon encountering the same initiating excitation of light energy or particle beam energy radiate an output energy complementary to the output of the first set of particles. With the energy augmentators, this invention can generate local regions of intense electric fields to enhance light emission from the light emitting particles in proximity to those local regions.

In one embodiment, there is provided a system for light stimulation within a medium with and without energy augmentators. The system has an x-ray source positioned at a distance from the medium and configured to generate radiation within an energy band bounded by a lower energy threshold capable of inducing desirable reactions and an upper energy threshold leading to denaturization of the medium. The system has an x-ray source control device configured to 1) calculate an x-ray exposure condition including the distance and the above-noted energy band and 2) operate the x-ray source within the x-ray exposure condition. The system has a plurality of energy-emitting particles in the medium which, upon radiation from the x-ray source with energy above the lower energy threshold, radiate with and without energy augmentation at a first lower energy than the x-ray source to interact with the medium or with at least one photoactivatable agent in the medium. With the energy augmentators, this invention can generate local regions of intense electric fields to enhance light emission from the plurality of energy-emitting particles in proximity to those local regions.

The computer-implemented system according to one embodiment of the present invention may have a central processing unit (CPU) connected to a memory unit, configured such that the CPU is capable of processing user inputs and selecting a combination of initiation source (or initiation energies or distances), activatable pharmaceutical agent, and energy transfer agents for use in a method of the present invention.

The computer-implemented system according to one embodiment of the present invention includes (or is programmed to act as) an x-ray source control device configured to calculate an x-ray exposure condition including a distance between the initiation energy source 1 and the subject 4 and the energy band bounded by the above-noted lower energy threshold capable of inducing desirable reactions and the above-noted upper energy threshold leading to denaturization of the medium. The x-ray source control device operates the x-ray source (the initiation energy source 1) within the x-ray exposure condition to provide a requisite energy and/or dose of x-rays to the subject or a target site of the subject with and without energy augmentation.

Light intensity plays a substantial role in photo-catalysis. The more light intensity that is available, the higher the chance of activating reactions that are suitable for photo-activation. Conversely, the lower the intensity, the lesser the chance of activating chemical reactions. In other words, usually, the photonic flux at a sufficient intensity (number of photons per unit time) is necessary to trigger reactions. The energy augmentators of this invention serve to enhance the photon flux or photons per nit time and/or serve to collect from (or distribute to) treatment sites photons for trigger of the photo-induced reactions.

Besides light intensity, a minimum level of spectral matching between the radiation(s) emanating from the conversion media and the radiation that can be absorbed by the photocatalyst being targeted is desired. In other words, the emitted radiation has to be suitable to the absorption of the chemical species under consideration.

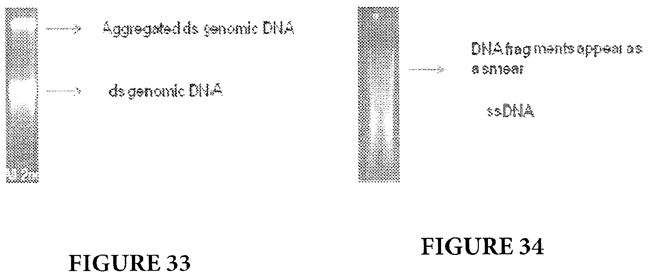

Prior work has shown the effect of psoralen on crosslinking DNA and showed the effectiveness of light modulating particles (phosphors, scintillators and combinations thereof) under X-Ray irradiation. Of particular interest were the crosslinking signals associated with DNA and in particular having a minimize effect of denaturing DNA while maximizing the density of desirable crosslinks such as those needed to engender an immune response. This effect is described below for the sake of completeness in the discussion associated with Figures 33-43, with the present invention expected to enhance the effectiveness of the light-induced cross-linking.

Gel electrophoresis is method for qualitatively analyzing DNA crosslinking. If no denaturing conditions are applied, then an observable pattern consisting of an aggregation of double stranded genomic DNA (or ds genomic DNA) are present as illustrated in Figure 33. On the other hand, if denaturing conditions are applied, then an observable signal represented by a smear pattern is observed since a distribution of species are present, not just a single stranded DNAs illustrated in Figure 34.

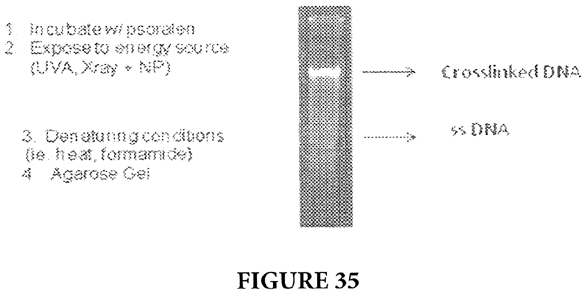

DNA was incubated with psoralen then exposed to X-Ray energy in the presence of nano particles and a biotherapeutic agent. Denaturing conditions were then applied in the form of heat, formamide. Agarose gel having an electric field gradient was used to force DNA to travel through its pores by a diffusion process. The signals resulting from the ds DNA and ss DNA are then recorded using the fluorescent dye technique described above (as illustrated in Figure 35). The intensity of the gel is directly related to the mass loading.

A DNA crosslinking test plan was devised for using X-Ray radiation as the initiating crosslinking radiation. The experimental space was mapped out, and variables were altered as part of the experimental plan. This work showed surprising results in that more ssDNA was generated at higher X-Ray intensity. The solutions were prepared using a Total volume per glass vial (2 mL DNA solution + AMT or phosphors). Dissolved stock lyophilized DNA (2 mg) in 20 mL of 1X PBS. The drug concentrations of AMT were kept at a fixed concentration of 0.1 ΦM. The phosphors were added to the solution as follows: 0.1 mg/mL final concentration in DNA. This was obtained by creating a suspension of 1 mg/mL BP7c suspension in PBS, adding 200 ΦL suspension to vial of 2mL DNA+TMPS solution and finally adding 200 ΦL suspension to vial of 2 mL DNA+AMT solution. After treatment, all the vials were transferred to ice, covered from the light, and stored in cold room on wet ice prior to the gel electrophoresis measurements.

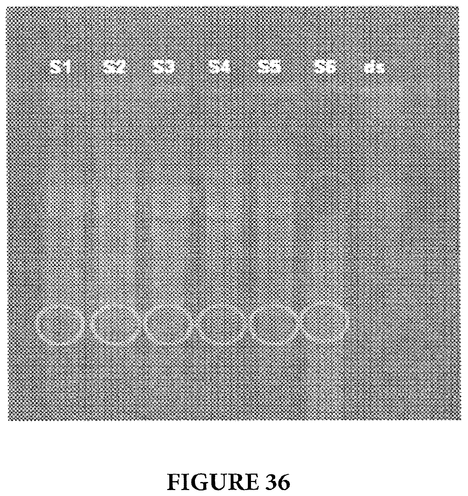

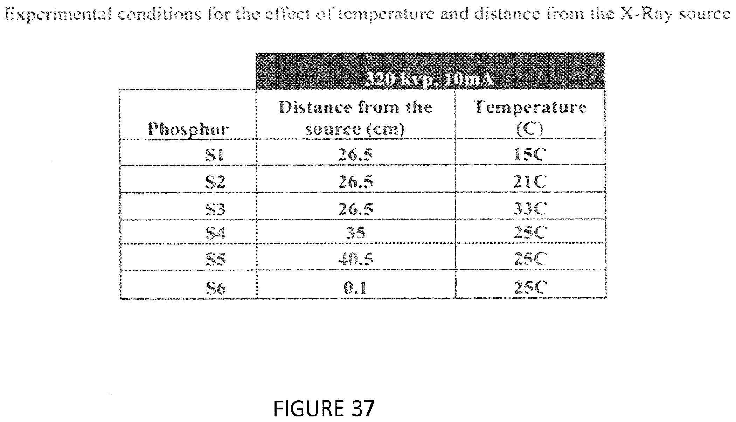

Figure 36 shows the gel electrophoresis results post DNA crosslinking attempts under X-Ray radiation and using temperature and distance from the source as variables. The experimental conditions are provided in Table 1 shown in Figure 37.

All the experiments were conducted using a constant source voltage and amperage. Sample S6 in Figure 37 had the most energy input from the irradiator. As shown in Figure 34, sample condition S6 revealed that more X-Ray intensity yielded more ssDNA than other conditions of lesser energy inputs. Production of ssDNA is the less desirable result. As noted above, the generation of more ssDNA at higher X-Ray intensity was the unexpected result.

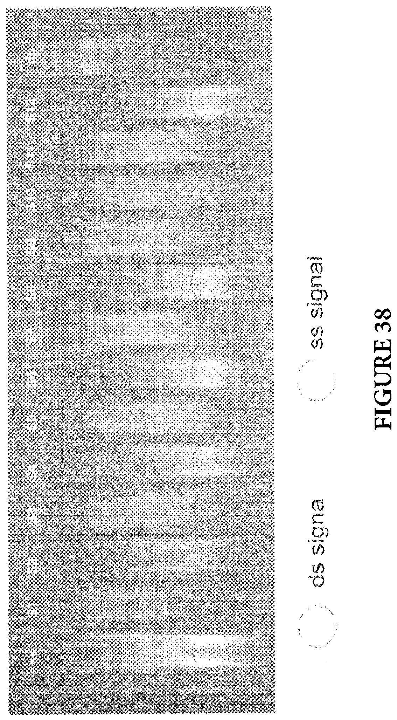

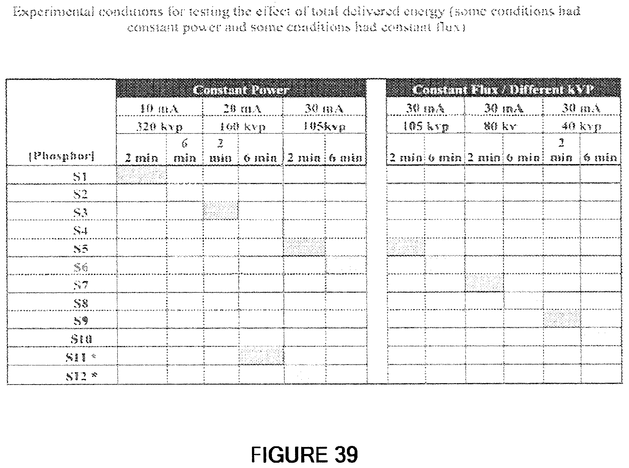

Figure 38 illustrates the results from gel electrophoresis post DNA. crosslinking attempts using various experimental conditions. Figure 39 shows Table 2 providing the experimental conditions for testing the effect of total delivered energy (some conditions had constant power and some conditions had constant flux).

The total delivered energy was an experimentally designed variable. The power was maintained constant by varying kVp (peak voltage on the x-ray cathode) and filament current accordingly. The impact of a constant flux was tested. For each of these conditions, time was fixed in two major intervals: e.g., a two minutes duration or a six minutes duration. As shown in Figure 36, all the two minute runs (regardless of the flux and kVp conditions) showed a strong ds DNA signal. On the other hand, all the six minute runs (regardless of the flux and kVp conditions) showed a strong ss-DNA signal. In effect the total energy delivered to the system makes a substantial difference in the formation of ss-DNA versus ds-DNA. Though the DNA crosslinking test is qualitative rather than quantitative, the exhibited trend is clear and unambiguous. More energy leads to the formation of smaller molecular weight species from the original DNA.

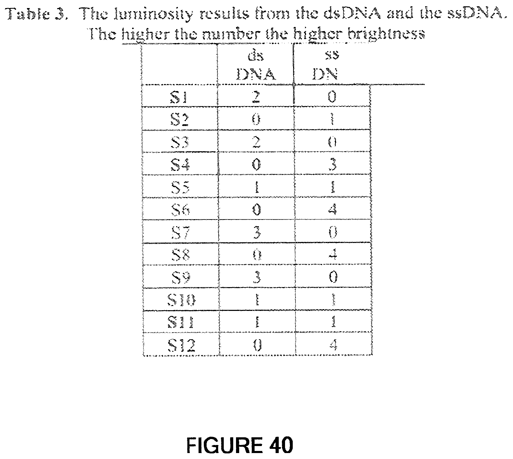

A visual ranking of brightness from the electrophoresis technique was adopted to rank the various conditions. The results are tabulated in Table 3 shown in Figure 40.

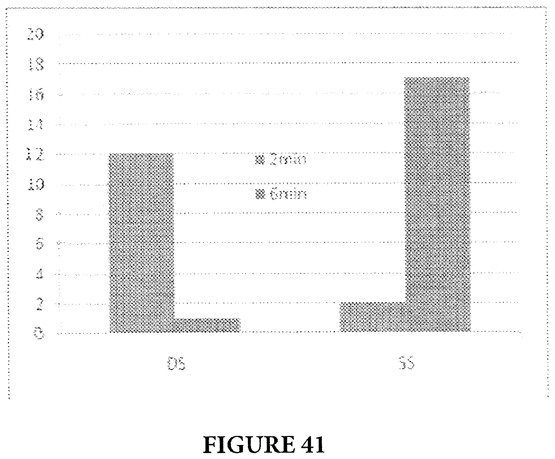

The sum total of all the brightness results in the "ds" column and the sum total of all the brightness in the "ss" column are plotted in Figure 41 for the two duration periods applied during the test. Clearly, the two minute duration X-ray irradiation treatments lead to more ds-DNA, and the six minute duration X-ray irradiation treatments lead to more ss-DNA.

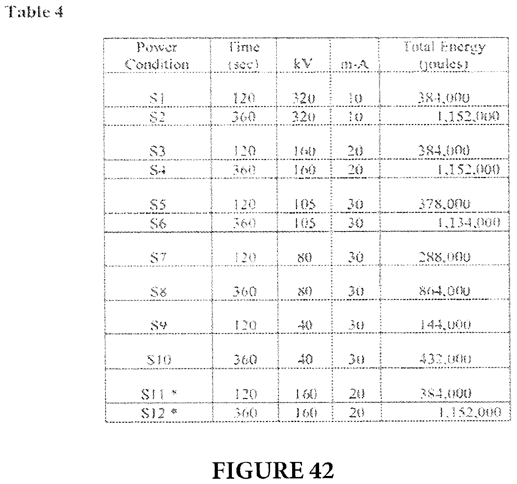

The total energy delivered during the X-Ray treatments was calculated by integrating the power delivery over the time period by multiplying the voltage and the amperage, as illustrated in Table 4 shown in Figure 42.

In order to test the impact of phosphor loading, a series of phosphor loadings were prepared for testing. The X-ray treatment was kept at two minutes for the conditions in this experiment (for the sake of confirming the repeatability of the fact that the lower level of energy delivery leads to ds-DNA signal). The phosphor concentration was varied from 0.1mg/ml to 0.15 mg/ml and 0.18mg/ml.

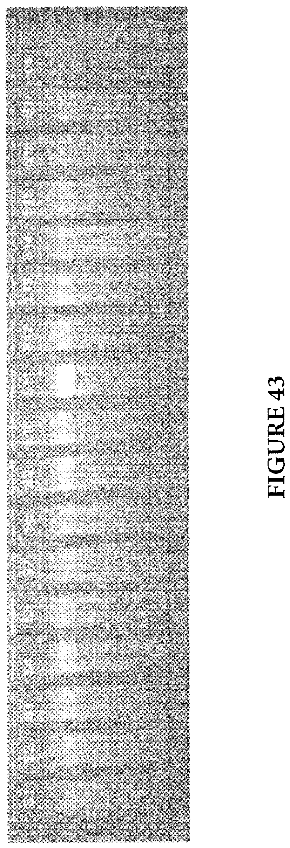

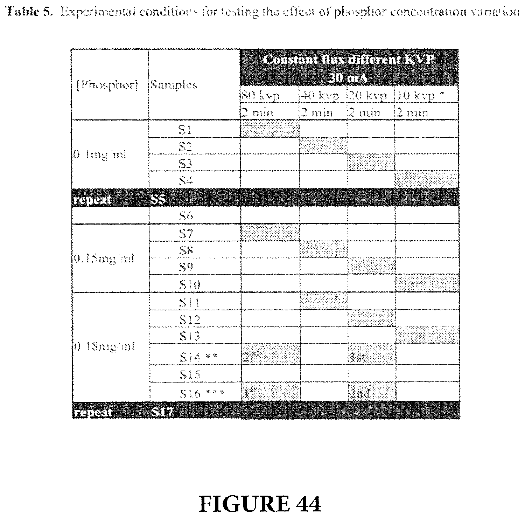

Figure 43 illustrates the results from gel electrophoresis post DNA crosslinking attempts using varying phosphor concentrations at kVp values at or below 80 kVp. As illustrated in Figure 43, the ds-DNA signal can be observed across the entire series of samples treated according to the experimental conditions in Table 5 (shown in Figure 44). This reinforces the utility of lower incident energy levels to avoid generating ssDNA.

Furthermore as illustrated in Figure 44, going to lower kVp values during the irradiation treatment can further be demonstrated in this subgroup from Table 5.

Sample S4 treated using 10 kVp exhibits a relatively stronger ds-DNA signal than S1 which was treated using 80 kVp. The lower the kVp results in stronger observable ds-DNA signal for the phosphor in 0.1mg/mL final concentration in DNA. The comparison of S1, S2, S3 and S4 conditions further reinforces that lower kVp values are helpful to the crosslinking process.

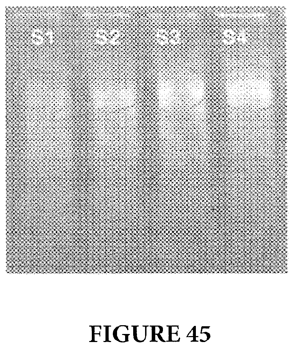

As illustrated in Figure 45, the condition that led to most crosslinking was sample S11. The phosphor in this case is 0.18 mg/mL final concentration in DNA and crosslinks best at 40 kVp. Besides the positive results at 80 kVp and below, positive results at 105 kVp have been obtained.

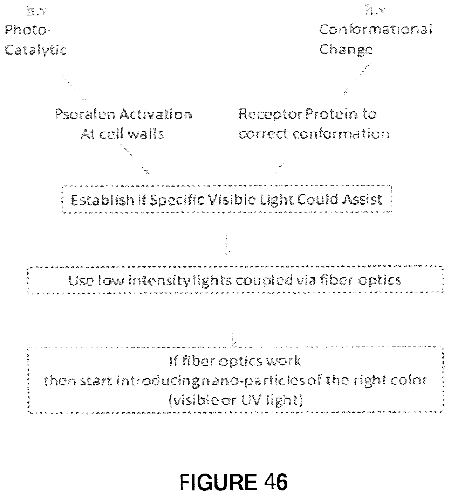

A non-limiting illustration of how photo-catalytic light can work cooperatively with non-ionizing radiation to potentiate the activation of bio-therapeutics is provided in Figure 46.

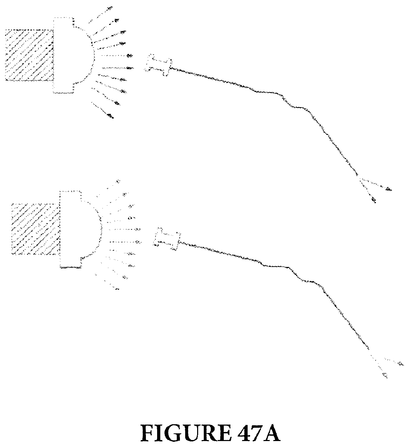

Prior work used a test set up to permit channeling of external radiation source into the x-ray radiation system as illustrated in Figure 47A.

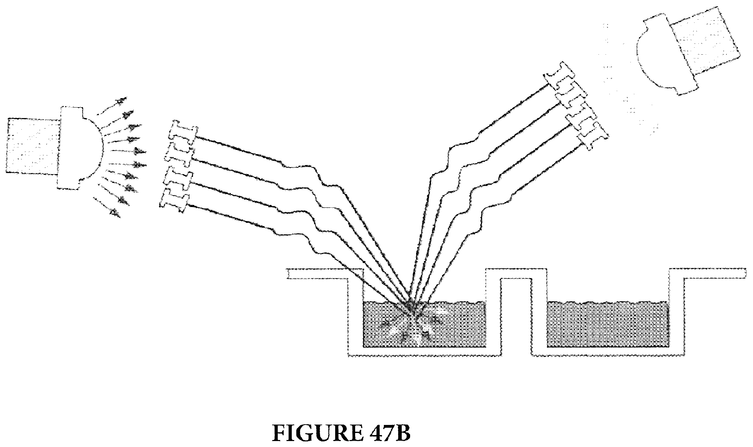

The coupled fibers coupled red light and while light, UV light, and LASER light (from outside the irradiator) to the inside of the irradiator where the X-Ray energy was turned on. Figure 47B provides an illustration of the coupled fiber permitting different wavelengths of ionizing and non-ionizing radiation to be applied in conjunction with X-Ray. In this invention while the sample depicted in Figure 47B is inside a petri dish, the concept relates to any sample regardless of the environment (including in vivo) where the activation occurs.

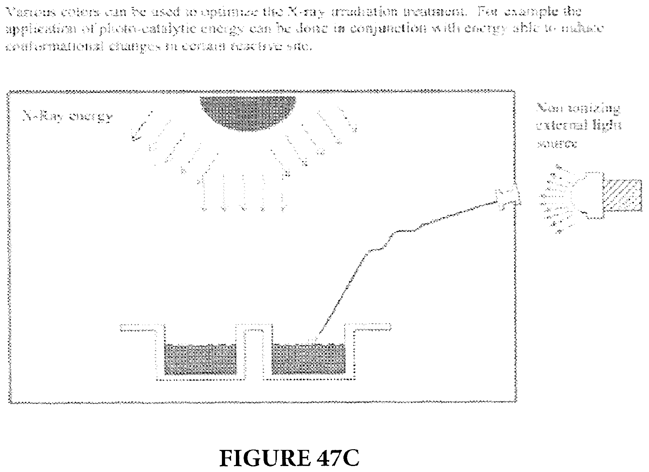

In one embodiment of this invention, various colors can be used to optimize an X-ray irradiation treatment. For example, the application of photo-catalytic energy can be done in conjunction with energy able to induce conformational changes in certain reactive site (i.e., a target site). Figure 47C illustrates the combination of X-Ray and a fiber optic allowing the simultaneous use of X-Ray energy with external light sources having potentiating effects. In one embodiment of the invention, the light from the external light source interacts with the energy augmentators of this invention, and in particular infrared light interacting with the folded resonator structures described above.

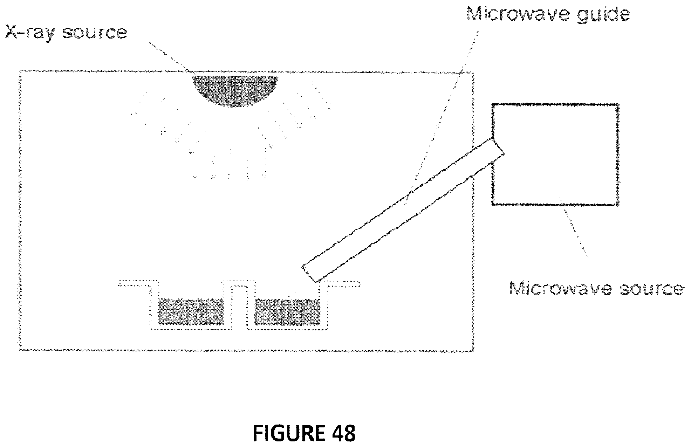

Figure 48 illustrates the combination of X-Ray and a microwave guide allowing the simultaneous use of X-Ray energy and microwave energy to interact with a target or reactive site. In one embodiment of the invention, the microwave energy interacts with the energy augmentators of this invention, and in particular the folded resonator structures described above.

Accordingly, as noted above, in one embodiment of this invention, there is provided a system or method for light stimulation within a medium. The system has a reduced-voltage x-ray source configured to generate x-rays from a peak applied cathode voltage at or below 105 kVp, and a first plurality of energy-emitting particles in the medium which, upon radiation from the x-ray source, radiate at a first lower energy than the x-ray source to interact with photoactivatable agent(s) in the medium with or without energy augmentation. The method accordingly introduces a first plurality of energy-emitting particles into the medium, radiates the first plurality of energy-emitting particles in the medium with x-rays generated from a peak applied cathode voltage at or below 105 kVp, and emits a first lower energy than the x-ray source to interact with photoactivatable agent(s) in the medium. In various aspects to the invention the peak applied cathode voltage is at or below 120 kVp, is at or below 105 kVp, is at or below 70 kVp, is at or below 60 kVp, is at or below 50 kVp, is at or below 40 kVp, is at or below 30 kVp, or is at or below 20 kVp, or is at or below 10 kVp or is at or below 5 kVp. In one aspect of the invention, the distance to the target is utilized to also alter the effect of varying the incident energy of the X-rays incident on the medium. The distance can be set to a value of less than 5 mm, less than 10 mm, less than 15 mm, or less than 20 mm. In other embodiments, the x-ray source can be positioned farther away from the target being irradiated.

"kVp" is peak accelerating voltage applied in an X-ray tube between the cathode and anode. The term and its definition derive from the fact that in some systems the accelerating potential is not constant, but varies over time (i.e., have a voltage ripple). The kVp (in units of kilovolts) is the kinetic energy (in keV) of the most energetic electrons arriving at the anode, and also the energy of the most energetic X-ray photon produced by bremsstrahlung.

The efficiency of X-ray production by bremsstrahlung increases with increasing kVp, and so therefore does X-ray tube output. If the kVp (in kilovolts) is higher than the binding energy of an electron shell of the X-ray tube target material, it is possible for the electron to ionize that shell and for characteristic radiation to be produced.

For any given kVp, the X-ray spectrum contains a spread of energies, the highest of which is proportional to the kVp. However, the number of photons in lower energy ranges is greater than at the very highest energies, and the average energy of the X-ray beam is lower than the kVp. Nonetheless, the average energy increases with increasing kVp and the beam becomes more penetrating.

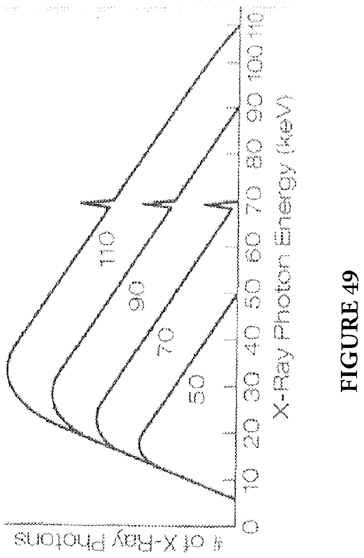

Figure 49 depicts the energy distribution of x-rays as a function of kVp. It shows a progressive reduction in the peak x-ray energy and a reduction in the number of x-rays as kVp is reduced. Accordingly, a computer system (or another x-ray source controller) controlling the initiation energy source can control the kVp setting to change the dose and average x-ray energies incident on a target of subject 4. While the x-ray energy used in the experimental results below were obtained without an aluminum filter on the x-ray source, an aluminum or other filter can be used to truncate a portion of the x-ray spectrum and selectively provide different x-ray doses and x-ray energies to the target.

### Phototherapy Excitation

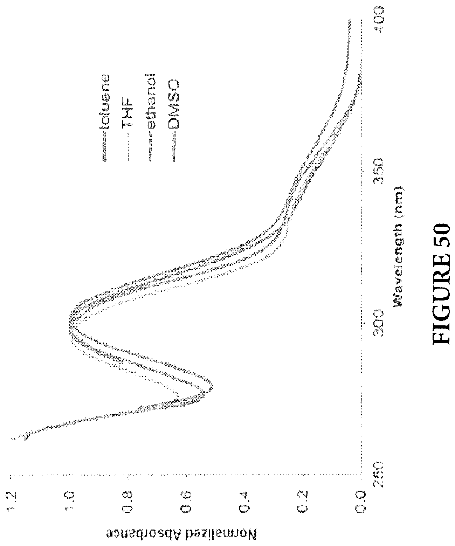

Regardless of method of treatment, psoralen is of interest for many of the biological applications of this invention. The absorption of psoralen was measured in different solvents including toluene, tetrahydrofuran (THF), ethanol, and dimethyl sulfoxide (DMSO). The UV-Vis absorption spectra are provided in Figure 50. In particular, Figure 50 shows the absorption spectrum of psoralen measured in different solvents and over a broad range extending from the UVB, the UVA and part of the visible.

The UV light emitted inside a cell or inside an organ depends on the light conversion capability of the utilized particle and on the number of particles residing close to the point of measurement. The higher the number of particles the higher the net intensity according to the superposition principles applicable to light in particular and to electromagnetic waves in general. The nano-particle conversion material can be selected to have a high probability of interaction with X-ray and strong emission in UV range with as much intensity as possible. Alternatively, the nano-particle conversion material can be a scintillator selected to have a high probability of interaction with an ionizing particle and strong emission in UV range with as much intensity as possible. A scintillator is a material which exhibits luminescence when excited by ionizing radiation, such as for example an incoming particle (electron or ion), absorb its energy and reemit the absorbed energy in the form of light.

Some phosphors can be doped with ionic species such that the material formed can exhibit fluorescence and phosphorescence at the same time. The materials can be formed in single crystal or poly-crystalline forms, in powders or monoliths.

However, once the conversion material selection is done, further improvement of intensity solely depends on the size, the number and the distribution of the nano-particles that are close to target or to the measurement point. The delivery of particles inside an organ can be gated by the organ's vasculature. The delivery of particles inside a cell can also be gated by the ion channels residing in the cell walls. Organs can accept larger particles than cells since the openings gated by the organ's vasculature are much larger than ion channels in the cell walls.

One embodiment of this invention deals with the delivery of the above described energy converters, e.g., phosphors or scintillators or a combination thereof having particle sizes below 40 nm and that can pass through the ion channels of cells. Once inside the cell, the phosphors of this invention are trapped in sufficient concentration. The entrapment of the phosphors of this invention can be facilitated by the combination of applying a magnetic coating to the particles and using magnetic fields that are imposed externally to a given mammalian body (or external to an artificial medium). In addition to entrapment of phosphors or scintillators or a combination thereof inside cells or organs, the phosphors of this invention can be made to assemble in patterns that increase their net UV light output under X-Ray excitation.

In one embodiment, there is provided a system for light stimulation within a medium with or without energy augmentation. The system has a first plurality of light-emitting particles which upon encountering an appropriate initiating excitation of light energy or particle beam energy radiate an output energy having photocatalysis potential to activate photoactivatable agents with minimized impact on the medium. The system further has a second plurality of light-emitting particles which upon encountering the same appropriate initiating excitation of light energy or particle beam energy radiate an output energy complementary to the output of the first set of particles With the energy augmentators, this invention can generate local regions of intense electric fields to enhance light emission from the plurality of light-emitting particles in proximity to those local regions.

A combination of energy emission from the first and second plurality of energy emitting particles produces a combined energy capable of activating chemical agents inside the medium more effectively than the first set of particles alone. The two sets of particles are interoperably complimentary to one another. The energy outputs can be of different natures. The first set of particles can output light energy and the second set of particles can output chemical energy.

The energy spectrum of the first set of particles has an energy distribution having a peak position in common with a peak in an absorption spectrum of the photoactivatable agent(s) and having a bandwidth overlapping the absorption spectrum of the photoactivatable chemical agents. The second energy potentiates the photoactivation by predisposing reactive sites to the photoactivatable chemical agent(s). The second energy can also be a light energy of different spectrum or a chemical energy resulting in the favorable alteration of the reaction potential of select reactive sites. For instance, light can cause excitation of photosensitizers, in the presence of oxygen, to produce various toxic species, such as singlet oxygen and hydroxyl radicals. Meanwhile, microwave and RF energy leads to dipolar alignment of molecular species having an asymmetrical charge distribution over their length.

More specific methods by which chemical pathways of photoactivatable chemistries can be altered is described below in at least the photo-treatment section and the photobiomodulation section.

The phosphors or scintillator particles of this invention can be synthesized from known material chemistries that possess the capability of fluorescence (caused by the instantaneous decay of electrons from a high energetic state to a lower one) or phosphorescence (delayed decay of electrons from a high energetic state).

The phosphors or scintillator particles of this invention can be further prepared using additive processes (i.e.; coatings) to gain the self-assembly capability inside cells when exposed to electrical field or magnetic fields stimulation. Externally imposed electrical field or magnetic fields can be applied in a cyclic manner of specific frequencies and magnitudes that promote the assembly into patterned configurations.

Besides phosphors and scintillator particles, this invention can also use other light emitting particles such as fluorescent particles and up-converting particles or others of the energy converters or light emitters described above. In those cases, the techniques described here for improving the efficiency of delivering light to a target or for spectrally matching the emitted light to a photoactivatable substance still apply regardless of energy augmentation. Moreover, the light emitters of the invention can utilize the above-described plasmonic metallic shell structures to increase the efficiency of absorption and light emission.

Some of the materials (which can be used with or without energy augmentation) include phosphors such as YTaO4, YVO₄, YNbO₄ and CaWO₄. Each of these lattice structures is an effective X-Ray absorber and a strong UV emitter. The absorption spectra exhibit strong and broad bands in the UV. The transition involved in these lattices is typically the result of a charge transfer from the oxygen to the d0 ion. An electron can be excited from a non-bonding orbital on the oxygen to an anti-bonding orbital (d on the metal ion). Another lattice structure of interest is Y₂O₃. All of these materials have been doped using ionic species to create color centers. Y₂O₃ can be doped with Gd and YTaO₄ can be doped with Nb. The specific influence of the host lattice on the luminescent center is different for different materials. This is not surprising since the direct surrounding of the luminescent center is changed. The influence of the lattice on optical centers is relatively well known for some materials such as YF₃:E³⁺ and Y₂O₃3:Eu³⁺.

One of the first factors is covalency. A high covalency translates to reduced interactions between electrons since they spread out over wider orbitals. Electronic transitions between energy levels are set by the difference in these energy levels which are in turn gated by electronic interactions. The difference in energy levels is lower for increasing covalency. Another factor for the influence of the lattice on the optical properties of an ion is the crystal field. Certain optical transitions are determined by the strength of the crystal field. This explains why Cr₂O₃ is green but Al₂O₃:Cr³⁺ is red even though both materials have the same crystalline structure. The Cr³⁺ ions occupy the smaller Al³⁺ sites and as a result feel a stronger crystal filed in Al₂O₃ than in Cr₂O₃. The synthesis of the materials influences the emission of the color centers. The defects as well as the particle size and particle size distribution all play a role.

Controllable and repeatable processes that can be utilized to produce nano-particles, and use thereof, have emerged as an area of science and engineering of considerable interest in recent years. The use of electric or magnetic field-assisted transport offers an approach for manipulating millimeter, micrometer and nanometer particles in a repeatable and controllable manner. The use of such electric fields is generally referred to as dielectro-phoresis (DEP).

The application of a field gradient gives rise to translation and orientation of particles exhibiting dipolar characteristics. The net asymmetrical distribution of charge along the dimension of a particle dictates the magnitude of the resultant dipole which has units of unit charge per unit length or Coulomb/meter. The same is true for magnetic fields as well as electric fields. In magnetic fields, this effect is characterized by the susceptibility of the material forming the particle. The net magnetization per unit length will define the strength of the magnetic dipole.

Phosphor or scintillator particles (which can be used with or without energy augmentation), such as those made of oxide materials, do not have a net dielectric dipole or magnetic dipole. However, according to one embodiment of the invention, phosphor or scintillator particles can be made to act in a dipolar fashion.

Phosphor selection criterions for this invention are based on peak intensity of the emission, peak position with UV of the emission, the need to have a workable phosphor with minimal storage requirements, handling and packaging, the ability of the phosphor to couple to X-Ray energy, the control over its particle size and particle size distribution; and, finally their surface chemistry.

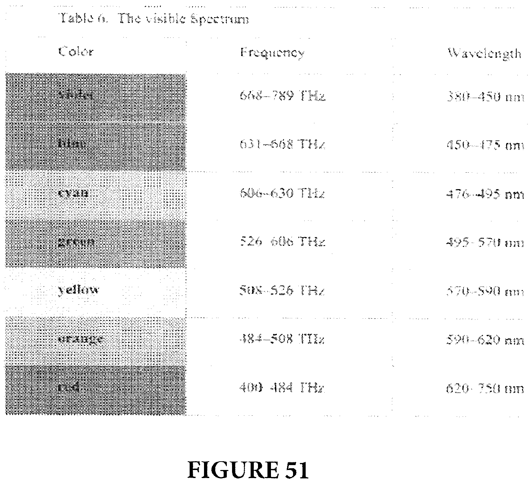

In one embodiment of the invention, the peak emission target is between 310 nm and 800 nm or simply the UVA spectrum. It is desirable to have the maximum conversion of X-ray intensity into UVA intensity and visible light (see Table 6 in Figure 51).

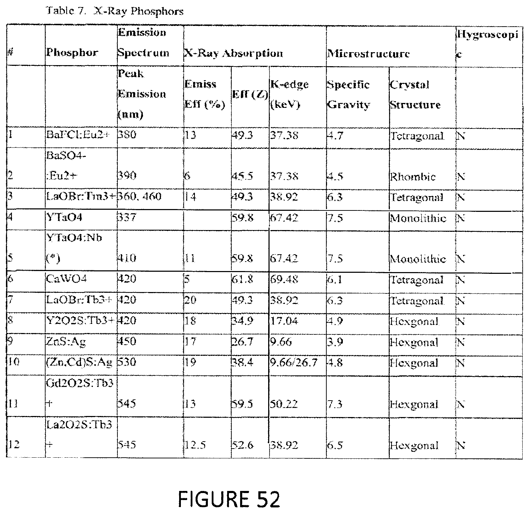

This conversion can be characterized in various interrelated terms. Sometimes the conversion is referred to as the quantum yield or probability of interaction between X-ray and phosphors. These interrelated terms include the coupling efficiency, emission effectiveness or the Effective-Z between the X-ray and the phosphor. A list of some of the X-ray phosphors emitting in the VIS range is reported in Table 7 in Figure 52.

Alternatively, as noted above, a variety of scintillator materials can also be used including organic scintillators, plastic scintillators, and inorganic crystals.

Organic scintillators (which can be used with or without energy augmentation) are usually aromatic hydrocarbon compounds which contain benzene ring structures interlinked in various ways. Their luminescence typically decays within a few nanoseconds. Some organic scintillators are pure crystals. The most common types are anthracene (C₁₄H₁₀, decay time ≈30 ns), stilbene (C₁₄H₁₂, few ns decay time), and naphthalene (C₁₀E₈, few ns decay time). These organic crystal scintillators are very durable, but their response is anisotropic. Anthracene has the highest light output of all organic scintillators

Plastic scintillators (which can be used with or without energy augmentation) are solutions of organic scintillators in a solvent which is subsequently polymerized to form a solid. Some of the common solutes are p-Terphenyl, PBD, b-PBD, PBO, POPOP. The most widely used plastic solvents are polyvinyltoluene and polystyrene. Plastics scintillators give a fast signal (a few ns) and a high light output. The number of emitted scintillation photons is best described by the convolution of an exponential decay and a Gaussian (rather than the exponential decay alone).

Plastics by their nature can very easily be shaped and machined to the forms (cylinders, rods, flat sheets, fibers, microspheres and thin films) and are relatively inexpensive. Plastics scintillators, while generally resistant, can be scratched and attacked by organic solvents (e.g. acetone). Also, bodily acids can cause cracking over time. Nonetheless, in one embodiment of the invention, plastic sheet scintillators can be inserted around or near a tumor site to provide light emission upon exposure to an electron beam.

Inorganic scintillator crystals (which can be used with or without energy augmentation) include materials such as tungstates and alkali metal halides, often with a small amount of activator impurity. The most widely used inorganic scintillator crystal is NaI(Tl) (sodium iodide doped with thallium). Other inorganic alkali halide crystals are: CsI(Tl), CsI(Na), CsI(pure), CsF, KI(Tl), LiI(Eu). Some non-alkali crystals include: BaF₂, CaF₂(Eu), ZnS(Ag), CaWO₄, CdWO₄, YAG(Ce) (Y₃Al₅O₁₂(Ce)), BGO bismuth germanate, GSO , LSO, LaCl₃(Ce), LaBr₃(Ce).

A disadvantage of some inorganic crystals, e.g., NaI, is their hygroscopicity, a property which requires them typically to be housed in an air-tight enclosure to protect them from moisture. CsI(Tl) and BaF₂ are only slightly hygroscopic and do not usually need protection. CsF, Nal(Tl), LaCₗ₃(Ce), LaBr₃(Ce) are hygroscopic, while BGO, CaF₂(Eu), LYSO, and YAG(Ce) are not. The hygroscopic inorganic crystals for application in this invention would typically be encapsulated with a silica or plastic.

Like the phosphors above, scintillators (which can be used with or without energy augmentation) each show typical emission peaks. BaF₂ or barium fluoride is reported to emit in the UV band (220 nm) and at longer wavelengths (310 nm) and has a 630 ns decay time. BaF₂ is not hygroscopic. CaF has a reported emission at 390 nm. CaF₂(Eu) or calcium fluoride doped with europium is not hygroscopic, has a 940 ns decay time, and has been reported to have an emission centered at 435 nm. BGO or bismuth germanate has a higher stopping power, but a lower optical yield than NaI(Tl). BGO has emission centered at 480 nm. CdWO₄ or cadmium tungstate has a relatively high light output (about 1/3 of that of NaI(Tl)). CdWO₄ has been reported to have an emission centered at 475 nm. CaWO₄ or calcium tungstate has been reported to have emission at centered at 420 nm. CsI(Tl) or cesium iodide doped with thallium crystals have been reported as one of the brightest scintillators. The maximum wavelength of light emission is centered at 550 nm. CsI(Tl) is only slightly hygroscopic. CsI(Na) or cesium iodide doped with sodium is less bright than CsI(Tl), but comparable in light output to NaI(Tl). The wavelength of maximum emission is at 420 nm. CsI(Na) is hygroscopic. CsI undoped cesium iodide emits predominantly at 315 nm, and is only slightly hygroscopic. The light output is relatively low. LaBr₃(Ce) (or lanthanum bromide doped with cerium is an alternative to NaI(Tl). LaBr₃(Ce) has been reported to have emission at centered at 370 nm. It is hygroscopic. LaCl₃(Ce) (or lanthanum chloride doped with cerium) is an alternative to LaBr₃(Ce). It is hygroscopic. It has been reported to have emissions centered at 350 and 390 nm.

U.S. Pat. No. 7,084,403 shows a variety of emission from lanthanum halides.

PbWO₄ or lead tungstate has a high stopping power. It has emission at 420 nm. LuI₃ or lutetium iodide has emission at 420 nm. LSO or lutetium oxyorthosilicate (Lu₂SiO₅) has emission around 420 nm. GSO or gadolinium oxyorthosilicate (Gd₂SiO₅) has emission around 430 nm. However, as reported by Mao et al, in "Emission Spectra of LSO and LYSO Crystals Excited by UV Light, X-Ray and gamma-ray" in IEEE TRANSACTIONS ON NUCLEAR SCIENCE, VOL. 55, NO. 3, JUNE 2008, the emission spectrum shifts depending on the source of excitation. Accordingly, in one embodiment of this invention, the choice of excitation source can be used to peak match to a particular photoactivatable substance such as to match the peak in the psoralen absorption.

LYSO (Lu_{1.8}Y_{0.2}SiO₅(Ce)) has a broad emission around 425 nm. LYSO is non-hygroscopic. NaI(Tl) or sodium iodide doped with thallium. NaI(Tl) is the most widely used scintillator material. It has an emission around 410 nm. NaI(Tl) is hygroscopic. YAG(Ce) or yttrium aluminum garnet: YAG(Ce) is non-hygroscopic. The wavelength of maximum emission is around 550 nm. Its light output is about 1/3 of that of NaI(Tl). ZnS(Ag) or zinc sulfide has emission at 450 nm. ZnWO₄ or zinc tungstate has a peak emission at 480 nm (with emission range between 380-660 nm).

In one embodiment of the present invention, mixtures of these scintillators (with or without energy augmentation) can provide a spectral output for photoactivation of photoactivatable agent(s) such as psoralen. In one embodiment of the invention, the amounts of each particular scintillator mixed into the composition is a weighted sum where the product of the emission intensity of each scintillator and the weight composition percentage provides at each emission wavelength a predetermined component of a spectral emission band. In one embodiment of the invention, light from the composition of scintillators simulates at least a part of an absorption spectrum of the photoactivatable agents. For example, a wavelength distribution of the light from the composition of scintillators can have a peak position in common with one of the peaks in the absorption spectra of the psoralens in different media. Further, the wavelength distribution of the light from the composition of scintillators can simulate an absorption edge of the absorption spectrum of the photoactivatable agents, such as for example the absorption edge to the higher wavelength side of the peaks. Further, the wavelength distribution of the light from the composition of scintillators can overlap the absorption spectrum of the photoactivatable agents in part or in whole as if a replicating the absorption spectra. With the energy augmentators, this invention can generate local regions of intense electric fields to enhance light emission from the scintillators in proximity to those local regions.

### UVA/UVB Emissions (with and without Energy Augmentation):

In some applications, the desirable incident or initiation energy is different than X-ray (such as EUV) while the desirable down-converted output intensity remains in the UVA and the visible. In other applications, the desirable incident or initiation energy is X-ray but the desirable down-converted energy output of the phosphor is in the UVB. Yet, in other cases, the desirable incident or initiation energy is X-ray but the desirable down-converted energy output of the phosphor is in the UVA and the UVB or the UV and the visible.

According to one embodiment of the invention, phosphors (which can be used with or without energy augmentation) were selected to work with excitation sources including X-Ray, Extreme UV and e-beam. Within the X-ray regime, the selected phosphors can couple to a flux of X-ray photons emanating from commercially available equipment sources used for therapeutic tumor treatments, medical imaging and semiconductor inspection. With the energy augmentators, this invention can generate local regions of intense electric fields to enhance light emission from the X-ray phosphors in proximity to those local regions.

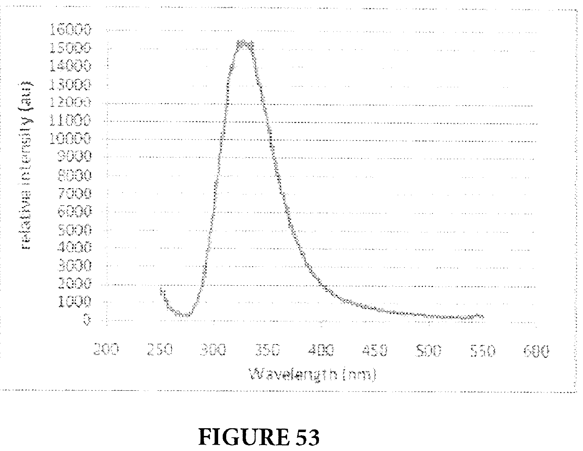

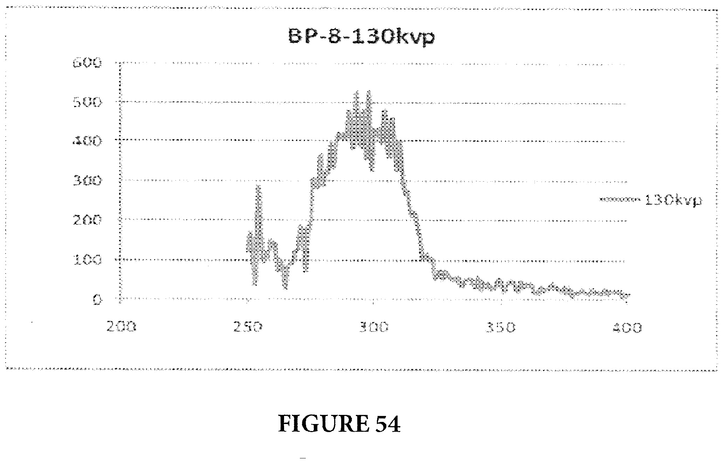

One example of a material that emits in the UVA regime is provided in Figure 53. The X-ray system used to carry out the excitation was the Faxitron X-Ray System. (Faxitron X-Ray LLC, 575 Bond St. Lincolnshire, IL 60069 USA). Figure 54 is a schematic of emission from YTaO₄ reported to have a peak emission at 337 nm under X-Ray excitation. However, here, emission at 327 nm was observed.

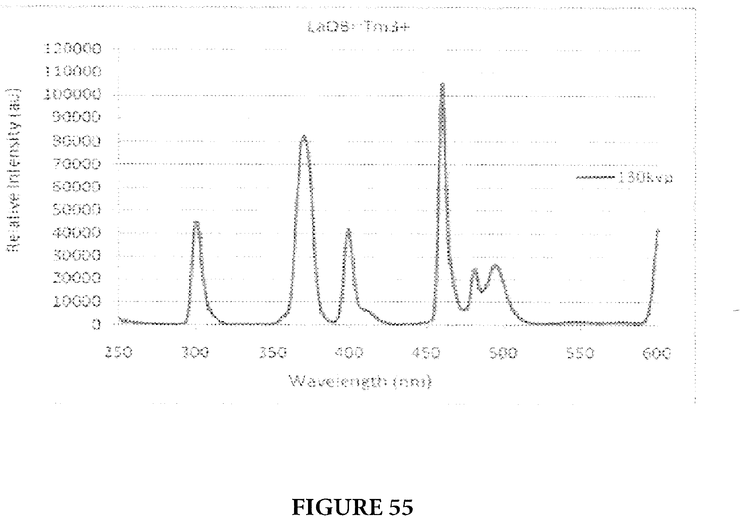

One example of a material having an output in the UVB is provided in Figure 55. Figure 55 is a schematic of emission from LaOBr:Tm₃⁺ reported to have a peak emission at 280 nm under X-Ray excitation. However, emission at 300 nm was observed in work by the inventors.

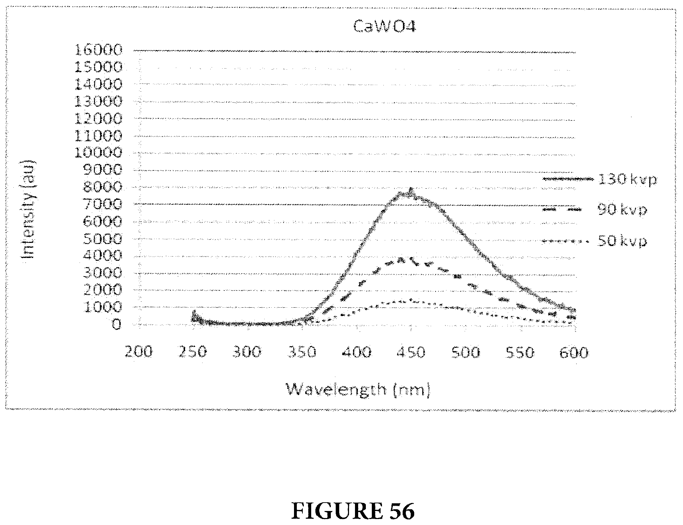

One example of a material having an output in the UVA, UVB and the visible is provided in Figure 56. Figure 56 is a schematic of emission from a CaWO₄ phosphor coated with silica and showing emission in UVB, UVA, and the visible.

### Impact of X-ray on UV output intensity:

The initiation energy (X-Ray in this example) influences the UV output of the phosphor. Both the intensity of X-Ray and the energy of the X-Ray photon excitation influence the UV light output. The following examples are provided to illustrate how modifying the photonic energy and intensity of X-Ray can modulate the light output of the UV and Visible light. These tests were made using three different voltages between the filament and the tungsten target of the X-ray generator. In each case, the emission peak and intensity of the phosphor emission was dependent on the voltage between the filament and the target (i.e., dependent on the intensity of X-Ray and the energy of the X-ray photon excitation).

In these tests, various phosphors were weighed to 12 grams and placed in UV transparent containers. These phosphors were activated under X-ray generated using different voltages (50 kVp, 90 kVp and 130 kVp). The term "kVp" as before represents the peak voltage in kilovolts. A photo-spectrometer was placed in the same position vis-à-vis the various containers.

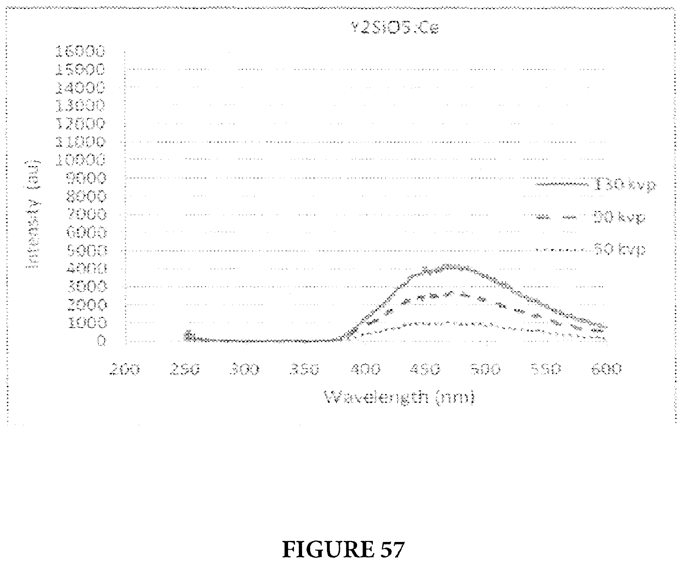

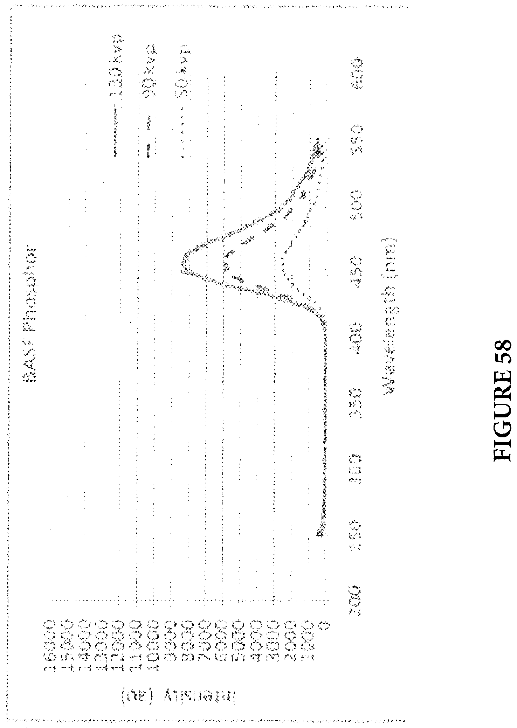

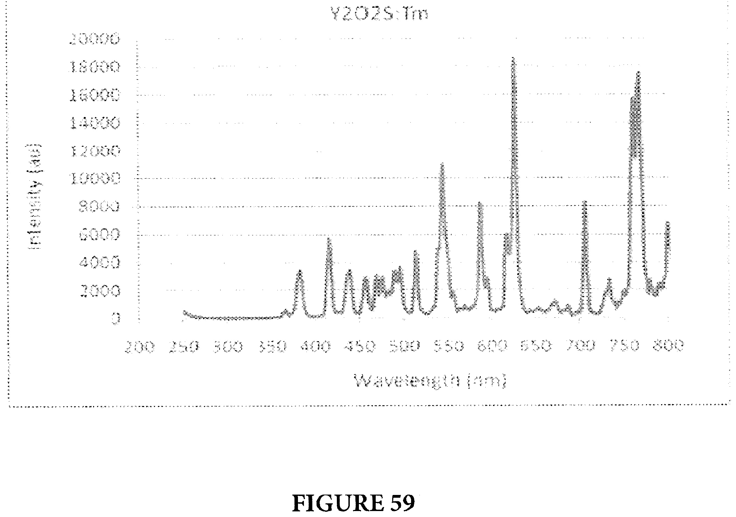

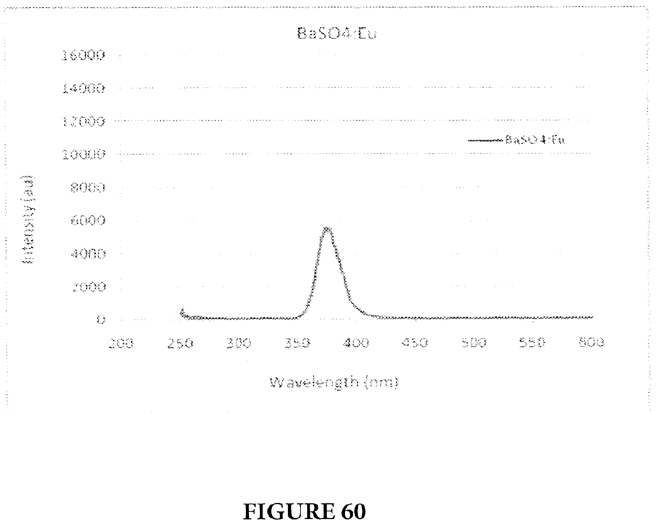

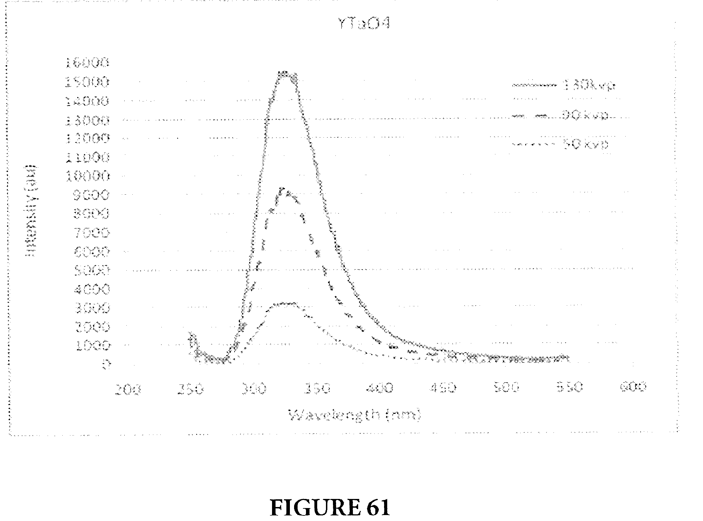

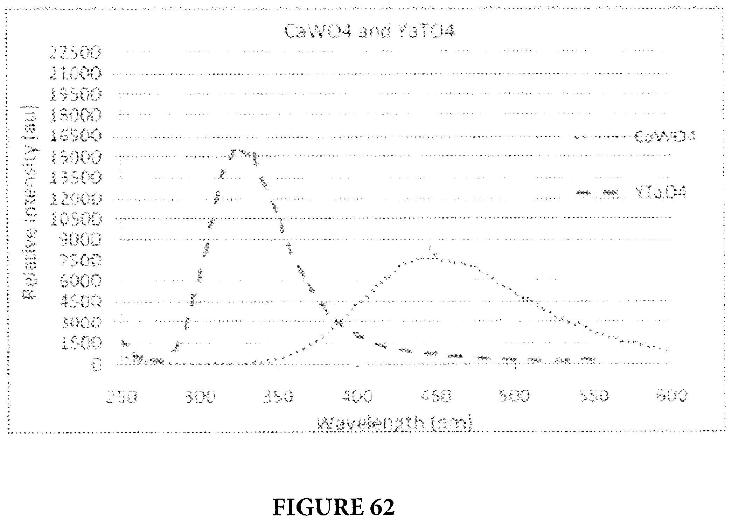

Figure 57 is the spectral output from a visible phosphor Y₂SiO₅:Ce under X-ray excitation using three different voltages between the filament and the target. Figure 58 is the spectral output of a visible phosphor (BASF commercial phosphor XYMARA MARKER BLUE LF2A) under X-Ray using three different voltages between the filament and the target of the X-ray generator. Figure 59 is the spectral output of a visible phosphor Y₂O₂S:Tm under X-Ray using three different voltages between the filament and the target of the X-ray generator. Figure 60 is the spectral output of a BaSO₄:Eu phosphor capable of emission in the UVA and in the visible. Figure 61 is the spectral output of an YTaO₄ phosphor capable of emission in the UVA and in the visible. Figure 62 is a schematic of the spectral output of an YTaO₄ phosphor chemistry capable of emission in the UVA and CaWO₄ capable of emitting in the UVA and in the visible.

### A Mixed or Alloyed Configuration of the Invention for use with or without energy augmentation

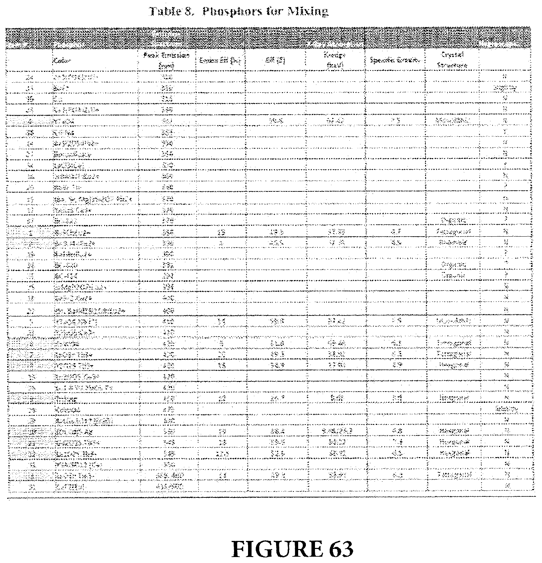

According to another embodiment of the invention, at least two phosphors are mixed to broaden the output of the mixture as compared to the individual starting phosphors. According to this embodiment, multi-peak output phosphors can be obtained from one phosphor chemistry or by combining multiple phosphor chemistries. All or any of the phosphor chemistries listed in Table 3 can be combined with one another to form multiple wavelengths of interest. These phosphors in Table 8 shown in Figure 63 are listed in an ascending order of wavelength emissions.

In one embodiment of the invention, the amounts of each particular phosphor mixed into the composition is a weighted sum where the product of the emission intensity of each phosphor and the weight composition percentage provides at each emission wavelength a predetermined component of a spectral emission band. In one embodiment of the invention, light from the composition of phosphors simulates at least a part of an absorption spectrum of the photoactivatable agents. For example, a wavelength distribution of the light from the composition of phosphors can have a peak position in common with one of the peaks in the absorption spectra of the psoralens in different media shown in Figure 50. Further, the wavelength distribution of the light from the composition of phosphors can simulate an absorption edge of the absorption spectrum of the photoactivatable agents, such as for example the absorption edge to the higher wavelength side of the peaks in Figure 50. Further, the wavelength distribution of the light from the composition of phosphors can overlap the absorption spectrum of the photoactivatable agents in part or in whole as if a replicating the absorption spectra.

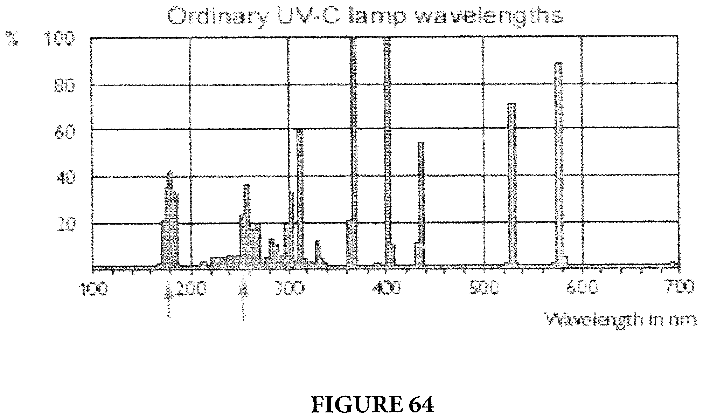

In one embodiment, the weighted product produces a spectral emission band which simulates a commercial UV light source. Figure 64 provides a typical spectrum from a commercial UV light source which has been used to activate psoralens. As can be seen, the commercial UV light source has a broader spectral width than the absorption line of psoralen.

Given the uncertainties of the mechanisms of psoralen activation and association with the cancer cells for cell death, the broader spectral width of the commercial UV light source may offer advantages in activation of the psoralen and promotion of cancer cell death. For example, the broader spectral width of the commercial UV light source may itself promote changes in the cancer cells themselves which promote attachment of the "activated" psoralen. Laskin et al, in a paper entitled "Psoralens potentiate ultraviolet light-induced inhibition of epidermal growth factor binding," in Proc. Nat. Acd. Sci. vol. 83, pp. 8211-8212, November 1996, show in their Figure 1 the effects of UV dose with and without trimethyl psoralen on inhibition of ¹²⁵I-EGF specific binding. The inhibition of promoted with and without trimethyl psoralen at higher UVA light doses, but with higher efficiency with trimethyl psoralen present. Moreover, the data shows that doses of greater than 1 Joule/cm² seem required to activate a cell response without psoralen, while with psoralen the activation occurs at lower doses. Indeed, the dose to induce a cell response seems inversely proportional to the psoralen concentration, that is the higher the psoralen concentration the lower the dose needed to promote a cell response. Laskin et al concluded that psoralen/UVA or UVA light-induced changes in the kinase activity of an epidermal growth factor receptor may initiate a cascade of biochemical events leading to cellular responses.

Varga et al in "Dose-related Effects of Psoralen and Ultraviolet Light on the Cell Cycle of Murine Melanoma Cells," in Cancer Res 1982;42:2223-2226, published online June 1, 1982 reported similar results with

Accordingly, in one embodiment of the invention, the mixed phosphors and scintillators of the invention provide a spectral response of higher UV dose and a closer spectral match to that of commercial UVA sources than for example single fluorescent emitters or single phosphor emitters.

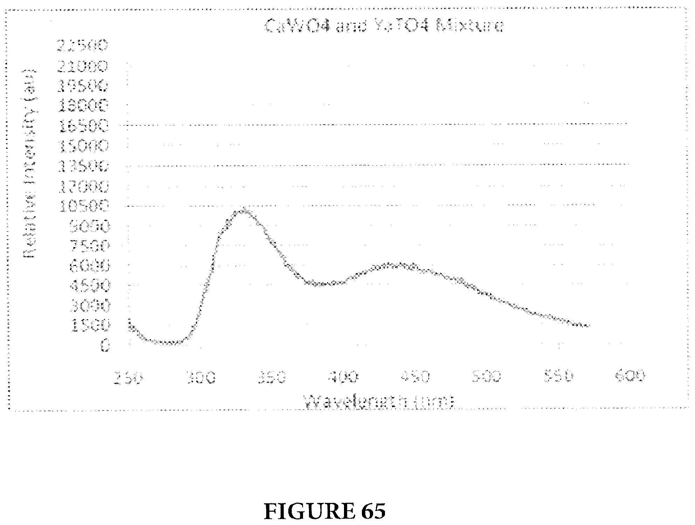

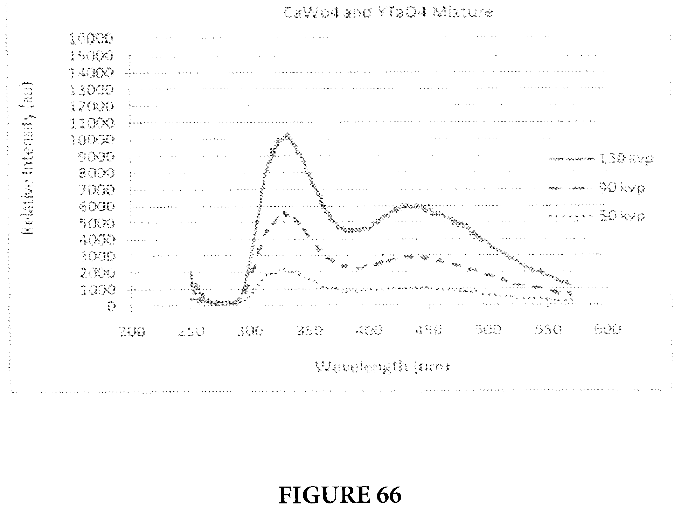

Figure 65 is the superimposed emission spectra under X-ray excitation for CaWO₄ phosphors and YTaO₄ phosphors. In the example illustrated in Figure 65, the two phosphors each emit in a distinct region. Figure 66 is the emission spectra under X-ray excitation (for various voltages between the filament and the target) for the combination of a mixture of CaWO₄ and YTaO₄ phosphors. The spectral output demonstrates the ability to influence the output intensity of the mixture as compared to the staring materials. The intensity of the initiation energy (X-ray in this case) influences the UV output of the phosphor. The following examples are provided to illustrate how modifying the intensity of photonic energy of X-ray can modulate the light output of the UV and Visible light. The relative intensity output of a phosphor (CaOW₄) was measured as a function of the energy of the X-ray photons. The X-ray energy was intensified by modifying the peak voltages that exist between the filament and the target. The target in this case was Tungsten. The measurements were carried out using the same mass of phosphor under 50 kVp, 90 kVp and 130 kVp. The relative intensity of the emission in arbitrary units is indicative but not conclusive in terms of comparing different materials. However, within the same conditions used to conduct measurements, it is clear that the higher X-ray intensity the higher the relative intensity of the emitted wavelength.

According to one embodiment of the invention, phosphors are synthesized from different chemicals and using different processes to control their morphology, in turn influence their properties and light intensity output, but more importantly their stability in ambient air environments. It is preferred in certain applications to have phosphors that are not hygroscopic. Phosphors are easier to handle and to work with when the phosphors are stable in water and do not contain dopants that are toxic; however, even when phosphors are not stable in water and do contain dopants that are toxic, particles of these phosphors in one embodiment of the invention can be coated using chemical synthesis methods to build-up a protective coating which shields the phosphor from the environment (water for example) and which shields the environment from the toxic dopant in the phosphor (Bromide for example). The protective coating can be silica or can be diamond or diamond-like carbon. Silica can be formed using sol-gel derived techniques. Diamond and diamond-like carbon can be derived from chemical vapor deposition (CVD) techniques based for example on Hydrogen-Methane gas mixtures. The handling and packaging of various phosphors and phosphor mixtures can be achieved through dispersion in solution or in powder form. It was found that silica coated phosphors do not have a tendency to agglomerate.

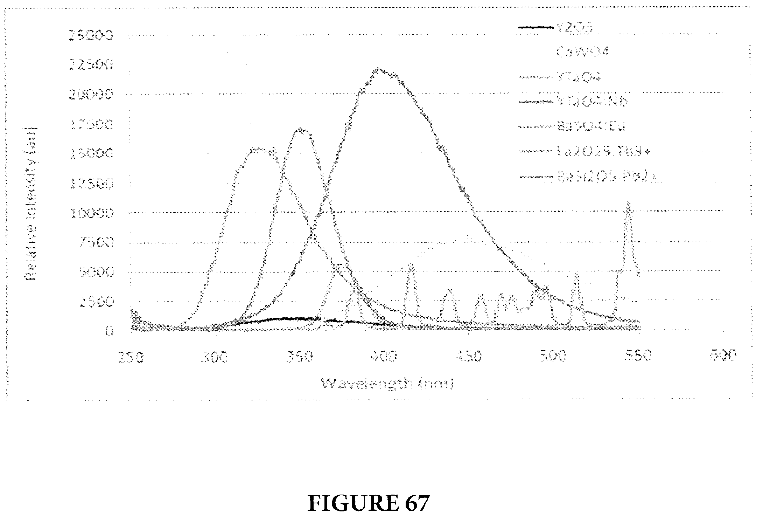

Figure 67 is the emission spectra under X-ray excitation for various materials includingY₂O₃, CaWO₄, YaTO₄, YaTO₄:Nb, BaSO₄:Eu, La₂O₂S:Tb, BaSi₂O₅:Pb. These materials yield various peak intensities and wavelengths. As seen from this figure, the phosphor and scintillator materials (CaWO₄, YaTO₄, YaTO₄:Nb, BaSO₄:Eu, La₂O₂S:Tb, BaSi₂O₅:Pb) are considerably brighter than that of Y₂O₃ a conventional fluorescent material.

Hence, in one embodiment, there is provided a system and method for light stimulation within a medium. The system includes an initiation source configured to an initiation energy and a plurality of energy-emitting particles in the medium which, upon radiation from the initiation source, radiate at a lower energy than the initiation source to interact with photoactivatable agents in the medium. The energy-emitting particles radiate with an intensity at least two times greater than that of intrinsic (or undoped) Y₂O₃, upon exposure of Y₂O₃ to the radiation from the initiation source. The method includes introducing a plurality of energy-emitting particles into the medium, radiating the plurality of energy-emitting particles in the medium with radiation from an initiation energy source, and emitting from the plurality of energy-emitting particles a lower energy than the radiation from the initiation energy source to interact with photoactivatable agents in the medium. In various aspects of the invention, the energy-emitting particles radiate with an intensity at least 10 times greater than that of intrinsic Y₂O₃, at least 50 times greater than that of intrinsic Y₂O₃, or at least 100 times greater than that of intrinsic Y₂O₃, or at least 500 times greater than that of intrinsic Y₂O₃, or at least 1000 times greater than that of intrinsic Y₂O₃.

In this and other embodiments, the plurality of energy-emitting particles can include at least one of phosphors, scintillators, fluorescent materials, and combinations and agglomerations thereof with or without plasmonic inducing agents. In this and other embodiments, the initiation energy source can be one of an X-ray source, a high energy source, a particle source, and extended UV source, and a radioactive source including at least one of a Cobalt 60 source, a Cesium-137 source, an Iridium-192 source, a Krypton-85 source, a Radium-226 source, and a Strontium-90 source or a combination thereof.

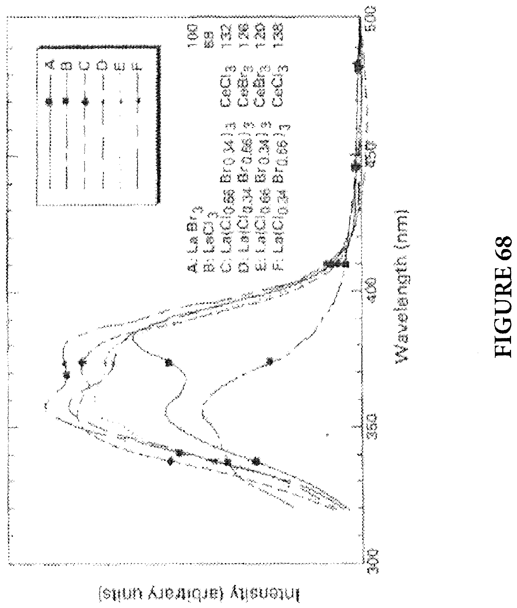

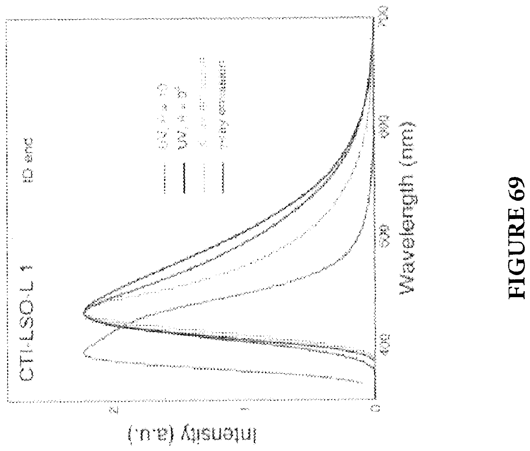

According to one embodiment of the invention, a combination of these materials can yield a spectrum with a specific signature. Phosphor emissions from these materials, as illustrated in Figures 67, 68, and 69, cover a broad range of the VIS and UV spectrum. Hence, in one embodiment, there is provided a system for light stimulation within a medium. The system includes an initiation source configured to radiate an initiation energy, a first plurality of energy-emitting particles in the medium which (upon radiation from the initiation source) radiate at a first lower energy than the initiation source to interact with photoactivatable agents in the medium, and a second plurality of energy-emitting particles in the medium which (upon radiation from the initiation source) radiate at a second lower energy than the initiation source to interact with photoactivatable agents in the medium. A combination of emission from the first and second plurality of energy-emitting particles produces a spectrum for illumination of the photoactivatable agents in the medium. The spectrum has a wavelength distribution simulating at least a part of an absorption spectrum of the photoactivatable agents or a spectrum of an ultraviolet discharge lamp.

In one aspect of the invention, the wavelength distribution can have a peak position in common with a peak in the absorption spectrum of the photoactivatable agents or can simulates an absorption edge of the absorption spectrum of the photoactivatable agents. In another aspect, the first and second plurality of energy-emitting particles can be a weighted composition of a plurality of different light-emitting particles, where light emitted from the weighted composition simulates part of the absorption spectrum of the photoactivatable agents.

In another aspect, the combination of the emission from the first and second plurality of energy-emitting particles with or without energy augmentation can be configured about a target site to form a light source illuminating the target site to treat the target site with the photoactivatable agents. In another aspect, an energy distribution emitted from the first and second plurality of energy-emitting particles resembles the absorption spectrum of the photoactivatable agents or the spectrum of the ultraviolet discharge lamp. The energy distribution can overlap with the absorption spectrum of the photoactivatable agents or the spectrum of the ultraviolet discharge lamp. With the energy augmentators, this invention can generate local regions of intense electric fields to enhance light emission from the energy-emitting particles in proximity to those local regions.

### Toxicity Testing:

Clonogenic Survival Assay (Low Density Protocol): In *low density clonogenics,* multiple cell densities are plated first and then treated. This clonogenic technique minimizes plating effects and pilot errors. In contrast, *high density clonogenics* have one stock plate of cells that is treated and then trypsinized and plated at different densities. This assay is more labor intensive and more prone to errors (e.g., pilot and plating) as well as contamination. However, this technique may more accurately depict the clinical situation as it allows cells to have more cell-to-cell contact.

The procedures followed for the clonogenic survival assays below are as follows:
**1.** Label plates (cells, treatments, date, initials).
a. Plate cells in triplicate at 3 different densities (such as 100, 300, and 1,000 cells/plate).
   i. The # of cells plated depends on:
      1. The cell line (for example HeLa, HT29, B16/F10 and most MEF cell lines, recommend using 100, 300 and 1,000 cells per plate).
      2. Treatments - the higher drug concentrations, higher IR doses or longer hypoxia treatments are usually more toxic compared to less stringent conditions, so use more cells for the more toxic treatments.

**2.** Calculate the drug concentrations and the amount of media needed for each treatment.
a. Media:
   i. In *6-wellplates,* use 3 mL media per well - so total amount of media needed is (3 mL/well) * (total # of plates) * (# of wells/plate)
   ii. In *6-well plates,* use 3 mL media per plate - so total amount of media needed is (3mL/well) * (total # of plates) * (# of wells/plate)
   iii. Also, take into account any media changes/washes - if using drug treatments, double the amount of media needed as you will need to add fresh media after the drugs are rinsed off the cells.
b. Drugs:
   i. Make fresh drug dilutions for every experiment
   ii. Make drug dilutions beforehand if adding drugs directly to the media, add greater 3µL volume per well. Any volume less than 3µL adds potential error to the experiment.

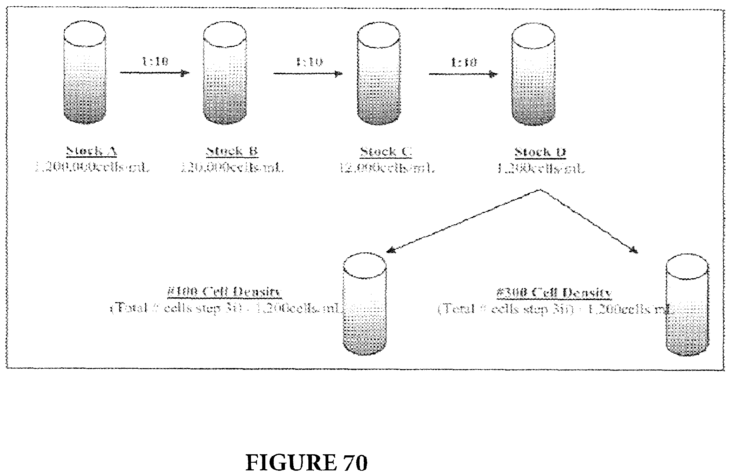

**3.** Plating:
a. Trypsinize cells.
b. Determine total # of cells for each cell density in a *6-well format*:
   i. (# of plates) * (3 well/plate) * (100 cells/ well) = Total # of cells needed to give 3 wells 100 cells/well in each plate.
   ii. (# of plates) * (3 well/plate) * (300 cells/ well) = Total # of cells needed to give 3 wells 300 cells/well in all plates
   iii. Calculate media needed to plate each density:
      1. (# of plates) * (3 well/plate) * (3mL/well) = Total # mL of media needed to plate each density.
c. Pellet cells - centrifuge @ 1,000 rpm / 2-3min / 4°C
d. Resuspend in media and count.
e. Make serial dilutions to obtain the number of cells needed to add to total volume of media (step 3iii).
   i. If 1,200,000 cells/ml are counted, plate #100 and #300 cells/well - dilute the total number of cells down to a more manageable volume.
   ii. Dilute (1:10) the main stock 1,200,000 cells/ml- to give 120,000 cells/ml - dilute (1:10) again to give 12,000 cells/ml - dilute (1:10) again gives 1,200 cells/ml. See Figure 70.
f. Plate 3 ml of media and cells in each well of all plates
g. Put in the incubator and allow cells ~18-24hr to attach.

**4.** Treat cells:
a. Treat cells according to the experimental design *i. Optional (depends on experiment): Remove media on all plates, rinse with 2mL 1X PBS and then add fresh 3mL of media.*
b. Incubate plates under normal conditions (37°C and 5% CO₂) for 7-14 days, or until visually detecting colonies of greater than 50 cells in the cell alone control plates.
c. Stain plates.

**5**. Staining (not necessarily under sterile conditions):
a. Decant media off plates.
b. Rinse plates with ~2 mL 1X PBS.
c. Add Fixation Solution and leave on for 10min/RT i. Typically, 2-3 mL is enough (i.e. enough to cover the bottom of the plate)
d. Decant Fixation Solution
e. Add Crystal Violet Stain (enough to cover bottom of plate) and leave on for 10min/RT.
f. Rinse plates with water.
i. Fill sink with water and drop plates in as you remove the crystal violet.
ii. Rinse off plates with water.

g. Allow plates to dry on bench paper
h. Count colonies using the ColCounter.
i. Count colonies that have >50 cells in them - look at colonies under the microscope if you are
unsure.

| **Fixation Solution:** |
|---|
| 10% Methanol |
| 10% Acetic Acid |
| 80% H₂O |

| **Crystal Violet** | |
|---|---|
| 500mL of working stock: | |
| | • 0.4% Crystal Violet (200mL of the 1% stock) |
| | • 20% Ethanol (100 mL) |
| | • 200 mL H₂O |

**6.** Data Analysis:
a. Record the number of colonies for each cell density and treatment group.
b. Correct for cell density (i.e. normalize all plates to 100 cells)
   i. Compare between groups to see if the groups are all corrected to reflect the same number of cells plated.
      1. To compare your treatment #1 on 300 cells to control/vehicle on 100 cells - divide your number of colonies from your 300 cell group by 3 since there are 3X as many cells.
c. Calculate the plating efficiency (survival of your control-plated cells)
   i. Average the # colonies in your control plates
d. Correct for plating efficiency (this removes effects just from plating your cells)
   i. Divide the surviving fractions normalized for cell density (Step6B) by the plating efficiency calculated in Step 6C.
e. Calculate survival fraction, which is the average of the corrected numbers in Step 6D, standard deviation as well as standard error (standard deviation divided by the square root of (n)).
f. Plot Surviving Fraction (semi-log plot; y-axis) vs. treatments (linear; x-axis).

### Solubilization Protocol:

(Used if working with cell lines that do not form tight, distinct colonies - like some GBM lines. Reference: Bernardi et al (2001) Clinical Cancer Research 7, 4164-73)
**1.** Add 33% acetic acid to each of your 60 mm plates 24 hr post-staining.
   a. Do not use less than 400 µL.
**2.** Aliquot 100 µL from each plate (in triplicate) to a 96-well plate.
**3.** Read the absorbance at 540 nm and average the 3 values.
**4.** Normalize all values based on the volume solubilized and then follow regular data analysis steps.

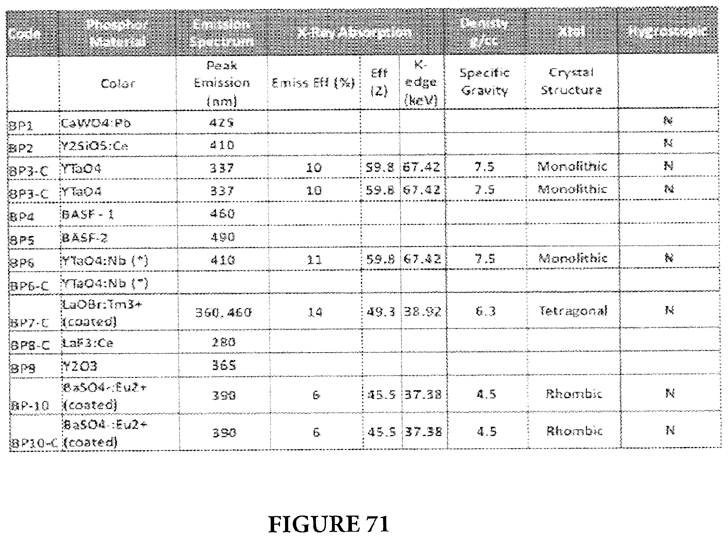

The phosphors were tested in two forms, coated and uncoated. All coated phosphors were designated by a "c" at the end for example BP7c (blue phosphor #7 coated). All uncoated phosphors were designated by a "u" at the end for example BP3u (blue phosphor #3 uncoated). Most of the coatings tested in our experiments consisted of silica. All uncoated phosphors were predominantly oxides. The assigned names to the various phosphors are provided in the following Table 9 in Figure 71.

### Toxicity testing of YTaO₄:Nb

Various phosphors including YTaO₄:Nb phosphors were tested for their inherent toxicity using a clonogenic survival assay (the details of which are provided below). Three different doses of YTaO₄:Nb were used in this case. In this particular case, the YTaO₄:Nb oxide phosphor was coated with a nano-meter size layer of silica.

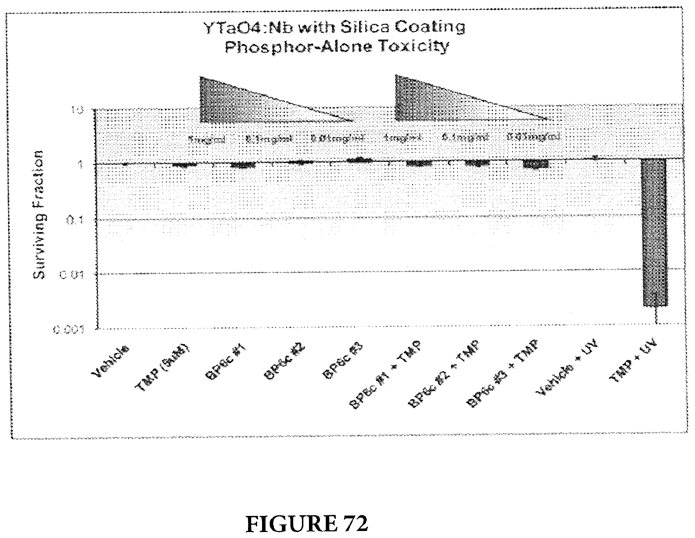

This clonogenic survival assay was plated using the B16 mouse melanoma cells with TMP (5 :m/ml) and/or silica coated YTaO4:Nb phosphor at three concentrations (1 mg/ml, 100 µg/ml, 10 µg/ml). The mixture sat on the cells for 3 hr, and then the media was removed. YTaO₄:Nb was found to be non-toxic up to a dose of 1 mg/ml alone or in combination with TMP. Figure 72 is a depiction of the results of YTaO₄:Nb Phosphor-Alone Toxicity using clonogenic assay. No inherent toxicity was observed. The YTaO₄:Nb with silica coating was found to be nontoxic even in high doses.

### Toxicity testing of BaSO₄:Eu:

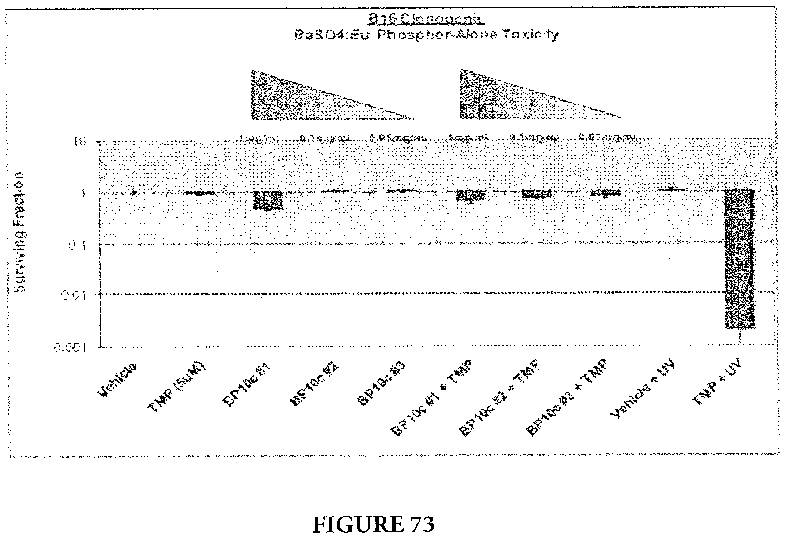

Three doses of BaSO₄:Eu were used to look for any inherent toxicity. Figure 73 is a depiction of the results of BaSO4:Eu phosphor-alone toxicity using the clonogenic assay. BaSO₄:Eu with silica coating was added in three different concentrations to B16 mouse melanoma cells with TMP. No inherent toxicity was observed. The clonogenic survival assay was plated using the B16 mouse melanoma cells with TMP (5 µm/ml) and/or BaSO₄:Eu phosphor (1mg/ml, 100 µg/ml, 10 µg/ml) sat on the cells for 3 hr, and then the media was removed. BaSO₄:Eu phosphor coated with silica coating was found to be non-toxic at 100 :g/ml and 10 :g/ml. It had moderately toxic at 1 mg/ml.

### Toxicity testing of BaSi₂O₅:Pb:

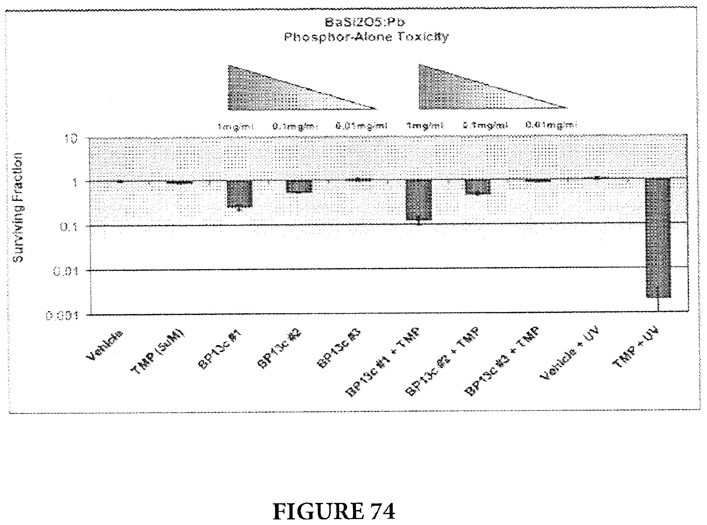

Three doses of BaSi₂O₅:Pb were used to look for any inherent toxicity. Figure 74 is a depiction of BaSi₂O₅:Pb phosphor-alone toxicity using the clonogenic assay. A BaSi₂O₅:Pb phosphor coated in silica containing trace amounts of Pb, is much more toxic at the highest concentration compared to either of the previous phosphors. This clonogenic survival assay was plated using the B16 mouse melanoma cells with TMP (5 µm/ml) and/or BaSi₂O₅:Pb phosphor (1mg/ml, 100 µg/ml, 10 µg/ml) sat on the cells for 3 hr, and then the media was removed. BaSi₂O₅:Pb was found to be non-toxic at 10 :g/ml, moderately toxic at 100 :g/ml, and markedly toxic at 1 mg/ml.

### YTaO₄ Phosphor Coated With Silica under X-ray in the Presence Of TMP:

Another clonogenic survival assay was plated using the B16 mouse melanoma cells. The testing was designed to determine if the YTaO₄ phosphor plus TMP lead to melanoma cells kill. Two levels of x-ray energy (filament to target voltage) were used. The TMP was added at a concentration of (5 µm/ml) and/or phosphor (1 mg/ml, 100 :g/ml, or 10 :g/ml). The mixture sat on the cells for 3 hr before the cells were exposed to radiation. The radiation was given to the indicated groups using the Orthovoltage machine where the 2 Gy total dose was delivered using 2 different energy levels (135 kVp, 160 kVp).

There is some degree of XRT + phosphor effect even at the lower doses of phosphor at 160 kVp. One effect of the X-ray radiation treatment with the YTaO₄ phosphor was observable though not as pronounced at 135 kVp. The cell kill results indicated a 30-40% 'inherent' toxicity with 1 mg/ml of phosphor (high concentration).

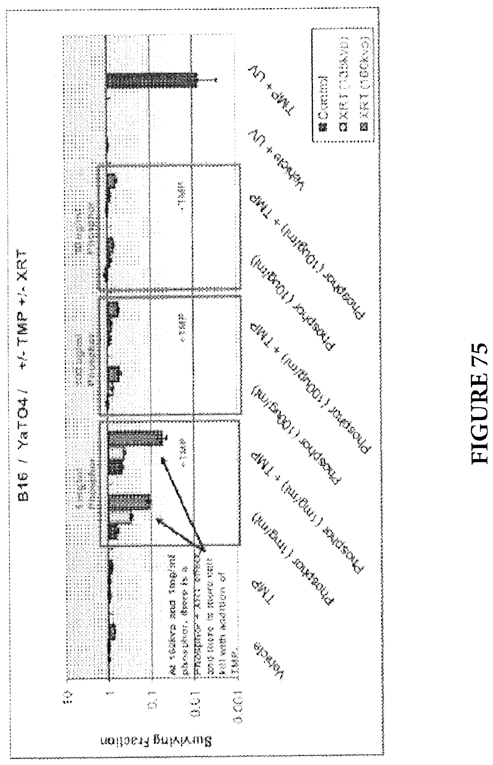

Figure 75 is a depiction of the results using a voltage of 160 kVp and 1 mg/ml concentration of the TaO₄ phosphor, which shows a marked XRT and Phosphor effect, and further cell kill when adding TMP.

Based on this data with YTaO₄, two concentrations of the YTaO₄ phosphors were evaluated to resolve with greater details the combined effect of phosphor plus X-Ray radiation plus TM at 160 kVp + TMP. This clonogenic survival assay was plated using the B16 mouse melanoma cells with TMP (5 µm/ml) and/or YTaO₄ phosphor (1 mg/ml, 500 :g/ml) sat on the cells for 3 hr before the cells were exposed to radiation. The radiation was given to the indicated groups using the Orthovoltage machine with the 2 Gy total dose at 160 kVp.

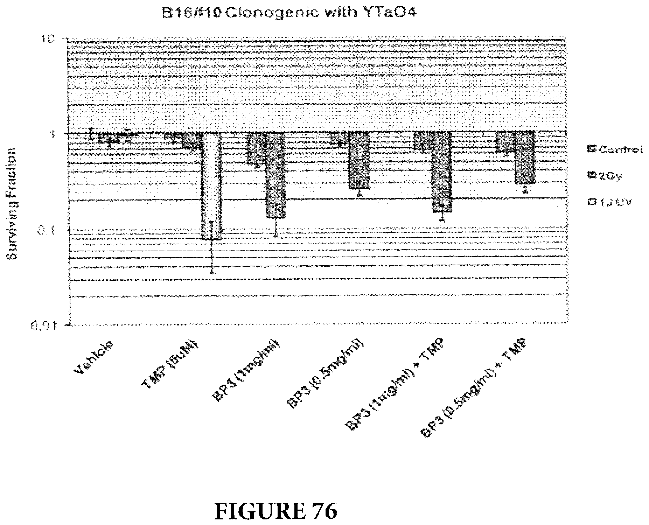

A repeatable and reproducible signal was observed based on the effect of radiation and phosphor. However, no significant added benefit of adding TMP was observed. In fact the data showed that (in this case) the addition of TMP lessened the surviving cell fraction. Perhaps, the TMP may have selectively adsorbed on the particle surfaces or the UV intensity was attenuated more in the presence of TMP. In either case, the phosphor effect was observable under X-ray. Figure 76 is a depiction of the YTaO₄ phosphor-alone toxicity - using clonogenic assay with three different concentrations added to B16 mouse melanoma cells with TMP.

### LTaO₄ Phosphor (with no coating) under X-ray in the Presence of TMP:

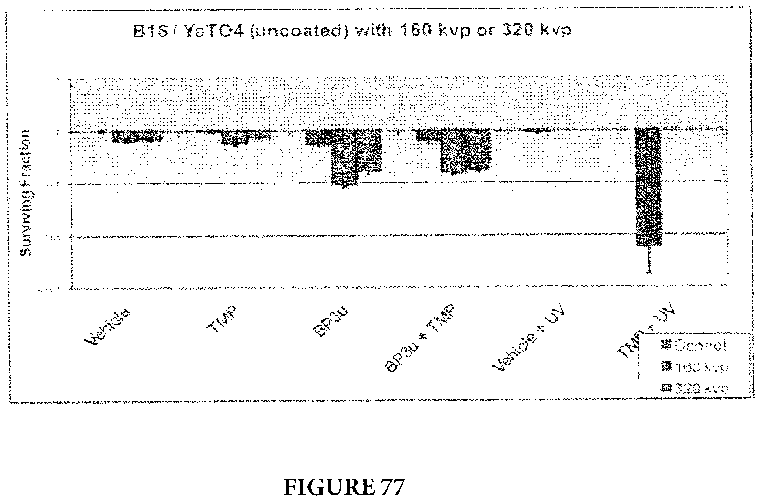

Another clonogenic test was carried out using an identical YTaO₄ (BP3u) without the SiO₂. In essence, the innate oxide was tested to resolve the impact of the surface finish of the phosphor. Figure 77 is a depiction of the results with YTaO₄ (uncoated) at 0.75 mg/ml +/- 2 gray XRT at 160 kVp or 320 kVp. 30-40% cell kill from radiation alone was observed. There is moderate toxicity with 0.75 mg/ml of YTaO₄ uncoated by itself (36-48% kill). There is a markedly enhanced cell kill with YTaO₄ plus XRT. However, similarly to the previous result shown in Figure 75, there is no added benefit from XRT + BP3u + TMP.

With YTaO₄ (uncoated) at a dose of 0.75 mg/ml, there is moderate toxicity from the phosphor alone. An enhanced cell kill was seen with BP3 + radiation. However, there was observed no added benefit of YTaO₄ + radiation + TMP at either 160 kVp or 320 kVp.

### YTaO₄:Nb Phosphor (with no Coating) under X-ray in the Presence

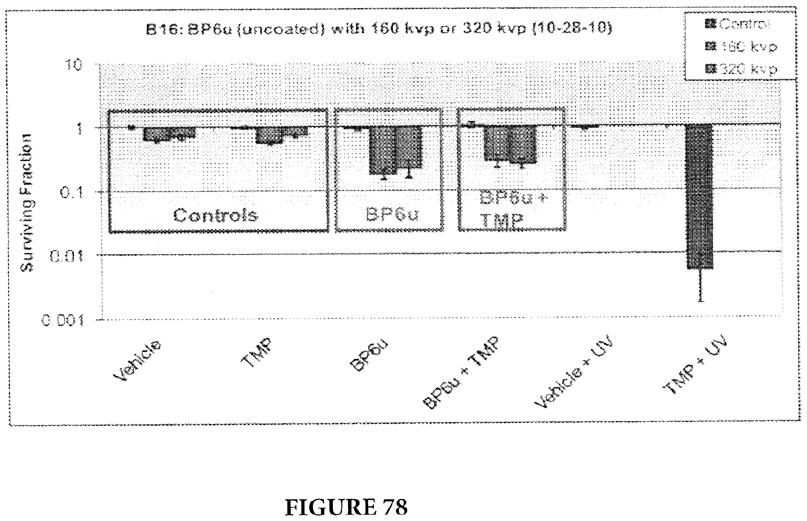

Another clonogenic test was carried out using the same phosphor base matrix with a doping that shifted the peak emission. This was achieved by adding niobium to the tantalate chemistry to form YTaO₄:Nb (BP6u). This evaluated phosphor was without the SiO₂ coating. In essence, the innate oxide was tested to resolve the impact of the surface finish of the phosphor. Figure 78 is a depiction of the results with YTaO₄:Nb (uncoated) at 0.75 mg/ml , +/- 2 gray XRT at 160 kVp and 320 kVp. 30-40% cell kill from radiation alone was observed. There is minimal toxicity with 0.75 mg/ml of BP6u by itself (0-7% kill). There is markedly enhanced cell kill with YTaO₄:Nb plus XRT. However, there is no added benefit from XRT plus YTaO₄:Nb plus TMP at these kVp levels.

### LaOBr:Tm³⁺ Phosphor (with SiO₂ Coating) under X-ray in the Presence of TMP:

Based on the previous data with YTaO4, three doses of LaOBr:Tm ³⁺ were evaluated to look for a phosphor plus radiation plus TMP effect. This clonogenic survival assay was plated using the B16 mouse melanoma cells with TMP (5 µm/ml) and/or LaOBr:Tm phosphor (1 mg/ml, 100 µg/ml, 10 µg/ml) sat on the cells for 3 hrs before the cells were exposed to radiation. The radiation was given to the indicated groups using the Orthovoltage machine (2 Gy total dose at 160 kVp or 80 kVp).

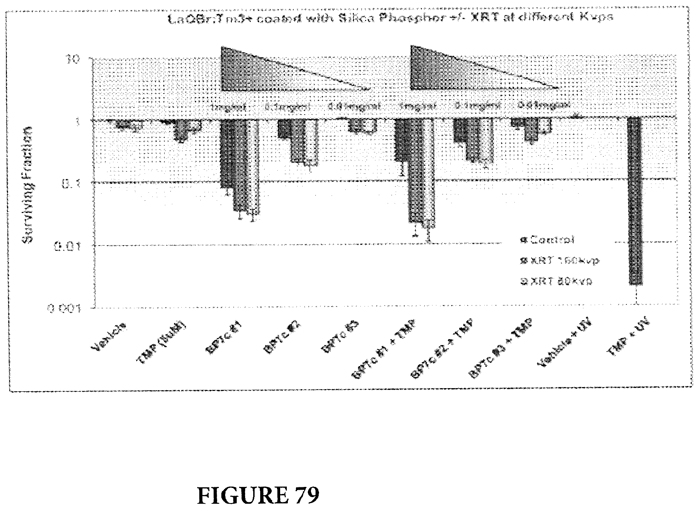

Figure 79 is a depiction of the results with LaOBr:Tm (coated with SiO₂) phosphor-alone toxicity - using a clonogenic assay with three different concentrations added to B16 mouse melanoma cells with TMP. LaOBr:Tm is toxic by itself (see the left bars in Figure 79). There was no additional benefit of adding TMP at these kVp levels. LaOBr:Tm while the brightest phosphor was found to be toxic by itself. This is not a surprise in the view of the bromine constituent which is toxic. Also, no TMP activation was seen, as with the previous experiment, at either 80 or 160 kVp. However, with this phosphor having a strong UV and visible light intensity, a lower X-Ray dose experiment was carried out. These experiments were carried out at 40 kVp and 80 kVp.

### LaOBr:Tm³⁺ Phosphor (with SiO₂ Coating) under X-ray using 80 kVp in the Presence of TMP

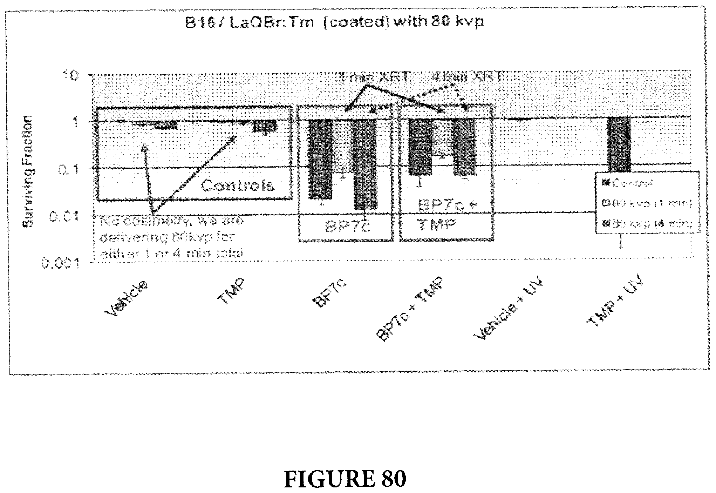

Figure 80 is a depiction of the results with a LaOBr:Tm (coated with SiO₂) phosphor-(BP7c) toxicity using a concentration of 0.75 mg/ml phosphor plus TMP concentration at 80 kVp for 1 or 4 minutes total. There is marked toxicity with 0.75 mg/ml of LaOBr:Tm by itself resulting in a 93-98% kill. The radiation bars are difficult to interpret in light of the severe, inherent toxicity of these phosphors

With BP7c (coated) at a dose of 0.75mg/ml, there is marked toxicity from the phosphor alone. It was difficult to interpret the radiation data in light of the marked inherent toxicity of this phosphor at the concentration of 0.75mg/ml. It is not possible to determine if there is a radiation plus phosphor effect, or an added benefit of TMP at 80 kVp for either 1 min or 4 min.

### LaOBr:Tm³⁺ Phosphor (with SiO₂ Coating) under X-ray using 40 kVp in the Presence of TMP

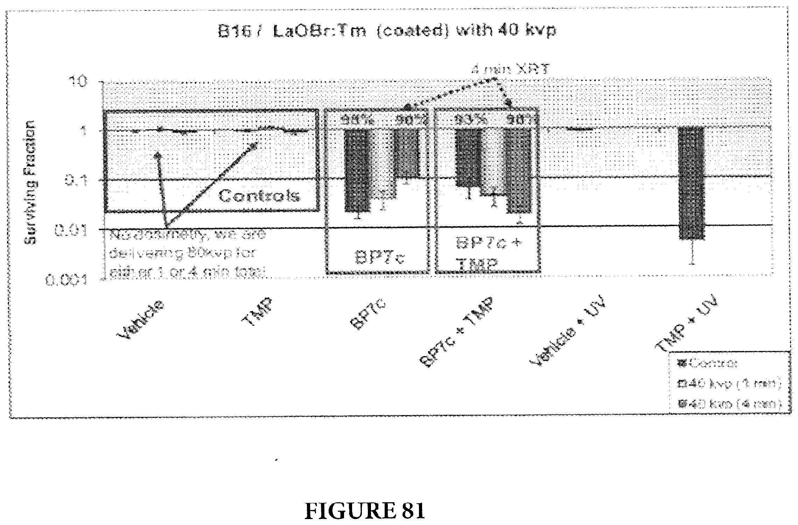

Figure 81 is a depiction of the results with a LaOBr:Tm (coated with SiO₂) phosphor-alone toxicity using a concentration of 0.75 mg/ml plus TMP at 40 kVp XRT for 1 or 4 minutes total. With LaOBr:Tm (coated) at a dose of 0.75 mg/ml, there is marked toxicity from the phosphor alone. It was difficult to interpret the radiation data in light of the marked inherent toxicity of this phosphor at 0.75 mg/ml. It is not possible to tell if there is a radiation + phosphor effect, or an added benefit of TMP in this study at 40 kVp for either 1 min or 4 min.

There is marked toxicity with 0.75 mg/ml of LaOBr:Tm by itself showing 93-98% cell kill. The plus radiation LaOBr:Tm radiation bars are difficult to interpret in light of the inherent toxicity. Though the brown bars (40 kVp for 4 min) may appear to be different, there is only an 8% difference between those bars. The LaOBr:Tm plus TMP plus XRT bar is not different from the toxicity of LaOBr:Tm alone.

### CaWO₄ Phosphor with no Coating) with surface modified Y₂O₃ under X-ray in the Presence of TMP:

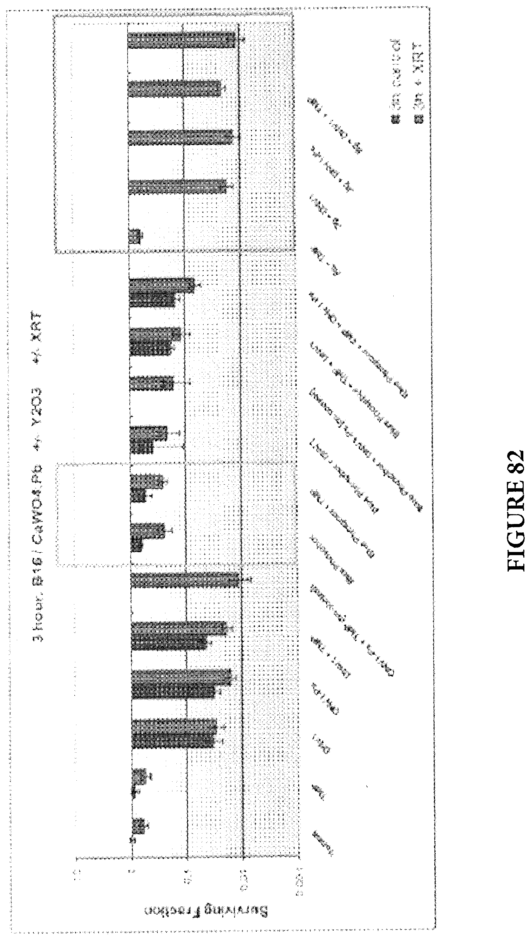

In this experiment, B16 mouse melanoma cells were plated in a 6-well format for a clonogenic survival assay. Cells were treated with combinations of TMP, downconverting nanoparticles, phosphor fixture used for processing in the irradiator or phosphor powder mixed into the media. Figure 82 is a depiction of the results of the cell kill assay performed with CaWO₄ combined with the Y₂O₃ particles in some cases. CaWO₄ plus TMP show an enhanced cell kill with radiation.

The cells were incubated with or without down-converting yttrium nanoparticles for 3 hours. These particles were either tethered to a tat-peptide or a tat-peptide conjugated with psoralen. X-ray exposure of the blue phosphor fixture results in UV emission which should activate TMP in the cell media. For the radiation set with CaWO₄ phosphor in the media, the cells were exposed to the phosphor and/or TMP and/or nanoparticles for 3 hours. The nanoparticle preparation was so toxic that an interpretation of enhanced cell kill with this nanoparticle combination was not possible.

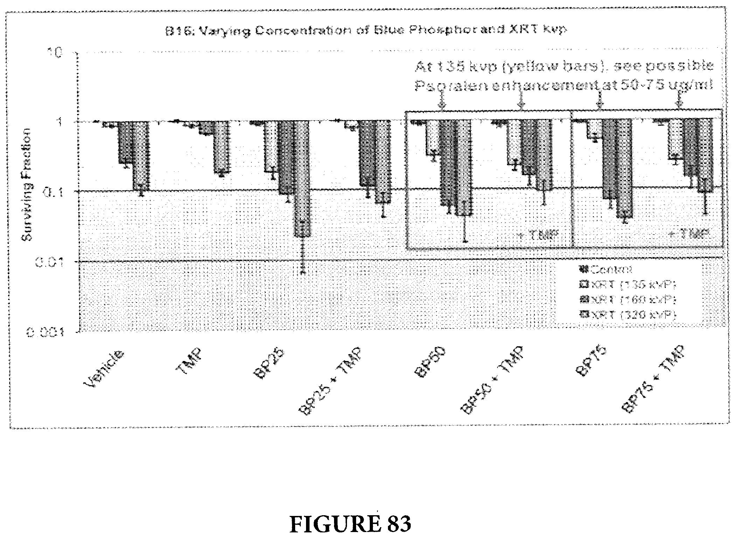

Another clonogenic survival assay was plated using the B16 mouse melanoma cells to test if the CaWO₄ phosphor at 3 intermediate concentrations can activate TMP to kill melanoma cells using 3 different energy levels of radiation. The cells were plated and allowed to attach to the plates overnight. The next day, CaWO₄ powder was suspended in water to give a 100 mg/ml stock and then added directly to the cells to give final concentrations of 0.25 mg/ml, 0.5 mg/ml and 0.75 mg/ml. TMP, previously dissolved in DMSO, was also added to the cells at the same time to give a final concentration of 5 :M. The drug and phosphor sat on the cells for 3 hr before the cells were exposed to radiation. The radiation was given to the indicated groups using the Orthovoltage machine where the 2 Gy total dose was delivered using three different energy levels (135 kVp, 160 kVp and 320 kVp). Figure 83 is a depiction of the results with B16 clonogenic assay for the CaWO₄ phosphor by varying the X-ray voltage (135 kVp, 160 kVp and 320 kVp) and phosphor doses 0.25 mg/ml, 0.5 mg/ml and 0.75 mg/ml. A signal of psoralen enhancement at 50 and 75 mg/ml was observed.

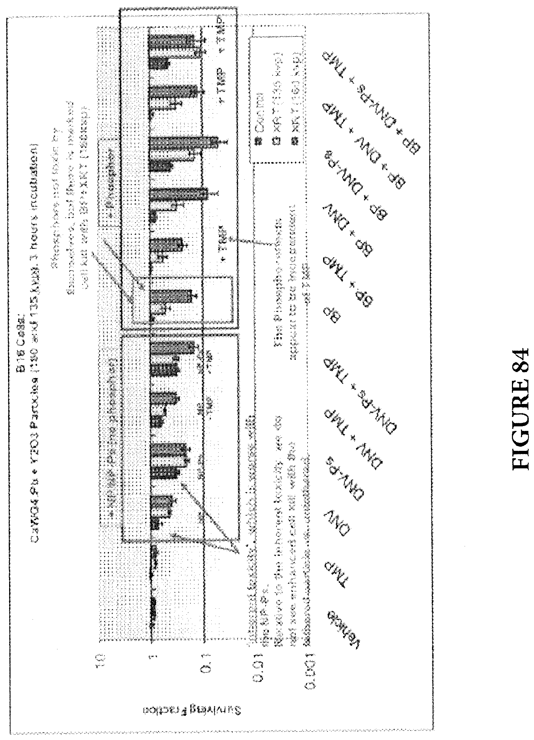

Another clonogenic survival assay was plated using the B16 mouse melanoma cells testing if the CaWO₄ phosphor plus TMP to kill melanoma cells using two different energy levels of radiation, to determine whether adding nanoparticles provides a benefit. The drug, particles, and phosphor sat on the cells for 3 hr before the cells were exposed to radiation. The radiation was given to the indicated groups using the Orthovoltage machine where the 2 Gy total dose was delivered using two different energy levels (135 kVp and 160 kVp). Figure 84 is a depiction of the results of a B16 clonogenic assay using the CaWO₄ phosphor and varying the X-ray voltage (135 kVp and 160 kVp).

There was significant toxicity from the nanoparticles, especially with the psoralentethered particles. The phosphor was not toxic by itself, but provided enhanced cell kill in the present of radiation. This phosphor + radiation effect was independent of TMP. The CaWO₄ phosphors have a very pronounced cell kill when treated with X-ray radiation. This effect does not seem to rely on TMP.

### Energy Modulation Agent Modifications:

In one embodiment of the invention, a phosphor production process for producing phosphor configurations is provided. The following depicts schematically this process and the resultant phosphor configurations. When or if the energy augmentators are present, this invention can generate local regions of intense electric fields to enhance light emission from these phosphor configurations in proximity to those local regions.

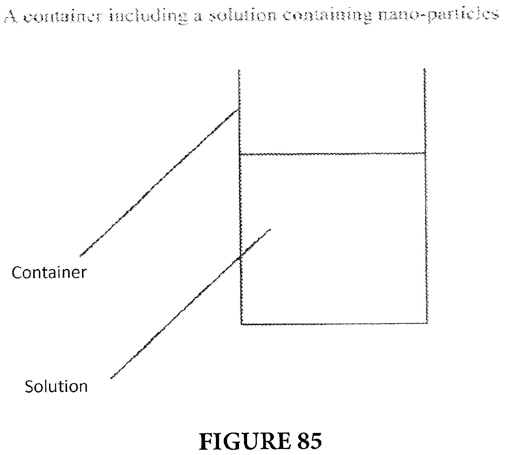

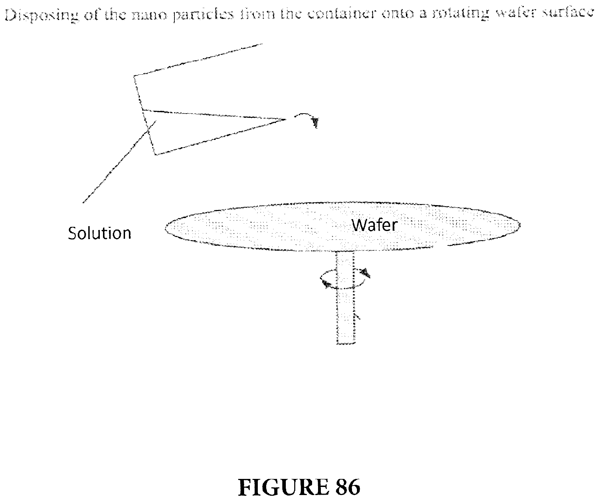

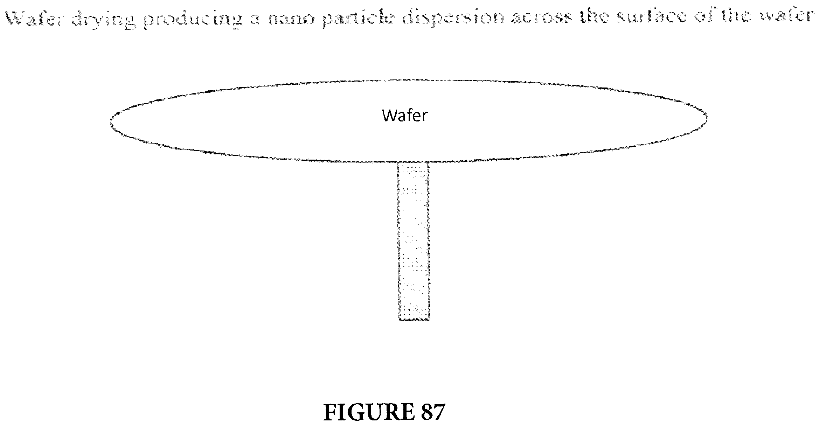

Figure 85 is a schematic of a container including a solution containing nano-particles. Figure 86 is a schematic of a solution containing nano particles applied to a quartz wafer through the process of spin coating. Figure 87 is a schematic of the wafer dried with a thin layer of the nanoparticles dispersed across the surface of the wafer.

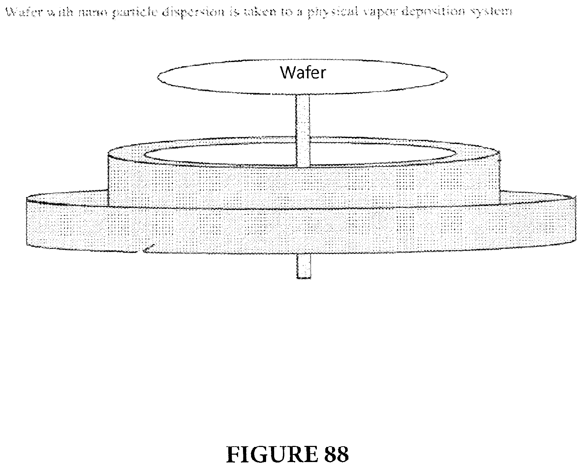

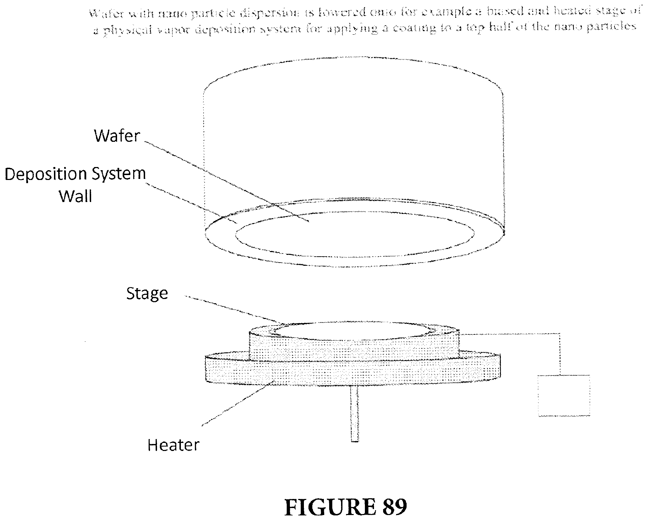

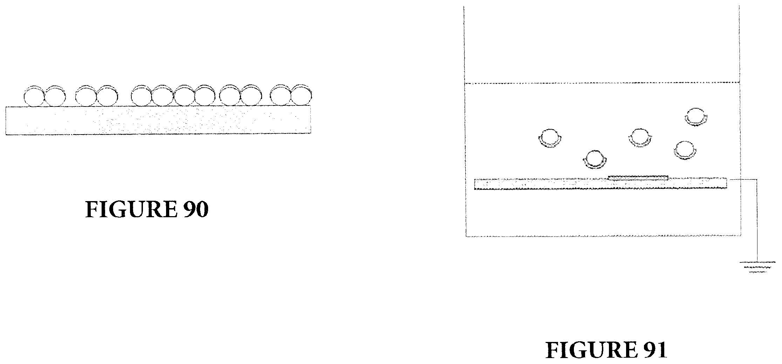

Figure 88 is a schematic of the wafer with nano particle dispersion taken to a physical vapor deposition system. Figure 89 is a schematic of the wafer lowered onto a biased and heated stage, and inserted into physical vapor deposition system for applying a coating on half of the nanoparticles. Figure 90 is a schematic of the cross section of the quartz wafer coated with nanoparticles. The coating applied in the PVD system is applied to a top half the particles.

Figure 91 is a schematic of the half coated phosphor particles placed back in a solution inside a container that has a biased stage. The biased stage contains metallic nano rods.

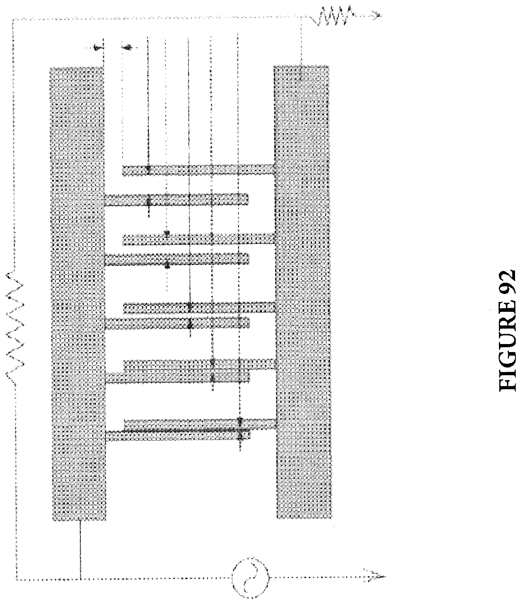

Figure 92 is a schematic of an alternative process where the solution containing phosphors with a metallic coating is placed in a micro-electrode structure having a RF feed energizing the electrodes. The electrodes are disposed to form various gaps ranging from the micron to submicron levels.

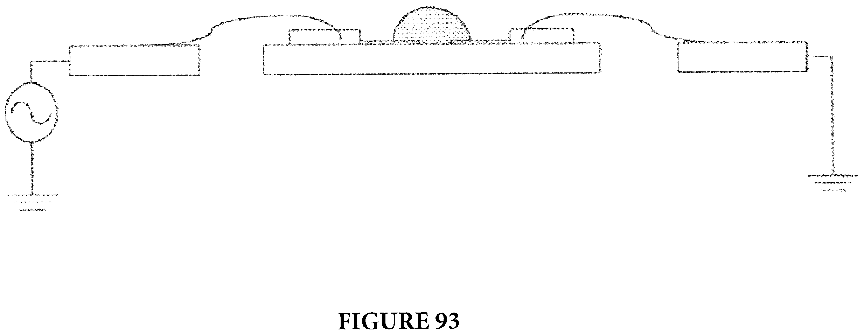

Figure 93 is a cross-sectional schematic of this alternative process where the solution containing phosphors with a metallic coating is placed in a micro-electrode structure having a RF feed energizing the electrodes. The electrodes are disposed to form various gaps ranging from the micron to submicron levels.

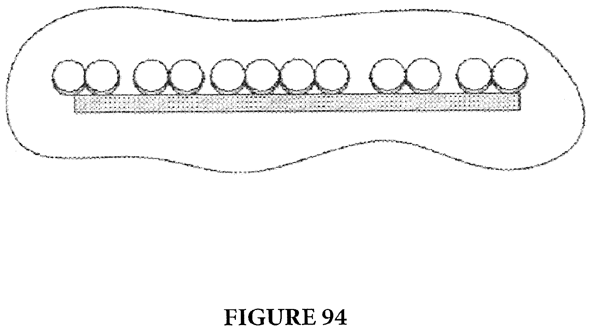

Figure 94 is a schematic depicting the half-coated phosphor particles disposed around a metallic nano rod and heated to sufficient temperatures to alloy the metallic coating with the metallic nano rod. Subsequently, a silica gel coating process is applied to coat the composite structure using silica.

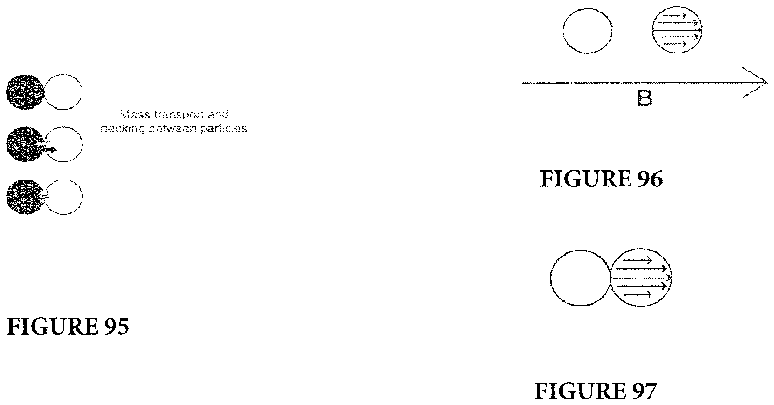

Figure 95 is a schematic depicting a mass transport process, necking the region between particles. Figure 96 is a schematic depicting alignment of a magnetic particle under a magnetic field and followed by joining the phosphor and the magnetic particles (lateral field configuration).

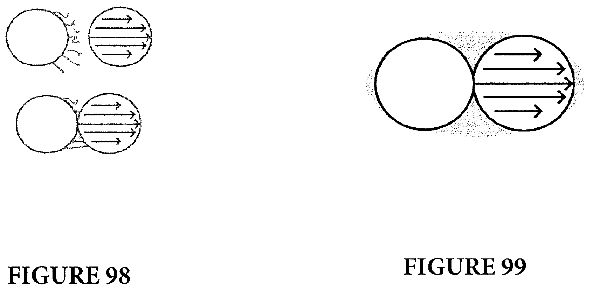

Figure 97 is a schematic depicting the joining of a magnetic particle and phosphor through a necking process. Figure 98 is a schematic depicting the joining of a magnetic particle and phosphor through an adhesion process by surface modification of at least one of the particles.

Figure 99 is a schematic depicting a lipid envelop around the adhered phosphor and nano magnetic particle.

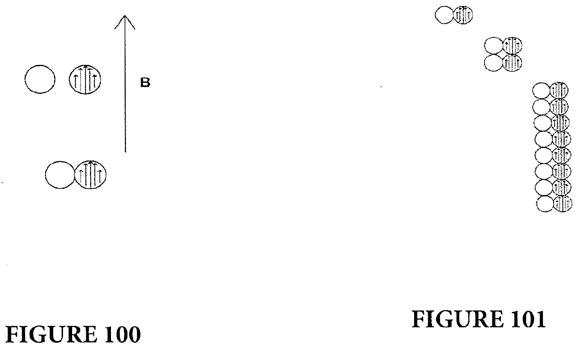

Figure 100 is a schematic depicting alignment of a magnetic particle under a magnetic field and followed by joining the phosphor and the magnetic particles (orthogonal field configuration). Figure 101 is a schematic depicting a situation where, after joining the particles in an orthogonal field configuration, the particles have a tendency to self-assemble in a recto-linear fashion.

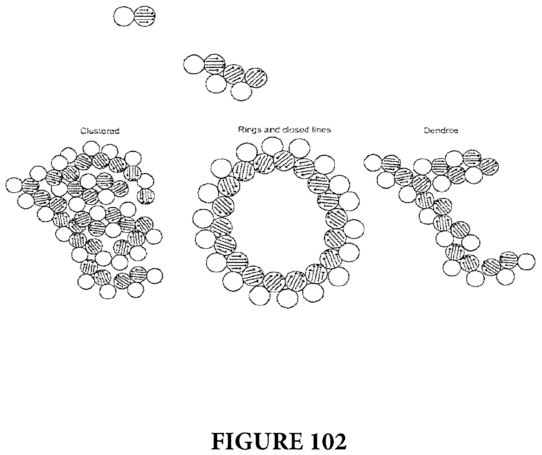

Figure 102 is a schematic depicting a situation where, after joining the particles in a lateral field configuration, the particles have a tendency to self-assemble in dendrite configurations, clusters and rings.

The phosphors (and energy converters) of this invention are not limited to those described above. Other phosphors are suitable and are applicable for various applications where mixtures of down converters are needed. For example, other down converters known in the art and suitable for this invention include TiO₂, ZnO, Fe₂O₃, CdTe, CdSe, ZnS, CaS, BaS, SrS and Y₂O₃. Other suitable down conversion materials known in the art include zinc sulfide, ZnS:Mn²⁺, ferric oxide, titanium oxide, zinc oxide, zinc oxide containing small amounts of Al₂O₃ and AgI nanoclusters encapsulated in zeolite. Other suitable down conversion materials include lanthanum and gadolinium oxyhalides activated with thulium; Er³⁺ doped BaTiO₃ nanoparticles, Yb³⁺ doped CsMnCl₃ and RbMnCl₃, BaFBr:Eu²⁺ nanoparticles, Cesium Iodine, Bismuth Germanate, Cadmium Tungstate, and CsBr doped with divalent Eu.

In various embodiments of the invention, the following luminescent polymers known in the art are also suitable as conversion materials: poly(phenylene ethynylene), poly(phenylene vinylene), poly(p-phenylene), poly(thiophene), poly(pyridyl vinylene), poly(pyrrole), poly(acetylene), poly(vinyl carbazole), poly(fluorenes), and the like, as well as copolymers and/or derivatives thereof.

In various embodiments of the invention, the following particles can be used similar to that detailed in U.S. Pat. No. 7,090,355. For down-conversion, the following materials can be used: inorganic or ceramic phosphors or nano-particles, including but not limited to metal oxides, metal halides, metal chalcogenides (e.g. metal sulfides), or their hybrids, such as metal oxo-halides, metal oxo-chalcogenides; laser dyes and small organic molecules, and fluorescent organic polymers; semiconductor nano-particles, such as II-VI or III-V compound semiconductors, e.g. fluorescent quantum dots; organometallic molecules including at least a metal center such as rare earth elements (e.g. Eu, Tb, Ce, Er, Tm, Pr, Ho) and transitional metal elements such as Cr, Mn, Zn, Ir, Ru, V, and main group elements such as B, Al, Ga, etc. The Garnet series of phosphors can be used: (Y ₘA ₁₋ₘ) ₃ (Al ₙ B ₁₋ₙ) ₅O ₁₂, doped with Ce; where 0≦m, n≦1, where A includes other rare earth elements, B includes B, Ga. In addition, phosphors containing metal silicates, metal borates, metal phosphates, and metal aluminates hosts can be used. In addition, phosphors containing common rare earth elements (e.g. Eu, Tb, Ce, Dy, Er, Pr, Tm) and transitional or main group elements (e.g. Mn, Cr, Ti, Ag, Cu, Zn, Bi, Pb, Sn, Tl) as the fluorescent activators, can be used. Materials such as Ca, Zn, Cd in tungstates, metal vanadates, ZnO, etc. can be used.

Semiconductor nanoparticles can be used. The term "semiconductor nanoparticles," in the art refers to an inorganic crystallite between 1 nm and 1000 nm in diameter, preferably between 2 nm to 50 nm. A semiconductor nano-particle is capable of emitting electromagnetic radiation upon excitation (i.e., the semiconductor nano-particle is luminescent). The nanoparticle can be either a homogeneous nano-crystal, or comprises of multiple shells. For example, the nanoparticle can include a "core" of one or more first semiconductor materials, and may be surrounded by a "shell" of a second semiconductor material. The core and/or the shell can be a semiconductor material including, but not limited to, those of the group II-VI (ZnS, ZnSe, ZnTe, CdS, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, and an alloy or a mixture thereof.

Other down converters include for example ZnS, PbS, SbS₃, MoS₂, PbTe, PbSe, BeO, MgO. Li₂CO₃, Ca(OH)₂, MoO₃, SiO₂, Al₂O₃, TeO₂, SnO₂, KBr, KCl, and NaCl. These materials can include dopants to tailor the emission properties. Examples of doped (or alloyed) glass systems suitable for the include Y₂O₃:Gd, Y₂O₃:Dy, Y₂O₃:Tb, Y₂O₃:Ho, Y₂O₃:Er, Y₂O₃:Tm, Gd₂O₃:Eu, Y₂O₂S:Pr, Y₂O₂S:Sm, Y₂O₂S:Eu, Y₂O₂S:Tb, Y₂O₂S:Ho, Y₂O₂S:Er, Y₂O₂S:Dy, Y₂O₂S:Tm, ZnS:Ag:Cl (blue), ZnS:Cu:Al (green), Y₂O₂S:Eu (red), Y₂O₃:Eu (red), YVO₄:Eu (red), and Zn₂SiO₄:Mn (green).

Alternatively, quantum dots can be used to tailor the down conversion process. As described in U.S. Pat. No. 6,744,960 different size quantum dots produce different color emissions. As applicable to this invention, quantum dots can comprise various materials including semiconductors such as zinc selenide (ZnSe), cadmium selenide (CdSe), cadmium sulfide (CdS), indium arsenide (InAs), and indium phosphide (InP). Another material that may suitably be employed is titanium dioxide (TiO₂). The size of the particle, i.e., the quantum dot, may range from about 2 to 10 nm. Since the size of these particles is so small, quantum physics governs many of the electrical and optical properties of the quantum dot. One such result of the application of quantum mechanics to the quantum dot is that quantum dots absorb a broad spectrum of optical wavelengths and re-emit radiation having a wavelength that is longer than the wavelength of the absorbed light. The wavelength of the emitted light is governed by the size of the quantum dot. For example, CdSe quantum dots 5.0 nm in diameter emit radiation having a narrow spectral distribution centered about 625 nm while quantum dots 18 including CdSe 2.2 nm in size emit light having a center wavelength of about 500 nm. Semiconductor quantum dots comprising CdSe, InP, and InAs, can emit radiation having center wavelengths in the range between 400 nm to about 1.5 µm. Titanium dioxide TiO₂ also emits in this range. The line width of the emission, i.e., full-width half-maximum (FWHM), for these semiconductor materials may range from about 20 to 30 nm. To produce this narrowband emission, quantum dots simply need to absorb light having wavelengths shorter than the wavelength of the light emitted by the dots. For example, for 5.0 nm diameter CdSe quantum dots, light having wavelengths shorter than about 625 nm is absorbed to produce emission at about 625 nm while for 2.2 nm quantum dots comprising CdSe light having wavelengths smaller than about 500 nm is absorbed and re-emitted at about 500 nm.

The converters in one embodiment can include a down converter including at least one of Y₂O₃; ZnS; ZnSe; MgS; CaS; Mn, Er ZnSe; Mn, Er MgS; Mn, Er CaS; Mn, Er ZnS; Mn, Yb ZnSe; Mn, Yb MgS; Mn, Yb CaS; Mn,Yb ZnS:Tb³⁺, Er³⁺; ZnS:Tb³⁺; Y₂O₃:Tb³⁺; Y₂O₃:Tb³⁺, Er3⁺; ZnS:Mn²⁺; ZnS:Mn,Er³⁺, alkali lead silicate including compositions of SiO₂, B₂O₃, Na₂O, K₂O, PbO, MgO, or Ag, and combinations or alloys or layers thereof.

In other embodiments, a metal coating or a metallic structure can exist inside the dielectric and the relative position of the metal structure to the dielectric structure can enhance plasmonic resonance. These structures with the metallic structure inside can be referred to as a metallic core up converter or a metallic core down converter. The metallic core technique for energy conversion is useful since it takes advantage of metal nanoparticles that have improved surface morphology compared to shell coatings on core dielectrics. The metal or metallic alloy in the inner core metallic energy converter can be selected to tune its plasmonic activity.

Such nanoparticle structures can exhibit in certain embodiments surface plasmonic resonance in the metallic shell to enhance upconversion of light from a first wavelength λ₁ to a second wavelength λ₂.

As described above, shell is in particular designed with a layer thickness to enhance the photon upconversion process through plasmonic enhancement. The thickness of the shell is "tuned" in its thickness to the absorption process by having a dimension in which plasmons (i.e., electrons oscillations) in shell have a resonance in frequency which provides spectral overlap with the absorption band of the incident light targeted. Thus, the thickness of the shell is "tuned" in a thickness to where a plasmon resonance resonates at a frequency of interest for stimulating a photoactivatable agent.

Such a plasmon resonating shell can be made of numerous transition metals, including though not limited to gold, silver, platinum, palladium, nickel, ruthenium, rhenium, copper, and cobalt. This capability of matching or tuning of the frequencies provides an enhancement of the absorption which would not be present with a dielectric core alone.

In one embodiment of this invention, the thickness of the metal shell is set depending on the emission frequency to enhance the total efficiency of the emission process. Accordingly, the thickness of the shell can be considered as a tool that in one instance enhances the absorption of λ₁, and in another instance can be considered as a tool that enhances the emission of λ₂, or in other situations can be considered an enhancement feature that in combination enhances the overall conversion process.

Additionally, plasmon-phonon coupling may be used to reduce a resonance frequency through the tuning of the bands to a degree off resonance. This may be useful in optimizing resonance energy transfer processes for the purpose of coupling the core-shell nanoparticles to sensitive chromophores or drug targets. Accordingly, when a recipient is outside of the shell, the recipient will receive enhanced light λ₂ by the above-described plasmonic effect than would occur if the shell were absent from the structure.

Accordingly, a plasmonics effect (from plasmonic inducing agents) is advantageous. A plasmonics effect can occur throughout the electromagnetic region provided the suitable nanostructures, nanoscale dimensions, metal types are used. Plasmonic effects are possible over a wide range of the electromagnetic spectrum, ranging from gamma rays and X rays throughout ultraviolet, visible, infrared, microwave and radio frequency energy.

### Photodynamic Therapy (PDT) with the Energy Modulation Agents (or Energy Converters) of the Invention (with or without Energy Augmentation):

In one embodiment of this invention, the above-described energy converters (phosphors, scintillators, fluorescent materials, and combinations and agglomerations thereof) with or without plasmonic inducing agents with and without energy augmentation can be used in photodynamic therapy for the light source. With the energy augmentators present, this invention can generate local regions of intense electric fields to enhance light emission from these energy converters in proximity to those local regions.

PDT is a light-based treatment, which has been approved by the United States Food & Drug Administration (FDA) for the treatment of both early and late-stage lung cancer. Other countries have approved PDT for treatment of various cancers as well. Unlike chemotherapy, radiation, and surgery, PDT is useful in treating all cell types, whether small cell or non-small cell carcinoma. PDT involves treatment of diseases such as cancer using light action on a special photoactive class of drugs, by photodynamic action in vivo to destroy or modify tissue [Dougherty T.J. and Levy J.G., "Photodynamic Therapy and Clinical Applications", in Biomedical Photonics Handbook, Vo-Dinh T., Ed., CRC Press, Boca Raton FL (2003)]. PDT, which was originally developed for treatment of various cancers, has now been used to include treatment of pre-cancerous conditions, e.g. actinic keratoses, high-grade dysplasia in Barrett's esophagus, and non-cancerous conditions, e.g. various eye diseases, e.g. age related macular degeneration (AMD). Photodynamic therapy (PDT) is approved for commercialization worldwide both for various cancers (lung, esophagus) and for AMD.

The PDT process requires three elements: (1) a PA drug (i.e., photosensitizer), (2) light that can excite the photosensitizer and (3) endogenous oxygen. The putative cytotoxic agent is singlet oxygen, an electronically excited state of ground state triplet oxygen formed according to the Type II photochemical process, as follows.

| | |
|---|---|
| PA + hv → ¹PA* (S) | Excitation |
| ¹PA* (S) → ³PA* (T) | Intersystem crossing for singlet to triplet state |
| ³PA* (T) + O₂ → ¹O*₂ + PA | Energy transfer from the drug to singlet oxygen |

where PA = photo-active drug at the ground state; ¹PA*(S) = excited singlet state; ³PA*(T) = excited triplet state; ¹O*₂ = singlet excited state of oxygen

Because the triplet state has a relatively long lifetime (µsec to seconds) only photosensitizers that undergo efficient intersystem crossing to the excited triplet state will have sufficient time for collision with oxygen in order to produce singlet oxygen. The energy difference between ground state and singlet oxygen is 94.2 kJ/mol and corresponds to a transition in the near-infrared at ~1270 nm. Most PA photosensitizers in clinical use have triplet quantum yields in the range of 40-60% with the singlet oxygen yield being slightly lower. Competing processes include loss of energy by deactivation to ground state by fluorescence or internal conversion (loss of energy to the environment or surrounding medium).

However, while a high yield of singlet oxygen is desirable it is by no means sufficient for a photosensitizer to be clinically useful. Pharmacokinetics, pharmacodynamics, stability in vivo and acceptable toxicity play critical roles as well [Henderson BW, Gollnick SO, "Mechanistic Principles of Photodynamic Therapy ", in Biomedical Photonics Handbook, Vo-Dinh T., Ed., CRC Press, Boca Raton FL (2003*)].* For example, it is desirable to have relatively selective uptake in the tumor or other tissue being treated relative to the normal tissue that necessarily will be exposed to the exciting light as well. Pharmacodynamic issues such as the subcellular localization of the photosensitizer may be important as certain organelles appear to be more sensitive to PDT damage than others (e.g. the mitochondria). Toxicity can become an issue if high doses of photosensitizer are necessary in order to obtain a complete response to treatment. An important mechanism associated with PDT drug activity involves apoptosis in cells. Upon absorption of light, the photosensitizer (PS) initiates chemical reactions that lead to the direct or indirect production of cytotoxic species such as radicals and singlet oxygen. The reaction of the cytotoxic species with subcellular organelles and macromolecules (proteins, DNA, etc.) leads to apoptosis and/or necrosis of the cells hosting the PDT drug. The preferential accumulation of PDT drug molecules in cancer cells combined with the localized delivery of light to the tumor, results in the selective destruction of the cancerous lesion. Compared to other traditional anticancer therapies, PDT does not involve generalized destruction of healthy cells. In addition to direct cell killing, PDT can also act on the vasculature, reducing blood flow to the tumor causing its necrosis. In particular cases it can be used as a less invasive alternative to surgery.

There are several chemical species used for PDT including porphyrin-based sensitizers. A purified hematoporphyrin derivative, Photofrin^{®}, has received approval of the US Food and Drug Administration. Porphyrins are generally used for tumors on or just under the skin or on the lining of internal organs or cavities because theses drug molecules absorbs light shorter than 640 nm in wavelength. For tumors occurring deep in tissue, second generation sensitizers, which have absorbance in the NIR region, such as porphyrin-based systems [R.K. Pandey, "Synthetic Strategies in designing Porphyrin-Based Photosensitizers', in Biomedical Photonics Handbook, Vo-Dinh T., Ed., CRC Press, Boca Raton FL (2003)], chlorines, phthalocyanine, and naphthalocyanine have been investigated.

PDT retains several photosensitizers in tumors for a longer time than in normal tissues, thus offering potential improvement in treatment selectivity. See Comer C., "Determination of [3H]- and [14C] hematoporphyrin derivative distribution in malignant and normal tissue," Cancer Res 1979, 3 9: 146- 15 1 ; Young SW, et al., "Lutetium texaphyrin (PCI-0123) a near-infrared, water-soluble photosensitizer," Photochem Photobiol 1996, 63:892-897; and Berenbaum MC, et al., "Meso-Tetra(hydroxyphenyl)porphyrins, a new class of potent tumor photosensitizers with favorable selectivity," Br J Cancer 1986, 54:717-725. Photodynamic therapy uses light of a specific wavelength to activate the photosensitizing agent. Various light sources have been developed for PDT, which include dye lasers and diode lasers. Light generated by lasers can be coupled to optical fibers that allow the light to be transmitted to the desired site. See Pass 1-11, "Photodynamic therapy in oncology: mechanisms and clinical use," J Natl Cancer Inst 1993, 85:443-456. According to researchers, the cytotoxic effect of PDT is the result of photooxidation reactions, as disclosed in Foote CS, "Mechanisms of photooxygenation," Proa Clin Biol Res 1984, 170:3-18. Light causes excitation of the photosensitizer, in the presence of oxygen, to produce various toxic species, such as singlet oxygen and hydroxyl radicals. It is not clear that direct damage to DNA is a major effect; therefore, this may indicate that photoactivation of DNA crosslinking is not stimulated efficiently.

### Photoactivation Treatments with the Energy Modulation Agents (or Energy Converters) of the Invention (with or without Energy Augmentation):

For the treatment of cell proliferation disorders, an initiation energy source (e.g., light from the phosphors or scintillators of the invention) can provide with or without energy augmentation an initiation energy that activates an activatable pharmaceutical agent to treat target cells within a subject. In one embodiment, the initiation energy source is applied indirectly to the activatable pharmaceutical agent, preferably in proximity to the target cells. Within the context of the present invention, the phrase "applied indirectly" (or variants of this phrase, such as "applying indirectly", "indirectly applies", "indirectly applied", "indirectly applying", etc.), when referring to the application of the initiation energy, means the penetration by the initiation energy into the subject beneath the surface of the subject and to the activatable pharmaceutical agent within a subject.

Although not intending to be bound by any particular theory or be otherwise limited in any way, the following theoretical discussion of scientific principles and definitions are provided to help the reader gain an understanding and appreciation of the present invention. As used herein, the term "subject" is not intended to be limited to humans, but may also include animals, plants, or any suitable biological organism.

As used herein, the phrase "cell proliferation disorder" refers to any condition where the growth rate of a population of cells is less than or greater than a desired rate under a given physiological state and conditions. Although, preferably, the proliferation rate that would be of interest for treatment purposes is faster than a desired rate, slower than desired rate conditions may also be treated by methods of the present invention. Exemplary cell proliferation disorders may include, but are not limited to, cancer, bacterial infection, immune rejection response of organ transplant, solid tumors, viral infection, autoimmune disorders (such as arthritis, lupus, inflammatory bowel disease, Sjogrens syndrome, multiple sclerosis) or a combination thereof, as well as aplastic conditions wherein cell proliferation is low relative to healthy cells, such as aplastic anemia. Particularly preferred cell proliferation disorders for treatment using the present methods are cancer, staphylococcus aureus (particularly antibiotic resistant strains such as methicillin resistant staphylococcus aureus or MRSA), and autoimmune disorders.

As used herein, an "activatable pharmaceutical agent" (alternatively called a "photoactive agent" or PA) is an agent that normally exists in an inactive state in the absence of an activation signal. When the agent is activated by a matching activation signal under activating conditions, it is capable of affecting the desired pharmacological effect on a target cell (i.e. preferably a predetermined cellular change).

Signals that may be used to activate a corresponding agent may include, but are not limited to, photons of specific wavelengths (e.g. x-rays, or visible light), together with or without electromagnetic energy (e.g. radio or microwave), thermal energy, acoustic energy, or any combination thereof.

Activation of the agent may be as simple as delivering the signal to the agent or may further premise on a set of activation conditions. For example, in the former case, an activatable pharmaceutical agent, such as a photosensitizer, may be activated by UV-A radiation (e.g., UV-A light from the phosphors or scintillators of the invention). Once activated, the agent in its active-state may then directly proceed to effect a cellular change.

Where activation may further premise upon other conditions, mere delivery of the activation signal may not be sufficient to bring about the desired cellular change. For example, a photoactive compound that achieves its pharmaceutical effect by binding to certain cellular structure in its active state may require physical proximity to the target cellular structure when the activation signal is delivered. For such activatable agents, delivery of the activation signal under non-activating conditions will not result in the desired pharmacologic effect. Some examples of activating conditions may include, but are not limited to, temperature, pH, location, state of the cell, presence or absence of co-factors. Selection of an activatable pharmaceutical agent greatly depends on a number of factors such as the desired cellular change, the desired form of activation, as well as the physical and biochemical constraints that may apply.

When activated, the activatable pharmaceutical agent may effect cellular changes that include, but are not limited to, apoptosis, redirection of metabolic pathways, up-regulation of certain genes, down-regulation of certain genes, secretion of cytokines, alteration of cytokine receptor responses, production of reactive oxygen species or combinations thereof.

The mechanisms by which an activatable pharmaceutical agent may achieve its desired effect are not particularly limited. Such mechanisms may include direct action on a predetermined target as well as indirect actions via alterations to the biochemical pathways. A preferred direct action mechanism is by binding the agent to a critical cellular structure such as nuclear DNA, mRNA, rRNA, ribosome, mitochondrial DNA, or any other functionally important structures. Indirect mechanisms may include releasing metabolites upon activation to interfere with normal metabolic pathways, releasing chemical signals (e.g. agonists or antagonists) upon activation to alter the targeted cellular response, and other suitable biochemical or metabolic alterations.

The treatment can be by those methods described in U.S .Application Serial No. 11/935,655, filed November 6, 2007 or by a modified version of a conventional treatment such as PDT, but using a plasmonics-active agent to enhance the treatment by modifying or enhancing the applied energy or, in the case of using an energy converter, modifying either the applied energy, the emitted energy from the energy converter, or both.

In one embodiment, the activatable pharmaceutical agent is capable of chemically binding to the DNA or mitochondria at a therapeutically effective amount. In this embodiment, the activatable pharmaceutical agent, preferably a photoactivatable agent, is exposed *in situ* to an activating energy emitted from an energy converter such as the phosphors or scintillators of the invention, which, in turn receives energy from an initiation energy source.

The activatable agent may be derived from a natural or synthetic origin. Any such molecular entity that may be activated by a suitable activation signal source to effect a predetermined cellular change may be advantageously employed in the present invention. Suitable photoactive agents include, but are not limited to: psoralens and psoralen derivatives, pyrene cholesteryloleate, acridine, porphyrin, fluorescein, rhodamine, 16-diazorcortisone, ethidium, transition metal complexes of bleomycin, transition metal complexes of deglycobleomycin, organoplatinum complexes, alloxazines such as 7,8-dimethyl-10-ribityl isoalloxazine (riboflavin), 7,8,10-trimethylisoalloxazine (lumiflavin), 7,8-dimethylalloxazine (lumichrome), isoalloxazine-adenine dinucleotide (flavine adenine dinucleotide [FAD]), alloxazine mononucleotide (also known as flavine mononucleotide [FMN] and riboflavine-5-phosphate), vitamin Ks, vitamin L, their metabolites and precursors, and napththoquinones, naphthalenes, naphthols and their derivatives having planar molecular conformations, porphyrins, dyes such as neutral red, methylene blue, acridine, toluidines, flavine (acriflanvine hydrochloride) and phenothiazine derivatives, coumarins, quinolones, quinones, and anthroquinones, aluminum (111) phthalocyanine tetrasulfonate, hematoporphyrin, and phthalocyanine, and compounds which preferentially adsorb to nucleic acids with little or no effect on proteins. The term "alloxazine" includes isoalloxazines.

Endogenously-based derivatives include synthetically derived analogs and homologs of endogenous photoactivated molecules, which may have or lack lower (1 to 5 carbons) alkyl or halogen substituents of the photosensitizers from which they are derived, and which preserve the function and substantial non-toxicity. Endogenous molecules are inherently nontoxic and may not yield toxic photoproducts after photoradiation.

Table 10 below lists some photoactivatable molecules capable of being photoactivated to induce an auto vaccine effect.

| SSET and TTET rate constants for bichromophoric peptides | | | | | | | | | | |
|---|---|---|---|---|---|---|---|---|---|---|
| Compound | λₑₓ (nm) | *E_{SSET}* | *kₛ* of donor (s⁻¹) | k*_{SSET}* (s⁻¹) | k*_{SSET}*(s⁻¹) (Average) | R₀ (Å) | R(Å) | R*_{model}*(Å) (Average) | E*_{TTET}⁻* | k*_{TTET}*(s⁻¹) |
| 1B | 224 | 96.3 | 9.5 × 10⁵ | 2.44 × 10⁸ | 1.87 × 10³ | 14.7 | 9 | 9.5 | | |
| | 266 | 95 | | 1.8 × 10⁸ | | | | | 2.5 | 5 × 10² |
| | 280 | 94 | | 1.36 × 10⁸ | | | | | | |
| 1A | 224 | 80 | 9.5 × 10⁶ | 3.8 × 10⁷ | 3.67 × 10⁷ | 14.7 | 11.8 | 14.1 | | |
| | 266 | 79 | | 3.6 × 10⁷ | | | | | 2 | 3.6 × 10² |
| | 280 | 79 | | 3.6 × 10⁷ | | | | | | |
| 2B | 224 | 77 | 9.5 × 10⁵ | 3.1 × 10⁷ | 3.9 × 10⁷ | 14.7 | 11.9 | 6.5 | | |
| | 266 | 81 | | 3.9 × 10⁷ | | | | | 32 | 9.4 × 10³ |
| | 280 | 83 | | 4.7 × 10⁷ | | | | | | |
| 2A | 224 | 69 | 9.5 × 10⁵ | 2.1 × 10⁷ | 3 × 10⁷ | 14.7 | 12.2 | 8.1 | 74.3 | 5.7 × 10⁴ |
| | 266 | 80 | | 1.7 × 10⁷ | | | | | | |
| | 280 | 77 | | 3.2 × 10⁷ | | | | | | |

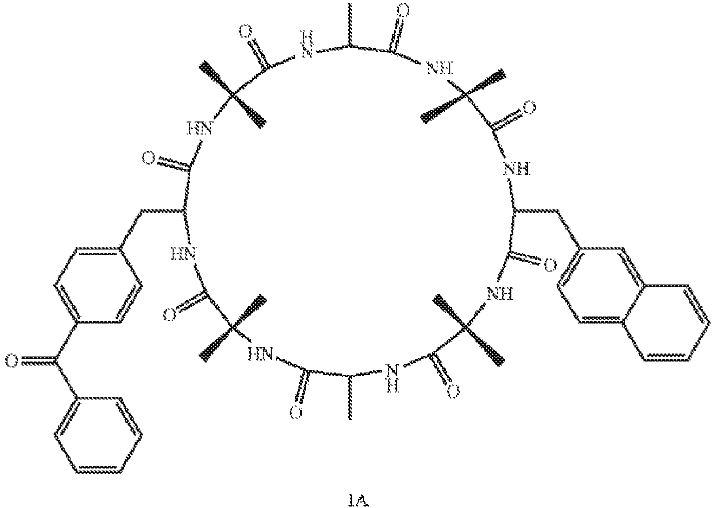

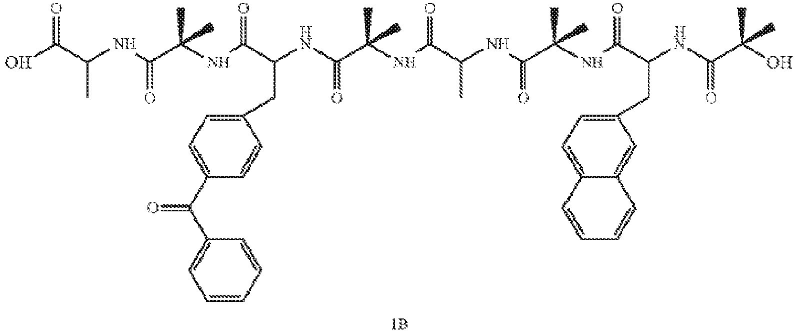

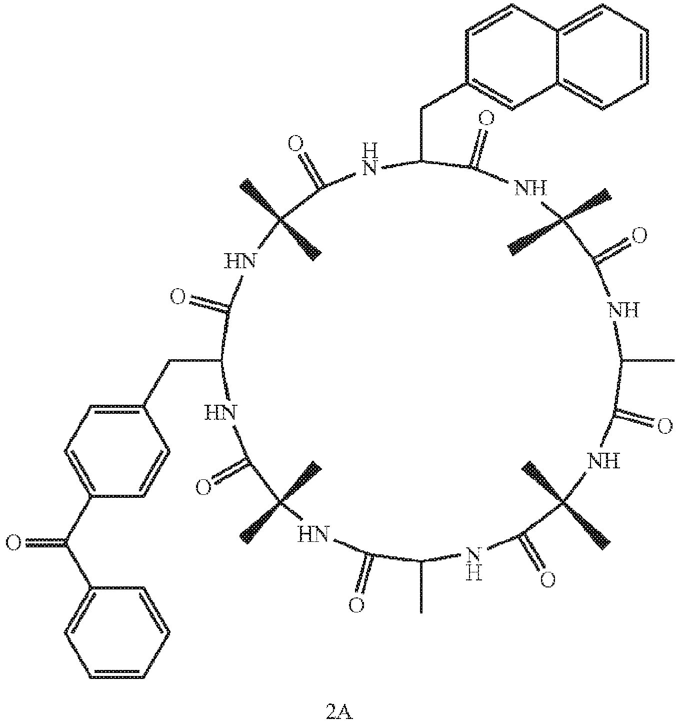

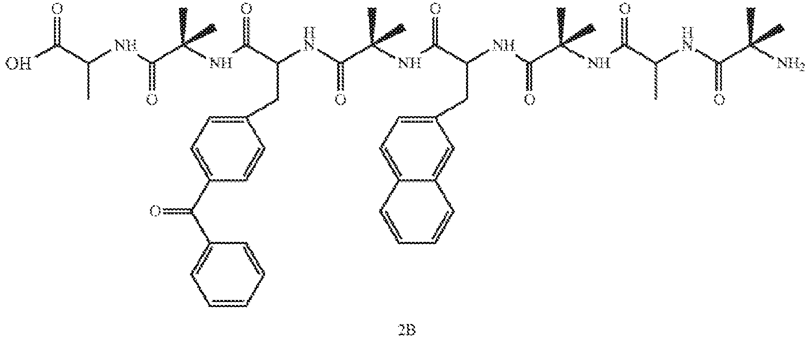

Table 11 below lists some additional endogenous photoactivatable molecules.

Biocompatible, endogenous fluorophore emitters.

| Endogenous Fluorophores | Excitation Max. (nm) | Emission Max. (nm) |
|---|---|---|
| Amino acids: | | |
| Tryptophan | 280 | 350 |
| Tyrosine | 275 | 300 |
| Phenylalanine | 260 | 280 |
| Structural Proteins: | | |
| Collagen | 325, 360 | 400, 405 |
| Elastin | 290, 325 | 340, 400 |
| Enzymes and Coenzymes: | | |
| flavin adenine dinucleotide | 450 | 535 |
| reduced nicotinamide dinucelotide | 290, 351 | 440, 460 |
| reduced nicotinamide dinucelotide | 336 | 464 |
| phosphate | | |
| Vitamins: | | |
| Vitamins A | 327 | 510 |
| Vitamins K | 335 | 480 |
| Vitamins D | 390 | 480 |
| Vitamins B₆ compounds: | | |
| Pyridoxine | 332, 340 | 400 |
| Pyridoxamine | 335 | 400 |
| Pyridoxal | 330 | 385 |
| Pyridoxic acid | 315 | 425 |
| Pyridoxal phosphate | 5'-330 | 400 |
| Vitamin B₁₂ | 275 | 305 |
| Lipids: | | |
| Phospholipids | 436 | 540, 560 |
| Lipofuscin | 340-395 | 540, 430-460 |
| Ceroid | 340-395 | 430-460, 540 |
| Porphyrins | 400-450 | 630, 690 |

The nature of the predetermined cellular change will depend on the desired pharmaceutical outcome. Exemplary cellular changes may include, but are not limited to, apoptosis, necrosis, up-regulation of certain genes, down-regulation of certain genes, secretion of cytokines, alteration of cytokine receptor responses, or a combination thereof.

Energy from light emitted from the phosphors, scintillators, fluorescent materials, and combinations and agglomerations thereof, with or without plasmonic inducing agents, of the invention may be transferred from one molecule to another (intermolecular transfer) or from one part of a molecule to another part of the same molecule (intramolecular transfer). For example, the electromagnetic energy may be converted into thermal energy. Energy transfer processes are also referred to as molecular excitation.

Additionally, energy converters may be included in the medium to be treated. The energy converters may upon receiving of light from the phosphors or scintillators of the invention re-emit a light specific to a desired photo-driven reaction. Energy converters can have a very short energy retention time (on the order of fs-ns, e.g. fluorescent molecules) whereas others may have a very long half-life (on the order of seconds to hours, e.g. luminescent inorganic molecules or phosphorescent molecules). Various exemplary uses of these are described below in preferred embodiments.

The converters may further be coupled to a carrier for cellular targeting purposes. For example, a biocompatible molecule, such as a fluorescing metal nanoparticle or fluorescing dye molecule that emits in the UV-A band, may be selected as the energy converter.

The energy converter of the invention such as the phosphors, scintillators, fluorescent materials, and combinations and agglomerations thereof, with or without plasmonic inducing agents and with or without energy augmentation may be preferably directed to the desired site (e.g. a tumor) by systemic administration to a subject. For example, a UV-A emitting energy converter may be concentrated in the tumor site by physical insertion or by conjugating the UV-A emitting energy converter with a tumor specific carrier, such as an antibody, nucleic acid, peptide, a lipid, chitin or chitin-derivative, a chelate, a surface cell receptor, molecular imprints, aptamers, or other functionalized carrier that is capable of concentrating the UV-A emitting source in a specific target tumor. With the energy augmentators present, this invention can generate local regions of intense electric fields to enhance light emission from the UV-A emitting energy converters in proximity to those local regions.

Additionally, the energy converter can be used alone or as a series of two or more energy converters wherein the energy converters provide an energy cascade from the light of the phosphors or scintillators. Thus, the first energy converter in the cascade will absorb the activation energy, convert it to a different energy which is then absorbed by the second energy modulation in the cascade, and so forth until the end of the cascade is reached with the final energy converter in the cascade emitting the energy necessary to activate the activatable pharmaceutical agent.

Although the activatable pharmaceutical agent and the energy converter can be distinct and separate, it will be understood that the two agents need not be independent and separate entities. In fact, the two agents may be associated with each other via number of different configurations. Where the two agents are independent and separately movable from each other, they generally interact with each other via diffusion and chance encounters within a common surrounding medium. Where the activatable pharmaceutical agent and the energy converter are not separate, they may be combined into one single entity.

In general, photoactivatable agents may be stimulated by light of from the phosphors or scintillators of the invention or the other above-described energy converters, leading to subsequent irradiation, resonance energy transfer, exciton migration, electron injection, or chemical reaction, to an activated energy state that is capable of effecting the predetermined cellular change desired. In a one embodiment, the photoactivatable agent, upon activation, binds to DNA or RNA or other structures in a cell. The activated energy state of the agent is capable of causing damage to cells, inducing apoptosis. The mechanism of apoptosis is associated with an enhanced immune response that reduces the growth rate of cell proliferation disorders and may shrink solid tumors, depending on the state of the patient's immune system, concentration of the agent in the tumor, sensitivity of the agent to stimulation, and length of stimulation.

A preferred method of treating a cell proliferation disorder of the invention administers a photoactivatable agent to a patient, stimulates the photoactivatable agent by light from the phosphors or scintillators of the invention to induce cell damage, and generates an auto vaccine effect. In one further preferred embodiment, the photoactivatable agent is stimulated via resonance energy transfer occurring with or without energy augmentation. With the energy augmentators present, this invention can generate local regions of intense electric fields to enhance resonance energy transfer in proximity to those local regions.

One advantage is that multiple wavelengths of emitted radiation from the light from the phosphors or scintillators of the invention may be used to selectively stimulate one or more photoactivatable agents or energy converters capable of stimulating the one or more photoactivatable agents. The energy converter is preferably stimulated at a wavelength and energy that causes little or no damage to healthy cells, with the energy from one or more energy converters being transferred, such as by Foerster Resonance Energy Transfer, to the photoactivatable agents that damage the cell and cause the onset of the desired cellular change, such as apoptosis of the cells.

Another advantage is that side effects can be greatly reduced by limiting the production of free radicals, singlet oxygen, hydroxides and other highly reactive groups that are known to damage healthy cells. Furthermore, additional additives, such as antioxidants, may be used to further reduce undesired effects of irradiation.

Resonance Energy Transfer (RET) is an energy transfer mechanism between two molecules having overlapping emission and absorption bands. In the present invention, the energy augmentators produce regions of intensified electric fields which can enhance or accelerate the transfer. In general, electromagnetic emitters are capable of converting an arriving wavelength to a longer wavelength. For example, UV-B energy absorbed by a first molecule may be transferred by a dipole-dipole interaction to a UV-A-emitting molecule in close proximity to the UV-B-absorbing molecule.

Alternatively, a material absorbing a shorter wavelength may be chosen to provide RET to a non-emitting molecule that has an overlapping absorption band with the transferring molecule's emission band. Alternatively, phosphorescence, chemiluminescence, or bioluminescence may be used to transfer energy to a photoactivatable molecule.

In another embodiment, the invention includes the administration of the activatable pharmaceutical agent, along with administration of a source of chemical energy such as chemiluminescence, phosphorescence or bioluminescence. With the energy augmentators present, this invention can generate local regions of intense electric fields to enhance luminescence from these sources of chemical energy in proximity to those local regions. The source of chemical energy can be a chemical reaction between two or more compounds, or can be induced by activating a chemiluminescent, phosphorescent or bioluminescent compound with an appropriate activation energy, either outside the subject or inside the subject, with the chemiluminescence, phosphorescence or bioluminescence being allowed to activate the activatable pharmaceutical agent *in vivo* after administration. The administration of the activatable pharmaceutical agent and the source of chemical energy can be performed sequentially in any order or can be performed simultaneously. In the case of certain sources of such chemical energy, the administration of the chemical energy source can be performed after activation outside the subject, with the lifetime of the emission of the energy being up to several hours for certain types of phosphorescent materials for example. There are no known previous efforts to use resonance energy transfer of any kind to activate an intercalator to bind DNA.

When drug molecules absorb excitation light, electrons undergo transitions from the ground state to an excited electronic state. The electronic excitation energy subsequently relaxes via radiative emission (luminescence) and radiationless decay channels. When a molecule absorbs excitation energy, it is elevated from *Sₒ* to some vibrational level of one of the excited singlet states, *Sₙ,* in the manifold *S₁* ,..., *Sₙ.* In condensed media (tissue), the molecules in the *Sₙ* state deactivate rapidly, within 10⁻¹³ to 10⁻¹¹ s via vibrational relaxation (VR) processes, ensuring that they are in the lowest vibrational levels of *Sₙ* possible. Since the VR process is faster than electronic transitions, any excess vibrational energy is rapidly lost as the molecules are deactivated to lower vibronic levels of the corresponding excited electronic state. This excess VR energy is released as thermal energy to the surrounding medium. From the *Sₙ* state, the molecule deactivates rapidly to the isoenergetic vibrational level of a lower electronic state such as *S*_{*n* -1} via an internal conversion (IC) process. IC processes are transitions between states of the same multiplicity. The molecule subsequently deactivates to the lowest vibronic levels of *S*_{*n*-1} via VR process. By a succession of IC processes immediately followed by VR processes, the molecule deactivates rapidly to the ground state *S*_{*1*.}. This process results in excess VR and IC energy released as thermal energy to the surrounding medium leading to the overheating of the local environment surrounding the light absorbing drug molecules. The heat produced results in local cell or tissue destruction. The light absorbing species include natural chromophores in tissue or exogenous dye compounds such as indocyanine green, naphthalocyanines, and porphyrins coordinated with transition metals and metallic nanoparticles and nanoshells of metals. Natural chromophores, however, suffer from very low absorption. The choice of the exogenous photothermal agents is made on the basis of their strong absorption cross sections and highly efficient light-to-heat conversion. This feature greatly minimizes the amount of energy needed to induce local damage of the diseased cells, making therapy method less invasive.

### Various Light-Activated Pharmaceuticals Activatable with the Energy Modulation Agents (or Energy Converters) of the Invention (with and without energy augmentation):

Another object of the invention is to treat with or without energy augmentation a condition, disorder or disease in a subject using an activatable pharmaceutical agent activated using the above-described energy converters (phosphors, scintillators, fluorescent materials, and combinations and agglomerations thereof) with or without plasmonic inducing agents. With the energy augmentators present, this invention can generate local regions of intense electric fields to enhance light emission from these energy converters in proximity to those local regions.

It has been reported that ferritin could be internalized by some tumor tissues, and the internalization was associated with the membrane-specific receptors [S. Fargion, P. Arosio, A. L. Fracanzoni, V. Cislaghi, S. Levi, A. Cozzi, A Piperno and A. G. Firelli, Blood, 1988, 71, 753-757; P. C. Adams, L. W. Powell and J. W. Halliday, Hepatology, 1988, 8, 719-721]. Previous studies have shown that ferritin-binding sites and the endocytosis of ferritin have been identified in neoplastic cells [M. S. Bretscher and J. N. Thomson, EMBO J., 1983, 2, 599-603]. Ferritin receptors have the potential for use in the delivery of anticancer drugs into the brain [S. W. Hulet, S. Powers and J. R. Connor, J. Neural. Sci., 1999, 165, 48-55]. In one embodiment, the invention uses ferritin or apoferritin to both encapsulate PA and energy converter-PA systems and also target tumor cells selectively for enhanced drug delivery and subsequent phototherapy. In this case, no additional bioreactors are needed.

The photoactive drug molecules can be given to a patient by oral ingestion, skin application, or by intravenous injection. The photoactive drug molecules drugs travel through the blood stream inside the body towards the targeted tumor (either via passive or active targeting strategies). The invention treatment may also be used for inducing an auto vaccine effect for malignant cells, including those in solid tumors. To the extent that any rapidly dividing cells or stem cells may be damaged by a systemic treatment, then it may be preferable to direct the stimulating energy directly toward the tumor, preventing damage to most normal, healthy cells or stem cells by avoiding photoactivation or resonant energy transfer of the photoactivatable agent.

Alternatively, a treatment may be applied that slows or pauses mitosis. Such a treatment is capable of slowing the division of rapidly dividing healthy cells or stem cells during the treatment, without pausing mitosis of cancerous cells. Alternatively, a blocking agent is administered preferentially to malignant cells prior to administering the treatment that slows mitosis.

In one embodiment, an aggressive cell proliferation disorder has a much higher rate of mitosis, which leads to selective destruction of a disproportionate share of the malignant cells during even a systemically administered treatment. Stem cells and healthy cells may be spared from wholesale programmed cell death, even if exposed to photoactivated agents, provided that such photoactivated agents degenerate from the excited state to a lower energy state prior to binding, mitosis or other mechanisms for creating damage to the cells of a substantial fraction of the healthy stem cells. Thus, an auto-immune response may not necessarily be induced or required.

Alternatively, a blocking agent may be used that prevents or reduces damage to stem cells or healthy cells, selectively, which would otherwise be impaired. The blocking agent is selected or is administered such that the blocking agent does not impart a similar benefit to malignant cells, for example.

In one embodiment, stem cells are targeted, specifically, for destruction with the intention of replacing the stem cells with a donor cell line or previously stored, healthy cells of the patient. In this case, no blocking agent is used. Instead, a carrier or photosensitizer is used that specifically targets the stem cells.

Work in the area of photodynamic therapy has shown that the amount of singlet oxygen required to cause cell lysis, and thus cell death, is 0.32 × 10⁻³ mol/liter or more, or 10⁹ singlet oxygen molecules/cell or more. However, in one embodiment of the invention, it is most preferable to avoid production of an amount of singlet oxygen that would cause cell lysis, due to its indiscriminate nature of attack, lysing both target cells and healthy cells. Accordingly, it is most preferred in the invention that the level of singlet oxygen production caused by the initiation energy used or activatable pharmaceutical agent upon activation is less than level needed to cause cell lysis.

In a further embodiment, methods in accordance with the invention may further include adding an additive to alleviate treatment side-effects. Exemplary additives may include, but are not limited to, antioxidants, adjuvant, or combinations thereof. In one exemplary embodiment, psoralen is used as the activatable pharmaceutical agent, UV-A is used as the activating energy, and antioxidants are added to reduce the unwanted side-effects of irradiation.

The activatable pharmaceutical agent and derivatives thereof as well as the energy converter and plasmonics compounds and structures and with or without energy augmentation, can be incorporated into pharmaceutical compositions suitable for administration. Such compositions typically comprise the activatable pharmaceutical agent and a pharmaceutically acceptable carrier. The pharmaceutical composition also comprises at least one additive having a complementary therapeutic or diagnostic effect, wherein the additive is one selected from an antioxidant, an adjuvant, or a combination thereof.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions. Modifications can be made to the compound of the present invention to affect solubility or clearance of the compound. These molecules may also be synthesized with D-amino acids to increase resistance to enzymatic degradation. If necessary, the activatable pharmaceutical agent can be co-administered with a solubilizing agent, such as cyclodextran.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, rectal administration, and direct injection into the affected area, such as direct injection into a tumor. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811, the entire contents of which are incorporated herein by reference.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, kit or dispenser together with instructions for administration.

Methods of administering agents are not limited to the conventional means such as injection or oral infusion, but include more advanced and complex forms of energy transfer. For example, genetically engineered cells that carry and express energy converters may be used. Cells from the host may be transfected with genetically engineered vectors that express bioluminescent agents. Transfection may be accomplished via *in situ* gene therapy techniques such as injection of viral vectors or gene guns, or may be performed *ex vivo* by removing a sample of the host's cells and then returning to the host upon successful transfection. Such transfected cells may be inserted or otherwise targeted at the site where diseased cells are located.

It will also be understood that the order of administering the different agents is not particularly limited. It will be appreciated that different combinations of ordering may be advantageously employed depending on factors such as the absorption rate of the agents, the localization and molecular trafficking properties of the agents, and other pharmacokinetics or pharmacodynamics considerations.

An advantage of the methods of this approach is that by specifically targeting cells affected by a cell proliferation disorder, such as rapidly dividing cells, and triggering with or without energy augmentation a cellular change, such as apoptosis, in these cells *in situ,* the immune system of the host may be stimulated to have an immune response against the diseased cells. Once the host's own immune system is stimulated to have such a response, other diseased cells that are not treated by the activatable pharmaceutical agent may be recognized and be destroyed by the host's own immune system. Such autovaccine effects may be obtained, for example, in treatments using psoralen and UV-A.

The methods described here can be used alone or in combination with other therapies for treatment of cell proliferation disorders. Additionally, the methods described can be used, if desired, in conjunction with recent advances in chronomedicine, such as that detailed in Giacchetti et al, Journal of Clinical Oncology, Vol 24, No 22 (August 1), 2006: pp. 3562-3569,

In chronomedicine, it has been found that cells suffering from certain types of disorders, such as cancer, respond better at certain times of the day than at others. Thus, chronomedicine could be used in conjunction with the present methods in order to augment the effect of the treatments of the invention.

### Photo-treatment with the Energy Modulation Agents (or Energy Converters) of the Invention (with and without Energy Augmentation)

Another object of the invention is to treat a condition, disorder or disease in a subject using an activatable pharmaceutical agent activated using the above-described energy converters (phosphors, scintillators, fluorescent materials, and combinations and agglomerations thereof) with or without plasmonic inducing agents and with or without energy augmentation. Exemplary conditions, disorders or diseases may include, but are not limited to, cancer, autoimmune diseases, cardiac ablation (e.g., cardiac arrhythmia and atrial fibrillation), photoangioplastic conditions (e.g., *de novo* atherosclerosis, restenosis), intimal hyperplasia, arteriovenous fistula, macular degeneration, psoriasis, acne, alopecia areata, portwine spots, hair removal, rheumatoid and inflammatory arthritis, joint conditions, lymph node conditions, and cognitive and behavioral conditions. With the energy augmentators present, this invention can generate local regions of intense electric fields to enhance light emission from these energy converters or energy converters in proximity to those local regions.

Accordingly, the invention in one embodiment provides methods utilizing the principle of energy transfer to and among molecular agents to control delivery and activation of pharmaceutically active agents such that delivery of the desired pharmacological effect is more focused, precise, and effective than the conventional techniques. Here, the energy transfer can include light from the phosphors or scintillators.

Although not intending to be bound by any particular theory or be otherwise limited in any way, the following theoretical discussion of scientific principles and definitions are provided to help the reader gain an understanding and appreciation of the present invention.

As used here, the term "subject" is not intended to be limited to humans, but may also include animals, plants, or any suitable biological organism.

As used herein, the phrase "a disease or condition" refers to a condition, disorder or disease that may include, but are not limited to, cancer, soft and bone tissue injury, chronic pain, wound healing, nerve regeneration, viral and bacterial infections, fat deposits (liposuction), varicose veins, enlarged prostate, retinal injuries and other ocular diseases, Parkinson's disease, and behavioral, perceptional and cognitive disorders. Exemplary conditions also may include nerve (brain) imaging and stimulation, a direct control of brain cell activity with light, control of cell death (apoptosis), and alteration of cell growth and division. Yet other exemplary a condition, disorder or disease may include, but are not limited to, cardiac ablation (e.g., cardiac arrhythmia and atrial fibrillation), photoangioplastic conditions (e.g., *de novo* atherosclerosis, restenosis), intimal hyperplasia, arteriovenous fistula, macular degeneration, psoriasis, acne, alopecia areata, portwine spots, hair removal, rheumatoid and inflammatory arthritis, joint conditions, and lymph node conditions.

As used here, the term "target structure" refers to a eukaryotic cell, prokaryotic cell, a subcellular structure, such as a cell membrane, a nuclear membrane, cell nucleus, nucleic acid, mitochondria, ribosome, or other cellular organelle or component, an extracellular structure, virus or prion, and combinations thereof.

The nature of the predetermined cellular change will depend on the desired pharmaceutical outcome. Exemplary cellular changes may include, but are not limited to, apoptosis, necrosis, up-regulation of certain genes, down-regulation of certain genes, secretion of cytokines, alteration of cytokine receptor responses, regulation of cytochrome c oxidase and flavoproteins, activation of mitochondria, stimulation antioxidant protective pathway, modulation of cell growth and division, alteration of firing pattern of nerves, alteration of redox properties, generation of reactive oxygen species, modulation of the activity, quantity, or number of intracellular components in a cell, modulation of the activity, quantity, or number of extracellular components produced by, excreted by, or associated with a cell, or a combination thereof. Predetermined cellular changes may or may not result in destruction or inactivation of the target structure.

As used here, an "energy converter" refers to an agent that is capable of receiving an energy input from a source and then re-emitting a different energy to a receiving target. Energy transfer among molecules may occur in a number of ways. The form of energy may be electronic, thermal, electromagnetic, kinetic, or chemical in nature. Energy may be transferred from one molecule to another (intermolecular transfer) or from one part of a molecule to another part of the same molecule (intramolecular transfer). For example, a converter may receive electromagnetic energy and re-emit the energy in the form of thermal energy. In various embodiments, the energy converters receive higher energy (e.g. x-ray) and re-emits in lower energy (e.g. UV-A). Some converters may have a very short energy retention time (on the order of fs, e.g. fluorescent molecules) whereas others may have a very long half-life (on the order of minutes to hours, e.g. luminescent or phosphorescent molecules). Suitable energy converters include, but are not limited to, a phosphor, a scintillator, a biocompatible fluorescing metal nanoparticle, fluorescing dye molecule, gold nanoparticle, a water soluble quantum dot encapsulated by polyamidoamine dendrimers, a luciferase, a biocompatible phosphorescent molecule, a combined electromagnetic energy harvester molecule, an up-converter, and a lanthanide chelate capable of intense luminescence. Various exemplary uses of these are described below.

U.S. Pat. No. 7,008,559 describes the upconversion performance of ZnS where excitation at 767 nm produces emission in the visible range. The materials described in U.S. Pat. No. 7,008,559 (including the ZnS as well as Er³⁺ doped BaTiO₃ nanoparticles and Yb³⁺ doped CsMnCl₃) are suitable in various embodiments of the invention.

Further materials suitable as energy converters include, but are not limited to, CdTe, CdSe, ZnO, CdS, Y₂O₃, MgS, CaS, SrS and BaS. Such materials may be any semiconductor and more specifically, but not by way of limitation, sulfide, telluride, selenide, and oxide semiconductors and their nanoparticles, such as Zn₁₋ₓMnₓS_{y}, Zn₁₋ₓMnₓSe_{y}, Zn₁₋ₓMnₓTe_{y}, Cd₁₋ₓMnS_{y}, Cd₁₋ₓMnₓSe_{y}, Cd₁₋ₓMnₓTe_{y}, Pb₁₋ₓMnₓS_{y}, Pb₁₋ₓMnₓSe_{y}, Pb₁₋ₓMnₓTe_{y}, Mg₁₋ₐMnS_{y}, Ca₁₋ₓMnₓS_{y}, Ba₁₋ₓMnₓS_{y} and Sr₁₋ₓ, etc. (wherein, 0<x≦1, and 0<y≦1). Complex compounds of the above-described semiconductors are also contemplated for use in the invention--e.g. (M_{1-z}N_{z})₁₋ₓMnₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, Se, Te, O; 0<x≦1, 0<y≦1, 0<z≦1). Two examples of such complex compounds are Zn_{0.4}Cd_{0.4}Mn_{0.2}S and Zn_{0.9}Mn_{0..1}S_{0.8}Se_{0.2}. Additional energy modulation materials include insulating and nonconducting materials such as BaF₂, BaFBr, and BaTiO₃, to name but a few exemplary compounds. Transition and rare earth ion co-doped semiconductors suitable for the invention include sulfide, telluride, selenide and oxide semiconductors and their nanoparticles, such as ZnS; Mn; Er; ZnSe; Mn, Er; MgS; Mn, Er; CaS; Mn, Er; ZnS; Mn, Yb; ZnSe; Mn, Yb; MgS; Mn, Yb; CaS; Mn,Yb etc., and their complex compounds: (M_{1-z}N_{z})₁₋ₓ(Mn_{q}R_{1-q})ₓA_{1-y}B_{y} (M=Zn, Cd, Pb, Ca, Ba, Sr, Mg; N=Zn, Cd, Pb, Ca, Ba, Sr, Mg; A=S, Se, Te, O; B=S, ...0<z<1, o<q<1).

Some nanoparticles such as ZnS:Tb³⁺, Er³⁺; ZnS:Tb³⁺; Y₂O₃:Tb³⁺; Y₂O₃Tb³⁺, Er3⁺; ZnS:Mn²⁺; ZnS:Mn,Er³⁺ are known in the art to function for both down-conversion luminescence and upconversion luminescence, and can thus be used in various embodiments of the present invention.

The converters may further be coupled to a carrier for cellular targeting purposes. For example, a biocompatible molecule, such as a fluorescing metal nanoparticle or fluorescing dye molecule that emits in the UV-A band, may be selected as the energy converter.

The energy converter may be preferably directed to the desired site (e.g. a tumor) by systemic administration to a subject. For example, a UV-A emitting energy converter may be concentrated in the tumor site by physical insertion or by conjugating the UV-A emitting energy converter with a tumor specific carrier, such as a lipid, chitin or chitin-derivative, a chelate or other functionalized carrier that is capable of concentrating the UV-A emitting source in a specific target tumor.

Additionally, the energy converter can be used alone or as a series of two or more other energy converters wherein the energy converters provide an energy cascade. Thus, the first energy converter in the cascade will absorb the activation energy, convert it to a different energy which is then absorbed by the second energy modulation in the cascade, and so forth until the end of the cascade is reached with the final energy converter in the cascade emitting the energy necessary to activate the activatable pharmaceutical agent.

Signals that may be used to activate a corresponding agent may include, but are not limited to, photons of specific wavelengths (e.g. x-rays, ultraviolet, or visible light), electromagnetic energy (e.g. radio or microwave), thermal energy, acoustic energy, or any combination thereof.

Activation of the agent may be as simple as delivering the signal to the agent or may further premise on a set of activation conditions. For example, an activatable pharmaceutical agent, such as a photosensitizer, may be activated by UV-A radiation from the phosphors or scintillators of the invention. Once activated, the agent in its active-state may then directly proceed to effect a cellular change.

Where activation may further premise upon other conditions, mere delivery of the activation signal may not be sufficient to bring about the desired cellular change. For example, a photoactive compound that achieves its pharmaceutical effect by binding to certain cellular structure in its active state may require physical proximity to the target cellular structure when the activation signal is delivered. For such activatable agents, delivery of the activation signal under non-activating conditions will not result in the desired pharmacologic effect. Some examples of activating conditions may include, but are not limited to, temperature, pH, location, state of the cell, presence or absence of co-factors.

Selection of an activatable pharmaceutical agent greatly depends on a number of factors such as the desired cellular change, the desired form of activation, as well as the physical and biochemical constraints that may apply. Exemplary activatable pharmaceutical agents may include, but are not limited to, agents that may be activated by photonic energy, electromagnetic energy, acoustic energy, chemical or enzymatic reactions, thermal energy, or any other suitable activation mechanisms. Suitable activatable agents include, but are not limited to, photoactive agents, sono-active agents, thermo-active agents, and radio/microwave-active agents. An activatable agent may be a small molecule; a biological molecule such as a protein, a nucleic acid or lipid; a supramolecular assembly; a nanoparticle; or any other molecular entity having a pharmaceutical activity once activated.

When activated, the activatable pharmaceutical agent may effect cellular changes that include, but are not limited to, apoptosis, redirection of metabolic pathways, up-regulation of certain genes, down-regulation of certain genes, secretion of cytokines, alteration of cytokine receptor responses, or combinations thereof.

The mechanisms by which an activatable pharmaceutical agent may achieve its desired effect are not particularly limited. Such mechanisms may include direct action on a predetermined target as well as indirect actions via alterations to the biochemical pathways. A preferred direct action mechanism is by binding the agent to a critical cellular structure such as nuclear DNA, mRNA, rRNA, ribosome, mitochondrial DNA, or any other functionally important structures. Indirect mechanisms may include releasing metabolites upon activation to interfere with normal metabolic pathways, releasing chemical signals (e.g. agonists or antagonists) upon activation to alter the targeted cellular response, and other suitable biochemical or metabolic alterations.

In one embodiment, the activatable pharmaceutical agent is capable of chemically binding to the DNA or mitochondria at a therapeutically effective amount. In this embodiment, the activatable pharmaceutical agent, preferably a photoactivatable agent, is exposed *in situ* to an activating energy emitted from an energy converter, which, in turn receives energy from an initiation energy source. An activatable agent may be a small molecule; a biological molecule such as a protein, a nucleic acid or lipid; a supramolecular assembly; a nanoparticle; or any other molecular entity having a pharmaceutical activity once activated.

The activatable agent may be derived from a natural or synthetic origin. Any such molecular entity that may be activated by a suitable activation signal source to effect a predetermined cellular change may be advantageously employed in the present invention.

Suitable photoactive agents include, but are not limited to: psoralens and psoralen derivatives, pyrene cholesteryloleate, acridine, porphyrin, fluorescein, rhodamine, 16-diazorcortisone, ethidium, transition metal complexes of bleomycin, transition metal complexes of deglycobleomycin, organoplatinum complexes, alloxazines such as 7,8-dimethyl-10-ribityl isoalloxazine (riboflavin), 7,8,10-trimethylisoalloxazine (lumiflavin), 7,8-dimethylalloxazine (lumichrome), isoalloxazine-adenine dinucleotide (flavine adenine dinucleotide [FAD]), alloxazine mononucleotide (also known as flavine mononucleotide [FMN] and riboflavine-5-phosphate), vitamin Ks, vitamin L, their metabolites and precursors, and napththoquinones, naphthalenes, naphthols and their derivatives having planar molecular conformations, porphyrins, dyes such as neutral red, methylene blue, acridine, toluidines, flavine (acriflavine hydrochloride) and phenothiazine derivatives, coumarins, quinolones, quinones, and anthroquinones, aluminum (111) phthalocyanine tetrasulfonate, hematoporphyrin, and phthalocyanine, and compounds which preferentially adsorb to nucleic acids with little or no effect on proteins. The term "alloxazine" includes isoalloxazines.

Endogenously-based derivatives include synthetically derived analogs and homologs of endogenous photoactivated molecules, which may have or lack lower (1 to 5 carbons) alkyl or halogen substituents of the photosensitizers from which they are derived, and which preserve the function and substantial non-toxicity. Endogenous molecules are inherently nontoxic and may not yield toxic photoproducts after photoradiation.

The initiation energy source can be any energy source capable of providing energy at a level sufficient to cause cellular changes directly or via converter which transfer the initiation energy to energy capable of causing the predetermined cellular changes. Also, the initiation energy source can be any energy source capable of providing energy at a level sufficient activate the activatable agent directly, or to provide the energy to a converter with the input needed to emit the activation energy for the activatable agent (indirect activation). In one embodiment, the initiation energy is capable of penetrating completely through the subject. Within the context of the invention, the phrase "capable of penetrating completely through the subject" is used to refer to energy that can penetrate to any depth within the subject to activate the activatable pharmaceutical agent It is not required that the any of the energy applied actually pass completely through the subject, merely that it be capable of doing so in order to permit penetration to any desired depth to activate the activatable pharmaceutical agent. Exemplary initiation energy sources that are capable of penetrating completely through the subject include, but are not limited to, UV light, visible light, IR radiation, x-rays, gamma rays, electron beams, microwaves and radio waves.

An additional embodiment of the invention is to provide a method for treatment of a condition, disease or disorder by the in-situ generation of energy in a subject in need thereof, where the energy generated with or without energy augmentation can be used directly to effect a change thereby treating the condition, disease or disorder, or the energy can be used to activate an activatable pharmaceutical agent, which upon activation effects a change thereby treating the condition, disease or disorder. The energy can be generated in-situ by any desired method, including, but not limited to, chemical reaction such as chemiluminescence, or by conversion of an energy applied to the subject externally, which is converted in-situ to a different energy (of lower or higher energy than that applied), through the use of one or more energy converters. With the energy augmentators present, this invention can generate local regions of intense electric fields to enhance light emission from these chemical reactions in proximity to those local regions.

A further embodiment of the invention combines the treatment of a condition, disease or disorder with the generation with or without energy augmentation of heat in the affected target structure in order to enhance the effect of the treatment. For example, in the treatment of a cell proliferation disorder using a photoactivatable pharmaceutical agent (such as a psoralen or derivative thereof), one can activate the photoactivatable pharmaceutical agent by applying an initiation energy which, directly or indirectly, activates the pharmaceutical agent. As noted elsewhere in this application, this initiation energy can be of any type, so long as it can be converted to an energy suitable for activating the pharmaceutical compound. In addition to applying this initiation energy, in this embodiment of the present invention, an energy is applied that causes heating of the target structure. In the case of a cell proliferation disorder such as cancer, the heating would increase the proliferation rate of the cancer cells. While this may seem counterintuitive at first, when the cell proliferation disorder is being treated using a DNA intercalation agent, such as psoralen or a derivative thereof, this increase in cell proliferation can actually assist the psoralen in causing apoptosis. In particular, when psoralen becomes intercalated into DNA, apoptosis occurs when the cell goes through its next division cycle. By increasing the rate at which the cells divide, one can use the present invention methods to enhance the onset of apoptosis.

In one embodiment, heat can be generated by any desired manner. Preferably, the heat can be generated using the application of microwaves or NIR energy to the target structure or by the use of use of nanoparticles of metal or having metal shells. In the case of NIR energy, the folded resonator structures or the other resonant structures described above can be used. Heat can also be generated by the absorption of light from the phosphors or scintillators of the invention. Alternatively, as is done in tumor thermotherapy, magnetic metal nanoparticles can be targeted to cancer cells using conventional techniques, then used to generate heat by application of a magnetic field to the subject under controlled conditions. (DeNardo SJ, DeNardo GL, Natarajan A et al.: Thermal dosimetry predictive of efficacy of 111In-ChL6 NPAMF-induced thermoablative therapy for human breast cancer in mice. J. Nucl. Med. 48(3),437-444 (2007).)

In one embodiment, the source of the initiation energy can be a radiowave emitting nanotube, such as those described by K. Jensen, J. Weldon, H. Garcia, and A. Zettl in the Department of Physics at the University of California at Berkeley (see http://socrates.berkeley.edu/~argon/nanoradio/radio.html,).

These nanotubes can be administered to the subject, and preferably would be coupled to the activatable pharmaceutical agent or the energy converter, or both, or be located in proximity of a target cell such that upon application of the initiation energy, the nanotubes would accept the initiation energy (preferably radiowaves), then emit radiowaves in close proximity to the activatable pharmaceutical agent, or in close proximity to the energy converter, or to the target cell to then cause the predetermined cellular changes or activation of the activatable pharmaceutical agent. In such an embodiment, the nanotubes would act essentially as a radiowave focusing or amplification device in close proximity to the activatable pharmaceutical agent or energy converter or the target cell.

Alternatively, the energy emitting source may be an energy converter that emits energy in a form suitable for absorption by the transfer agent or a target cell. For example, the initiation energy source may be acoustic energy and one energy converter may be capable of receiving acoustic energy and emitting photonic energy (e.g. sonoluminescent molecules) to be received by another energy converter that is capable of receiving photonic energy. Other examples include transfer agents that receive energy at x-ray wavelength and emit energy at UV wavelength, preferably at UV-A wavelength. As noted above, a plurality of such energy converters may be used to form a cascade to transfer energy from initiation energy source via a series of energy converters to activate the activatable agent or the predetermined cellular change.

Signal transduction schemes as a drug delivery vehicle may be advantageously developed by careful modeling of the cascade events coupled with metabolic pathway knowledge to sequentially or simultaneously cause the predetermined cellular change or activate multiple activatable pharmaceutical agents to achieve multiple-point alterations in cellular function.

Alternatively, one can administer the initiation energy source to the subject. Within the context of the present invention, the administering of the initiation energy source means the administration of an agent, that itself produces the initiation energy, in a manner that permits the agent to arrive at the target cell within the subject without being surgically inserted into the subject. The administration can take any form, including, but not limited to, oral, intravenous, intraperitoneal, inhalation, etc. Further, the initiation energy source in this embodiment can be in any form, including, but not limited to, tablet, powder, liquid solution, liquid suspension, liquid dispersion, gas or vapor, etc. In this embodiment, the initiation energy source includes, but is not limited to, chemical energy sources, nanoemitters, nanochips, and other nanomachines that produce and emit energy of a desired frequency. Recent advances in nanotechnology have provided examples of various devices that are nanoscale and produce or emit energy, such as the Molecular Switch (or Mol-Switch) work by Dr. Keith Firman of the EC Research and Development Project, or the work of Cornell et al. (1997) who describe the construction of nanomachines based around ion-channel switches only 1.5 nm in size, which use ion channels formed in an artificial membrane by two gramicidin molecules: one in the lower layer of the membrane attached to a gold electrode and one in the upper layer tethered to biological receptors such as antibodies or nucleotides. When the receptor captures a target molecule or cell, the ion channel is broken, its conductivity drops, and the biochemical signal is converted into an electrical signal. These nanodevices (and/or for example the mechano-luminescent devices described above) could also be coupled with the present invention to provide targeting of the target cell, to deliver the initiation energy source directly at the desired site.

In another embodiment, the present invention includes the administration of a source of chemical energy such as chemiluminescence, phosphorescence or bioluminescence. The source of chemical energy can be a chemical reaction between two or more compounds, or can be induced by activating a chemiluminescent, phosphorescent or bioluminescent compound with an appropriate activation energy, either outside the subject or inside the subject.

In another embodiment, the patient's own cells are removed and genetically modified to provide photonic emissions. For example, tumor or healthy cells may be removed, genetically modified to induce bioluminescence and may be reinserted at the site of the disease or condition to be treated. The modified, bioluminescent cells may be further modified to prevent further division of the cells or division of the cells only so long as a regulating agent is present. In a further embodiment, UV emitting bioluminescent materials can be injected into the site of the tumor without first having removed any of the tumor or healthy cells.

In a further embodiment, a biocompatible emitting source, such as a fluorescing metal nanoparticle or fluorescing dye molecule or the phosphors or scintillators of the invention, is selected that emits in the UV-A band. The UV-A emitting source is directed to the site of a disease or condition. The UV-A emitting source may be directed to the site of the disease or condition by systemically administering the UV-A emitting source. Preferably, the UV-A emitting source is concentrated in the target site, such as by physical insertion or by conjugating the UV-A emitting molecule with a specific carrier that is capable of concentrating the UV-A emitting source in a specific target structure, as is known in the art.

In another embodiment, a UV- or light-emitting luciferase is selected as the emitting source for exciting a photoactivatable agent. A luciferase may be combined with ATP or another molecule, which may then be oxygenated with additional molecules to stimulate light emission at a desired wavelength. The luciferase may be used with or without the energy augmentators. Alternatively, a phosphorescent emitting source may be used with or without the energy augmentators. One advantage of a phosphorescent emitting source is that the phosphorescent emitting molecules or other source may be electroactivated or photoactivated prior to insertion into a target site either by systemic administration or direct insertion into the region of the target site. Alternatively, some of these materials can be activated, with the energy being "stored" in the activated material, until emission is stimulated by application of another energy. For example, see the discussion in U.S. Patent 4,705,952 regarding infrared-triggered phosphors.

Phosphorescent materials may have longer relaxation times than fluorescent materials, because relaxation of a triplet state is subject to forbidden energy state transitions, storing the energy in the excited triplet state with only a limited number of quantum mechanical energy transfer processes available for returning to the lower energy state. Energy emission is delayed or prolonged from a fraction of a second to several hours. Otherwise, the energy emitted during phosphorescent relaxation is not otherwise different than fluorescence, and the range of wavelengths may be selected by choosing a particular phosphor.

Alternatively, a phosphorescent emitting source may be used as the energy converter. One advantage of a phosphorescent emitting source is that the phosphorescent emitting molecules or other source may be electroactivated or photoactivated prior to insertion into the tumor either by systemic administration or direct insertion into the region of the tumor. Phosphorescent materials may have longer relaxation times than fluorescent materials, because relaxation of a triplet state is subject to forbidden energy state transitions, storing the energy in the excited triplet state with only a limited number of quantum mechanical energy transfer processes available for returning to the lower energy state. Energy emission is delayed or prolonged from a fraction of a second to several hours. Otherwise, the energy emitted during phosphorescent relaxation is not otherwise different than fluorescence, and the range of wavelengths may be selected by choosing a particular phosphor.

In one embodiment, the energy converters of the invention can include persistent after-glow phosphor materials emitting light in the visible to near ultraviolet and ultraviolet range. In one embodiment, Eu-doped strontium aluminate is used as an energy converter in which deep UV light or x-ray or electron beans "charge" the photoluminescence such that these phosphors can be charged outside for example a patient and then injected into target or diseased site where UV photons would be emitted. In another embodiment, gadolinium strontium magnesium aluminate is used as an energy converter in which deep UV light or x-ray or electron beans "charge" the photoluminescence such that these phosphors can be charged outside for example a patient and then injected into target or diseased site where UV photons would be emitted. U.S. Pat. Appl. Publ. No. 20070221883 describes specifically gadolinium-activated strontium magnesium aluminate having an excitation maximum at about 172 nm, and which emits in a narrow-band UV emission at about 310 nm. The '883 publication also describes other useful energy converters for this invention, making note of emission spectra between 300 nm and 320 nm for a Sr(Al,Mg)₁₂O₁₉:Gd phosphor and two 312 nm line emitting phosphors, YMgB₅O₁₀;Gd, Ce and YMgB₅O₁₀:Gd, Ce, Pr. WO2016200349 describes long lasting yellowish-green emitting phosphorescent pigments in the strontium aluminate (SrAl2O4) system, which could serve as energy converters in the present invention. WO 2016200348 describes long lasting bluish-green emitting phosphorescent pigments in the strontium aluminate (Sr4Al14O25) system, which could serve as energy converters in the present invention. Xiong et al in "Recent advances in ultraviolet persistent phosphors," Optical Materials X 2 (2019) describes a number of ultraviolet persistent phosphors that could as energy converters in the present invention. The table below provides a listing of such persistent phosphors:

| | | |
|---|---|---|
| SrO:Pb²⁺ | 390 | > 1 h |
| CaAl₂O₄:Ce³⁺ Tb³⁺ | 400 | > 10 h |
| CaAl₂O₄:Ce²⁺ Tb³⁺ | 413 | > 10 h |
| Sr₂Al₂SiO₄:Ce³⁺ | 400 | sevent minutes |
| SrZrO₂ | 395 | < 1000 s |
| BaZrO₃:Mg²⁺ | 400 | > 2400 s |
| SrZrO_{3:}Pr³⁺ | 356 | |
| CdSiO₃:Bi³⁺ | 360 | |
| CdSiO₃:Bi³⁺ Dy³⁺ | 360 | |
| CdSiO₃:Bi³⁺ Gd³⁺ | 344 | > 6 h |
| Sr₂MgGe₂O₇:Pb²⁺ | 370 | > 12 h |
| NaLuGeO₄:Bi³⁺ Eu³⁺ | 400 | > 63 h |
| CaZnGe₂O₆: Bi³⁺ | 300-700 | > 12 h |
| Cs₂NaYF₆:Pr³⁺ | 250 | > 2 h |

In one embodiment, the phosphor described by Xiong et al as CaAl₂O₄:Ce³⁺having an emission peak of 400 nm and a persistent time of more than 10 h could be used, where it would be charged by x-ray irradiation outside a patient and then injected at a diseased site to provide internally generated UV light.

In one embodiment, the persistent phosphors noted could be activated *ex vivo* and introduced along with psoralen (or other photoactivatable drug) into the patient by exchange of a bodily fluid or for example by supplying the persistent phosphors and the photoactivatable drug into a patient's blood stream.

In one embodiment, the persistent phosphors noted could be activated *in vivo* by injection of the phosphors into a diseased site and then exposure to x-rays.

Among various materials, luminescent nanoparticles have attracted increasing technological and industrial interest. In the context of the invention, nanoparticle refers to a particle having a size less than one micron. While the description of the invention describes specific examples using nanoparticles, the invention in many embodiments is not limited to particles having a size less than one micron. However, in many of the embodiments, the size range of having a size less than one micron, and especially less than 100 nm produces properties of special interest such as for example emission lifetime luminescence quenching, luminescent quantum efficiency, and concentration quenching and such as for example diffusion, penetration, and dispersion into mediums where larger size particles would not migrate.

U.S. Pat. No. 4,705,952 (the contents of which are hereby incorporated herein by reference) describes an infrared-triggered phosphor that stored energy in the form of visible light of a first wavelength and released energy in the form of visible light of a second wavelength when triggered by infrared light. In some cases, U.S. Pat. No. 4,705,952 describes that "the upconversion continues for as long as several days before a new short recharge is required." The phosphors in U.S. Pat. No. 4,705,952 were compositions of alkaline earth metal sulfides, rare earth dopants, and fusible salts. The phosphors in U.S. Pat. No. 4,705,952 were more specifically phosphors made from strontium sulfide, barium sulfide and mixtures thereof; including a dopant from the rare earth series and europium oxide, and mixtures thereof; and including a fusible salt of fluorides, chlorides, bromides, and iodides of lithium, sodium, potassium, cesium, magnesium, calcium, strontium, and barium, and mixtures thereof. The materials described in U.S. Pat. No. 4,705,952 are useful in various embodiments of the invention.

In some cases, U.S. Pat. No. 4,705,952 describes that "the storage times become extremely long, on the order of years." The material is thus adapted to receive infrared photons and to emit higher energy photons in a close to 1:1 relation. With storage times this long, these infrared-triggered phosphors can be used in various embodiments of the present invention as a viable mechanism where commercial IR lasers are used to activate phosphorescence in a medium, thereby in a patient generating visible or ultraviolet light.

In another embodiment, a combined electromagnetic energy harvester molecule is designed, such as the combined light harvester disclosed in J. Am. Chem. Soc. 2005, 127, 9760-9768, the entire contents of which are hereby incorporated by reference. By combining a group of fluorescent molecules in a molecular structure, a resonance energy transfer cascade with or without the energy augmentators may be used to harvest a wide band of electromagnetic radiation resulting in emission of a narrow band of fluorescent energy. By pairing a combined energy harvester with a photoactivatable molecule, a further energy resonance transfer excites the photoactivatable molecule, when the photoactivatable molecule is nearby stimulated combined energy harvester molecules. Another example of a harvester molecule is disclosed in Figure 4 of "Singlet-Singlet and Triplet-Triplet Energy Transfer in Bichromophoric Cyclic Peptides," M.S. Thesis by M.O. Guler, Worcester Polytechnic Institute, May 18, 2002, which is incorporated herein by reference.

In another embodiment, a Stokes shift of an emitting source or a series of emitting sources arranged in a cascade with or without the energy augmentators is selected to convert a shorter wavelength energy, such as X-rays, to a longer wavelength fluorescence emission such as optical or UV-A, which is used to stimulate a photoactivatable molecule at the location of the target structure. Preferably, the photoactivatable molecule is selected to cause the predetermined change in target structure without causing substantial harm to normal, healthy cells.

In an additional embodiment, the photoactivatable agent can be a photocaged complex having an active agent contained within a photocage. The active agent is bulked up with other molecules that prevent it from binding to specific targets, thus masking its activity. When the photocage complex is photoactivated, the bulk falls off, exposing the active agent. In such a photocage complex, the photocage molecules can be photoactive (i.e. when photoactivated, they are caused to dissociate from the photocage complex, thus exposing the active agent within), or the active agent can be the photoactivatable agent (which when photoactivated causes the photocage to fall off), or both the photocage and the active agent are photoactivated, with the same or different wavelengths. For example, a toxic chemotherapeutic agent can be photocaged, which will reduce the systemic toxicity when delivered. Once the agent is concentrated in the tumor, the agent is irradiated with an activation energy. This causes the "cage" to fall off, leaving a cytotoxic agent in the tumor cell. Suitable photocages include those disclosed by Young and Deiters in "Photochemical Control of Biological Processes", Org. Biomol. Chem., 5, pp. 999 - 1005 (2007) and "Photochemical Hammerhead Ribozyme Activation", Bioorganic & Medicinal Chemistry Letters, 16(10) ,pp. 2658-2661 (2006), the contents of which are hereby incorporated by reference.

In one embodiment, the use of light (e.g. light emitted from the phosphor or scintillator particles or combination thereof or the energy converters described above) for uncaging a compound or agent is used for elucidation of neuron functions and imaging, for example, two-photon glutamine uncaging (Harvey CD, et al., Nature, 450:1195-1202 (2007); Eder M, et al., Rev. Neurosci., 15:167-183 (2004)). Other signaling molecules can be released by UV light stimulation, e.g., GABA, secondary messengers (e.g., Ca²⁺ and Mg²⁺), carbachol, capsaicin, and ATP (Zhang F., et al., 2006). Chemical modifications of ion channels and receptors may be carried out to render them light-responsive. Ca²⁺ is involved in controlling fertilization, differentiation, proliferation, apoptosis, synaptic plasticity, memory, and developing axons. In yet another preferred embodiment, Ca²⁺ waves can be induced by UV irradiation (single-photon absorption) and NIR irradiation (two-photon absorption) by releasing caged Ca²⁺, an extracellular purinergic messenger InsP3 (Braet K., et al., Cell Calcium, 33:37-48 (2003)), or ion channel ligands (Zhang F., et al., 2006).

Genetic targeting allows morphologically and electrophysiologically characterization of genetically defined cell populations. Accordingly, in an additional embodiment, a light-sensitive protein is introduced into cells or live subjects via number of techniques including electroporation, DNA microinjection, viral delivery, liposomal transfection, creation of transgenic lines and calcium-phosphate precipitation. For example, lentiviral technology provides a convenient combination a conventional combination of stable long-term expression, ease of high-titer vector production and low immunogenicity. The light-sensitive protein may be, for example, channelrhodopsin-2 (ChR2) and chloride pump halorhodopsin (NpHR). The light protein encoding gene(s) along with a cell-specific promoter can be incorporated into the lentiviral vector or other vector providing delivery of the light-sensitive protein encoding gene into a target cell. ChR2 containing a light sensor and a cation channel, provides electrical stimulation of appropriate speed and magnitude to activate neuronal spike firing, when the cells harboring Ch2R are pulsed with light.

In one embodiment, a lanthanide chelate with or without the energy augmentators capable of intense luminescence can be used. For example, a lanthanide chelator may be covalently joined to a coumarin or coumarin derivative or a quinolone or quinolone-derivative sensitizer. Sensitizers may be a 2- or 4-quinolone, a 2- or 4- coumarin, or derivatives or combinations of these examples. A carbostyril 124 (7-amino-4-methyl-2-quinolone), a coumarin 120 (7-amino-4-methyl-2-coumarin), a coumarin 124 (7-amino-4-(trifluoromethy1)-2-coumarin), aminoinethyltrimethylpsoralen or other similar sensitizer may be used. Chelates may be selected to form high affinity complexes with lanthanides, such as terbium or europium, through chelator groups, such as DTPA. Such chelates may be coupled to any of a wide variety of well-known probes or carriers, and may be used for resonance energy transfer to a psoralen or psoralen-derivative, such as 8-MOP, or other photoactive molecules capable of binding DNA. In one alternative example, the lanthanide chelate is localized at the site of the disease using an appropriate carrier molecule, particle or polymer, and a source of electromagnetic energy is introduced by minimally invasive procedures (e.g., the gas containing upconverters of the invention) to irradiate the target structure, after exposure to the lanthanide chelate and a photoactive molecule.

In another embodiment, a biocompatible, endogenous fluorophore emitter with or without the energy augmentators can be selected to stimulate resonance energy transfer to a photoactivatable molecule. A biocompatible emitter (e.g. the phosphors or scintillators) with an emission maxima within the absorption range of the biocompatible, endogenous fluorophore emitter may be selected to stimulate an excited state in fluorophore emitter. One or more halogen atoms may be added to any cyclic ring structure capable of intercalation between the stacked nucleotide bases in a nucleic acid (either DNA or RNA) to confer new photoactive properties to the intercalator. Any intercalating molecule (psoralens, coumarins, or other polycyclic ring structures) may be selectively modified by halogenation or addition of non-hydrogen bonding ionic substituents to impart advantages in its reaction photochemistry and its competitive binding affinity for nucleic acids over cell membranes or charged proteins, as is known in the art.

Skin photosensitivity is a major toxicity of photosensitizers. Severe sunburn occurs if skin is exposed to direct sunlight for even a few minutes. Early murine research hinted at a vigorous and long term stimulation of immune response; however, actual clinical testing has failed to achieve the early promises of photodynamic therapies. The early photosensitizers for photodynamic therapies targeted type II responses, which created singlet oxygen when photoactivated in the presence of oxygen. The singlet oxygen caused cellular necrosis and was associated with inflammation and an immune response. Some additional photosensitizers have been developed to induce type I responses, directly damaging cellular structures.

Porfimer sodium (Photofrin; QLT Therapeutics, Vancouver, BC, Canada), is a partially purified preparation of hematoporphyrin derivative (HpD). Photofrin has been approved by the US Food and Drug Administration for the treatment of obstructing esophageal cancer, microinvasive endobronchial non-small cell lung cancer, and obstructing endobronchial non-small cell lung cancer. Photofrin is activated with 630 nm, which has a tissue penetration of approximately 2 to 5 mm. Photofrin has a relatively long duration of skin photosensitivity (approximately 4 to 6 weeks).

Tetra (m-hydroxyphenyl) chlorin (Foscan; Scotia Pharmaceuticals, Stirling, UK), is a synthetic chlorine compound that is activated by 652 nm light. Clinical studies have demonstrated a tissue effect of up to 10 mm with Foscan and 652 nm light. Foscan is more selectively a photosensitizer in tumors than normal tissues, and requires a comparatively short light activation time. A recommended dose of 0.1 mg/kg is comparatively low and comparatively low doses of light may be used. Nevertheless, duration of skin photosensitivity is reasonable (approximately 2 weeks). However, Foscan induces a comparatively high yield of singlet oxygen, which may be the primary mechanism of DNA damage for this molecule.

Motexafin lutetium (Lutetium texaphryin) is activated by light in the near infrared region (732 nm). Absorption at this wavelength has the advantage of potentially deeper penetration into tissues, compared with the amount of light used to activate other photosensitizers. Lutetium texaphryin also has one of the greatest reported selectivities for tumors compared to selectivities of normal tissues. Young SW, et al.: Lutetium texaphyrin (PCI-0123) a near-infrared, water-soluble photosensitizer. Photochem Photobiol 1996, 63:892-897. In addition, its clinical use is associated with a shorter duration of skin photosensitivity (24 to 48 hours). Lutetium texaphryin has been evaluated for metastatic skin cancers. It is currently under investigation for treatment of recurrent breast cancer and for locally recurrent prostate cancer. The high selectivity for tumors promises improved results in clinical trials.

In general, the approach may be used with any source for the excitation an activatable molecule. The process may be a photopheresis process or may be similar to photophoresis. While photophoresis is generally thought to be limited to photonic excitation, such as by UV-light, other forms of radiation may be used as a part of a system to activate an activatable molecule. Light emission can stimulate the activation of an activatable molecule, such as 8-MOP. In one example, light emission from the phosphors or scintillators of the invention is directed at a solid tumor and stimulates, directly or indirectly, activation of 8-MOP.

In yet another embodiment, the activatable pharmaceutical agent, preferably a photoactive agent, is directed to a receptor site by a carrier having a strong affinity for the receptor site. The carrier may be a polypeptide and may form a covalent bond with a photo active agent, for example. The polypeptide may be an insulin, interleukin, thymopoietin or transferrin, for example. Alternatively, a photoactive pharmaceutical agent may have a strong affinity for the target cell without a binding to a carrier.

For example, a treatment may be applied that acts to slow or pause mitosis. Such a treatment is capable of slowing the division of rapidly dividing healthy cells or stem cells without pausing mitosis of cancerous cells. Thus, the difference in growth rate between the non-target cells and target cells are further differentiated to enhance the effectiveness of the methods of the invention.

In a further embodiment, methods in accordance with the invention may further include adding an additive to alleviate treatment side-effects. Exemplary additives may include, but are not limited to, antioxidants, adjuvant, or combinations thereof. In one exemplary embodiment, psoralen is used as the activatable pharmaceutical agent, UV-A is used as the activating energy, and antioxidants are added to reduce the unwanted side-effects of irradiation.

In another aspect, the invention also provides methods for producing an autovaccine, including: (1) providing a population of targeted cells; (2) treating the cells *ex vivo* with a psoralen or a derivative thereof; (3) activating the psoralen with an initiation energy source to induce a predetermined change in a target structure in the population of the target cells; and (4) returning the treated cells back to the host to induce an autovaccine effect against the targeted cell, wherein the treated cells cause an autovaccine effect.

### Photobiomodulation (with and without Energy Augmentation)

Photobiomodulation also known as low level laser therapy (LLLT), cold laser therapy, and laser biostimulation, is an emerging medical and veterinary technique in which exposure to low-level laser light can stimulate or inhibit cellular function leading to beneficial clinical effects. The "best" combination of wavelength, intensity, duration and treatment interval is complex and sometimes controversial with different diseases, injuries and dysfunctions needing different treatment parameters and techniques.

In one embodiment of this invention, the above-described energy converters (phosphors, scintillators, fluorescent materials, and combinations and agglomerations thereof or other of the described energy converters) with or without plasmonic inducing agents and with and without energy augmentation provide the light for producing photobiomodulation. Certain wavelengths of light emitted from the phosphor or scintillator configurations of the invention at certain intensities will, for example, aid tissue regeneration, resolve inflammation, relieve pain and boost the immune system. Observed biological and physiological effects to be expected include changes in cell membrane permeability, and up-regulation and down-regulation of adenosine triphosphate and nitric oxide. With the energy augmentators present, this invention can generate local regions of intense electric fields to enhance light emission from the energy converters in proximity to those local regions.

All light-induced biological effects depend on the parameters of the irradiation (wavelength, dose, intensity, irradiation time, depth of a target cell, and continuous wave or pulsed mode, pulse parameters). (See, e.g., Karu IT, Low-Power Laser Therapy", in Biomedical Photonics Handbook, Vo-Dinh T. Ed., CRC Press, Boca Raton, FL, pp. 48-1 to 48-25, (2003)). The phosphor or scintillator configurations of the invention can be programmed or instructed to deliver light comparable to that of known photobiomodulation treatments. For example, the phosphor or scintillator configurations of the invention can be programmed or instructed to deliver light with an average power typically in the range of 1-500 mW; or with peak power and short pulse width in the range of 1-100 W with 200 ns pulse widths. In this example, the average beam irradiance would typically be 10 mW/cm² - 5 W/cm². The phosphor or scintillator configurations of the invention (or the other energy converters or light emitters described above) can be programmed or instructed to or configured to deliver light at a wavelength typically in the range 600-1000 nm. The red-to-near infrared (NIR) region is preferred for photobiomodulation. Other wavelengths may be also used, e.g., UV light for neurons and green light for prostate tissue. Maximum biological responses have been seen to occur from prior work when the tissues were irradiated at 620, 680, 760, and 820-830 nm (Karu TI, et al., (1998).

In another embodiment, a plurality of sources for supplying electromagnetic radiation energy or energy transfer are provided by one or more molecules administered to a patient. The molecules may emit stimulating radiation in the correct band of wavelength to stimulate the target structure directly or to simulate the photoactivatable agents, or the molecules may transfer energy by a resonance energy transfer or other mechanism directly to the target structure or the photoactivatable agent or indirectly by a cascade effect via other molecular interactions.

The phenomenon of ultra-weak emission from cellular systems has been a topic of various inquiries since the 1900s. This topic can be traced back to the early investigations of the Russian biologist Gurwitsch Alexander G. Gurwitsch more than seventy years ago, who speculated that ultraweak photon emission transmit information in cells [A. G. Gurwitsch, S. S. Grabje, and S. Salkind, "Die Natur des spezifischen Erregers der Zellteilung," Arch. Entwicklungsmech. Org. 100, 11-40, 1923].

In the 1970s, this area of research was investigated by a number of investigators. The presence of biological radiation from a variety of cells was later investigated by several research groups in Europe and Japan using low-noise, sensitive photon-counting detection systems [B. Ruth and F.-A. Popp, "Experimentelle Untersuchungen zur ultraschwachen Photonenemission biologischer Systeme," Z. Naturforsch., A: Phys. Sci. 31c, 741-745, 1976; T. I. Quickenden and S. S. Que-Hee, "The spectral distribution of the luminescence emitted during growth of the yeast Saccharomyces cerevisiae and its relationship to mitogenetic radiation," Photochem. Photobiol. 23, 201-204, 1976; H. Inaba, Y. Shimizu, Y. Tsuji, and A. Yamagishi, "Photon counting spectral analysing system of extra-weak chemi- and bioluminescence for biochemical applications," Photochem. Photobiol. 30, 169-175, 1979]. Popp and coworkers suggested the evidence of some 'informational character' associated with the ultra-weak photon emission from biological systems, often referred by Popp as "bio-photons". Other studies reported ultra-weak photon emission from various species including plant, and animals cells [H. J. Niggli, C. Scaletta, Y. Yan, F.-A. Popp, and L. A. Applegate, "Ultraweak photon emission in assessing bone growth factor efficiency using fibroblastic differentiation," J. Photochem. Photobiol., B, 64, 62-68, 2001;]. Results of experiments of UV-irradiated skin fibroblasts indicated that repair deficient xeroderma pigmentosum cells show an efficient increase of ultraweak photon emission in contrast to normal cells. [H. J. Niggli, "Artificial sunlight irradiation induces ultraweak photon emission in human skin fibroblasts," J. Photochem. Photobiol., B 18, 281-285 (1993)].

A delayed luminescence emission was also observed in biological systems [F.-A. Popp and Y. Yan, "Delayed luminescence of biological systems in terms of coherent states," Phys. Lett. A 293, 93-97 (2002); A. Scordino, A. Triglia, F. Musumeci, F. Grasso, and Z. Rajfur, "Influence of the presence of Atrazine in water on in-vivo delayed luminescence of acetabularium acetabulum," J. Photochem. Photobiol., B, 32, 11-17 (1996); This delayed luminescence was used in quality control of vegetable products [ A. Triglia, G. La. Malfa, F. Musumeci, C. Leonardi, and A. Scordino, "Delayed luminescence as an indicator of tomato fruit quality," J. Food. Sci. 63, 512-515 (1998)] or for assessing the quality or quality changes of biological tissues [Yu Yan, Fritz-Albert Popp *, Sibylle Sigrist, Daniel Schlesinger, Andreas Dolf, Zhongchen Yan, Sophie Cohen, Amodsen Chotia, "Further analysis of delayed luminescence of plants", Journal of Photochemistry and Photobiology B: Biology 78, 235-244 (2005)].

It was reported that UV excitation can further enhance the ultra-weak emission and a method for detecting UV-A-laser-induced ultra-weak photon emission was used to evaluate differences between cancer and normal cells. [H. J. Niggli et al, Laser-ultraviolet-A-induced ultraweak photon emission in mammalian cells, Journal of Biomedical Optics 10(2), 024006 (2005)].

Accordingly, in one embodiment of the present invention, upon applying an initiation energy from at least one source to a target structure in a subject in need of treatment, the initiation energy with and without energy augmentation contacts the target structure and induces a predetermined change in said target structure *in situ,*
wherein the predetermined change is the enhancement of energy emission from the target, which then mediates, initiates or enhances a biological activity of other target structures in the subject, or of a second type of target structure (e.g., a different cell type).

In another embodiment, the initiation energy can itself be energy emitted by at least one cell with and without energy augmentation excited by metabolic processes or some other internal or external trigger, and said applying is conducted via cell-to-cell energy transfer. There are those that maintain that the health of the body depends on certain bioelectric vibrations that are susceptible to chemical or physical toxic factors. Fröhlich notes that there are coherent electric vibrations in the frequency range 100 GHz to 1 THz, excited in cells by metabolic processes (see Fröhlich H. Coherent electric vibrations in biological systems and the cancer problem, IEEE Transactions on Microwave Theory and Techniques, Vol. MTT-26, No. 8, August, 1978, pp 613-617). This idea is based on observation of the inhibition or stimulation of the growth of yeast and bacterial functions of the applied frequency, showing very stable and repetitive resonances. If such vibrational states are indeed metabolically excited, then they should be manifested in Raman spectroscopy. Actually, their existence has been demonstrated during periods of metabolic activity of lysozyme and E.coli (700 GHz to 5 THz). Emissions have also been observed at lower frequencies (150 GHz or less). These vibrations occur in the tissue of higher organisms and they have been hypothesized exercise some control on cellular growth (see also S. J. Webb et al, Nature, Vol. 218, April 27, 1968, pp. 374-375; and S. J. Webb et al et al, Nature Vol. 222, June 21, 1969, pp. 1199-1200). Cancerization could result from a modification of these vibrations by the invasion of foreign molecules, e.g., the presence of free electrons in the condition bands of proteins. There is some evidence for the presence of double spectral lines at 1.5 and 6 THz in breast carcinoma, which may be an indication of an interaction between normal cellular vibrations and free electrons. In such coherent frequency communication between cells, it is believed that the medium through which the communication is transmitted is the water within and around the cells (see Smith, Coherent Frequencies, Consciousness and the Laws of Life, 9th International Conference CASYS '09 on Computing Anticipatory Systems, Liege, Belgium, August 3-8, 2009).

Accordingly, in a further embodiment of the present invention, the initiation energy is an energy capable of triggering with and without energy augmentation an altered metabolic activity in one or more cells, preferably in the 100 GHz to 10 THz region, and is applied directly to one or more cells, to trigger the cell(s) to undergo altered metabolic activity, and optionally, to further trigger emissions from the cell(s) to thereby cascade the effects of the emissions to other similar or different cell types adjacent thereto, in essentially a triggered entry into the natural emissions process described above, preferably where the medium through which the emissions are communicated is water-based, most preferably where the medium is the water contained within and surrounding the cells.

Indeed, a combination of x-ray and microwave energy (e.g., 100 GHz to 10 THz region) can be applied to a target site. In this embodiment, the x-ray irradiation triggers light emission with and without energy augmentation from energy converters in the medium (phosphors, scintillators, fluorescent materials, and combinations and agglomerations thereof) with or without plasmonic inducing agents to activate photoactivatable agents in the medium (as discussed above), and the microwave and or RF radiation can cause the alignment of dipoles or alter the mass transport across ionic channels which in turn could trigger the cell(s) to undergo altered metabolic activity, or optionally, to further trigger emissions from the cell(s) to thereby cascade the effects of the emissions to other similar or different cell types adjacent thereto (as described above) to complement the photoactivated photoactivatable agents in the medium. With the energy augmentators present, this invention can generate local regions of intense electric fields to enhance light emission from the energy converters in proximity to those local regions.

While not bound to the particular following theory, a photoacceptor first absorbs the light used for the irradiation. After promotion of electronically excited states, primary molecule processes from these states can lead to a measurable biological effect (via secondary biochemical reaction, or photosignal transduction cascade, or cellular signaling) at the cellular level. A photoacceptor for eukaryotic cells in red-to-NIR region is believed to be the terminal enzyme of the respiratory chain cytochrome c oxidase located in cell mitochondrion. In the violet-to blue spectra region, flavoprotein (e.g., NADHdehydrogenase in the beginning of the respiratory chain) is also among the photoacceptors. The phosphor configurations of the invention can be programmed or instructed to or configured to deliver light at these wavelengths.

Clinical applications of photobiomodulation include, for example, treating soft tissue and bone injuries, chronic pain, wound healing and nerve and sensory regeneration/restoration, and possibly even resolving viral and bacterial infections, treating neurological and psychiatric diseases (e.g., epilepsy and Parkinson's disease) (e.g., Zhang F., et al., Nature, 446:617-9 (April 5, 2007; Han X., et al., PloS ONE, 2(3):e299 (March 21, 2007); Arany PR, et al., Wound Repair Regen., 15(6):866-74 (2007); Lopes CB, et al., Photomed. Laser Surg., 25(2):96-101 (2007)). One clinical application showing great promise is the treatment of inflammation, where the anti-inflammatory effect of location-and-dose-specific laser irradiation produces similar outcomes as NSAIDs, but without the potentially harmful side-effects (Bjordal JM, Couppé C, Chow RT, Tunér J, Ljunggren EA (2003). "A systematic review of low level laser therapy with location-specific doses for pain from chronic joint disorders". The Australian journal of physiotherapy 49(2): 107-16). The phosphor configurations of the invention can be programmed or instructed to or configured to deliver light at the wavelengths and illuminations reported in this work.

An NIR light treatment can prevent cell death (apoptosis) in cultured neurons (brain) cells (Wong-Reiley MT, et al., JBC, 280(6):4761-71 (2005)). Specific wavelengths of light can promote cellular proliferation to the activation of mitochondria, the energy-producing organelles within the cell via cytochrome c oxidase. An NIR treatment can augment mitochondrial function and stimulate antioxidant protective pathways. The evidence that the NIR treatment can augment mitochondrial function and stimulate antioxidant protective pathways comes from photobiomodulation experiments carried out using a laboratory model of Parkinson's disease (PD) (cultures of human dopaminergic neuronal cells) (Whelan H., et. al., SPIE, Newsroom, pages 1-3 (2008)). The phosphor or scintillator configurations of the invention can be programmed or instructed to or configured to deliver light at these NIR wavelengths.

It has also been shown that light has both inductive and inhibitory effect on cell growth and division in a red tide flagellate, *Chattonellantique* (Nemote Y., Plant and Cell Physiol., 26(4):669-674 (1985)). The phosphor or scintillator configurations of the invention can be programmed or instructed to or configured to deliver light at these wavelengths.

When the excitable cells (e.g., neurons, cardiomyocytes) are irradiated with monochromatic visible light, the photoacceptors are also believed to be components of respiratory chain. It is clear from experimental data (Karu, T.I., (2002). Low-power laser therapy. In: CRC Biomedical Photonics Handbook, T. Vo-Dinh, Editor- in-Chief, CRC Press, Boca Raton (USA)) that irradiation can cause physiological and morphological changes in nonpigmental excitable cells via absorption in mitochondria. Later, similar irradiation experiments were performed with neurons in connection with low-power laser therapy. It was shown in 80's that He-Ne laser radiation alters the firing pattern of nerves; it was also found that transcutaneous irradiation with HeNe laser mimicked the effect of peripheral stimulation of a behavioral reflex. These findings were found to be connected with pain therapy (Karu TI, et al., (2002)). The phosphor configurations of the invention can be programmed or instructed to or configured to deliver light at these wavelengths.

When photoacceptors absorb photons, electronic excitation followed by photochemical reactions occurring from lower excitation states (first singlet and triplet) takes place. It is also known that electronic excitation of absorbing centers alters their redox properties. Until yet, five primary reactions have been discussed in literature (Karu TI, et al., (2002)). Two of them are connected with alteration of redox properties and two mechanisms involve generation of reactive oxygen species (ROE). Also, induction of local transient (very short time) heating of absorbing chromophores is possible. Details of these mechanisms can be found in (Karu TI, et. al., (2002); Karu TI, et al., (1998). The Science of Low Power Laser Therapy. Gordon and Breach Sci. Publ., London). The phosphor or scintillator configurations of the invention can be programmed or instructed to or configured to deliver light at these wavelengths.

Photobiological action via activation of respiratory chain is believed to be a general mechanism occurring in cells. Crucial events of this type of cell metabolism activation are occurring due to a shift of cellular redox potential into more oxidized direction as well as due to ATP extrasynthesis. Susceptibility to irradiation and capability for activation depend on physiological status of irradiated cells: the cells, which overall redox potential is shifted to more reduced state (example: some pathological conditions) are more sensitive to the irradiation. The specificity of final photobiological response is determined not at the level of primary reactions in the respiratory chain but at the transcription level during cellular signaling cascades. In some cells, only partial activation of cell metabolism happens by this mechanism (example: redox priming of lymphocytes). The phosphor or scintillator configurations of the invention can be programmed or instructed to or configured to deliver light at these wavelengths.

Far red and NIR radiation have been shown to promote wound healing, e.g., infected, ischemic, and hypoxic wounds (Wong-Reley, WTT, JBC, 280(6):4761-4771 (2005)). Red-to-NIR radiation also protects the retina against the toxic actions of methanol-derived formic acid in a rodent model of methanol toxicity and may enhance recovery from retinal injury and other ocular diseases in which mitochondrial dysfunction is postulated to play a role (Eells JT., PNAS, 100(6):3439-44 (2003)). Another clinical application of photobiomodulation is repair of soft and bone tissues by IR laser irradiation (Martinez ME, et al., Laser in Med. Sci., 2007). Invasive laser assisted liposuction is a recently developed method, wherein a laser fiber is introduced through a tube into the skin and directly to the fat cells causing the cells to rapture and drain away as liquid (Kim KH, Dermatol. Surg., 32(2):241-48 (2006)). Tissue around the area is coagulated. Yet, another application of photobiomodulation is a non-surgical varicose vein treatment (an endovenous laser therapy), wherein a laser is threaded through an incision and the full length of the varicose vein (Kim HS, J. Vasc. Interv. Radiol., 18(6):811 (2007)). When the laser is slowly withdrawn, heat is applied to the vein walls, causing the vein to permanently close and disappear. The phosphor or scintillator configurations of the invention can be programmed or instructed to or configured to deliver light at these wavelengths.

The green light laser is a laser that vaporizes and removes the enlarged prostate tissue (Heinrich E., Eur. Urol., 52(6):1632-7 (2007)). The significance of the color of the laser light (green) is that this results in absorption by hemoglobin which is contained within red blood cells and not absorbed by water. The procedure may also be known as laser prostatectomy or laser Transurethral resection of the prostate (TURP). The technique involves painting the enlarged prostate with the laser until the capsule of the prostate is reached. By relieving this portion of the prostate, patients are able to void much easier through a wide-open channel in the prostate. The procedure needs to be performed under general or spinal anesthesia. An advantage of the procedure is that even patients taking blood thinners (e.g., aspirin to prevent stroke) can be treated because there is less bleeding compared to a traditional surgery. The phosphor configurations of the invention can be programmed or instructed to or configured to deliver light at these wavelengths.

Yet, another area of application of photobiomodulation is a direct control of brain cell activity with light. The technique is based upon NIR spectroscopy and is simpler to use and less expensive than other methods such as functional magnetic resonance imaging and positron emission tomography.

Whenever a region of the brain is activated, that part of the brain uses more oxygen. This technique works by measuring the blood flow and oxygen consumption in the brain. The light emitted by NIR laser diodes is carried through optical fibers to a person's head. The light penetrates the skull where it assesses the brain's oxygen level and blood volume. The scattered light is then collected by optical fibers, sent to detectors and analyzed by a computer. By examining how much of the light is scattered and how much is absorbed, portions of the brain and extract information about brain activity can be mapped. By measuring the scattering, it is determined where the neurons are firing. This means that scientists can simultaneously detect both blood profusion and neural activity. The technique could be used in many diagnostic, prognostic and clinical applications. For example, it could be used to find hematomas in children, to study blood flow in the brain during sleep apnea, and to monitor recovering stroke patients on a daily, or even hourly, basis (that would be impractical to do with MRI). To validate the technique, hemoglobin oxygen concentrations in the brain obtained simultaneously by NIR spectroscopy and by functional MRI, the current "gold standard" in brain studies, was compared. Both methods were used to generate functional maps of the brain's motor cortex during a periodic sequence of stimulation by finger motion and rest. Spatial congruence between the hemoglobin signal and the MRI signal in the motor cortex related to finger movement was demonstrated. The researchers also demonstrated collocation between hemoglobin oxygen levels and changes in scattering due to brain activities. The changes in scattering associated with fast neuron signals came from exactly the same locations. The phosphor or scintillator configurations of the invention can be programmed or instructed to or configured to deliver light at these wavelengths.

A low-intensity laser light-oxygen cancer therapy is another application of photobiomodulation. The light-oxygen effect (LOE), which involves activation of or damage to biosystems by optical radiation at low optical doses by direct photoexcitation of molecular oxygen dissolved in a biosystem so that it is converted to the singlet state, i.e., by photogeneration of molecular singlet oxygen from O₂ dissolved in cells, similar to photodynamic effect (Zakharov SD, et al., Quantum Electronics, 29(12):1031-53 (1999)). It was shown that the He-Ne laser radiation destroys tumor cells in the presence or absence of the photosensitizer. The LOE can be activated by small optical doses, which are 4-5 orders of magnitude lower that those found if a comparison is made with the familiar analogue in the form of the photodynamic effect (PDE). The phosphor or scintillator configurations of the invention can be programmed or instructed to or configured to deliver light at these wavelengths.

Another type of photobiomodulation methods fall into two general categories: one set of methods uses light to uncage a compound that then becomes biochemically active, binding to a downstream effector; the other set uses light to activate a light-sensitive protein such as rhodopsin (ChR2), which can then excite the cell expressing the opsin. The phosphor or scintillator configurations of the invention can be programmed or instructed to or configured to deliver light for these types of photobiomodulation.

In the first set, this method involves applying "caged" chemicals to a sample and then using light to open the cage to invoke a reaction. Modified glutamate is useful for finding excitatory connections between neurons, since the uncaged glutamate mimics the natural synaptic activity of one neuron impinging upon another. This method is used for elucidation of neuron functions and imaging in brain slices using, for example, two-photon glutamine uncaging (Harvey CD, et al., Nature, 450:1195-1202 (2007); Eder M, et al., Rev. Neurosci., 15:167-183 (2004)). Other signaling molecules can be released by UV light stimulation, e.g., GABA, secondary messengers (e.g., Ca²⁺ and Mg²⁺), carbachol, capsaicin, and ATP (Zhang F., et al., 2006). Chemical modifications of ion channels and receptors may be carried out to render them light-responsive. Ca²⁺ is involved in controlling fertilization, differentiation, proliferation, apoptosis, synaptic plasticity, memory, and developing axons. In yet another preferred embodiment, Ca²⁺ waves can be induced by UV irradiation (single-photon absorption) and NIR irradiation (two-photon absorption) by releasing caged Ca²⁺, an extracellular purinergic messenger InsP3 (Braet K., et al., Cell Calcium, 33:37-48 (2003)), or ion channel ligands (Zhang F., et al., 2006).

In the second set which uses light to activate a light-sensitive protein such as rhodopsin (ChR2), which can then excite the cell expressing the opsin, It has been shown that channelrhodopsin-2, a monolithic protein containing a light sensor and a cation channel, provides electrical stimulation of appropriate speed and magnitude to activate neuronal spike firing. Recently, photoinhibition, the inhibition of neural activity with light, has become feasible with the application of molecules such as the light-activated chloride pump halorhodopsin to neural control. Together, blue-light activated channelrhodopsin-2 and the yellow light-activated chloride pump halorhodopsin enable multiple-color, optical activation and silencing of neural activity.

ChR2 photostimulation involves genetic targeting ChR2 to neurons and light pulsing the neurons expressing ChR2 protein. The experiments have been conducted *in vitro* and *in vivo* in mice by *in vivo* deep-brain photostimulation using optical fibers to deliver light into the lateral hypothalamus (Adamantidis AR, et al., Nature 450:420-425 (2007)). Genetic targeting of ChR2 allows exclusive stimulation of defined cellular subsets and avoids the need for addition of the caged glutamate, facilitating photostimulation *in vivo* (Wang H., et al., PNAS, 104(19):8143-48 (2007)). ChR2 photostimulation has been used for restoring visual activity in mice with impaired vision, to evoke behavioral responses in worms and flies (Wang H., et al., 2007). The robust associative learning induced by ChR2-assisted photostimulation in mice opens the door to study the circuit basis of perception and cognition *in vivo* (Huber D., et al., 2007). This kind of neuronal targeting and stimulation might have clinical application, e.g., deep brain stimulation to treat Parkinson's disease and other disorders, controlling behavioral, perceptional and cognitive characteristics, and for imaging and studying how the brain works (Zhang F., et al., Nature Methods, 3(10):785-792 (2006); Wong-Riley MT., et al., JBC, 280(6):4761-4771 (2005)).

Another gene, chloride pump (NpHR), which is borrowed from a microbe called an archaebacterium, can make neurons less active in the presence of yellow light. Combined, the two genes ChR2 and NpHR can now make neurons obey pulses of light like drivers obey a traffic signal: Blue means "go" (emit a signal), and yellow means "stop" (don't emit).

Light-sensitive proteins can be introduced into cells or live subjects via number of techniques including electroporation, DNA microinjection, viral delivery, liposomal transfection and calcium-phosphate precipitation.

Hence, in one embodiment of the invention, there is provided a system for modulating biological activity within a medium with and without energy augmentation. The system includes a reduced-voltage x-ray source configured to generate x-rays from a peak applied cathode voltage at or below 80 kVp, and a plurality of energy-emitting particles in the medium with and without energy augmentation which, upon radiation from the x-ray source, radiate at a lower energy than the x-ray source to alter the biological activity of the medium by photobiomodulation (as discussed above). The ranges of peak applied cathode voltage discussed above are applicable for photobiomodulation. The use of energy-emitting particles radiate with an intensity at least 10 times greater than that of Y₂O₃, upon exposure of Y₂O₃ to the radiation from an initiation source (or with the other greater intensities described above) are applicable for photobiomodulation. The use of first and second energy-emitting particles with and without energy augmentation can produce a combination of emission from the first and second plurality of energy-emitting particles which produces a spectrum for illumination in the medium (as described above) applicable for direct or indirect (via a photoactivated agent) photobiomodulation. With the energy augmentators present, this invention can generate local regions of intense electric fields to enhance light emission from the energy-emitting particles in proximity to those local regions.

### Photostimulation (with and without Energy Augmentation)

A photostimulation technique involves chemical modification of ion channels and receptors to render them light-responsive. The above-described energy converters or light emitters described above (e.g., phosphors, scintillators, fluorescent materials, and combinations and agglomerations thereof) with or without plasmonic inducing agents and with or without energy augmentation can be programmed or instructed to or configured to deliver light for this technique. Some of the most fundamental signaling mechanisms in a cell involve the release and uptake of Ca²⁺ ions. Ca²⁺ is involved in controlling fertilization, differentiation, proliferation, apoptosis, synaptic plasticity, memory, and developing axons. It has been shown that Ca²⁺ waves can be induced by UV irradiation (single-photon absorption) and NIR irradiation (two-photon absorption) by releasing caged Ca²⁺, an extracellular purinergic messenger InsP3 (Braet K., et al., Cell Calcium, 33:37-48 (2003)), or ion channel ligands (Zhang F., et al., 2006).

Directly controlling a brain cell activity with light is a novel means for experimenting with neural circuits and could lead to therapies for some disorders. This accomplishment is a step toward the goal of mapping neural circuit dynamics on a millisecond timescale to see if impairments in these dynamics underlie severe psychiatric symptoms. Knowing the effects that different neurons have could ultimately help researchers figure out the workings of healthy and unhealthy brain circuits. If use of the technique can show that altered activity in a particular kind of neuron underlies symptoms, for example, this insight will allow development of targeted genetic or pharmaceutical treatments to fix those neurons. Conceivably, direct control of neuronal activity with light could someday become a therapy in itself. Here, the phosphor configurations of the invention can be programmed or instructed to or configured to deliver light for direct control of neuronal activity.

In living organisms, scientists have been able to cause worms, C. *elegans,* to stop swimming while their genetically altered motor neurons were exposed to pulses of yellow light intensified through a microscope. In some experiments, exposure to blue light caused the worms to wiggle in ways they weren't moving while unperturbed. When the lights were turned off, the worms resumed their normal behavior.

Meanwhile, in experiments in living brain tissues extracted from mice, the researchers were able to use the technique to cause neurons to signal or stop on the millisecond timescale, just as they do naturally. Other experiments showed that cells appear to suffer no ill effects from exposure to the light. The mice resume their normal function once the exposure ends.

The most direct application of an optical neuron control is experimenting with neural circuits to determine why unhealthy ones fail and how healthy ones work.

In patients with Parkinson's disease, for example, researchers have shown that electrical "deep brain stimulation" of cells can help patients, but they don't know precisely why. By allowing researchers to selectively stimulate or dampen different neurons in the brain, the light stimulation techniques could help in determining which particular neurons are benefiting from deep brain stimulation. That could lead to making the electrical treatment, which has some unwanted side effects, more targeted.

Another potential application is experimenting with simulating neural communications. Because neurons communicate by generating patterns of signals-sometimes on and sometimes off like the 0s and 1s of binary computer code-flashing blue and yellow lights in these patterns could compel neurons to emit messages that correspond to real neural instructions. In the future, this could allow researchers to test and tune sophisticated neuron behaviors. Much farther down the road, the ability to artificially stimulate neural signals, such as movement instructions, could allow doctors to bridge blockages in damaged spinal columns, perhaps restoring some function to the limbs of paralyzed patients.

Finally, the technique could be useful in teasing out the largely unknown functioning of healthy brains. Here, the phosphor or scintillator configurations of the invention can be programmed or instructed to or configured to deliver light for control of these and other neuron activities.

Hence, in one embodiment of the invention, there is provided a system for modulating biological activity within a medium with or without energy augmentation. The system includes a reduced-voltage x-ray source configured to generate x-rays from a peak applied cathode voltage at or below 80 kVp, and a plurality of energy-emitting particles in the medium which, upon radiation from the x-ray source, radiate at a lower energy than the x-ray source to alter the biological activity of the medium by photostimulation (as discussed above). The ranges of peak applied cathode voltage discussed above are applicable for photobiomodulation. The use of energy-emitting particles radiate with an intensity at least 10 times greater than that of Y₂O₃, upon exposure of Y₂O₃ to the radiation from an initiation source (or with the other greater intensities described above) are applicable for photostimulation. The use of first and second energy-emitting particles with or without energy augmentation can produce a combination of emission from the first and second plurality of energy-emitting particles to produce a spectrum for illumination in the medium (as described above) applicable for direct or indirect (via a photoactivated agent) photostimulation. With the energy augmentators present, this invention can generate local regions of intense electric fields to enhance light emission from the energy-emitting particles in proximity to those local regions.

### Drug Packaging:

The reagents and chemicals useful for methods and systems of the present invention may be packaged in kits to facilitate application of the present invention. In one exemplary embodiment, a kit including a psoralen, and fractionating containers for easy fractionation and isolation of autovaccines is contemplated. In a different embodiment, a kit for performing a condition, disorder or disease treatment, comprises at least one energy converter or light emitters described above (e.g., phosphors, scintillators, fluorescent materials, and combinations and agglomerations thereof) capable of adsorbing, intensifying or modifying an initiation energy into an energy that is capable of causing a predetermined change in a target structure; and containers suitable for storing the agents in stable form and for storing the energy augmentators. In yet another embodiment, the kit may further comprise instructions for administering the at least one energy converter to a subject. A further embodiment of kit would comprise at least one activatable pharmaceutical agent capable of causing a predetermined cellular change, at least one energy converter capable of activating the at least one activatable agent when energized, and containers suitable for storing the agents in stable form, and preferably further comprising instructions for administering the at least one activatable pharmaceutical agent and at least one energy converter to a subject, and for applying an initiation energy from an initiation energy source to activate the activatable pharmaceutical agent. The instructions could be in any desired form, including but not limited to, printed on a kit insert, printed on one or more containers, as well as electronically stored instructions provided on an electronic storage medium, such as a computer readable storage medium. Also optionally included is a software package on a computer readable storage medium that permits the user to integrate the information and calculate a control dose, to calculate and control intensity of the irradiation or initiation source.

Accordingly, the energy augmentation structures of the present invention can be used in conjunction with the energy converters described herein in a wide variety of applications, including but not limited to, medical treatments using energy generated in situ within a subject being treated (whether using an energy converter or not), solar cells, adhesives and other resins, sterilization treatment for various materials (such as wastewater, beverages, etc). The use of energy converters in such applications has been described in the following: US Published Application No. 2008/0248001; US Published Application No. 2009/0104212; US Published Application No. 2009/0294692; US Published Application No. 2010/0003316; US Published Application No. 2010/0016783; US Published Application No. 2010/0261263; US Published Application No. 2010/0266621, US Published Application No. 2011/0021970; US Published Application No. 2011/0117202; US Published Application No. 2011/0126889; US Published Application No. 2011/0129537; US Published Application No. 2011/0263920; US Published Application No. 2012/0064134; US Published Application No. 2012/0089180; US Published Application No. 2013/0102054; US Published Application No. 2013/0129757; US Published Application No. 2013/0131429; US Published Application No. 2013/0156905; US Published Application No. 2013/0171060; US Published Application No. 2013/0240758; US Published Application No. 2014/0134307; US Published Application No. 2014/0163303; US Published Application No. 2014/0166202; US Published Application No. 2014/0222117; US Published Application No. 2014/0242035; US Published Application No. 2014/0243934; US Published Application No. 2014/0272030; US Published Application No. 2014/0323946; US Published Application No. 2014/0341845; US Published Application No. 2014/0343479; US Published Application No. 2015/0182934; US Published Application No. 2015/0202294; US Published Application No. 2015/0246521; US Published Application No. 2015/0251016; US Published Application No. 2015/0265706; US Published Application No. 2015/0283392; US Published Application No. 2015/0290614; US Published Application No. 2016/0005503; US Published Application No. 2016/0067524; US Published Application No. 2016/0159065; US Published Application No. 2016/0243235; US Published Application No. 2016/0263393; US Published Application No. 2016/0325111; US Published Application No. 2016/0331731; US Published Application No. 2016/0354467; US Published Application No. 2016/0362534; US Published Application No. 2017/0027197; US Published Application No. 2017/0043178; US Published Application No. 2017/0050046; US Published Application No. 2017/0096585; US Published Application No. 2017/0113061; US Published Application No. 2017/0121472; US Published Application No. 2017/0154866; US Published Application No. 2017/0157418; US Published Application No. 2017/0162537; US Published Application No. 2017/0173350; US Published Application No. 2017/0186720; US Published Application No. 2017/0190166; US Published Application No. 2017/0196977; US Published Application No. 2017/0239489; US Published Application No. 2017/0239637; US Published Application No. 2017/0240717; US Published Application No. 2017/0258908; US Published Application No. 2017/0319868; US Published Application No. 2017/0319869; US Published Application No. 2018/0036408; US Published Application No. 2018/0154171; US Published Application No. 2018/0154178; US Published Application No. 2018/0169433; US Published Application No. 2018/0170028; US Published Application No. 2018/0269174; US Published Application No. 2018/0271121; US Published Application No. 2018/0304225; US Published Application No. 2018/0311355; US Published Application No. 2018/0317307; US Published Application No. 2018/0344850; US Published Application No. 2018/0358327; US Published Application No. 2019/0016869; US Published Application No. 2019/0022221; US Published Application No. 2019/0100680; US Published Application No. 2019/0134419; US Published Application No. 2019/0134595; US Published Application No. 2019/0134596; US Published Application No. 2019/0157234; US Published Application No. 2019/0168015; US Published Application No. 2019/0184190; US Published Application No. 2019/308030; US Published Application No. 2019/0336605; US Published Application No. 2019/0336785; US Published Application No. 2019/0336786; US Published Application No. 2019/0341364; U.S. Application Serial No. 16/074,707, filed 8/1/2018; U.S. Application Serial No. 16/516,463, filed 7/19/2019; U.S. Application Serial No. 16/554,831, filed 8/29/2019 U.S. Application Serial No. 16/599,732, filed 10/11/2019; U.S. Application Serial No. 16/674,435, filed 11/5/2019; and U.S. Application Serial No. 16/728,803, filed 12/27/2019;

## Claims

1. A kit for modifying a target structure which mediates or is associated with a biological activity, comprising:
- at least one energy augmentation structure, wherein the at least one energy augmentation structure is capable of capturing one or more wavelengths of electromagnetic energy representing an incident energy wave, and having captured the incident energy wave, augmenting the one or more wavelengths of captured electromagnetic energy in at least one property, and then outputting an energy wave with the at least one property augmented,
wherein "energy augmentation" means effecting some change in one or more wavelengths of electromagnetic energy in at least one property, wherein the at least one property is intensity, power, associated electrical field, associated magnetic field, wave amplitude, photonic flux, magnetic flux, phase, coherence, or propagation direction,
- an energy converter capable of receiving the energy wave output by the at least one energy augmentation structure, converting said energy wave and emitting therefrom an emitted light of a higher or lower energy than the received energy, and the energy converter being disposed in a vicinity of the at least one energy augmentation structure such that the emitted light is emitted with an intensity larger than if the energy converter were remote from the at least one energy augmentation structure, or if the energy augmentation structure were not present;
wherein the energy converter upgrades or downgrades an initiation energy to an activation energy capable of causing, either directly or indirectly, a predetermined change in the target structure;
and, optionally, a plasmonics-active agent, wherein the plasmonics-active agent, if present, enhances or modifies the applied initiation energy or the activation energy generated by the energy converter, or both; and one or more containers suitable for storing the agents in stable forms;
**characterised in that**
the at least one energy augmentation structure is an antenna.

2. The kit of claim 1, (I) further comprising instructions for administering the at least one agent to a subject; or:
wherein (II) wherein the at least one energy converter is one or more members selected from a biocompatible fluorescing metal nanoparticle, fluorescing metal oxide nanoparticle, fluorescing metal coated metal oxide nanoparticle, fluorescing dye molecule, gold nanoparticle, silver nanoparticle, gold-coated silver nanoparticle, a water soluble quantum dot encapsulated by polyamidoamine dendrimers, a luciferase, a biocompatible phosphorescent molecule, a combined electromagnetic energy harvester molecule, and a lanthanide chelate exhibiting intense luminescence; or:
wherein (III) the target structure is a target cell and the predetermined change is apoptosis in the target cell.

3. The kit of claim 1, further comprising at least one activatable pharmaceutical agent.

4. The kit of claim 3, (I) further comprising instructions for administering the at least one activatable pharmaceutical agent and at least one energy augmentation structure, and energy converter, to a subject, and for activating the at least one activatable pharmaceutical agent by application of an initiation energy; or:
wherein (II) the at least one activatable pharmaceutical agent is a photoactivatable agent; or:
wherein (III) the at least one activatable pharmaceutical agent is selected from psoralens, pyrene cholesteryloleate, acridine, porphyrin, fluorescein, rhodamine, 16-diazorcortisone, ethidium, transition metal complexes of bleomycin, transition metal complexes of deglycobleomycin organoplatinum complexes, alloxazines, vitamin Ks, vitamin L, vitamin metabolites, vitamin precursors, naphthoquinones, naphthalenes, naphthols and derivatives thereof having planar molecular conformations, porphorinporphyrins, dyes and phenothiazine derivatives, coumarins, quinolones, quinones, and anthroquinones; or:
wherein (IV) the at least one activatable pharmaceutical agent is a psoralen, a coumarin, a porphyrin or a derivative thereof; or:
wherein (V) the at least one activatable pharmaceutical agent is 8-MOP or AMT; or:
wherein (VI) the at least one activatable pharmaceutical agent is one selected from 7,8-dimethyl-10-ribityl, isoalloxazine, 7,8,10-trimethylisoalloxazine, 7,8-dimethylalloxazine, isoalloxazine-adenine dinucleotide, alloxazine mononucleotide, aluminum (III) phthalocyanine tetrasulonate, hematophorphyrin, and phthadocyanine; or:
wherein (VII) the at least one activatable pharmaceutical agent has affinity for the target structure; or:
wherein (VIII) the target structure is a target cell and the at least one activatable pharmaceutical agent is capable of being preferentially absorbed by the target cell; or:
wherein (IX) the at least one activatable pharmaceutical agent, upon activation, causes an auto-vaccine effect in the subject that reacts with a target structure; or:
wherein (X) the at least one activatable pharmaceutical agent is a DNA intercalator or a halogenated derivative thereof; or:
wherein (XI) the at least one activatable pharmaceutical agent comprises an active agent contained within a photocage, wherein upon exposure to a reemitted energy by the at least one energy converter as an activation energy of the at least one activatable pharmaceutical agent, the photocage disassociates from the active agent, rendering the active agent available.

5. The kit of claim 3, wherein the at least one activatable pharmaceutical agent is coupled to a carrier that is capable of binding to a receptor site on or near the target structure.

6. The kit of claim 5, wherein (I) the carrier is one selected from insulin, interleukin, thymopoietin or transferrin; or:
wherein (II) the at least one activatable pharmaceutical agent is coupled to the carrier by a covalent bond; or:
wherein (III) the at least one activatable pharmaceutical agent is coupled to the carrier by non-covalent bond; or:
wherein (IV) the receptor site is one selected from nucleic acids of nucleated cells, antigenic sites on nucleated cells, or epitopes.

7. The kit of claim 1, wherein (I) the at least one energy converter is a single energy converter; or:
wherein (II) said at least one energy converter is a plurality of the energy converters, and wherein the initiation energy is converted, through a cascade energy transfer between the plurality of the energy converters, to the activation energy; or:
wherein (III) said at least one energy converter is a single energy converter, and is coupled to at least one activatable pharmaceutical agent; or:
said kit (IV) comprising a plurality of energy converters, capable of converting, through a cascade energy transfer between the plurality of energy converters, the initiation energy to an activation energy that activates at least one activatable pharmaceutical agent.

8. The kit of claim 1, wherein the at least one plasmonics-active agent is present; optionally said kit further comprising at least one activatable pharmaceutical agent.

9. The kit of any one of the preceding claims, wherein the antenna is a fractal antenna.

## Patentansprüche

1. Kit zum Modifizieren einer Zielstruktur, welche eine biologische Aktivität vermittelt oder mit dieser in Verbindung steht, welches aufweist:
- mindestens eine Energieverstärkungsstruktur, wobei die mindestens eine Energieverstärkungsstruktur in der Lage ist, eine oder mehrere Wellenlängen elektromagnetischer Energie, die eine einfallende Energiewelle darstellen, aufzunehmen, und nach dem Aufnehmen der einfallenden Energiewelle die eine oder mehreren Wellenlängen der aufgenommenen elektromagnetischen Energie in mindestens einer Eigenschaft verstärkt, und dann eine Energiewelle mit der mindestens einen verstärkten Eigenschaft ausgibt,
wobei "Energieverstärkung" bedeutet, dass eine Änderung bei einer oder mehreren Wellenlängen der elektromagnetischen Energie in mindestens einer Eigenschaft bewirkt wird, wobei die mindestens eine Eigenschaft eine Intensität, eine Leistung, ein zugehöriges elektrisches Feld, ein zugehöriges magnetisches Feld, eine Wellenamplitude, ein Photonenfluss, ein magnetischer Fluss, eine Phase, eine Kohärenz oder eine Ausbreitungsrichtung ist,
- einen Energiewandler, der in der Lage ist, die von der mindestens einen Energieverstärkungsstruktur abgegebene Energiewelle zu empfangen, die Energiewelle umzuwandeln, und daraus ein emittiertes Licht mit einer höheren oder niedrigeren Energie als die empfangene Energie zu emittieren,
wobei der Energiewandler in der Nähe der mindestens einen Energieverstärkungsstruktur auf eine solche Weise angeordnet ist, dass das emittierte Licht mit einer größeren Intensität emittiert wird, als wenn der Energiewandler von der mindestens einen Energieverstärkungsstruktur weiter entfernt wäre oder wenn die Energieverstärkungsstruktur nicht vorhanden wäre,
wobei der Energiewandler eine Initiierungsenergie auf eine Aktivierungsenergie erhöht oder erniedrigt, die in der Lage ist, entweder direkt oder indirekt eine vorbestimmte Veränderung in der Zielstruktur zu bewirken, und
- und gegebenenfalls ein plasmonisch aktives Mittel,
wobei das plasmonisch aktive Mittel, falls vorhanden, die angewandte Initiierungsenergie oder die durch den Energiekonverter erzeugte Aktivierungsenergie, oder beide, verstärkt oder modifiziert, und einen oder mehrere Behälter, die zur Speicherung der Mittel in stabilen Formen geeignet sind,
**dadurch gekennzeichnet, dass** die mindestens eine Energieverstärkungsstruktur eine Antenne ist.

2. Kit nach Anspruch 1, welches (I) ferner Anweisungen zur Verabreichung des mindestens einen Mittels an einen Patienten aufweist, oder:
wobei (II) der mindestens eine Energiewandler eines oder mehrere Elemente ist, die aus einer Gruppe ausgewählt worden sind, die ein biokompatibles fluoreszierendes Metallnanopartikel, ein fluoreszierendes Metalloxidnanopartikel, ein fluoreszierendes metallbeschichtetes Metalloxidnanopartikel, ein fluoreszierenden Farbstoffmolekül, ein Goldnanopartikel, ein Silbernanopartikel, ein goldbeschichtetes Silbernanopartikel, ein wasserlöslicher Quantenpunkt, der mit Polyamidoamindendrimeren verkapselt ist, eine Luciferase, ein biokompatibles phosphoreszierendes Molekül, ein kombiniertes elektromagnetisches Energieerzeugermolekül und ein Lanthanidchelat, das eine intensive Lumineszenz zeigt, umfasst, oder:
wobei (III) die Zielstruktur eine Zielzelle ist und die vorbestimmte Veränderung eine Apoptose in der Zielzelle ist.

3. Kit nach Anspruch 1, welches ferner mindestens ein aktivierbares pharmazeutisches Mittel aufweist.

4. Kit nach Anspruch 3, welches (I) ferner Anweisungen zur Verabreichung des mindestens einen aktivierbaren pharmazeutischen Mittels und mindestens einer Energieverstärkungsstruktur und eines Energiewandlers an einen Patienten und zur Aktivierung des mindestens einen aktivierbaren pharmazeutischen Mittels durch Anwendung einer Initiationsenergie aufweist, oder:
wobei (II) das mindestens eine aktivierbare pharmazeutische Mittel ein photoaktivierbares Mittel ist, oder:
wobei (III) das mindestens eine aktivierbare pharmazeutische Mittel aus einer Gruppe ausgewählt worden ist, die ein Psoralenen, ein Pyrencholesteryloat, ein Acridin, ein Porphyrin, ein Fluorescein, ein Rhodamin, ein 16-Diazorcortison, ein Ethidium, Übergangsmetallkomplexe von Bleomycin, Übergangsmetallkomplexe von Deglycobleomycin, Organoplatin-Komplexe, Alloxazine, ein Vitamin Ks, ein Vitamin L, Vitamin-Metabolitin, Vitamin-Vorstufe, Naphthochinone, Naphthaline, Naphthole und Derivate davon mit planaren molekularen Konformationen, Porphorinporphyrine, Farbstoffe und Phenothiazinderivate, Cumarine, Chinolone, Chinone und Anthrachinone umfasst, oder:
wobei (IV) das mindestens eine aktivierbare pharmazeutische Mittel ein Psoralen, ein Cumarin, ein Porphyrin oder ein Derivat davon ist, oder:
wobei (V) das mindestens eine aktivierbare pharmazeutische Mittel ein 8-MOP oder ein AMT ist, oder:
wobei (VI) das mindestens eine aktivierbare pharmazeutische Mittel eines ist, das aus einer Gruppe ausgewählt worden ist, die ein 7,8-Dimethyl-10-Ribityl, ein Isoalloxazin, ein 7,8,10-Trimethylisoalloxazin, ein 7,8-Dimethylalloxazin, ein Isoalloxazin-Adenin-Dinukleotid, ein Alloxazin-Mononukleotid, ein Aluminium(III)-Phthalocyanintetrasulonat, ein Hämatophorphyrin und ein Phthadocyanin umfasst, oder:
wobei (VII) der mindestens eine aktivierbare pharmazeutische Wirkstoff eine Affinität für die Zielstruktur aufweist, oder:
wobei (VIII) die Zielstruktur eine Zielzelle ist und der mindestens eine aktivierbare pharmazeutische Wirkstoff bevorzugt von der Zielzelle absorbiert werden kann, oder:
wobei (IX) der mindestens eine aktivierbare pharmazeutische Wirkstoff bei Aktivierung einen Autovakzine-Effekt bei dem Patienten bewirkt, das mit einer Zielstruktur reagiert, oder:
wobei (X) der mindestens eine aktivierbare pharmazeutische Wirkstoff ein DNA-Interkalator oder ein halogeniertes Derivat davon ist, oder:
wobei (XI) der mindestens eine aktivierbare pharmazeutische Wirkstoff einen in einem Photokäfig enthaltenen Wirkstoff aufweist, wobei sich der Photokäfig bei Einwirkung einer von dem mindestens einen Energiewandler als Aktivierungsenergie des mindestens einen aktivierbaren pharmazeutischen Wirkstoffs reemittierten Energie von dem Wirkstoff löst und den Wirkstoff verfügbar macht.

5. Kit nach Anspruch 3, wobei der mindestens eine aktivierbare pharmazeutische Wirkstoff mit einem Träger verbunden ist, der in der Lage ist, an eine Rezeptorstelle auf oder in der Nähe der Zielstruktur zu binden.

6. Kit nach Anspruch 5, wobei (I) der Träger aus einem Insulin, einem Interleukin, einem Thymopoietin oder einem Transferrin ausgewählt worden ist, oder:
wobei (II) der mindestens eine aktivierbare pharmazeutische Wirkstoff durch eine kovalente Bindung mit dem Träger verbunden ist, oder:
wobei (III) der mindestens eine aktivierbare pharmazeutische Wirkstoff durch eine nicht-kovalente Bindung mit dem Träger verbunden ist, oder:
wobei (IV) die Rezeptorstelle eine ist, die aus Nukleinsäuren von kernhaltigen Zellen, antigenen Stellen auf kernhaltigen Zellen oder Epitopen ausgewählt worden ist.

7. Kit nach Anspruch 1, wobei (I) der mindestens eine Energiewandler ein einzelner Energiewandler ist, oder:
(II) wobei der mindestens eine Energiewandler eine Vielzahl an Energiewandlern ist und wobei die Initiierungsenergie durch einen Kaskadenenergietransfer zwischen der Vielzahl an Energiewandlern in die Aktivierungsenergie umgewandelt wird, oder:
wobei (III) der mindestens eine Energiewandler ein einzelner Energiewandler ist und mit mindestens einem aktivierbaren pharmazeutischen Wirkstoff verbunden ist, oder:
das Kit (IV) eine Vielzahl an Energiewandlern aufweist, die in der Lage sind, durch eine Kaskadenenergieübertragung zwischen der Vielzahl an Energiewandlern die Initiierungsenergie in eine Aktivierungsenergie umzuwandeln, die mindestens einen aktivierbaren pharmazeutischen Wirkstoff aktiviert.

8. Kit nach Anspruch 1, wobei der mindestens eine plasmonisch aktive Wirkstoff vorhanden ist, wobei das Kit gegebenenfalls ferner mindestens einen aktivierbaren pharmazeutischen Wirkstoff aufweist.

9. Kit nach einem der vorhergehenden Ansprüche, wobei die Antenne eine fraktale Antenne ist.

## Revendications

1. **Kit pour** modifier une structure cible, qui médie ou est associée à une activité biologique, comprenant :
- au moins une structure d'augmentation d'énergie, dans lequel l'au moins une structure d'augmentation d'énergie est apte à capturer une ou plusieurs longueurs d'onde d'énergie électromagnétique représentant une onde d'énergie incidente, et après la capture de l'onde d'énergie incidente, à augmenter les une ou plusieurs longueurs d'onde d'énergie électromagnétique capturée en ce qui concerne au moins une propriété, puis à délivrer en sortie une onde d'énergie avec l'au moins une propriété augmentée,
où « augmentation d'énergie » signifie la mise en œuvre d'un changement pour une ou plusieurs longueurs d'onde d'énergie électromagnétique en ce qui concerne au moins une propriété, dans lequel l'au moins une propriété est l'intensité, la puissance, le champ électrique associé, le champ magnétique associé, l'amplitude d'onde, le flux photonique, le flux magnétique, la phase, la cohérence ou la direction de propagation,
- un convertisseur d'énergie apte à recevoir l'onde d'énergie délivrée par l'au moins une structure d'augmentation d'énergie, à convertir ladite onde d'énergie et de là, à émettre une lumière émise d'une énergie supérieure ou inférieure à l'énergie reçue, et le convertisseur d'énergie étant disposé à proximité de l'au moins une structure d'augmentation d'énergie de sorte que la lumière émise est émise avec une intensité plus grande que si le convertisseur d'énergie était éloigné de l'au moins une structure d'augmentation d'énergie, ou que si la structure d'augmentation d'énergie n'était pas présente ;
dans lequel le convertisseur d'énergie augmente ou abaisse une énergie d'amorçage en une énergie d'activation apte à entraîner, directement ou indirectement, un changement prédéterminé dans la structure cible ;
et, éventuellement, un agent plasmonique actif,
dans lequel l'agent plasmonique actif, s'il est présent, améliore ou modifie l'énergie d'amorçage appliquée ou l'énergie d'activation générée par le convertisseur d'énergie, ou les deux; et un ou plusieurs récipients appropriés pour stocker les agents sous des formes stables ;
**caractérisé en ce que**
l'au moins une structure d'augmentation d'énergie est une antenne.

2. Kit selon la revendication 1, (I) comprenant en outre des instructions pour l'administration de l'au moins un agent à un sujet ; ou :
dans lequel (II) l'au moins un convertisseur d'énergie est un ou plusieurs éléments choisis parmi une nanoparticule métallique fluorescente biocompatible, une nanoparticule d'oxyde métallique fluorescente, une nanoparticule d'oxyde métallique enrobée de métal fluorescente, une molécule de colorant fluorescent, une nanoparticule d'or, une nanoparticule d'argent, une nanoparticule d'argent enrobée d'or, un point quantique soluble dans l'eau encapsulé par des dendrimères de poly(amidoamine), une luciférase, une molécule phosphorescente biocompatible, une molécule récupératrice d'énergie électromagnétique combinée et un chélate de lanthanide présentant une luminescence intense ; ou :
dans lequel (III) la structure cible est une cellule cible et le changement prédéterminé est l'apoptose dans la cellule cible.

3. Kit selon la revendication 1, comprenant en outre au moins un agent pharmaceutique activable.

4. Kit selon la revendication 3, (I) comprenant en outre des instructions pour l'administration à un sujet de l'au moins un agent pharmaceutique activable et d'au moins une structure d'augmentation d'énergie, et d'un convertisseur d'énergie, et pour l'activation de l'au moins un agent pharmaceutique activable par application d'une énergie d'amorçage ; ou :
dans lequel (II) l'au moins un agent pharmaceutique activable est un agent photoactivable ; ou :
dans lequel (III) l'au moins un agent pharmaceutique activable est choisi parmi : psoralènes, pyrène cholestéryloléate, acridine, porphyrine, fluorescéine, rhodamine, 16-diazocortisone, éthidium, complexes de métaux de transition de bléomycine, complexes de métaux de transition de déglyco-bléomycine, complexes organoplatine, alloxazines, vitamine Ks, vitamine L, métabolites de vitamines, précurseurs de vitamines, naphtoquinones, naphtalènes, naphthols et dérivés de ceux-ci ayant des conformations moléculaires planes, porphorinporphyrines, colorants et dérivés de phénothiazine, coumarines, quinolones, quinones et anthraquinones ; ou :
dans lequel (IV) l'au moins un agent pharmaceutique activable est un psoralène, une coumarine, une porphyrine ou un dérivé de ceux-ci ; ou :
dans lequel (V) l'au moins un agent pharmaceutique activable est 8-MOP ou AMT ; ou :
dans lequel (VI) l'au moins un agent pharmaceutique activable est un élément choisi parmi 7,8-diméthyl-10-ribityle, isoalloxazine, 7,8,10-triméthylisoalloxazine, 7,8-diméthylalloxazine, isoalloxazine-adénine dinucléotide, alloxazine mononucléotide, phtalocyanine tétrasulfonate d'aluminium (III), hématophorphyrine, et phthalocyanine ; ou :
dans lequel (VII) l'au moins un agent pharmaceutique activable a une affinité pour la structure cible ; ou :
dans lequel (VIII) la structure cible est une cellule cible et l'au moins un agent pharmaceutique activable est apte à être absorbé préférentiellement par la cellule cible ; ou :
dans lequel (IX) l'au moins un agent pharmaceutique activable, lors de l'activation, provoque un effet d'auto-vaccin chez le sujet qui réagit avec une structure cible ; ou : dans lequel (X) l'au moins un agent pharmaceutique activable est un intercalant d'ADN ou un dérivé halogéné de celui-ci ; ou :
dans lequel (XI) l'au moins un agent pharmaceutique activable comprend un agent actif contenu dans une photocage, dans lequel lors de l'exposition à une énergie réémise par l'au moins un convertisseur d'énergie en tant qu'énergie d'activation de l'au moins un agent pharmaceutique activable, la photocage se dissocie de l'agent actif, rendant l'agent actif disponible.

5. Kit selon la revendication 3, dans lequel l'au moins un agent pharmaceutique activable est couplé à un vecteur qui est apte à se lier à un site récepteur sur la structure cible ou proche de la structure cible.

6. Kit selon la revendication 5, dans lequel (I) le vecteur est un élément choisi parmi l'insuline, l'interleukine, la thymopoïétine ou la transferrine ; ou :
dans lequel (II) l'au moins un agent pharmaceutique activable est couplé au vecteur par une liaison covalente ; ou :
dans lequel (III) l'au moins un agent pharmaceutique activable est couplé au vecteur par une liaison non covalente ; ou :
dans lequel (IV) le site récepteur est un élément choisi parmi des acides nucléiques de cellules nucléées, des sites antigéniques sur des cellules nucléées, ou des épitopes.

7. Kit selon la revendication 1, dans lequel (I) l'au moins un convertisseur d'énergie est un convertisseur d'énergie unique ; ou :
dans lequel (II) ledit au moins un convertisseur d'énergie est une pluralité des convertisseurs d'énergie, et dans lequel l'énergie d'amorçage est convertie, par un transfert d'énergie en cascade entre la pluralité des convertisseurs d'énergie, en l'énergie d'activation ; ou :
dans lequel (III) ledit au moins un convertisseur d'énergie est un convertisseur d'énergie unique, et est couplé à au moins un agent pharmaceutique activable ; ou :
ledit kit (IV) comprenant une pluralité de convertisseurs d'énergie, aptes à convertir, par un transfert d'énergie en cascade entre la pluralité de convertisseurs d'énergie, l'énergie d'amorçage en une énergie d'activation qui active au moins un agent pharmaceutique activable.

8. Kit selon la revendication 1, dans lequel l'au moins un agent plasmonique actif est présent ; éventuellement, ledit kit comprenant en outre au moins un agent pharmaceutique activable.

9. Kit selon l'une quelconque des revendications précédentes, dans lequel l'antenne est une antenne fractale.
